(19) Europäisches Patentamt
European Patent Office
Office européen des brevets

(11) EP 0 903 346 A1

(12) EUROPEAN PATENT APPLICATION
published in accordance with Art. 158(3) EPC

(43) Date of publication:
**24.03.1999 Bulletin 1999/12**

(21) Application number: **96935538.7**

(22) Date of filing: **05.11.1996**

(51) Int. Cl.[6]: **C07D 271/04**, C07D 413/04, C07D 413/12, C07D 417/12, A61K 31/41, A61K 31/495, A61K 31/535

(86) International application number:
**PCT/JP96/03226**

(87) International publication number:
**WO 97/17334 (15.05.1997 Gazette 1997/21)**

(84) Designated Contracting States:
**AT BE CH DE DK ES FI FR GB GR IE IT LI LU MC NL PT SE**

(30) Priority: **06.11.1995 JP 323490/95**
**19.12.1995 JP 354569/95**

(71) Applicant:
**Chugai Seiyaku Kabushiki Kaisha**
**Tokyo 115 (JP)**

(72) Inventors:
- **KOGA, Hiroshi**
**Gotenba-shi, Shizuoka-ken 412 (JP)**
- **SATO, Harukiko**
**Gotenba-shi, Shizuoka-ken 412 (JP)**
- **TAKAHASHI, Tadakatsu**
**Gotenba-shi, Shizuoka-ken 412 (JP)**
- **IMAGAWA, Junichi**
**Gotenba-shi, Shizuoka-ken 412 (JP)**

(74) Representative:
**VOSSIUS & PARTNER**
**Siebertstrasse 4**
**81675 München (DE)**

# (54) SYDNONE IMINE DERIVATIVES

(57) Compounds represented by the following general formula (1) or pharmaceutically acceptable salts thereof:

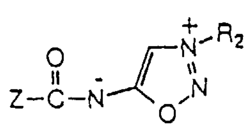

( 1 )

[wherein Z represents -A-X-$R_1$, a heterocycle or cycloalkyl; A represents a methylene group; X represents a sulfur atom; $R_1$ represents a benzoyl group; $R_2$ represents - $N(R_3)R_4$ (wherein $R_3$ represents a methyl group; and $R_4$ represents an optionally substituted alkyl group, etc.); or a group represented by the following general formula (2):

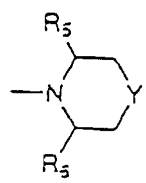

( 2 )

{wherein $R_5$ and $R_6$ represent each a hydrogen atom; and Y represents a group represented by the following general formula (3):

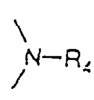

( 3 )

(wherein $R_4$ is as defined above)}].

Because of having vasodilative effect, myocardial



EP 0 903 346 A1

protective effect, antiplatelet aggregation effect, etc., the compounds represented by the above general formula (1) or pharmaceutically acceptable salts thereof are useful as drugs such as therapeutic medicines for angina pectoris.

## Description

TECHNICAL FIELD

This invention relates to novel sydnonimine derivatives which exhibit vasodilative effect, myocardial protective effect, antiplatelet aggregation effect, etc. and, therefore, are useful as drugs.

BACKGROUND ART

There have been widely employed nitrous acid agents and nitric acid agents in drug therapy for angina pectoris. The main pharmacological action of these compounds, which are generally called "NO donors", resides in the dilation of thick coronary arteries. The pharmacological mechanism of these compounds comprises forming nitrogen monoxide *in vivo* and then, owing to the subsequent increase in the cGMP level, expressing the vasodilative effect.

There have been known sydnonimine derivatives having vasodilative effects, etc. For example, various sydnonimine derivatives are disclosed in JP (Kokoku) Sho-45-6265, JP (Kokoku) Sho 46-10855, JP (Kokoku) Sho 47-34701, JP (Kokai) Sho 48-32890, JP (Kokoku) Sho 53-7433, JP (Kokai) Sho 56-25174, JP (Kokai) Sho 57-158768, JP (Kokai) Sho 58-59977, JP (Kokai) Sho 59-98076, JP (Kokai) Sho 62-73, JP (Kokai) Sho 62-22775, JP (Kokai) Sho 63-201177, JP (Kokai) Hei 1-106881, JP (Kokai) Hei 2-32069, JP (Kokai) Hei 2-36180, JP (Kokai) Hei 2-178275, JP (Kokai) Hei 3-44391, JP (Kokai) Hei 3-128367, JP (Kokai) Hei 3-178975, JP (Kokai) Hei 4-226978, JP (Kokai) Hei 4-244071, JP (Kokai) Hei 4-273873, JP (Kokai) Hei 4-312581, JP (Kokai) Hei 6-128156, WO 93/18767, EP No. 655450, USP No. 3833589, USP No. 4371539, German Patent No. 1813752, German Patent No. 1942854, Spanish Patent No. 547310, Spanish Patent No. 547311, etc. These compounds are also NO donors.

On the other hands, attention has been paid in recent years to attempts to prevent myocardial infarction attack in association with the worsening of angina pectoris by the antiplatelet aggregation effect of NO donors and, at the same time, to improve the prognosis of myocardial infraction attack by the myocardial protective effect of these drugs to thereby accelerate the prolongation of life. It is reported that sydnonimine derivatives typified by molsidomine, which are therapeutic medicines for angina pectoris, have myocardial protective effect [The Journal of Pharmacology and Experimental Therapeutics, 260 (2), 668, 1992; Journal of Cardiovascular Pharmacology, 25 (3), 424, 1995, etc.]. However, each of these compounds fails to exhibit any satisfactory effect and it has been therefore urgently required to achieve more potent myocardial protective effect.

It is also reported that FK-409, which is another NO donor, has antiplatelet aggregation effect (British Journal of Pharmacology, 113, 385, 1994; European Journal of Pharmacology, 272, 39, 1995, etc.). However, this compound fails to exhibit any satisfactory effect and it has been therefore urgently required to achieve more potent antiplatelet aggregation effect.

DISCLOSURE OF THE INVENTION

The present invention aims at providing sydnonimine derivatives which have vasodilative effect, myocardial protective effect, antiplatelet aggregation effect, etc. and, therefore, are useful as therapeutic medicines for angina pectoris, etc.

The present inventors have conducted extensive studies to develop compounds having excellent vasodilative effect, antiplatelet aggregation effect and myocardial protective effect and, as a result, found that sydnonimine derivatives represented by the following general formula (1) achieve the above-mentioned object and are useful as drugs. The present invention has been completed based on this finding.

Accordingly, the present invention provides compounds represented by the following general formula (1) or pharmaceutically acceptable salts thereof:

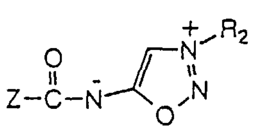

(1)

[wherein Z represents:

$$-A-X-R_1$$

{wherein A represents a linear or branched alkylene group having 1 to 6 carbon atoms optionally substituted by an optionally substituted aryl group having 6 to 12 carbon atoms or an alkylcarbonylamino group carrying, as the alkyl moiety, a linear or branched alkyl group having 1 to 6 carbon atoms;

X represents $S(O)_n$ (n being 0, 1 or 2); or a direct bond; and

$R_1$ represents an aryl group having 6 to 12 carbon atoms optionally mono-, di- or tri-substituted by 1 to 3 hydroxyl groups and/or 1 to 3 linear or branched alkyl groups having 1 to 6 carbon atoms and/or 1 to 3 linear or branched alkoxy groups having 1 to 6 carbon atoms and/or 1 to 3 halogen atoms and/or 1 or 2 nitro groups; an arylcarbonyl group carrying, as the aryl moiety, an aryl group having 6 to 12 carbon atoms optionally mono-, di- or tri-substituted by 1 to 3 linear or branched alkyl groups having 1 to 6 carbon atoms optionally halogenated and/or 1 to 3 linear or branched alkoxy groups having 1 to 6 carbon atoms and/or 1 to 3 halogen atoms; an alkylcarbonyl group having, as the alkyl moiety, a linear or branched alkyl group having 1 to 6 carbon atoms; a linear or branched alkyl group having 1 to 6 carbon atoms; an aralkyloxycarbonyl group carrying, as the aryl moiety, an optionally substituted aryl group having 6 to 12 carbon atoms and, as the alkylene moiety, an alkylene group having 1 or 2 carbon atoms; a cycloalkylcarbonyl group carrying, as the cycloalkyl moiety, an optionally substituted cycloalkyl group having 3 to 6 carbon atoms; an arylcarbamoyl group carrying, as the aryl moiety, an optionally substituted aryl group having 6 to 12 carbon atoms; an alkylcarbamoyl group carrying, as the alkyl moiety, a linear or branched alkyl group having 1 to 6 carbon atoms; or $-CO-R_{14}$ wherein $R_{14}$ represents an optionally substituted monocyclic heterocycle; or a bicyclic heterocycle optionally substituted by a hydroxyl group and/or a linear or branched alkyl group having 1 to 6 carbon atoms); an optionally substituted monocyclic heterocycle; a bicyclic heterocycle optionally substituted by a hydroxyl group and/or linear or branched alkyl groups having 1 to 6 carbon atoms; or an optionally substituted cycloalkyl group having 3 to 6 carbon atoms; and

$R_2$ represents:

$$-N(R_3)R_4$$

(wherein $R_3$ represents a hydrogen atom; or a linear or branched alkyl group having 1 to 6 carbon atoms; and $R_4$ represents a hydrogen atom; an optionally substituted linear or branched alkyl group having 1 to 6 carbon atoms; an optionally substituted linear or branched alkenyl group having 2 to 6 carbon atoms; an optionally substituted linear or branched alkynyl group having 2 to 6 carbon atoms; an optionally substituted aryl group having 6 to 12 carbon atoms; an optionally substituted cycloalkyl group having 3 to 6 carbon atoms; or $-CO-R_7$ wherein $R_7$ represents an optionally substituted linear or branched alkyl group having 1 to 6 carbon atoms; an optionally substituted linear or branched alkoxy group having 1 to 6 carbon atoms; an optionally substituted monocyclic heterocycle; or an optionally substituted bicyclic heterocycle); or a group represented by the following general formula (2):

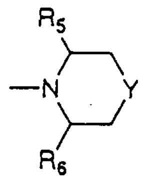

(2)

{wherein $R_5$ and $R_6$ are the same or different and each represents a hydrogen atom; or a linear or branched alkyl group having 1 to 6 carbon atoms; Y represents an oxygen atom; $S(O)_n$ (n being 0, 1 or 2); or a group represented by the following general formula (3):

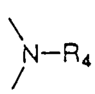

(3)

(Wherein $R_4$ represents a hydrogen atom; an optionally substituted linear or branched alkyl group having 1 to 6 carbon atoms; an optionally substituted linear or branched alkenyl group having 2 to 6 carbon atoms; an optionally substituted linear or branched alkynyl group having 2 to 6 carbon atoms; an optionally substituted aryl group having 6 to 12 carbon atoms; an optionally substituted cycloalkyl group having 3 to 6 carbon atoms; or $-CO-R_7$ wherein $R_7$ represents an optionally substituted linear or branched alkyl group having 1 to 6 carbon atoms; an optionally substituted linear or branched alkoxy group having 1 to 6 carbon atoms; an optionally substituted monocyclic heterocycle; or an optionally substituted bicyclic heterocycle); or a group represented by the following general formula (4):

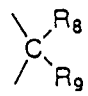

(4)

(wherein $R_8$ represents a hydrogen atom; or a linear or branched alkyl group having 1 to 6 carbon atoms; and $R_9$ represents a hydrogen atom; a hydroxyl group; a thiol group; an alkylcarbonylthio group carrying, as the alkyl moiety, a linear or branched alkyl group having 1 to 6 carbon atoms; an alkylcarbonyloxy group carrying, as the alkyl moiety, a linear or branched alkyl group having 1 to 6 carbon atoms; an arylcarbonylthio group carrying, as the aryl moiety, an aryl group having 6 to 12 carbon atoms; an arylcarbonyloxy group carrying, as the aryl moiety, an aryl group having 6 to 12 carbon atoms; or a linear or branched alkyl group having 1 to 6 carbon atoms)}].

In the definition of the compounds represented by the general formula (1), examples of the "linear or branched alkylene group having 1 to 6 carbon atoms" include methylene, ethylene, propylene, dimethylmethylene, trimethylene, tetramethylene, pentamethylene, hexamethylene and $-C(CH_3)_2-CH_2-$ groups.

Examples of the "linear or branched alkyl group having 1 to 6 carbon atoms" include methyl, ethyl, n-propyl, i-propyl, n-butyl, i-butyl, s-butyl, t-butyl, n-pentyl and n-hexyl groups.

Examples of the "linear or branched alkenyl or alkynyl group having 2 to 6 carbon atoms" include vinyl, ethynyl, allyl, i-propenyl and 2-propynyl groups.

Examples of the "linear or branched alkoxy group having 1 to 6 carbon atoms" include methoxy, ethoxy, n-propoxy, i-propoxy, n-butoxy and t-butoxy groups.

The "cycloalkyl group having 3 to 6 carbon atoms" means cyclopropyl, cyclobutyl, cyclopentyl and cyclohexyl groups.

The "alkylcarbonyl, alkylcarbonylthio, alkylcarbonyloxy, alkylcarbonylamino or alkylcarbamoyl group carrying, as the alkyl moiety, a linear or branched alkyl group having 1 to 6 carbon atoms" means any alkylcarbonyl, alkylcarbonylthio, alkylcarbonyloxy, alkylcarbonylamino or alkylcarbamoyl group carrying, as the alkyl moiety, the above-mentioned linear or branched alkyl group having 1 to 6 carbon atoms. Examples thereof include acetyl, pivaloyl, acetylthio, acetoxy, acetylamino, ethylcarbamoyl and t-butylcarbamoyl groups.

The "halogen atom" means chlorine, bromine, iodine and fluorine atoms.

Examples of the "aryl group having 6 to 12 carbon atoms" include phenyl and naphthyl groups.

The "arylcarbonyl, arylcarbonylthio, arylcarbonyloxy, aralkyloxycarbonyl or arylcarbamoyl group carrying, as the aryl moiety, an aryl group having 6 to 12 carbon atoms" means any arylcarbonyl, arylcarbonylthio, arylcarbonyloxy, aralkyloxycarbonyl or arylcarbamoyl group carrying, as the aryl moiety, the above-mentioned aryl group having 6 to 12 carbon atoms. Examples thereof include benzoyl, benzoylthio, benzoyloxy, benzyloxycarbonyl and phenylcarbamoyl groups.

Examples of the substituents in the optionally substituted aryl group having 6 to 12 carbon atoms include linear or branched alkoxy groups having 1 to 6 carbon atoms.

Examples of the substituents in the optionally substituted linear or branched alkyl and alkoxy group having 1 to 6 carbon atoms, the optionally substituted cycloalkyl group having 3 to 6 carbon atoms and the optionally substituted linear or branched alkenyl and alkynyl group having 2 to 6 carbon atoms include hydroxyl groups, halogen atoms, linear or branched alkoxy groups having 1 to 6 carbon atoms, optionally substituted aryl groups, optionally substituted arylthio, aryloxy, arylcarbonylthio, arylcarbonyloxy, alkylcarbonylthio, alkylcarbonyloxy, arylcarbonyl and alkylcarbonyl groups, siloxy groups substituted by linear or branched alkyl group(s) having 1 to 6 carbon atoms, tetrahydropyranyloxy group, benzyloxy group, 2-(methoxy)ethoxy group and 2-(trimethylsilyl)ethoxy group.

Examples of the substituents in the optionally substituted monocyclic heterocycle or the optionally substituted bicyclic heterocycle include hydroxyl, oxo, nitro, cyano and trifluoromethyl groups, optionally substituted linear or branched alkyl groups having 1 to 6 carbon atoms groups and heterocycles containing oxygen or sulfur atom(s) as the heteroatom.

It is preferable that A represents a linear or branched alkylene group having 1 to 4 carbon atoms optionally substituted by an aryl group having 6 to 12 carbon atoms or an alkylcarbonylamino group carrying, as the alkyl moiety, a linear or branched alkyl group having 1 to 3 carbon atoms; still preferably a linear or branched alkylene group having 1 to 4 carbon atoms optionally substituted by a phenyl group or an alkylcarbonylamino group carrying, as the alkyl moiety, a linear alkyl group having 1 to 3 carbon atoms; and particularly preferably a methylene, ethylene, trimethylene, propylene, benzylethylene, acetylaminoethylene or $-C(CH_3)_2-CH_2-$ group.

It is preferable that X represents a sulfur atom $[S(O)_n$ wherein n is 0] or a direct bond.

In $R_1$, preferable examples of the aryl group having 6 to 12 carbon atoms optionally mono-, di- or tri-substituted by 1 to 3 hydroxyl groups and/or 1 to 3 linear or branched alkyl groups having 1 to 6 carbon atoms and/or 1 to 3 linear or branched alkoxy groups having 1 to 6 carbon atoms and/or 1 to 3 halogen atoms and/or 1 or 2 nitro groups include phenyl groups optionally mono-, di- or tri-substituted by 1 to 3 hydroxyl groups and/or 1 to 3 linear or branched alkyl groups having 1 to 4 carbon atoms and/or 1 to 3 linear or branched alkoxy groups having 1 to 3 carbon atoms, still preferably phenyl groups mono-, di- or tri-substituted by a hydroxyl group, and/or 1 or 2 linear or branched alkyl groups having 1 to 4 carbon atoms and/or 1 to 3 alkoxy groups having 1 to 2 carbon atoms, and particularly preferably phenyl groups mono-, di- or tri-substituted a hydroxyl group and/or 1 or 2 t-butyl groups and/or 1 to 3 methoxy groups.

In $R_1$, preferable examples of the arylcarbonyl group carrying, as the aryl moiety, an aryl group having 6 to 12 carbon atoms optionally mono-, di- or tri-substituted by 1 to 3 linear or branched alkyl groups having 1 to 6 carbon atoms optionally halogenated and/or 1 to 3 linear or branched alkoxy groups having 1 to 6 carbon atoms and/or 1 to 3 halogen atoms include benzoyl groups optionally mono-, di- or tri-substituted by 1 to 3 linear or branched alkyl groups having 1 to 4 carbon atoms optionally halogenated and/or 1 to 3 linear or branched alkoxy groups having 1 to 3 carbon atoms and/or 1 to 3 halogen atoms, still preferably benzoyl groups optionally mono-, di- or tri-substituted by 1 or 2 linear or branched alkyl groups having 1 to 4 carbon atoms optionally halogenated and/or 1 to 3 linear or branched alkoxy groups having 1 or 2 carbon atoms and/or 1 to 3 halogen atoms, and particularly preferably benzoyl groups optionally mono-, di- or tri-substituted by 1 or 2 methyl groups and/or a trifluoromethyl group and/or 1 to 3 methoxy groups and/or halogen atoms.

In $R_1$, preferable examples of the alkylcarbonyl group carrying, as the alkyl moiety, a linear or branched alkyl group having 1 to 6 carbon atoms include alkylcarbonyl groups having, as the alkyl moiety, linear or branched alkyl groups having 1 to 4 carbon atoms, and particularly preferably acetyl and pivaloyl groups.

In $R_1$, preferable examples of the linear or branched alkyl group having 1 to 6 carbon atoms include linear or branched alkyl groups having 1 to 3 carbon atoms, still preferably methyl and ethyl groups, and particularly preferably a methyl group.

In $R_1$, preferable examples of the aralkyloxycarbonyl group carrying, as the aryl moiety, an optionally substituted aryl group having 6 to 12 carbon atoms and, as the alkylene moiety, an alkylene group having 1 or 2 carbon atoms include aralkyloxycarbonyl groups carrying, as the aryl moiety, an aryl group having 6 to 12 carbon atoms and as the alkylene moiety, an alkylene group having 1 or 2 carbon atoms, particularly preferably a benzyloxycarbonyl group.

In $R_1$, preferable examples of the cycloalkylcarbonyl group carrying, as the cycloalkyl moiety, an optionally substituted cycloalkyl group having 3 to 6 carbon atoms include cycloalkylcarbonyl groups carrying, as the cycloalkyl moiety, a cycloalkyl group having 3 to 6 carbon atoms, particularly preferably a cyclohexanecarbonyl group.

In $R_1$, preferable examples of the arylcarbamoyl group carrying, as the aryl moiety, an optionally substituted aryl group having 6 to 12 carbon atoms include arylcarbamoyl groups carrying, as the aryl moiety, an aryl group having 6 to 12 carbon atoms, particularly preferably a phenylcarbamoyl group.

In $R_1$, preferable examples of the alkylcarbamoyl group carrying, as the alkyl moiety, a linear or branched alkyl group having 1 to 6 carbon atoms include alkylcarbamoyl groups carrying, as the alkyl moiety, a linear or branched alkyl group having 1 to 4 carbon atoms, particularly preferably ethylcarbamoyl and t-butylcarbamoyl groups.

In $R_1$, preferable examples of $-CO-R_{14}$ include those derived from $R_{14}$ as will be described hereinafter.

In $R_{14}$, examples of the monocyclic heterocycle include pyridine, pyrrole, pyran, furan, thiophene, morpholine and oxothiazolidine rings.

In $R_{14}$, preferable examples of the substituents of the optionally substituted monocyclic heterocycle include hydroxy, oxo, nitro, cyano, trifluoromethyl, optionally substituted linear or branched alkyl having 1 to 6 carbon atoms and heterocycles containing oxygen or sulfur as the heteroatom.

In $R_{14}$, examples of the bicyclic heterocycle include 3,4-dihydrobenzopyran, 2,3-dihydrobenzofuran, quinoline and purine rings.

In $R_{14}$, preferable examples of the bicyclic heterocycle optionally substituted by a hydroxyl group and/or linear or branched alkyl groups having 1 to 6 carbon atoms include bicyclic heterocycles substituted by a hydroxyl group and/or linear or branched alkyl groups having 1 to 4 carbon atoms, still preferably bicyclic heterocycles substituted by a hydroxyl group and/or a linear or branched alkyl group having 1 to 4 carbon atoms and containing an oxygen atom as the heteroatom, and particularly preferably a 3,4-dihydrobenzopyran ring substituted by a hydroxyl group and/or 4 methyl groups.

Preferable examples of $R_1$ include 3,5-di-t-butyl-4-hydroxyphenyl, 3,4,5-trimethoxyphenyl, benzoyl, 2,6-dimethylbenzoyl, 4-fluorobenzoyl, 4-methoxybenzoyl, 4-trifluoromethylbenzoyl, 2,6-dichlorobenzoyl, 2,6-difluorobenzoyl, 2,6-dimethoxybenzoyl, acetyl, pivaloyl, benzyloxycarbonyl, cyclohexanecarbonyl, phenylcarbamoyl, ethylcarbamoyl and t-butylcarbamoyl groups. Particularly preferable examples thereof include 3,5-di-t-butyl-4-hydroxyphenyl, 3,4,5-trimethoxyphenyl, benzoyl, 2,6-dimethylbenzoyl, acetyl, pivaloyl, phenylcarbamoyl and ethylcarbamoyl groups.

In Z, preferable examples of the optionally substituted monocyclic heterocycle (excluding the optionally substituted monocyclic heterocycles in $R_{14}$) include monocyclic 5- or 6-membered heterocycles optionally having an oxo group and containing nitrogen and/or sulfur and/or oxygen as the heteroatom, still preferably monocyclic 5-membered heterocycle optionally having an oxo group and containing nitrogen and/or sulfur as the heteroatom, and particularly preferably an oxothiazolidine ring.

In Z, preferable examples of the optionally substituted bicyclic heterocycle optionally substituted by a hydroxyl group and/or linear or branched alkyl groups having 1 to 6 carbon atoms (excluding the bicyclic heterocycle optionally substituted by a hydroxyl group and/or linear or branched alkyl groups having 1 to 6 carbon atoms in $R_{14}$) include bicyclic heterocycles substituted by a hydroxyl group and/or linear or branched alkyl groups having 1 to 4 carbon atoms and containing oxygen as the heteroatom, particularly preferably a 3,4-dihydrobenzopyran group substituted by a hydroxyl group and/or 4 methyl groups.

In Z, preferable examples of the optionally substituted cyloalkyl group having 3 to 6 carbon atoms (excluding the optionally substituted cycloalkyl group in $R_1$) include optionally substituted cycloalkyl groups having 4 to 6 carbon atoms, still preferably cycloalkyl groups having 4 to 6 carbon atoms and particularly preferably a cyclohexyl group.

Preferable examples of $R_3$ include linear alkyl groups having 1 to 6 carbon atoms, still preferably linear alkyl groups having 1 to 3 carbon atoms and particularly preferably a methyl group.

In $R_4$, preferable examples of the optionally substituted linear or branched alkyl group having 1 to 6 carbon atoms include optionally substituted linear or branched alkyl group having 1 to 4 carbon atoms. Still preferable examples thereof include methyl, ethyl, isopropyl, benzyl, 2-hydroxyethyl, 2-chloroethyl, 2,2,2-trifluoroethyl, (3,4,5-trimethoxyphenyl)methyl, 2-(3,4,5-trimethoxyphenyl)ethyl, (3,4-dimethoxyphenyl)methyl, 2-(acetylthio)ethyl, 2-(3,5-di-t-butyl-4-hydroxyphenylthio)ethyl, 2-(benzoylthio)ethyl, benzyl and (2,3,4-trimethoxyphenyl)methyl groups. Particularly preferable examples thereof include methyl, ethyl, isopropyl, benzyl, 2-hydroxyethyl, 2-chloroethyl, (3,4,5-trimethoxyphenyl)methyl, 2-(3,4,5-trimethoxyphenyl)ethyl, (3,4-dimethoxyphenyl)methyl, 2-(acetylthio)ethyl, 2-(3,5-di-t-butyl-4-hydroxyphenylthio)ethyl, 2-(benzoylthio)ethyl and (2,3,4-trimethoxyphenyl)methyl groups.

In $R_4$, preferable examples of the optionally substituted linear or branched alkenyl or alkynyl group having 2 to 6 carbon atoms include optionally substituted linear or branched alkenyl groups having 2 to 4 carbon atoms, still preferably an allyl group.

In $R_4$, preferable examples of the optionally substituted aryl group having 6 to 12 carbon atoms include optionally substituted phenyl groups, still preferably phenyl and 2-methoxyphenyl groups and particularly preferably a 2-methoxyphenyl group.

In $R_4$, preferable examples of the optionally substituted cycloalkyl group having 3 to 6 carbon atoms include cycloalkyl groups having 3 to 6 carbon atoms, still preferably cycloalkyl groups having 4 to 6 carbon atoms and particularly preferably a cyclopentyl group.

In $R_4$, preferable examples of -CO-$R_7$ include those derived from $R_7$ as will be described hereinafter.

In $R_7$, preferable examples of the optionally substituted linear or branched alkyl group having 1 to 6 carbon atoms include optionally substituted linear or branched alkyl groups having 1 to 4 carbon atoms, particularly preferably (3,4,5-trimethoxyphenyl)methyl, (3,5-di-t-butyl-4-hydroxyphenylthio)methyl, 1-((acetylthio)methyl)ethyl, 1-((benzoylthio)methyl)ethyl, (acetylthio)methyl and (benzoylthio)methyl groups.

In $R_7$, preferable examples of the optionally substituted linear or branched alkoxy group having 1 to 6 carbon atoms include optionally substituted linear or branched alkoxy groups having 1 to 4 carbon atoms, still preferably a benzyloxy group.

In $R_7$, a preferable example of the optionally substituted monocyclic heterocycle is an oxothiazolidine ring.

In $R_7$, preferable examples of the optionally substituted bicyclic heterocycle include those represented by the following general formula (5):

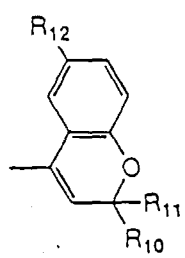

(5)

(wherein $R_{10}$ and $R_{11}$ are the same or different and each represents a hydrogen atom, a linear or branched alkyl group having 1 to 6 carbon atoms optionally substituted by a linear or branched alkoxy group having 1 to 6 carbon atoms or a halogen atom, or $R_{10}$ and $R_{11}$ may form together a heterocycle containing oxygen or sulfur; and $R_{12}$ repre-

sents a nitro, cyano or trifluoromethyl group). Particularly preferable examples thereof include 2,2-dimethyl-6-nitro-2H-1-benzopyran-4-yl and 6-cyano-2,2-dimethyl-2H-1-benzopyran-4-yl groups.

Therefore, preferable examples of -CO-$R_7$ in $R_4$ include (3,4,5-trimethoxyphenyl)acetyl, (3,5-di-t-butyl-4-hydroxyphenylthio)acetyl, 3-(acetylthio)isobutyryl, 3-(benzoylthio)isobutyryl, (acetylthio)acetyl, (benzoylthio)acetyl, 2-oxo-4-thiazolinecarbonyl, 2,2-dimethyl-6-nitro-2H-1-benzopyran-4-carbonyl, 6-cyano-2,2-dimethyl-2H-1-benzopyran-4-carbonyl and benzyloxycarbonyl groups. Particularly preferable examples thereof include (3,4,5-trimethoxyphenyl)acetyl, (3,5-di-t-butyl-4-hydroxyphenylthio)acetyl, 3-(acetylthio)isobutyryl, 3-(benzoylthio)isobutyryl, (acetylthio)acetyl and (benzoylthio)acetyl groups.

Preferable examples of $R_4$ include a hydrogen atom and methyl, ethyl, isopropyl, benzyl, allyl, 2-hydroxyethyl, 2-chloroethyl, 2,2,2-trifluoroethyl, (3,4,5-trimethoxyphenyl)methyl, 2-(3,4,5-trimethoxyphenyl)ethyl, (3,4-dimethoxyphenyl)methyl, 2-(acetylthio)ethyl, 2-(3,5-di-t-butyl-4-hydroxyphenylthio)ethyl, 2-(benzoylthio)ethyl, (2,3,4-trimethoxyphenyl)methyl, phenyl, 2-methoxyphenyl, cyclobutyl, cyclopentyl, cyclohexyl, (3,4,5-trimethoxyphenyl)acetyl, (3,5-di-t-butyl-4-hydroxyphenylthio)acetyl, 3-(acetylthio)isobutyryl, 3-(benzoylthio)isobutyryl, (acetylthio)acetyl, (benzoylthio)acetyl, 2-oxo-4-thiazolinecarbonyl, 2,2-dimethyl-6-nitro-2H-1-benzopyran-4-carbonyl, 6-cyano-2,2-dimethyl-2H-1-benzopyran-4-carbonyl and benzyloxycarbonyl groups. Still preferable examples thereof include methyl, ethyl, isopropyl, benzyl, 2,2,2-trifluoroethyl, 2-methoxyphenyl, cyclopentyl, (3,4,5-trimethoxyphenyl)acetyl, (3,5-di-t-butyl-4-hydroxyphenylthio)acetyl, 3-(acetylthio)isobutyryl, 3-(benzoylthio)isobutyryl, (acetylthio)acetyl and (benzoylthio)acetyl groups. Particularly preferable examples thereof include methyl, ethyl, isopropyl and benzyl groups.

Preferable examples of $R_5$ and $R_6$ include a hydrogen atom and linear alkyl groups having 1 to 6 carbon atoms, still preferably a hydrogen atom and linear alkyl groups having 1 to 3 carbon atoms and particularly preferably a hydrogen atom and a methyl group.

In Y, preferable examples of the oxygen atom or $S(O)_n$ (n being 0, 1 or 2) include an oxygen atom and a sulfur atom, (namely, $S(O)_n$ wherein n is 0). An oxygen atom is particularly preferable therefor.

In Y, preferable examples of the group represented by the following general formula (3):

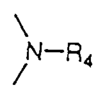

(3)

(Wherein $R_4$ is as defined above); include those derived from $R_4$ as described above.

In Y, preferable examples of the group represented by the following general formula (4):

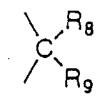

(4)

(Wherein $R_8$ and $R_9$ are each as defined above); include those derived from $R_8$ and $R_9$ as will be described hereinbelow.

$R_8$ preferably represents a hydrogen atom; or a linear alkyl group having 1 to 3 carbon atoms, still preferably a hydrogen atom or a methyl group and particularly preferably a hydrogen atom.

In $R_9$, preferable examples of the alkylcarbonylthio group carrying, as the alkyl moiety, a linear or branched alkyl group having 1 to 6 carbon atoms include alkylcarbonylthio groups carrying, as the alkyl moiety, a linear alkyl group having 1 to 6 carbon atoms, still preferably alkylcarbonylthio groups carrying, as the alkyl moiety, a linear alkyl group having 1 to 3 carbon atoms, and particularly preferably an acetylthio group.

In $R_9$, a benzoyl group is particularly preferable as the arylcarbonylthio group carrying, as the aryl moiety, an aryl group having 6 to 12 carbon atoms.

In $R_9$, linear alkyl groups having 1 to 3 carbon atoms are preferable as the linear or branched alkyl group having 1 to 6 carbon atoms and a methyl group is still preferable therefor.

In $R_9$, an acetoxy group is preferable as the alkylcarbonyloxy group carrying, as the alkyl moiety, a linear or branched alkyl group having 1 to 6 carbon atoms.

In $R_9$, a benzoyloxy group is preferable as the arylcarbonyloxy group carrying, as the aryl moiety, an aryl group having 6 to 12 carbon atoms.

Particularly preferable examples of $R_9$ include a hydrogen atom and hydroxyl, thiol, acetylthio and benzoylthio groups.

In $R_2$, preferable examples of the group represented by -N($R_3$)$R_4$ include methylamino, dimethylamino, N-allyl-N-methylamino, N-(3-(acetylthio)isobutyryl)-N-methylamino, N-(3-(benzoylthio)isobutyryl)-N-methylamino, N-((3,4,5-trimethoxyphenyl)acetyl)-N-methylamino, N-((3,5-di-t-butyl-4-hydroxyphenylthio)acetyl)-N-methylamino and N-(2-(3,4,5-trimethoxyphenyl)ethyl)-N-methylamino groups. Still preferable examples thereof include dimethylamino, N-(3-(acetylthio)isobutyryl)-N-methylamino, N-(3-(benzoylthio)isobutyryl)-N-methylamino, N-((3,4,5-trimethoxyphenyl)acetyl)-N-methylamino and N-((3,5-di-t-butyl-4-hydroxyphenylthio)acetyl)-N-methylamino groups.

In $R_2$, preferable examples of the group represented by the general formula (2):

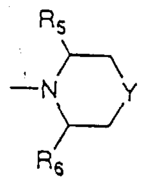

(2)

wherein $R_5$, $R_6$ and Y are each as defined above; include morpholino, 2,6-dimethylpiperidin-1-yl, 4-(acetylthio)piperidin-1-yl, piperazin-1-yl, 4-methylpiperazin-1-yl, 4-ethylpiperazin-1-yl, 4-(2,2,2-trifluoroethyl)piperazin-1-yl, 4-(2-hydroxyethyl)piperazine-1-yl, 4-(2-chloroethyl)piperazin-1-yl, 4-benzylpiperazin-1-yl, 4-(3,4-dimethoxyphenylmethyl)piperazin-1-yl, 4-(2-(acetylthio)ethyl)piperazin-1-yl, 4-(2-(3,4,5-trimethoxyphenyl)ethyl)piperazin-1-yl, 4-(2-(3,5-di-t-butyl-4-hydroxyphenylthio)ethyl)piperazin-1-yl, 4-(2-(benzoylthio)ethyl)piperazin-1-yl, 4-(2,3,4-trimethoxyphenylmethyl)piperazin-1-yl, 4-(3,4,5-trimethoxyphenylmethyl)piperazin-1-yl, 4-phenylpiperazin-1-yl, 4-(2-methoxyphenyl)piperazin-1-yl, 4-isopropylpiperazin-1-yl, 4-cyclobutylpiperazin-1-yl, 4-cyclopentylpiperazin-1-yl, 4-cyclohexylpiperazin-1-yl, 4-benzyloxycarbonylpiperazin-1-yl, 4-(2-oxo-4-thiazolidinecarbonyl)piperazin-1-yl, 4-(2,2-dimethyl-6-nitro-2H-1-benzopyran-4-carbonyl)piperazin-1-yl, 4-(6-cyano-2,2-dimethyl-2H-1-benzopyran-4-carbonyl)piperazin-1-yl, 4-(3-(acetylthio)isobutyryl)piperazin-1-yl, 4-((acetylthio)acetyl)piperazin-1-yl, 4-(3-(benzoylthio)isobutyryl)piperazin-1-yl, 4-((3,4,5-trimethoxyphenyl)acetyl)piperazin-1-yl, 4-((3,5-di-t-butyl-4-hydroxyphenylthio)acetyl)piperazin-1-yl and 4-((benzoylthio)acetyl)piperazin-1-yl groups. Still preferable examples thereof include morpholino, 2,6-dimethylpiperidin-1-yl, 4-(acetylthio)piperidin-1-yl, 4-methylpiperazin-1-yl, 4-ethylpiperazin-1-yl, 4-isopropylpiperazin-1-yl, 4-cyclopentylpiperazin-1-yl, 4-(2,2,2-trifluoroethyl)piperazin-1-yl, 4-benzylpiperazin-1-yl, 4-(3-(acetylthio)isobutyryl)piperazin-1-yl, 4-((acetylthio)acetyl)piperazin-1-yl, 4-(3-(benzoylthio)-isobutyryl)piperazin-1-yl, 4-((3,4,5-trimethoxyphenyl)acetyl)piperazin-1-yl, 4-((3,5-di-t-butyl-4-hydroxyphenylthio)acetyl)piperazin-1-yl and 4-((benzoylthio)acetyl)piperazin-1-yl groups. Particularly preferable examples thereof include morpholino, 4-methylpiperazin-1-yl, 4-ethylpiperazin-1-yl, 4-isopropylpiperazin-1-yl and 4-benzylpiperazin-1-yl groups.

Preferable examples of $R_2$ include methylamino, dimethylamino, N-allyl-N-methylamino, N-(3-(acetylthio)isobutyryl)-N-methylamino, N-(3-(benzoylthio)isobutyryl)-N-methylamino, N-(3,4,5-trimethoxyphenyl)acetyl-N-methylamino, N-((3,5-di-t-butyl-4-hydroxyphenylthio)acetyl)-N-methylamino, N-(2-(3,4,5-trimethoxyphenyl)ethyl)-N-methylamino, morpholino, 2,6-dimethylpiperidin-1-yl, 4-(acetylthio)piperidin-1-yl, piperazin-1-yl, 4-methylpiperazin-1-yl, 4-ethylpiperazin-1-yl, 4-isopropylpiperazin-1-yl, 4-(2,2,2-trifluoroethyl)piperazin-1-yl, 4-(2-hydroxyethyl)piperazin-1-yl, 4-(2-chloroethyl)piperazin-1-yl, 4-benzylpiperazin-1-yl, 4-(3,4-dimethoxyphenylmethyl)piperazin-1-yl, 4-(2-(acetylthio)ethyl)piperazin-1-yl, 4-(2-(3,4,5-trimethoxyphenyl)ethyl)piperazin-1-yl, 4-(2-(3,5-di-t-butyl-4-hydroxyphenylthio)ethyl)piperazin-1-yl, 4-(2-(benzoylthio)ethyl)piperazin-1-yl, 4-(2,3,4-trimethoxyphenylmethyl)piperazin-1-yl, 4-(3,4,5-trimethoxyphenylmethyl)piperazin-1-yl, 4-phenylpiperazin-1-yl, 4-(2-methoxyphenyl)piperazin-1-yl, 4-cyclobutylpiperazin-1-yl, 4-cyclopentylpiperazin-1-yl, 4-cyclohexylpiperazin-1-yl, 4-benzyloxycarbonylpiperazin-1-yl, 4-(2-oxo-4-thiazolidinecarbonyl)piperazin-1-yl, 4-(2,2-dimethyl-6-nitro-2H-1-benzopyran-4-carbonyl)piperazin-1-yl, 4-(6-cyano-2,2-dimethyl-2H-1-benzopyran-4-carbonyl)piperazin-1-yl, 4-(3-(acetylthio)isobutyryl)piperazin-1-yl, 4-((acetylthio)acetyl)piperazin-1-yl, 4-(3-(benzoylthio)isobutyryl)piperazin-1-yl, 4-((3,4,5-trimethoxyphenyl)acetyl)piperazin-1-yl, 4-((3,5-di-t-butyl-4-hydroxyphenylthio)acetyl)piperazin-1-yl and 4-((benzoylthio)acetyl)piperazin-1-yl groups. Still preferable examples thereof include dimethylamino, N-(3-(acetylthio)isobutyryl)-N-methylamino, N-(3-(benzoylthio)isobutyryl)-N-methylamino, N-(3,4,5-trimethoxyphenyl)acetyl-N-methylamino, N-((3,5-di-t-butyl-4-hydroxyphenylthio)acetyl)-N-methylamino, morpholino, 2,6-dimethylpiperidin-1-yl, 4-(acetylthio)piperidin-1-yl, 4-methylpiperazin-1-yl, 4-ethylpiperazin-1-yl, 4-isopropylpiperazin-1-yl, 4-(2,2,2-trifluoroethyl)piperazin-1-yl, 4-benzylpiperazin-1-yl, 4-cyclopentylpiperazin-1-yl, 4-(3-(acetylthio)isobutyryl)piperazin-1-yl, 4-((acetylthio)acetyl)piperazin-1-yl, 4-(3-(benzoylthio)isobutyryl)piperazin-1-yl, 4-((3,4,5-trimethoxyphenyl)acetyl)piperazin-1-yl, 4-((3,5-di-t-butyl-4-hydroxyphenylthio)acetyl)piperazin-1-yl and 4-((benzoylthio)acetyl)piperazin-1-yl groups. Particularly preferable examples thereof include morpholino, 4-methylpiperazin-1-yl, 4-ethylpiperazin-1-yl, 4-isopropylpiperazin-1-yl and 4-benzylpiperazin-1-yl groups.

Preferable examples of the compounds represented by the general formula (1):

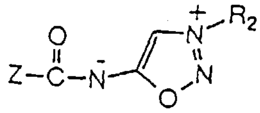

(1)

include N-((3,5-di-t-butyl-4-hydroxyphenylthio)acetyl)-3-(4-methylpiperazin-1-yl)sydnonimine, N-((S)-3-(benzoylthio)isobutyryl)-3-(4-methylpiperazin-1-yl)sydnonimine, N-((S)-3-(acetylthio)isobutyryl)-3-morpholinosydnonimine, N-((S)-3-(benzoylthio)isobutyryl)-3-(4-benzylpiperazine-1-yl)sydnonimine, N-((benzoylthio)acetyl)-3-(4-methylpiperazin-1-yl)sydnonimine, N-((3,4,5-trimethoxyphenyl)acetyl)-3-(4-methylpiperazin-1-yl)sydnonimine, N-((S)-3-(benzoylthio)isobutyryl)-3-(4-ethylpiperazin-1-yl)sydnonimine, N-((S)-3-(benzoylthio)isobutyryl)-3-(4-(2,2,2-trifluoroethyl)piperazin-1-yl)sydnonimine, N-((S)-3-(benzoylthio)isobutyryl)-3-(4-cyclopentylpiperazin-1-yl)sydnonimine, N-((S)-3-(acetylthio)isobutyryl)-3-(4-methylpiperazin-1-yl) sydnonimine, N-((S)-3-(acetylthio)isobutyryl)-3-(4-ethylpiperazin-1-yl)sydnonimine, N-((S)-3-(acetylthio)-isobutyryl)-3-(4-isopropylpiperazin-1-yl)sydnonimine, 3-(4-(acetylthio)piperidin-1-yl)-N-((3,4,5-trimethoxyphenyl)acetyl)sydnonimine, N-(acetylthioacetyl)-3-(4-ethylpiperazin-1-yl)sydnonimine, N-(acetylthioacetyl)-3-(4-isopropylpiperazin-1-yl)sydnonimine, N-(benzoylthioacetyl)-3-(4-isopropylpiperazin-1-yl)sydnonimine, N-(benzoylthioacetyl)-3-(4-(2,2,2-trifluoroethyl)piperazin-1-yl)sydnonimine, N-(benzoylthioacetyl)-3-(4-cyclopentylpiperazin-1-yl)sydnonimine, N-(2-(acetylthio)isobutyryl)-3-(4-benzylpiperazin-1-yl)sydnonimine, N-(3-(acetylthio)propionyl)-3-(4-methylpiperazin-1-yl)sydnonimine, N-(3-(acetylthio)propionyl)-3-(4-benzylpiperazine-1-yl)sydnonimine, N-(3-(acetylthio)propionyl)-3-(4-ethylpiperazin-1-yl)sydnonimine, N-(3-(acetylthio)propionyl)-3-(4-isopropylpiperazin-1-yl)sydnonimine, N-(3-(benzoylthio)propionyl)-3-(4-methylpiperazin-1-yl)sydnonimine, N-(3-(benzoylthio)propionyl)-3-(4-isopropylpiperazin-1-yl)sydnonimine, N-(4-(acetylthio)butyryl)-3-(4-methylpiperazin-1-yl)sydnonimine, N-(3-(cyclohexanecarbonylthio)propionyl)-3-(4-methylpiperazin-1-yl)sydnonimine, N-((2,6-dimethylbenzoylthio)acetyl)-3-(4-methylpiperazin-1-yl)sydnonimine, N-((2,6-dimethylbenzoylthio)acetyl)-3-(4-ethylpiperazin-1-yl)sydnonimine, N-((2,6-dimethylbenzoylthio)acetyl)-3-(4-isopropylpiperazin-1-yl)sydnonimine, N-(3-(4-fluorobenzoylthio)propionyl)-3-(4-methylpiperazin-1-yl)sydnonimine, N-(3-(4-fluorobenzoylthio)propionyl)-3-(4-ethylpiperazin-1-yl)sydnonimine, N-(3-(4-fluorobenzoylthio)propionyl)-3-(4-isopropylpiperazin-1-yl)sydnonimine, N-(3-(4-methoxybenzoylthio)propionyl)-3-(4-methylpiperazin-1-yl)sydnonimine, N-(3-(2,6-dimethylbenzoylthio)propionyl)-3-(4-methylpiperazin-1-yl)sydnonimine, N-(3-(2,6-dimethylbenzoylthio)propionyl)-3-(4-ethylpiperazin-1-yl)sydnonimine, N-(3-(2,6-dimethylbenzoylthio)propionyl)-3-(4-isopropylpiperazin-1-yl)sydnonimine, N-(3-(2,6-dichlorobenzoylthio)propionyl)-3-(4-methylpiperazin-1-yl)sydnonimine, N-(3-(2,6-difluorobenzoylthio)propionyl)-3-(4-methylpiperazin-1-yl)sydnonimine, N-(3-(2,6-difluorobenzoylthio)propionyl)-3-(4-ethylpiperazin-1-yl)sydnonimine, N-(3-(2,6-difluorobenzoylthio)propionyl)-3-(4-isopropylpiperazin-1-yl)sydnonimine, N-(3-(2,6-dimethoxybenzoylthio)propionyl)-3-(4-methylpiperazin-1-yl)sydnonimine, N-(pivaloylthio-acetyl)-3-(4-methylpiperazin-1-yl)sydnonimine, N-(pivaloylthioacetyl)-3-(4-ethylpiperazin-1-yl)sydnonimine, N-(pivaloylthioacetyl)-3-(4-isopropylpiperazin-1-yl)sydnonimine, N-(3-(pivaloylthio)propionyl)-3-(4-methylpiperazin-1-yl)sydnonimine, N-(3-(pivaloylthio)propionyl)-3-(4-ethylpiperazin-1-yl)sydnonimine, N-(3-(pivaloylthio)propionyl)-3-(4-isopropylpiperazin-1-yl)sydnonimine, N-(pivaloylthiopivaloyl)-3-(4-methylpiperazin-1-yl)sydnonimine, N-(pivaloylthiopivaloyl)-3-(4-ethylpiperazin-1-yl)sydnonimine, N-(pivaloylthiopivaloyl)-3-(4-isopropylpiperazin-1-yl)sydnonimine, N-(3-(benzyloxycarbonylthio)propionyl)-3-(4-methylpiperazin-1- yl)sydnonimine, N-(ethylcarbamoylthioacetyl)-3-(4-methylpiperazin-1-yl)sydnonimine, N-(3-(ethylcarbamoylthio)propionyl)-3-(4-methylpiperazin-1-yl)sydnonimine, N-(3-(ethylcarbamoylthio)propionyl)-3-(4-ethylpiperazin-1-yl)sydnonimine, N-(3-(ethylcarbamoylthio)propionyl)-3-(4-isopropylpiperazin-1-yl)sydnonimine, N-(ethylcarbamoylthiopivaloyl)-3-(4-methylpiperazin-1-yl)sydnonimine, N-(ethylcarbamoylthiopivaloyl)-3-(4-ethylpiperazin-1-yl)sydnonimine, N-(ethylcarbamoylthiopivaloyl)-3-(4-isopropylpiperazin-1-yl)sydnonimine, N-(3-(t-butylcarbamoylthio)propionyl)-3-(4-methylpiperazin-1-yl)sydnonimine, N-(3-(t-butylcarbamoylthio)propionyl)-3-(4-ethylpiperazin-1-yl)sydnonimine, N-(3-(t-butylcarbamoylthio)propionyl)-3-(4-isopropylpiperazin-1-yl)sydnonimine, N-(3-(phenylcarbamoylthio)propionyl)-3-(4-methylpiperazin-1-yl)sydnonimine, N-(3-(phenylcarbamoylthio)propionyl)-3-(4-ethylpiperazin-1-yl)sydnonimine, N-(3-(phenylcarbamoylthio)propionyl)-3-(4-isopropylpiperazin-1-yl)sydnonimine, N-(4-benzoylthio)butyryl)-3-(4-methylpiperazin-1-yl)sydnonimine, N-(3-acetylthio-2-benzylpropionyl)-3-(4-methylpiperazin-1-yl)sydnonimine, N-(3-(3,5-di-t-butyl-4-hydroxyphenylthio)propionyl)-3-(4-methylpiperazin-1-yl)sydnonimine, N-((R)-2-acetylamino-3-(acetylthio)propionyl)-3-(4-methylpiperazin-1-yl)sydnonimine, N-((R)-2-acetylamino-3-(acetylthio)propionyl)-3-(4-ethylpiperazin-1-yl)sydnonimine, N-((R)-2-acetylamino-3-(acetylthio)propionyl)-3-(4-isopropylpiperazin-1-yl)sydnonimine, N-((R)-2-acetylamino-3-(benzoylthio)propionyl)-3-(4-methylpiperazin-1- yl)sydnonimine, N-((R)-2-acetylamino-3-(4-fluorobenzoylthio)propionyl)-3-(4-methylpiperazin-1-yl)sydnonimine, N-((R)-2-acetylamino-3-(4-trifluoromethylbenzoylthio)propionyl)-3-(4-methylpiperazin-1-yl)sydnonimine, N-((6-hydroxy-2,5,7,8-tetramethylchroman-2-carbonylthio)acetyl)-3-(4-methylpiperazin-1-yl)sydnonimine, N-((6-hydroxy-2,5,7,8-tetramethylchroman-2-carbonylthio)acetyl)-3-(4-ethylpiperazin-1-yl)sydnonimine, N-((6-hydroxy-2,5,7,8-tetramethylchroman-2-carbonylthio)acetyl)-3-(4-isopropylpiperazin-1-yl)sydnonimine, N-(6-hydroxy-2,5,7,8-tetramethylchroman-2-carbonyl)-3-(4-methylpiperazin-1-yl)sydnonimine, N-(6-hydroxy-2,5,7,8-tetramethylchroman-2-carbonyl)-3-(4-isopropylpiperazin-1-yl)sydnonimine and N-((R)-2-oxo-4-thiazolidinecarbonyl)-3-(4-methylpiperazin-1-yl)sydnonimine. Particularly preferable examples thereof include N-((3,5-di-t-butyl-4-hydroxyphenylthio)acetyl)-3-(4-methylpiperazin-1-yl)sydnonimine, N-((S)-3-(benzoylthio)isobutyryl)-3-(4-methylpiperazin-1-yl)sydnonimine, N-((S)-3-(acetylthio)isobutyryl)-3-(4-isopropylpiperazin-1-yl)sydnonimine, N-(acetylthioacetyl)-3-(4-ethylpiperazin-1-yl)sydnonimine, N-(3-(acetylthio)propionyl)-3-(4-ethylpiperazin-1-yl) sydnonimine, N-((2,6-dimethylbenzoylthio)acetyl)-3-(4-methylpiperazin-1-yl)sydnonimine, N-(3-(2,6-dimethylbenzoylthio)propionyl)-3-(4-methylpiperazin-1-yl)sydnonimine, N-(3-(pivaloylthio)propionyl-3-(4-methylpiperazin-1-yl)sydnonimine, N-(3-(pivaloylthio)propionyl)-3-(4-ethylpiperazin-1-yl) sydnonimine, N-(3-(ethylcarbamoylthio)propionyl)-3-(4-methylpiperazin-1-yl)sydnonimine, N-(3-(ethylcarbamoylthio)propionyl)-3-(4-ethylpiperazin-1-yl)sydnonimine, N-(3-(ethylcarbamoylthio)propionyl)-3-(4-isopropylpiperazin-1-yl)sydnonimine, N-(3-(phenylcarbamoylthio)propionyl)-3-(4-methylpiperazin-1-yl)sydnonimine, N-(4-benzoylthio)butyryl)-3-(4-methylpiperazin-1-yl)sydnonimine, N-(3-(3,5-di-t-butyl-4-hydroxyphenylthio)propionyl)-3-(4-methylpiperazin-1-yl)sydnonimine, N-((R)-2-acetylamino-3-(acetylthio)propionyl)-3-(4-methylpiperazin-1-yl)sydnonimine and N-(6-hydroxy-2,5,7,8-tetramethylchroman-2-carbonyl)-3-(4-isopropylpiperazin-1-yl)sydnonimine.

Moreover, it is possible to use, as intermediates in synthesizing the compounds of the present invention, compounds of the general formula (1) wherein $R^2$ represents, for example, methylamino, N-allyl-N-methylamino, piperazine-1-yl, 4-(2-hydroxyethyl)piperazine-1-yl, 4-(2-chloroethyl)piperazine-1-yl, 4-benzylpiperazine-1-yl, 4-benzyloxycarbonylpiperazin-1-yl, etc. or pharmaceutically acceptable salts thereof.

Furthermore, it is possible to use, as intermediates in synthesizing the compounds of the present invention, compounds represented by the following general formula (11) or pharmaceutically acceptable salts thereof:

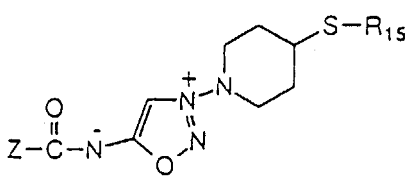

(11)

wherein Z is as defined in the above general formula (1); and $R_{15}$ represents an optionally substituted linear or branched alkyl group having 1 to 6 carbon atoms; a monocyclic heterocycle containing an oxygen atom as the heteroatom; an alkylcarbonyl group carrying, as the alkyl moiety, a linear or branched alkyl group having 1 to 6 carbon atoms; an alkyloxycarbonyl group carrying, as the alkyl moiety, an optionally substituted alkyl group having 1 to 6 carbon atoms; or an arylcarbonyl group carrying, as the aryl moiety, an optionally substituted aryl group having 6 to 12 carbon atoms;

or compounds represented by the following general formula (12) or pharmaceutically acceptable salts thereof:

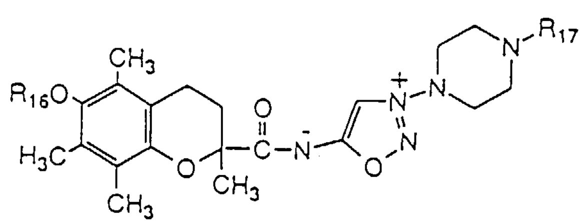

(12)

wherein $R_{16}$ represents an optionally substituted linear or branched alkyl group having 1 to 6 carbon atoms; a monocyclic heterocycle containing an oxygen atom as the heteroatom; an alkylcarbonyl group carrying, as the alkyl moiety, a linear or branched alkyl group having 1 to 6 carbon atoms; an arylcarbonyl group carrying, as the aryl moiety, an optionally substituted aryl group having 6 to 12 carbon atoms; or a silyl group substituted by a linear or branched alkyl group having 1 to 6 carbon atoms; and $R_{17}$ represents an optionally substituted linear or branched alkyl group having 1 to 6 carbon atoms; or an alkyloxycarbonyl group carrying, as the alkyl moiety, an optionally substituted linear or branched alkyl group having 1 to 6 carbon atoms.

Preferable examples of $R_{15}$ include optionally substituted linear or branched alkyl groups having 1 to 4 carbon atoms, monocyclic 5- or 6-membered heterocycles containing an oxygen atom as the heteroatom, alkylcarbonyl groups carrying, as the alkyl moiety, a linear or branched alkyl group having 1 to 4 carbon atoms, alkyloxycarbonyl groups carrying, as the alkyl moiety, an optionally substituted linear alkyl group having 1 to 4 carbon atoms and arylcarbonyl

groups carrying, as the aryl moiety, an aryl group having 6 to 12 carbon atoms. Still preferable examples thereof include 4-methoxybenzyl, benzyl, t-butyl, methoxymethyl, benzyloxymethyl, 2-tetrahydropyran-1-yl, acetyl, t-butoxycarbonyl, benzyloxycarbonyl, 4-methoxybenzyloxycarbonyl and benzoyl groups. Among all, a 4-methoxybenzyl group is particularly preferable therefor.

Preferable examples of $R_{16}$ include optionally substituted linear or branched alkyl groups having 1 to 4 carbon atoms, monocyclic 5- or 6-membered heterocycles containing an oxygen atom as the heteroatom, alkylcarbonyl groups carrying, as the alkyl moiety, a linear or branched alkyl group having 1 to 4 carbon atoms, arylcarbonyl groups carrying, as the aryl moiety, an aryl group having 6 to 12 carbon atoms and silyl groups substituted by a linear or branched alkyl group having 1 to 4 carbon atoms. Still preferable examples thereof include methoxymethyl, (4-methoxyphenoxy)methyl, t-butoxymethyl, 2-methoxyethoxymethyl, 2-(trimethylsilyl)ethoxymethyl, benzyl, 1-ethoxyethyl, t-butyl, 2-tetrahydropyran-1-yl, acetyl benzoyl, trimethylsilyl and t-butyldimethylsilyl groups. Among all, a methoxymethyl group is particularly preferable therefor.

Preferable examples of $R_{17}$ include optionally substituted linear or branched alkyl groups having 1 to 4 carbon atoms and alkyloxycarbonyl groups carrying, as the alkyl moiety, an optionally substituted alkyl group having 1 to 4 carbon atoms. Still preferable examples thereof include benzyl and benzyloxycarbonyl groups.

The medically or pharmaceutically acceptable salts to be used in the present invention are exemplified by inorganic acid salts such as hydrochlorides, hydrobromides, phosphates and sulfates and organic acid salts such as acetates, oxalates, fumarates, citrates and tartarates, though the present invention is not restricted thereto.

MODE FOR CARYYING OUT THE INVENTION

The compounds of the present invention can be produced by, for example, the following process.

Namely, compounds represented by the general formula (6) are acylated to give the aimed compounds:

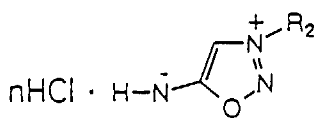

(6)

{wherein $R_2$ is as defined in the above general formula (1); and n is 1 or 2}.

As the acylating agents, use can be made of, for example, the following ones (1) and (2).

(1) Combination of linear or branched carboxylic acids having 1 to 6 carbon atoms preferably exemplified by 3-(acetylthio)isobutyric acid, 3-(benzoylthio)isobutyric acid, 3-(acetylthio)-2-methylisobutyric acid, acetylthioacetic acid, benzoylthioacetic acid, 2-oxo-4-thiazolidinecarboxylic acid, (3,5-di-t-butyl-4-hydroxyphenyl)thioacetic acid, (3,4,5-trimethoxyphenyl)acetic acid, etc., with condensing agents such as N,N'-dicyclohexylcarbodiimide, 1-ethyl-3-(3-dimethylaminopropyl)carbodiimide hydrochloride, N,N-carbonyldiimidazole, etc.

(2) Combination of linear or branched carboxylic acids having 1 to 6 carbon atoms preferably exemplified by 3-(acetylthio)isobutyric acid, 3-(benzoylthio)isobutyric acid, 3-(acetylthio)-2-methylisobutyric acid, acetylthioacetic acid, benzoylthioacetic acid, 2-oxo-4-thiazolidinecarboxylic acid, (3,5-di-t-butyl-4-hydroxyphenyl)thioacetic acid, (3,4,5-trimethoxyphenyl)acetic acid, etc., with ethyl chlorocarbonate and triethylamine.

It is preferable that this reaction is effected in an alcohol (preferably methanol or ethanol), or an inert solvent (preferably methylene chloride, tetrahydrofuran, N,N-dimethylformamide, dimethyl sulfoxide, dioxane, water, etc. or a mixture thereof) optionally in the presence of a base. Examples of the base usable herein include inorganic metal bases (preferably potassium carbonate, sodium carbonate, sodium hydrogencarbonate, potassium hydroxide, sodium hydroxide, sodium hydride, etc.), organic metal bases (preferably sodium alkoxides, potassium alkoxides, alkyllithium, etc.) and organic bases (preferably pyridine, triethylamine, N,N-dimethylaminopyridine, etc.).

Alternatively, the compounds of the present invention can be obtained by subjecting compounds represented by the following general formula (7) or (8) to acylation or reductive alkylation:

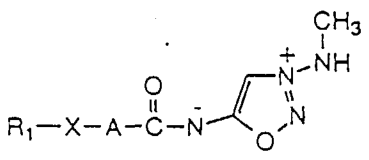

(7)

(wherein A, X and $R_1$ are each as defined above);

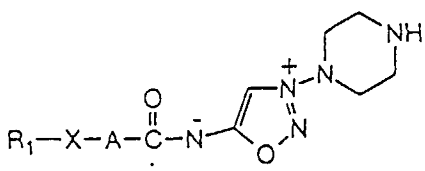

(8)

(wherein A, X and $R_1$ are each as defined above).

As the acylating agents, use can be made of, for example, the following ones (1) to (4).

(1) Combination of linear or branched carboxylic acids having 1 to 6 carbon atoms preferably exemplified by 3-(acetylthio)isobutyric acid, 3-(benzoylthio)isobutyric acid, 3-(acetylthio)-2-methylisobutyric acid, acetylthioacetic acid, benzoylthioacetic acid, 2-oxo-4-thiazolidinecarboxylic acid, (3,5-di-t-butyl-4-hydroxyphenyl)thioacetic acid, (3,4,5-trimethoxyphenyl)acetic acid, etc., with condensing agents such as N,N'-dicyclohexylcarbodiimide, 1-ethyl-3-(3-dimethylaminopropyl)carbodiimide hydrochloride, N,N-carbonyldiimidazole, etc.

(2) Combination of linear or branched carboxylic acids having 1 to 6 carbon atoms preferably exemplified by 3-(acetylthio)isobutyric acid, 3-(benzoylthio)isobutyric acid, 3-(acetylthio)-2-methylisobutyric acid, acetylthioacetic acid, benzoylthioacetic acid, 2-oxo-4-thiazolidine-carboxylic acid, (3,5-di-t-butyl-4-hydroxyphenyl)thioacetic acid, (3,4,5-trimethoxyphenyl)acetic acid, etc., with ethyl chlorocarbonate and triethylamine.

(3) Acid anhydrides such as acetic anhydride and propionic anhydride.

(4) Acyl halides such as acetyl chloride, propionyl chloride and pivaloyl chloride.

Examples of reductive alkylating agents include combination of aldehydes [preferably formalin, paraformaldehyde, 3,4-dimethoxybenzaldehyde, (3,4,5-trimethoxyphenyl)acetaldehyde, (3,5-di-t-butyl-4-hydroxyphenylthio)acetaldehyde, etc.] with reducing agents such as metal hydrogen complexes (preferably sodium borohydride, potassium borohydride, sodium cyanoborohydride, etc.).

It is preferable that this reaction is effected in an alcohol (preferably methanol or ethanol), or an inert solvent (preferably methylene chloride, tetrahydrofuran, N,N-dimethylformamide, dimethyl sulfoxide, dioxane, water, etc. or a mixture thereof) optionally in the presence of a base. Examples of the base usable herein include inorganic metal bases (preferably potassium carbonate, sodium carbonate, sodium hydrogencarbonate, potassium hydroxide, sodium hydroxide, sodium hydride, etc.), organic metal bases (preferably sodium alkoxides, potassium alkoxides, alkyllithium, etc.) and organic bases (preferably pyridine, triethylamine, N,N-dimethylaminopyridine, etc.).

Alternatively, the compounds of the present invention can be produced by reacting compounds represented by the following general formula (9) with thiol metal salts such as potassium thioacetate and potassium thiobenzoate:

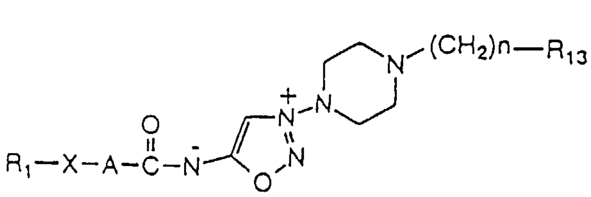

(9)

(wherein A, X and $R_1$ are each as defined above; n is an integer of from 1 to 4; and $R_{13}$ represents a halogen atom such as a chlorine or bromine atom or a leaving group such as a methanesulfonyl or p-toluenesulfonyl group.

Alternatively, the compounds of the present invention can be produced by subjecting compounds represented

by the following general formula (10) to the Mitsunobu reaction with the use of thiocarboxylic acids such as thiobenzoic acid, diethyl azodicarboxylate and triphenylphosphine:

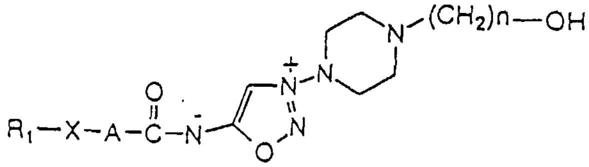

(10)

(wherein A, X, $R_1$ and n are each as defined above.

Moreover, the compounds of the present invention can be obtained by applying the production processes as will be described in detail in the following Examples.

Examples

To further illustrate the processes for producing the compounds of the present invention in greater detail, and not by way of limitation, the following Examples will be given.

To exhibit the usefulness of the compounds of the present invention, the results of the pharmacological tests of the following Test Examples will be given to show the excellent vasodilative, myocardial protective and antiplatelet aggregation effects of typical examples of the compounds of the present invention.

Tables A-1 to A-24 show the chemical structural formulae of the compounds given in Examples.

TABLE A-1

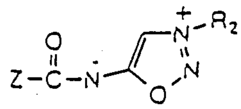

Z=R1-X-A

| Ex.No. | $R_1$ | X | A | $R_2$ |
|---|---|---|---|---|
| 1 | 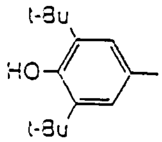 | -S- | $-CH_2-$ | 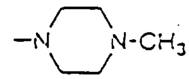 |
| 2 | 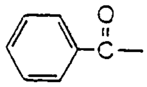 | -S- | 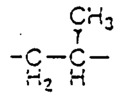 | 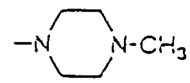 |
| 3 | 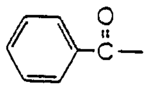 | -S- | $-CH_2-$ | 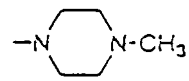 |
| 4 | 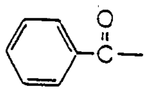 | -S- | 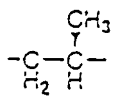 | 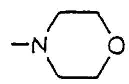 |
| 5 | 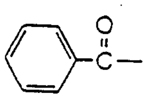 | -S- | 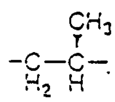 | 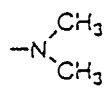 |
| 6 | 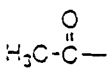 | -S- | 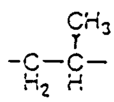 | 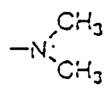 |
| 7 | 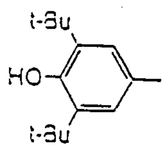 | -S- | $-CH_2-$ | 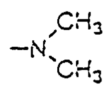 |
| 8 | 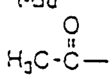 | -S- | $-CH_2-$ | 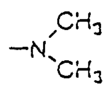 |
| 9 | 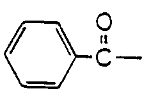 | -S- | $-CH_2-$ | 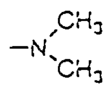 |
| 10 | 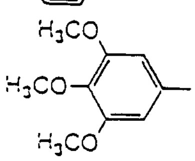 | — | $-CH_2-$ | 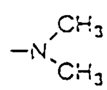 |
| 11 | 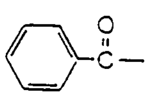 | -S- | 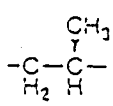 | 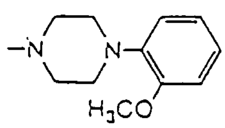 |
| 12 | 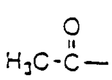 | -S- | 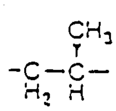 | 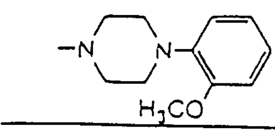 |

TABLE A-2 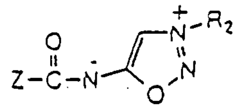 Z=R₁-X-A

| Ex.No. | $R_1$ | X | A | $R_2$ |
|---|---|---|---|---|
| 13 | 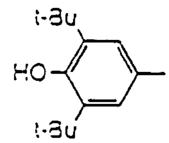 | -S- | $-CH_2-$ | 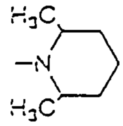 |
| 14 | 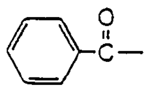 | -S- | 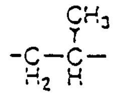 | 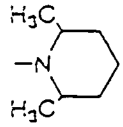 |
| 15 | 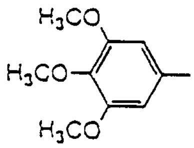 | — | $-CH_2-$ | 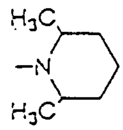 |
| 16 | 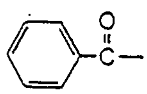 | -S- | $-CH_2-$ | 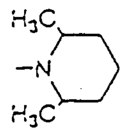 |
| 17 | 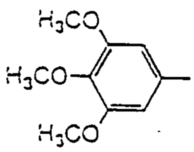 | — | $-CH_2-$ | 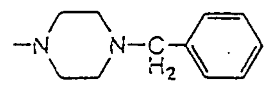 |
| 18 | 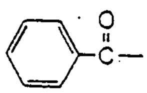 | -S- | 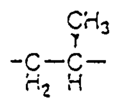 | 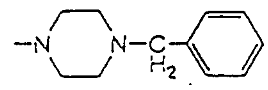 |
| 19-A | 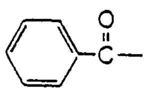 | -S- | 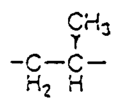 | 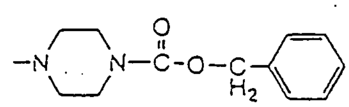 |
| 19-B | 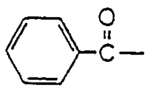 | -S- | 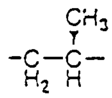 | 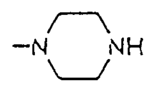 |
| 19-C | 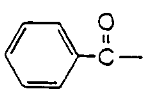 | -S- | 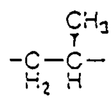 | 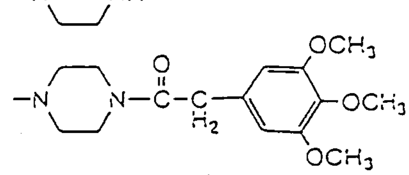 |
| 20 | 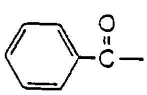 | -S- | 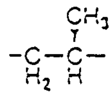 | 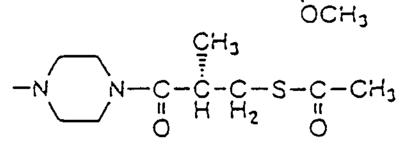 |
| 21 | 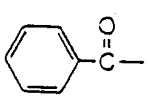 | -S- | 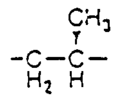 | 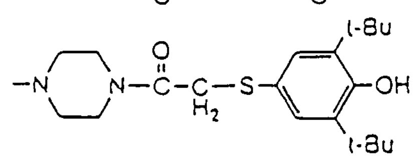 |

TABLE A-3

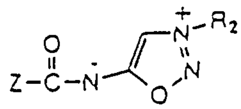

Z=R₁-X-A

| Ex.No. | R₁ | X | A | R₂ |
|---|---|---|---|---|
| 22 | 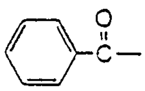 | -S- | 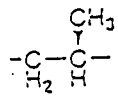 | 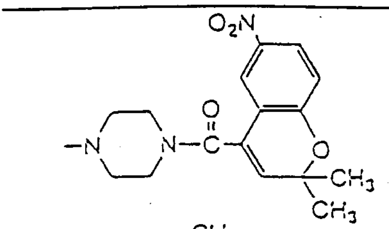 |
| 23 | 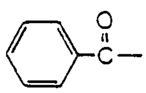 | -S- | 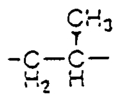 | 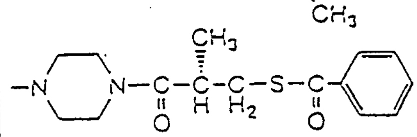 |
| 24-A | 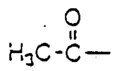 | -S- | 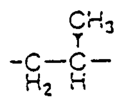 | 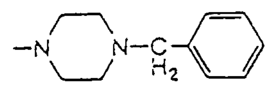 |
| 24-B | 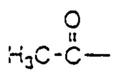 | -S- | 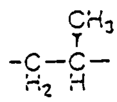 | 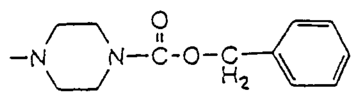 |
| 24-C | 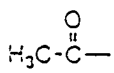 | -S- | 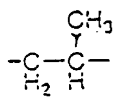 | 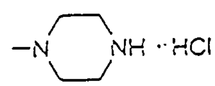 |
| 24-D | 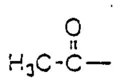 | -S- | 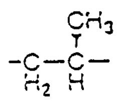 | 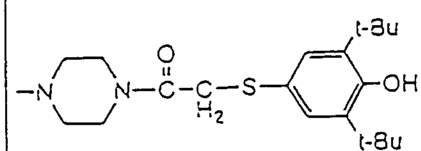 |
| 25 | 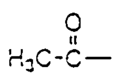 | -S- | 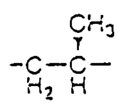 | 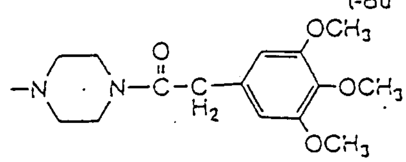 |
| 26 | 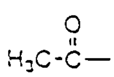 | -S- | 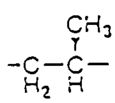 | 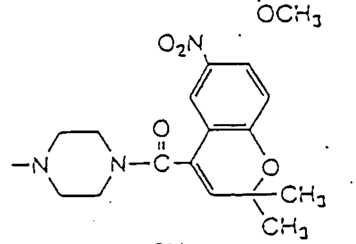 |
| 27 | 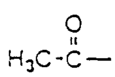 | -S- | 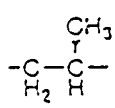 | 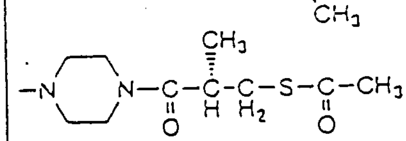 |
| 28 | 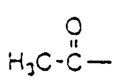 | -S- | 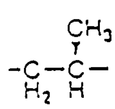 | 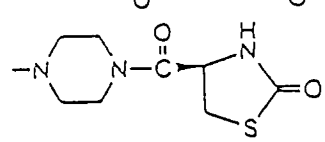 |

TABLE A-4

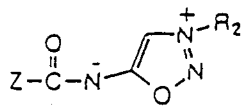

$Z=R_1$-X-A

| Ex.No. | $R_1$ | X | A | $R_2$ |
|---|---|---|---|---|
| 29 | 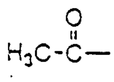 | -S- | 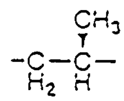 | 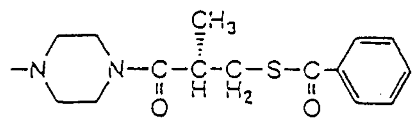 |
| 30-A | 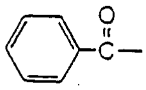 | -S- | $-CH_2-$ | 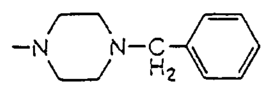 |
| 30-B | 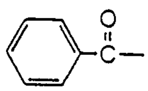 | -S- | $-CH_2-$ | 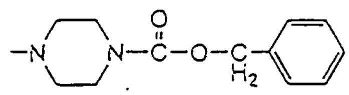 |
| 30-C | 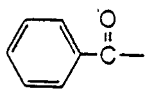 | -S- | $-CH_2-$ | 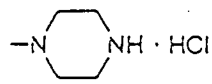 |
| 30-D | 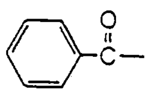 | -S- | $-CH_2-$ | 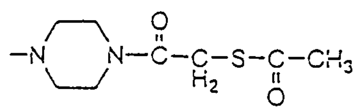 |
| 31 | 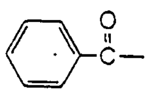 | -S- | $-CH_2-$ | 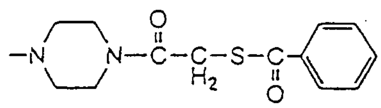 |
| 32 | 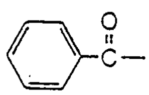 | -S- | $-CH_2-$ | 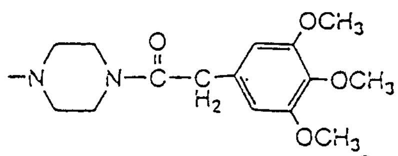 |
| 33 | 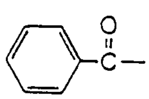 | -S- | $-CH_2-$ | 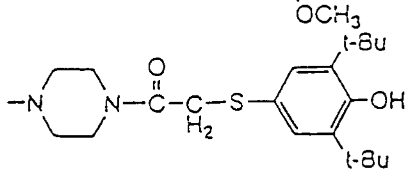 |
| 34-A | 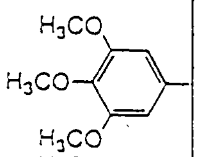 | — | $-CH_2-$ | 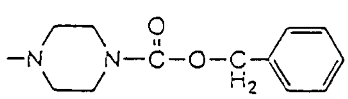 |
| 34-B | 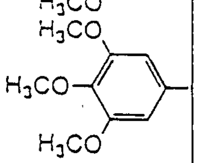 | — | $-CH_2-$ | 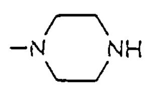 |
| 34-C | 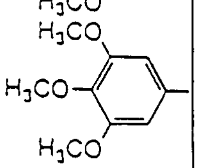 | — | $-CH_2-$ | 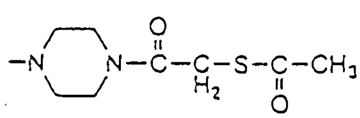 |

TABLE A-S 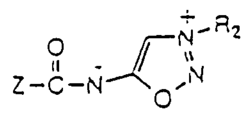 Z=R1-X-A

| Ex.No. | $R_1$ | X | A | $R_2$ |
|---|---|---|---|---|
| 35-A | 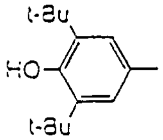 | -S- | $-CH_2-$ | 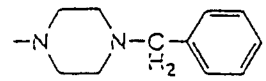 |
| 35-B | 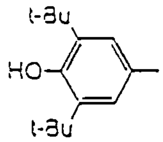 | -S- | $-CH_2-$ | 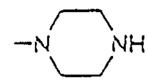 |
| 35-C | 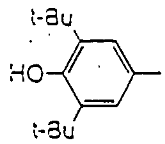 | -S- | $-CH_2-$ | 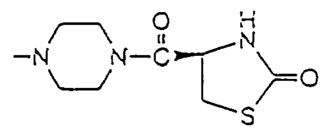 |
| 36 | 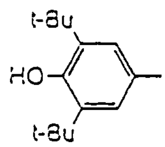 | -S- | $-CH_2-$ | 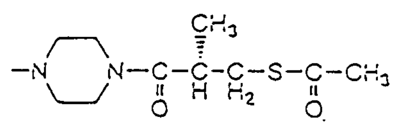 |
| 37 | 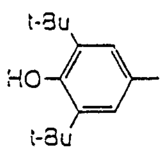 | -S- | $-CH_2-$ | 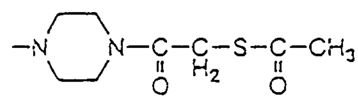 |
| 38-A | 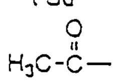 | -S- | $-CH_2-$ | 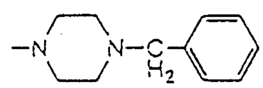 |
| 38-B | 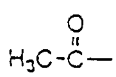 | -S- | $-CH_2-$ | 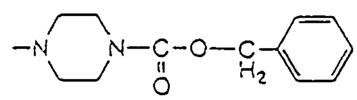 |
| 38-C | 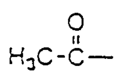 | -S- | $-CH_2-$ | 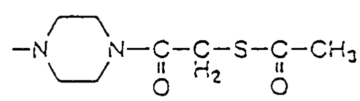 |
| 39 | 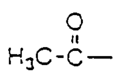 | -S- | $-CH_2-$ | 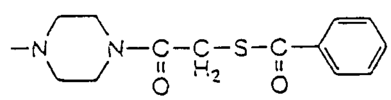 |
| 40 | 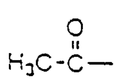 | -S- | $-CH_2-$ | 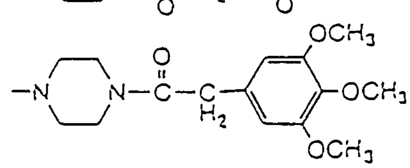 |
| 41 | 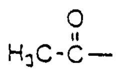 | -S- | $-CH_2-$ | 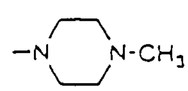 |

TABLE A-6 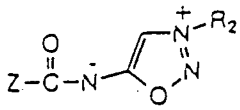 Z=R1-X-A

| Ex.No. | R1 | X | A | R2 |
|---|---|---|---|---|
| 42 | 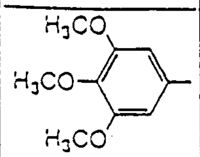 | — | $-CH_2-$ | 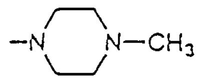 |
| 43 | 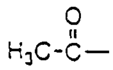 | -S- | 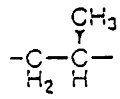 | 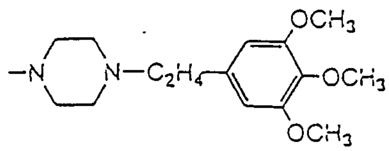 |
| 44 | 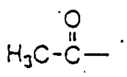 | -S- | 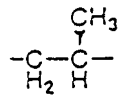 | 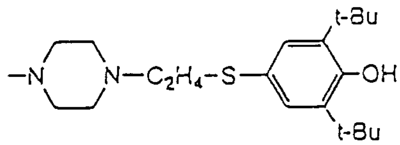 |
| 45 | 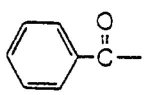 | -S- | 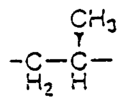 | 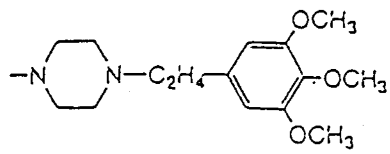 |
| 46 | 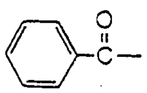 | -S- | 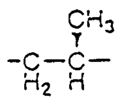 | 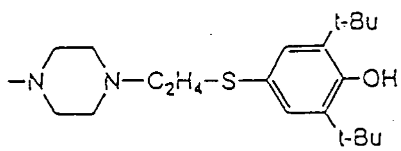 |
| 47 | 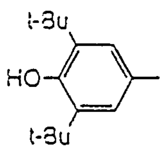 | -S- | $-CH_2-$ | 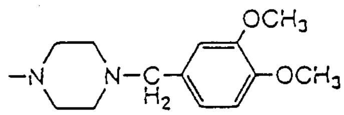 |
| 48 | 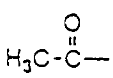 | -S- | $-CH_2-$ | 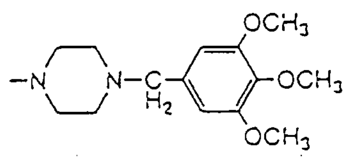 |
| 49 | 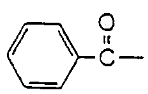 | -S- | $-CH_2-$ | 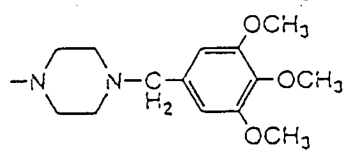 |
| 50 | 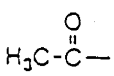 | -S- | $-CH_2-$ | 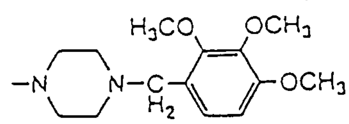 |
| 51 | 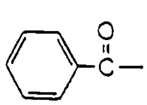 | -S- | $-CH_2-$ | 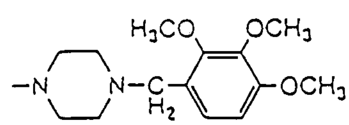 |

TABLE A-7

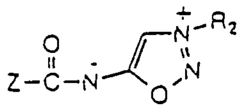

Z=R₁-X-A

| Ex.No. | R₁ | X | A | R₂ |
|---|---|---|---|---|
| 52-A | 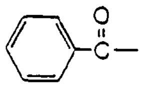 | -S- | 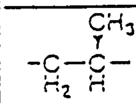 | 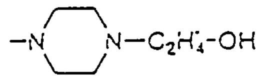 |
| 52-B | 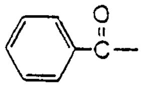 | -S- | 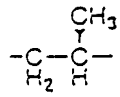 | 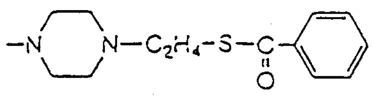 |
| 53-A | 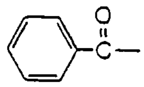 | -S- | $-CH_2-$ | 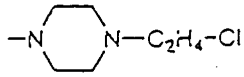 |
| 53-B | 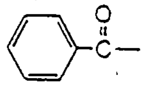 | -S- | $-CH_2-$ | 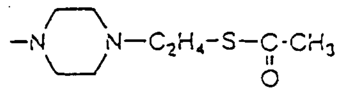 |
| 54-A | 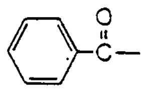 | -S- | 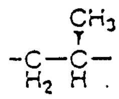 | 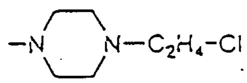 |
| 54-B | 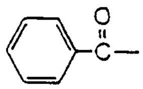 | -S- | 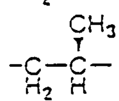 | 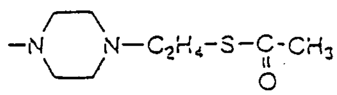 |
| 55-A | 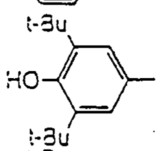 | -S- | $-CH_2-$ | 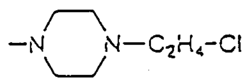 |
| 55-B | 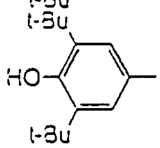 | -S- | $-CH_2-$ | 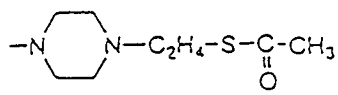 |
| 56-A | 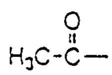 | -S- | 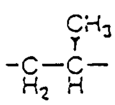 | 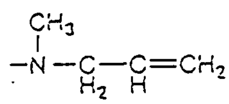 |
| 56-B | 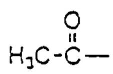 | -S- | 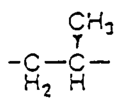 | 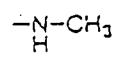 |
| 56-C | 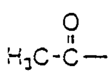 | -S- | 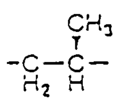 | 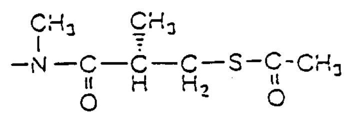 |
| 57 | 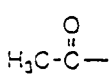 | -S- | 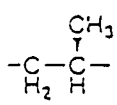 | 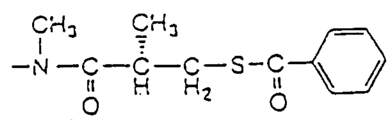 |
| 58 | 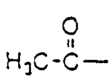 | -S- | 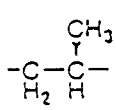 | 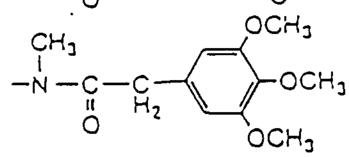 |

TABLE A-8

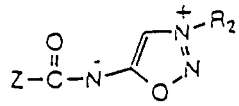

$Z=R_1-X-A$

| Ex.No. | $R_1$ | X | A | $R_2$ |
|---|---|---|---|---|
| 59 | 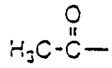 | -S- | 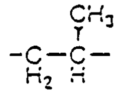 | 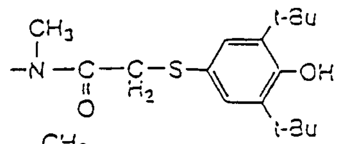 |
| 60-A | 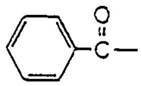 | -S- | 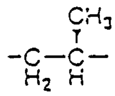 | 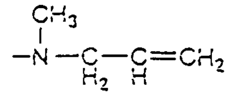 |
| 60-B | 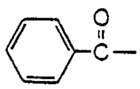 | -S- | 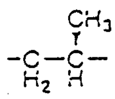 | 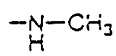 |
| 60-C | 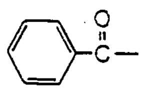 | -S- | 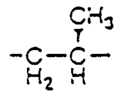 | 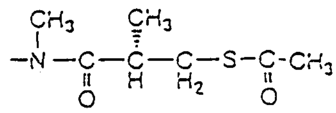 |
| 61 | 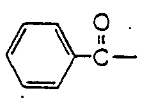 | -S- | 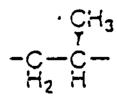 | 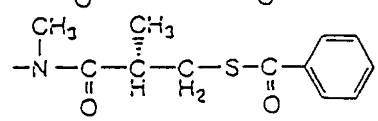 |
| 62 | 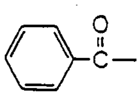 | -S- | 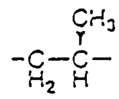 | 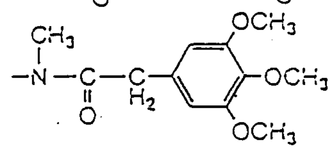 |
| 63-A | 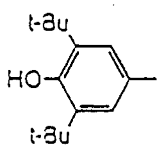 | -S- | $-CH_2-$ | 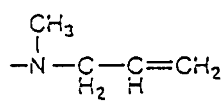 |
| 63-B | 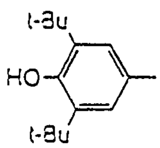 | -S- | $-CH_2-$ | 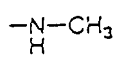 |
| 63-C | 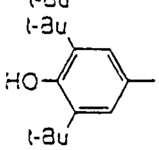 | -S- | $-CH_2-$ | 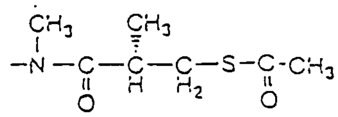 |
| 64 | 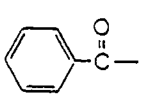 | -S- | 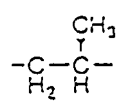 | 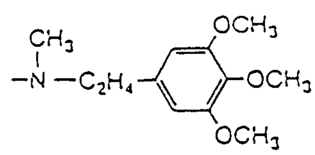 |
| 65 | 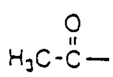 | -S- | 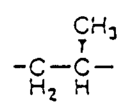 | 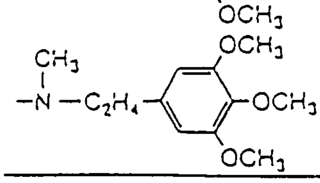 |

TABLE A-9

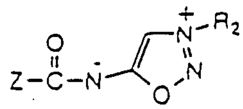

$Z=R_1-X-A$

| Ex.No. | $R_1$ | X | A | $R_2$ |
|---|---|---|---|---|
| 66 | 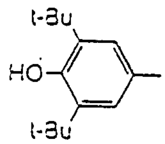 | -S- | $-CH_2-$ | 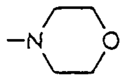 |
| 67 | 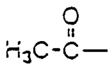 | -S- | 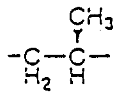 | 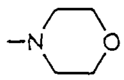 |

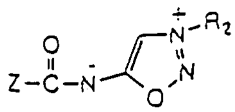

TABLE A-10 Z=R1-X-A

| Ex.No. | R1 | X | A | R2 |
|---|---|---|---|---|
| 68 | 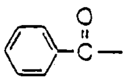 | -S- | 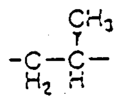 | 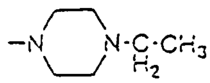 |
| 69 | 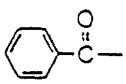 | -S- | 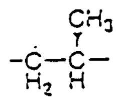 | 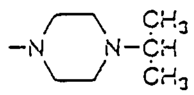 |
| 70 | 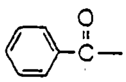 | -S- | 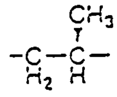 | 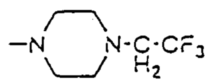 |
| 71 | 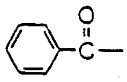 | -S- | 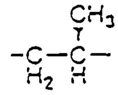 | 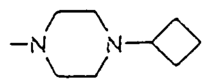 |
| 72 | 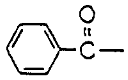 | -S- | 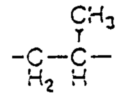 | 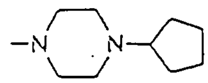 |
| 73 | 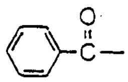 | -S- | 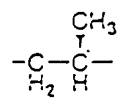 | 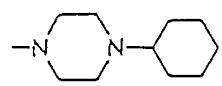 |
| 74 | 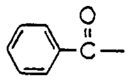 | -S- | 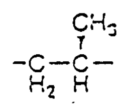 | 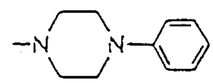 |
| 75 | 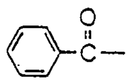 | -S- | 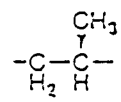 | 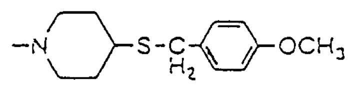 |
| 76 | 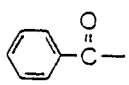 | -S- | 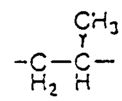 | 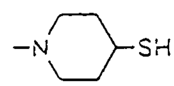 |
| 77 | 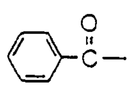 | -S- | 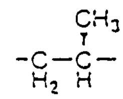 | 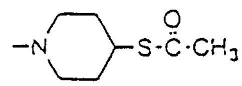 |
| 78 | 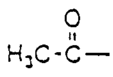 | -S- | 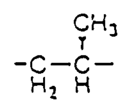 | 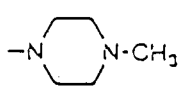 |

TABLE A-11

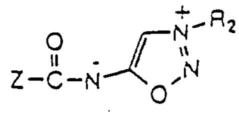

$Z=R_1-X-A$

| Ex.No. | $R_1$ | X | A | $R_2$ |
|---|---|---|---|---|
| 79 | 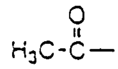 | -S- | 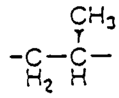 | 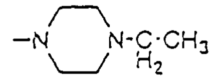 |
| 80 | 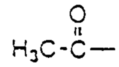 | -S- | 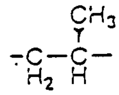 | 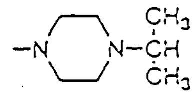 |
| 81 | 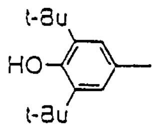 | -S- | 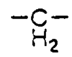 | 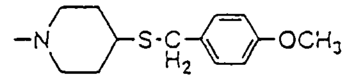 |
| 82 | 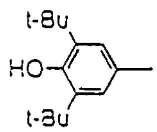 | -S- | 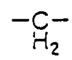 | 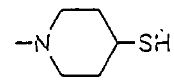 |
| 83 | 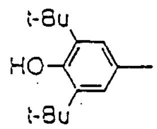 | -S- | 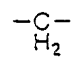 | 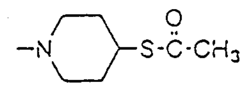 |
| 84 | 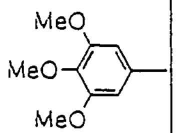 | — | 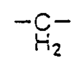 | 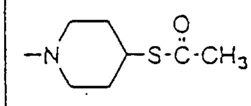 |
| 85 | 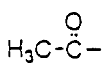 | -S- | 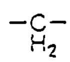 | 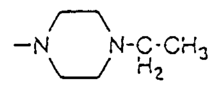 |
| 86 | 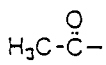 | -S- | 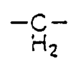 | 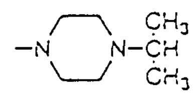 |
| 87 | 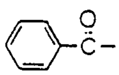 | -S- | 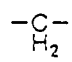 | 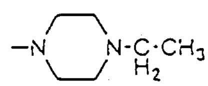 |
| 88 | 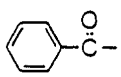 | -S- | 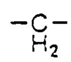 | 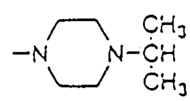 |
| 89 | 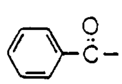 | -S- | 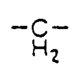 | 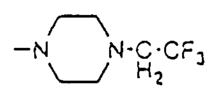 |

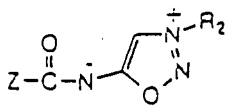

TABLE A-12

Z=R$_1$-X-A

| Ex.No. | R$_1$ | X | A | R$_2$ |
|---|---|---|---|---|
| 90 | 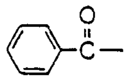 | -S- | 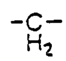 | 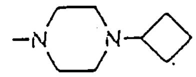 |
| 91 | 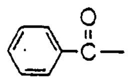 | -S- | 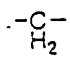 | 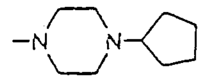 |
| 92 | 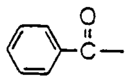 | -S- | 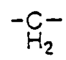 | 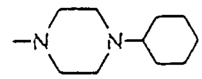 |
| 93 | 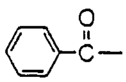 | -S- | 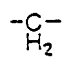 | 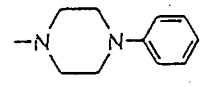 |
| 94 | 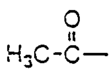 | -S- | 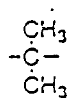 | 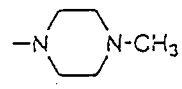 |
| 95 | 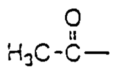 | -S- | 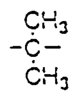 | 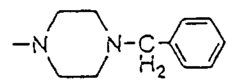 |
| 96 | 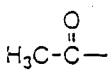 | -S- | 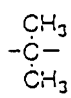 | 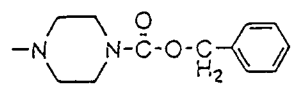 |
| 97 | 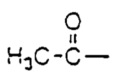 | -S- | 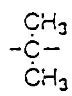 | 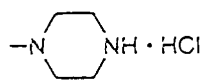 |
| 98 | 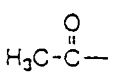 | -S- | 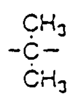 | 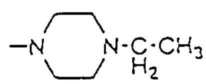 |
| 99 | 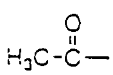 | -S- | 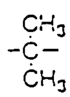 | 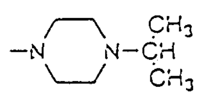 |
| 100 | 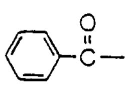 | -S- | 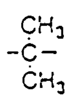 | 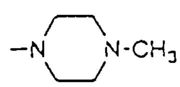 |
| 101 | 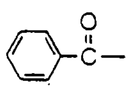 | -S- | 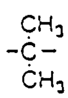 | 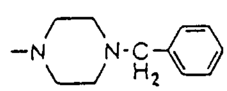 |

TABLE A-13 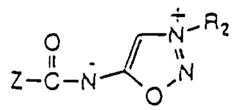 Z=$R_1$-X-A

| Ex.No. | $R_1$ | X | A | $R_2$ |
|---|---|---|---|---|
| 102 | 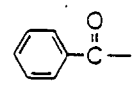 | -S- | 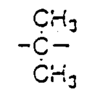 | 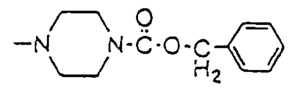 |
| 103 | 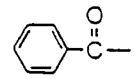 | -S- | 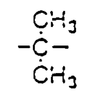 | 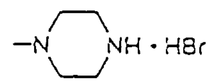 |
| 104 | 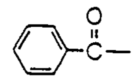 | -S- | 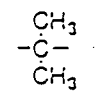 | 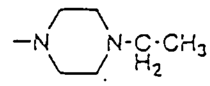 |
| 105 | 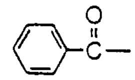 | -S- | 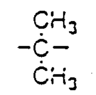 | 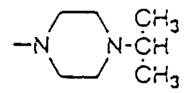 |
| 106 | 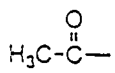 | -S- | 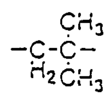 | 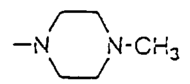 |
| 107 | 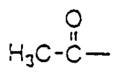 | -S- | 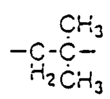 | 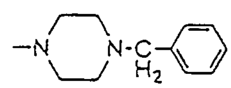 |
| 108 | 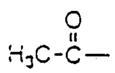 | -S- | 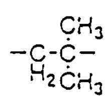 | 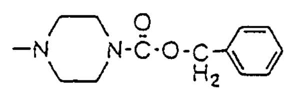 |
| 109 | 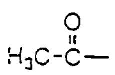 | -S- | 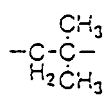 | 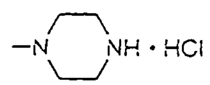 |
| 110 | 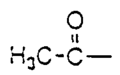 | -S- | 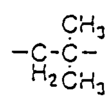 | 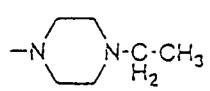 |
| 111 | 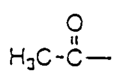 | -S- | 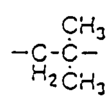 | 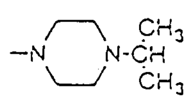 |
| 112 | 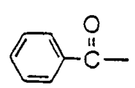 | -S- | 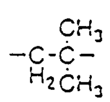 | 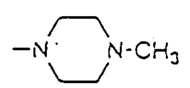 |
| 113 | 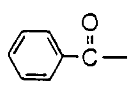 | -S- | 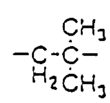 | 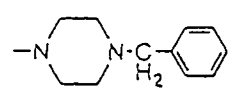 |
| 114 | 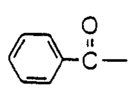 | -S- | 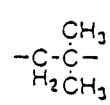 | 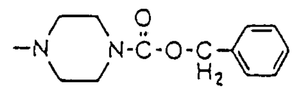 |

TABLE A-14

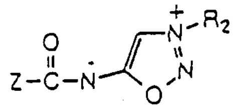

Z=R$_1$-X-A

| Ex.No. | $R_1$ | X | A | $R_2$ |
|---|---|---|---|---|
| 115 | 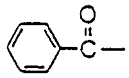 | -S- | $-\underset{H_2}{C}-\overset{CH_3}{\underset{CH_3}{C}}-$ | 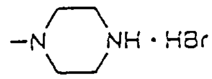 |
| 116 | 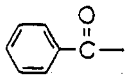 | -S- | $-\underset{H_2}{C}-\overset{CH_3}{\underset{CH_3}{C}}-$ | 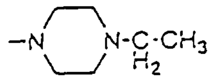 |
| 117 | 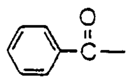 | -S- | $-\underset{H_2}{C}-\overset{CH_3}{\underset{CH_3}{C}}-$ | 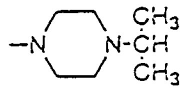 |
| 118 | $H_3C-\overset{O}{\overset{\|}{C}}-$ | -S- | $-\underset{H_2}{C}-\underset{H_2}{C}-$ | 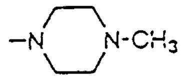 |
| 119 | $H_3C-\overset{O}{\overset{\|}{C}}-$ | -S- | $-\underset{H_2}{C}-\underset{H_2}{C}-$ | 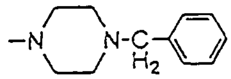 |
| 120 | $H_3C-\overset{O}{\overset{\|}{C}}-$ | -S- | $-\underset{H_2}{C}-\underset{H_2}{C}-$ | 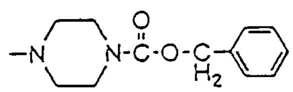 |
| 121 | $H_3C-\overset{O}{\overset{\|}{C}}-$ | -S- | $-\underset{H_2}{C}-\underset{H_2}{C}-$ | 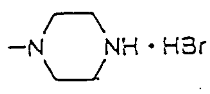 |
| 122 | $H_3C-\overset{O}{\overset{\|}{C}}-$ | -S- | $-\underset{H_2}{C}-\underset{H_2}{C}-$ | 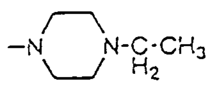 |
| 123 | $H_3C-\overset{O}{\overset{\|}{C}}-$ | -S- | $-\underset{H_2}{C}-\underset{H_2}{C}-$ | 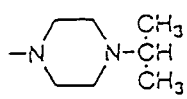 |
| 124 | 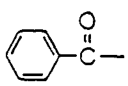 | -S- | $-\underset{H_2}{C}-\underset{H_2}{C}-$ | 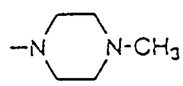 |
| 125 | 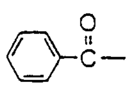 | -S- | $-\underset{H_2}{C}-\underset{H_2}{C}-$ | 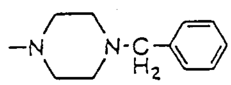 |
| 126 | 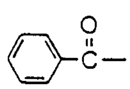 | -S- | $-\underset{H_2}{C}-\underset{H_2}{C}-$ | 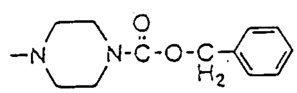 |
| 127 | 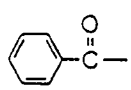 | -S- | $-\underset{H_2}{C}-\underset{H_2}{C}-$ | 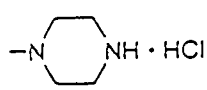 |
| 128 | 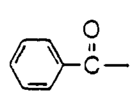 | -S- | $-\underset{H_2}{C}-\underset{H_2}{C}-$ | 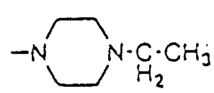 |

TABLE A-15 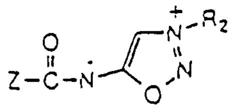 $Z=R_1-X-A$

| Ex.No. | $R_1$ | X | A | $R_2$ |
|---|---|---|---|---|
| 129 | 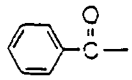 | -S- | $-\underset{H_2}{C}-\underset{H_2}{C}-$ | 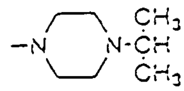 |
| 130 | 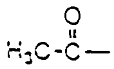 | -S- | $-\underset{H_2}{C}-\underset{H_2}{C}-\underset{H_2}{C}-$ | 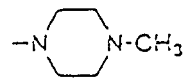 |
| 131 | 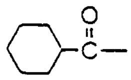 | -S- | $-\underset{H_2}{C}-\underset{H_2}{C}-$ | 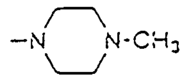 |
| 132 | 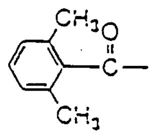 | -S- | $-\underset{H_2}{C}-$ | 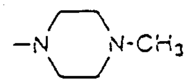 |
| 133 | 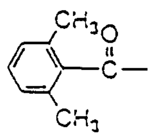 | -S- | $-\underset{H_2}{C}-$ | 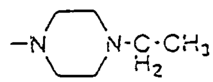 |
| 134 | 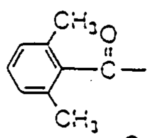 | -S- | $-\underset{H_2}{C}-$ | 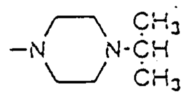 |
| 135 | 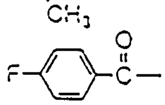 | -S- | $-\underset{H_2}{C}-\underset{H_2}{C}-$ | 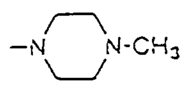 |
| 136 | 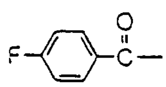 | -S- | $-\underset{H_2}{C}-\underset{H_2}{C}-$ | 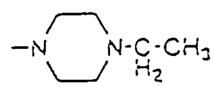 |
| 137 | 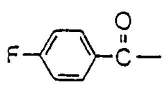 | -S- | $-\underset{H_2}{C}-\underset{H_2}{C}-$ | 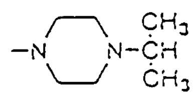 |
| 138 | 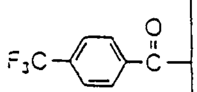 | -S- | $-\underset{H_2}{C}-\underset{H_2}{C}-$ | 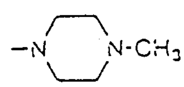 |
| 139 | 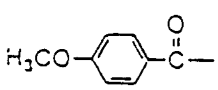 | -S- | $-\underset{H_2}{C}-\underset{H_2}{C}-$ | 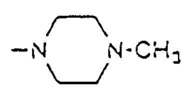 |
| 140 | 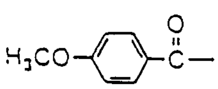 | -S- | $-\underset{H_2}{C}-\underset{H_2}{C}-$ | 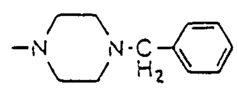 |

TABLE A-16 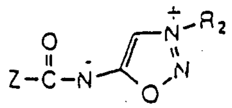 $Z=R_1-X-A$

| Ex.No. | $R_1$ | X | A | $R_2$ |
|---|---|---|---|---|
| 141 | 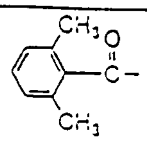 | -S- | $-\underset{H_2}{C}-\underset{H_2}{C}-$ | 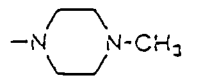 |
| 142 | 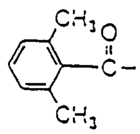 | -S- | $-\underset{H_2}{C}-\underset{H_2}{C}-$ | 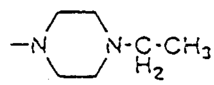 |
| 143 | 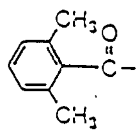 | -S- | $-\underset{H_2}{C}-\underset{H_2}{C}-$ | 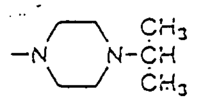 |
| 144 | 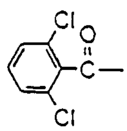 | -S- | $-\underset{H_2}{C}-\underset{H_2}{C}-$ | 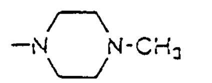 |
| 145 | 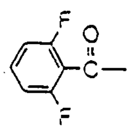 | -S- | $-\underset{H_2}{C}-\underset{H_2}{C}-$ | 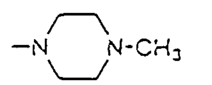 |
| 146 | 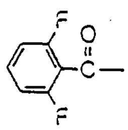 | -S- | $-\underset{H_2}{C}-\underset{H_2}{C}-$ | 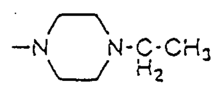 |
| 147 | 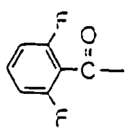 | -S- | $-\underset{H_2}{C}-\underset{H_2}{C}-$ | 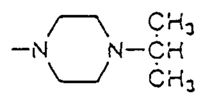 |
| 148 | 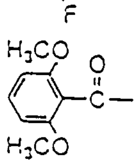 | -S- | $-\underset{H_2}{C}-\underset{H_2}{C}-$ | 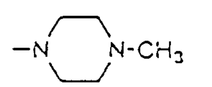 |
| 149 | 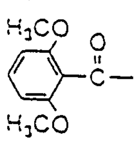 | -S- | $-\underset{H_2}{C}-\underset{H_2}{C}-$ | 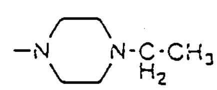 |
| 150 | 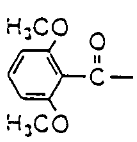 | -S- | $-\underset{H_2}{C}-\underset{H_2}{C}-$ | 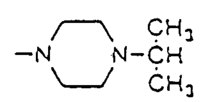 |

TABLE A-17 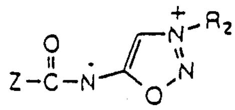 Z=$R_1$-X-A

| Ex.No. | $R_1$ | X | A | $R_2$ |
|---|---|---|---|---|
| 151 | $t\text{-}Bu\text{-}\overset{O}{\overset{\|}{C}}-$ | -S- | $-\underset{H_2}{C}-$ | 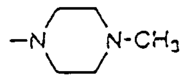 |
| 152 | $t\text{-}Bu\text{-}\overset{O}{\overset{\|}{C}}-$ | -S- | $-\underset{H_2}{C}-$ | 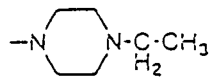 |
| 153 | $t\text{-}Bu\text{-}\overset{O}{\overset{\|}{C}}-$ | -S- | $-\underset{H_2}{C}-$ | 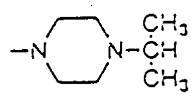 |
| 154 | $t\text{-}Bu\text{-}\overset{O}{\overset{\|}{C}}-$ | -S- | $-\underset{H_2}{C}-\underset{H_2}{C}-$ | 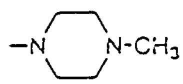 |
| 155 | $t\text{-}Bu\text{-}\overset{O}{\overset{\|}{C}}-$ | -S- | $-\underset{H_2}{C}-\underset{H_2}{C}-$ | 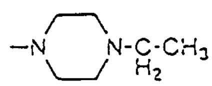 |
| 156 | $t\text{-}Bu\text{-}\overset{O}{\overset{\|}{C}}-$ | -S- | $-\underset{H_2}{C}-\underset{H_2}{C}-$ | 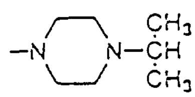 |
| 157 | $t\text{-}Bu\text{-}\overset{O}{\overset{\|}{C}}-$ | -S- | $-\underset{H_2}{C}-\overset{CH_3}{\underset{CH_3}{C}}-$ | 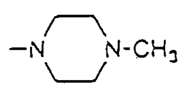 |
| 158 | $t\text{-}Bu\text{-}\overset{O}{\overset{\|}{C}}-$ | -S- | $-\underset{H_2}{C}-\overset{CH_3}{\underset{CH_3}{C}}-$ | 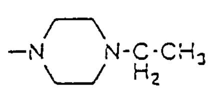 |
| 159 | $t\text{-}Bu\text{-}\overset{O}{\overset{\|}{C}}-$ | -S- | $-\underset{H_2}{C}-\overset{CH_3}{\underset{CH_3}{C}}-$ | 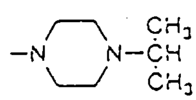 |
| 160 | 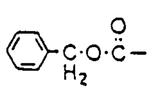 | -S- | $-\underset{H_2}{C}-\underset{H_2}{C}-$ | 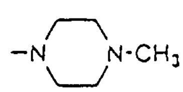 |

TABLE A-18 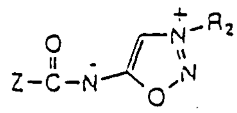 Z=$R_1$-X-A

| Ex.No. | $R_1$ | X | A | $R_2$ |
|---|---|---|---|---|
| 161 | $H_3C\text{-}\underset{H_2}{C}\text{-}\underset{H}{N}\text{-}\overset{O}{\overset{\|}{C}}-$ | -S- | $-\underset{H_2}{C}-$ | 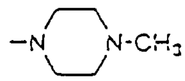 |
| 162 | $H_3C\text{-}\underset{H_2}{C}\text{-}\underset{H}{N}\text{-}\overset{O}{\overset{\|}{C}}-$ | -S- | $-\underset{H_2}{C}-\underset{H_2}{C}-$ | 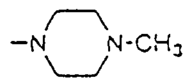 |
| 163 | $H_3C\text{-}\underset{H_2}{C}\text{-}\underset{H}{N}\text{-}\overset{O}{\overset{\|}{C}}-$ | -S- | $-\underset{H_2}{C}-\underset{H_2}{C}-$ | 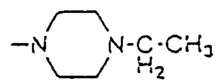 |
| 164 | $H_3C\text{-}\underset{H_2}{C}\text{-}\underset{H}{N}\text{-}\overset{O}{\overset{\|}{C}}-$ | -S- | $-\underset{H_2}{C}-\underset{H_2}{C}-$ | 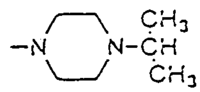 |
| 165 | $H_3C\text{-}\underset{H_2}{C}\text{-}\underset{H}{N}\text{-}\overset{O}{\overset{\|}{C}}-$ | -S- | $-\underset{H_2}{C}-\underset{CH_3}{\overset{CH_3}{C}}-$ | 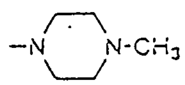 |
| 166 | $H_3C\text{-}\underset{H_2}{C}\text{-}\underset{H}{N}\text{-}\overset{O}{\overset{\|}{C}}-$ | -S- | $-\underset{H_2}{C}-\underset{CH_3}{\overset{CH_3}{C}}-$ | 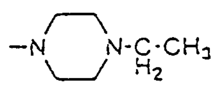 |
| 167 | $H_3C\text{-}\underset{H_2}{C}\text{-}\underset{H}{N}\text{-}\overset{O}{\overset{\|}{C}}-$ | -S- | $-\underset{H_2}{C}-\underset{CH_3}{\overset{CH_3}{C}}-$ | 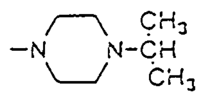 |
| 168 | $t\text{-}Bu-\underset{H}{N}\text{-}\overset{O}{\overset{\|}{C}}-$ | -S- | $-\underset{H_2}{C}-\underset{H_2}{C}-$ | 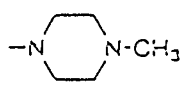 |
| 169 | $t\text{-}Bu-\underset{H}{N}\text{-}\overset{O}{\overset{\|}{C}}-$ | --S- | $-\underset{H_2}{C}-\underset{H_2}{C}-$ | 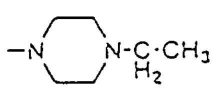 |
| 170 | $t\text{-}Bu-\underset{H}{N}\text{-}\overset{O}{\overset{\|}{C}}-$ | -S- | $-\underset{H_2}{C}-\underset{H_2}{C}-$ | 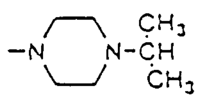 |

TABLE A-19 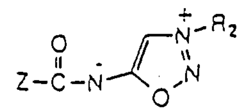 $Z=R_1\text{-}X\text{-}A$

| Ex.No. | $R_1$ | X | A | $R_2$ |
|---|---|---|---|---|
| 171 | 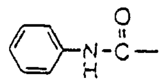 | -S- | $-\underset{H_2}{C}-\underset{H_2}{C}-$ | 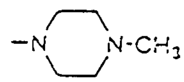 |
| 172 | 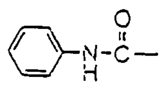 | -S- | $-\underset{H_2}{C}-\underset{H_2}{C}-$ | 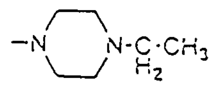 |
| 173 | 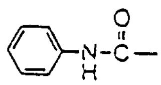 | -S- | $-\underset{H_2}{C}-\underset{H_2}{C}-$ | 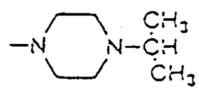 |
| 174 | 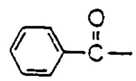 | -S- | $-\underset{H_2}{C}-\underset{H_2}{C}-\underset{H_2}{C}-$ | 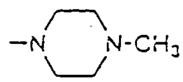 |
| 175 | 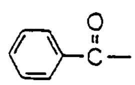 | -S- | $-\underset{H_2}{C}-\underset{H_2}{C}-\underset{H_2}{C}-$ | 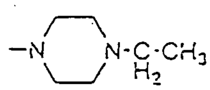 |
| 176 | 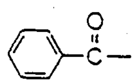 | -S- | $-\underset{H_2}{C}-\underset{H_2}{C}-\underset{H_2}{C}-$ | 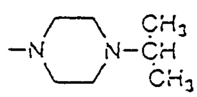 |
| 177 | 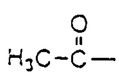 | -S- | 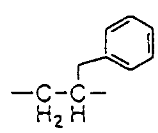 | 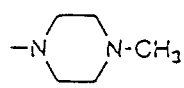 |
| 178 | $H_3C-$ | -S- | $-\underset{H_2}{C}-\underset{H_2}{C}-$ | 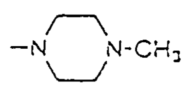 |
| 179 | 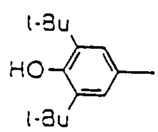 | -S- | $-\underset{H_2}{C}-\underset{H_2}{C}-$ | 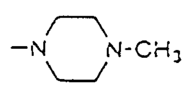 |
| 180 | 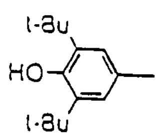 | -S- | $-\underset{H_2}{C}-\underset{H_2}{C}-\underset{H_2}{C}-$ | 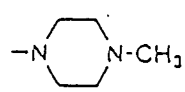 |

TABLE A-20

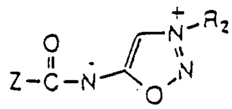

Z=R1-X-A

| Ex.No. | $R_1$ | X | A | $R_2$ |
|---|---|---|---|---|
| 181 | 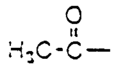 | -S- | 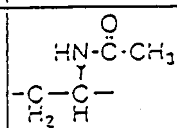 | 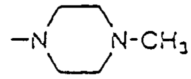 |
| 182 | 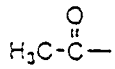 | -S- | 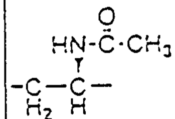 | 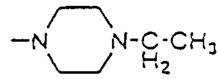 |
| 183 | 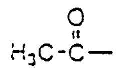 | -S- | 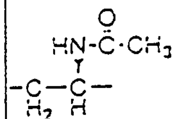 | 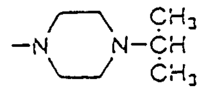 |
| 184 | 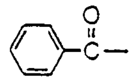 | -S- | 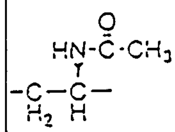 | 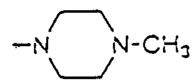 |
| 185 | 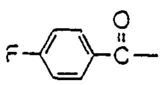 | -S- | 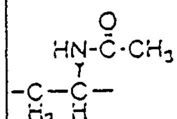 | 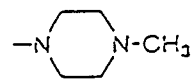 |
| 186 | 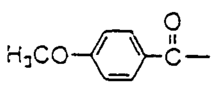 | -S- | 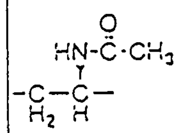 | 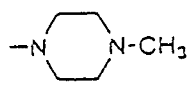 |
| 187 | 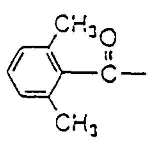 | -S- | 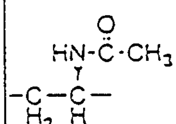 | 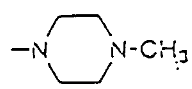 |
| 188 | 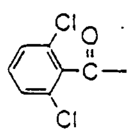 | -S- | 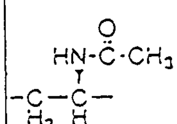 | 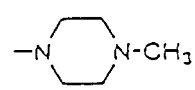 |
| 189 | 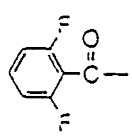 | -S- | 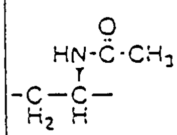 | 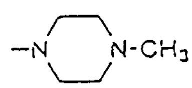 |
| 190 | 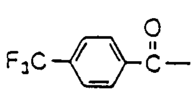 | -S- | 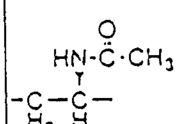 | 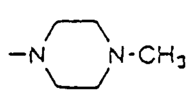 |

TABLE A-21 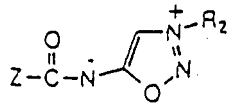 Z=$R_1$-X-A

| Ex.No. | $R_1$ | X | A | $R_2$ |
|---|---|---|---|---|
| 191 | 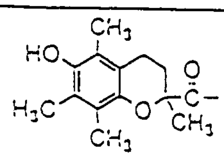 | -S- | $-\underset{H_2}{C}-$ | 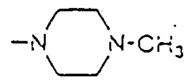 |
| 192 | 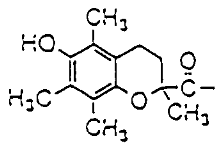 | -S- | $-\underset{H_2}{C}-$ | 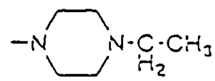 |
| 193 | 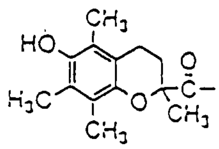 | -S- | $-\underset{H_2}{C}-$ | 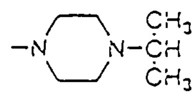 |

TABLE A-22

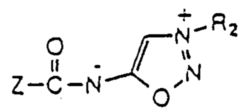

| Ex.No. | Z | $R_2$ |
|---|---|---|
| 194 | 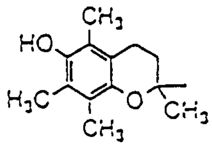 | 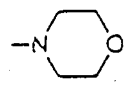 |
| 195 | 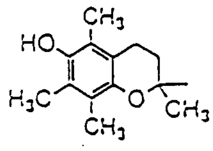 | 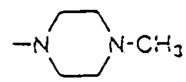 |
| 196 | 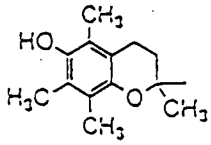 | 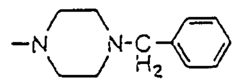 |
| 197 | 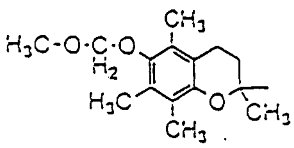 | 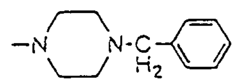 |
| 198 | 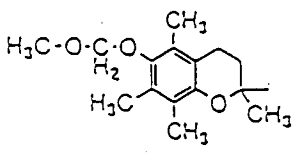 | 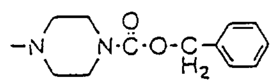 |
| 199 | 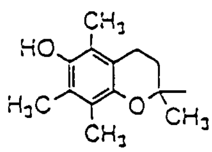 | 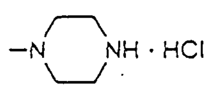 |
| 200 | 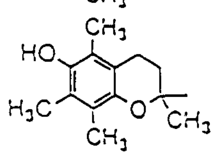 | 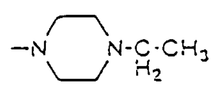 |
| 201 | 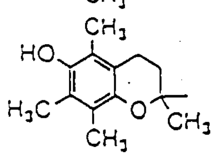 | 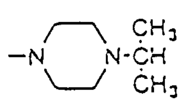 |
| 202 | 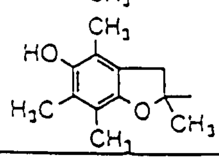 | 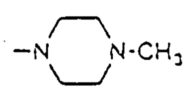 |

TABLE A-23

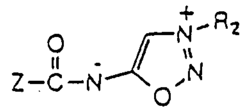

| Ex.No. | Z | $R_2$ |
|---|---|---|
| 203 | 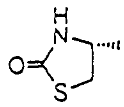 | 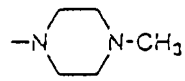 |
| 204 | 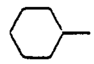 | 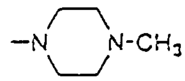 |
| 205 | 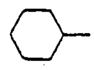 | 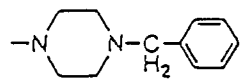 |
| 206 | 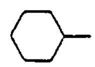 | 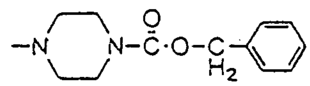 |
| 207 | 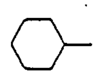 | 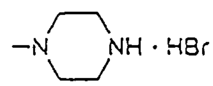 |
| 208 | 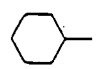 | 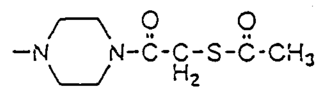 |
| 209 | 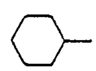 | 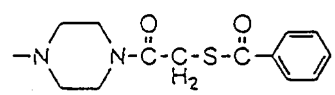 |
| 210 | 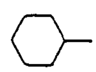 | 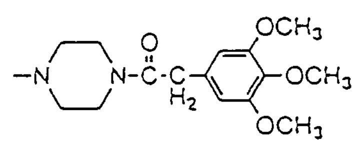 |
| 211 | 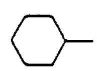 | 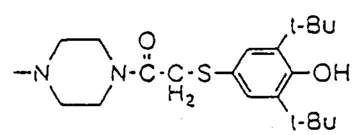 |
| 212 | 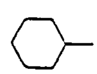 | 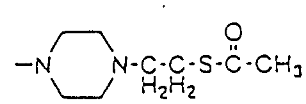 |
| 213 | 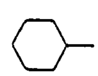 | 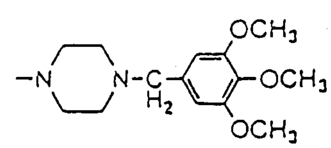 |

TABLE A-24

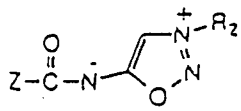

| Ex.No. | Z | $R_2$ |
|---|---|---|
| 214 | 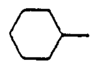 | 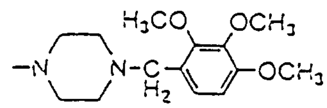 |
| 215 | 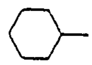 | 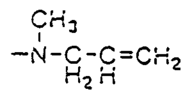 |
| 216 | 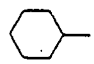 | 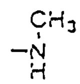 |
| 217 | 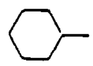 | 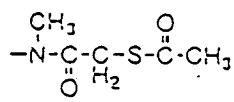 |

[Example 1]

N-((3,5-Di-t-butyl-4-hydroxyphenylthio)acetyl)-3-(4-methylpiperazin-1-yl)sydnonimine

A mixture of 0.74 g of (3,5-di-t-butyl-4-hydroxyphenylthio)acetic acid, 0.57 g of 1-ethyl-3-(3-dimethylaminopropyl)carbodiimide hydrochloride, 0.45 g of 1-hydroxybenzotriazole hydrate and 20 ml of N,N-dimethylformamide was stirred under ice cooling for 1 hour. Next, 0.71 g of 3-(4-methylpiperazin-1-yl)sydnonimine hydrochloride and 1.3 ml of pyridine were added thereto and the resultant mixture was stirred for 1 hr and then at room temperature for additional 20 hours. After adding water, the reaction mixture was extracted with ether. The organic layer was washed with water and dried and the solvent was distilled off. The residue thus obtained was purified by silica gel column chromatography (developing solution: chloroform : methanol : aqueous ammonia = 190 : 9 : 1) to give 0.80 g of N-((3,5-di-t-butyl-4-hydroxyphenylthio)acetyl)-3-(4-methylpiperazin-1-yl)sydnonimine (m.p.: 160 - 162°C).

270 MHz-NMR ($CDCl_3$) $\delta$ : 1.40 (18H, s), 2.38 (3H, s), 2.70-2.73 (4H, m), 3.53-3.57 (4H, m), 3.81 (2H, s), 5.15 (1H, s), 7.32 (2H, s), 8.00 (1H, s)
MS m/z : 461 ($M^+$)

[Example 2]

N-((S)-3-(Benzoylthio)isobutyryl)-3-(4-methylpiperazin-1-yl)sydnonimine

The procedure of Example 1 was repeated but starting with (S)-3-(benzoylthio)isobutyric acid to give N-((S)-3-(benzoylthio)isobutyryl)-3-(4-methylpiperazin-1-yl)sydnonimine (m.p.: 139 - 141°C).

270 MHz-NMR ($CDCl_3$) $\delta$ : 1.34 (3H, d), 2.38 (3H, s), 2.69-2.73 (4H, m), 2.88-2.95 (1H, m), 3.30-3.46(2H, m), 3.53-3.56 (4H, m), 7.38-7.44 (2H, m), 7.50-7.56 (1H, m), 7.94-7.98 (2H, m), 8.01 (1H, s)

[Example 3]

N-((Benzoylthio)acetyl)-3-(4-methylpiperazin-1-yl)sydnonimine

The procedure of Example 1 was repeated but starting with (benzoylthio)acetic acid to give N-((benzoylthio)acetyl)-3-(4-methylpiperazin-1-yl)sydnonimine (m.p.: 149 - 151°C).

60 MHz-NMR ($CDCl_3$) $\delta$ : 2.31 (3H, s), 2.56-2.77 (4H, m), 3.37-3.63 (4H, m), 4.01 (2H, s), 7.17-7.46 (3H, m), 7.73-7.95 (2H, m), 7.88 (1H, s)

[Example 4]

N-((S)-3-(Benzoylthio)isobutyryl)-3-morpholinosydnonimine

A mixture of 0.32 g of (S)-3-(benzoylthio)isobutyric acid, 0.31 g of 1-ethyl-3-(3-dimethylaminopropyl)carbodiimide hydrochloride, 0.24 g of 1-hydroxybenzotriazole hydrate and 10 ml of N,N-dimethylformamide was stirred under ice cooling for 50 minutes. Next, 0.30 g of 3-morpholinosydnonimine hydrochloride and 0.6 ml of pyridine were added thereto and the resultant mixture was stirred for 50 minutes and then at room temperature for additional 8 hours. After adding water, the reaction mixture was extracted with ether. The organic layer was washed with water and dried and the solvent was distilled off. The residue thus obtained was purified by silica gel column chromatography (developing solution: dichloromethane : methanol = 99 : 1) to give 0.42 g of N-((S)-3-(benzoylthio)isobutyryl)-3-morpholinosydnonimine as an oily substance.

60 MHz-NMR ($CDCl_3$) $\delta$ : 1.30 (3H, d), 2.50-3.18 (1H, m), 3.18-3.65 (6H, m), 3.72-4.07 (4H, m), 7.10-7.50 (3H, m), 7.70-7.90 (2H, m), 7.95 (1H, s)
MS m/z : 377 ($M^++1$)

[Example 5]

N-((S)-3-(Benzoylthio)isobutyryl)-3-dimethylaminosydnonimine

A mixture of 0.31 g of (S)-3-(benzoylthio)isobutyric acid, 0.30 g of 1-ethyl-3-(3-dimethylaminopropyl)carbodiimide hydrochloride, 0.25 g of 1-hydroxybenzotriazole hydrate and 10 ml of N,N-dimethylformamide was stirred under ice cooling for 50 minutes. Next, 0.24 g of 3-dimethylaminosydnonimine hydrochloride and 0.6 ml of pyridine were added thereto and the resultant mixture was stirred for 1 hour and then at room temperature for additional 14 hours. After adding water, the reaction mixture was extracted with ether. The organic layer was washed with water and dried and the solvent was distilled off. The residue thus obtained was purified by silica gel column chromatography (developing solution: chloroform : methanol : aqueous ammonia = 190 : 9 : 1) to give 0.45 g of N-((S)-3-(benzoylthio)isobutyryl)-3-dimethylaminosydnonimine as an oily substance.

60 MHz-NMR ($CDCl_3$) δ : 1.32 (3H, d), 2.60-3.05 (1H, m), 3.05-3.44 (2H, m), 3.17 (6H, s), 7.20-7.47 (3H, m), 7.70-7.84 (2H, m), 7.88 (1H, s)
MS m/z : 335 ($M^+$+1)

[Example 6]

N-((S)-3-(Acetylthio)isobutyryl)-3-dimethylaminosydnonimine

The procedure of Example 5 was repeated but starting with (S)-3-(acetylthio)isobutyric acid to give N-((S)-3-(acetylthio)isobutyryl)-3-dimethylaminosydnonimine (m.p.: 72 - 73°C).

60 MHz-NMR ($CDCl_3$) δ : 1.25 (3H, d), 2.26 (3H, s), 2.50-2.93 (1H, m), 3.02-3.19 (2H, m), 3.20 (6H, s), 7.88 (1H, s)
MS m/Z : 273 ($M^+$+1)

[Example 7]

N-((3,5-Di-t-butyl-4-hydroxyphenylthio)acetyl)-3-dimethylaminosydnonimine

The procedure of Example 5 was repeated but starting with (3,5-di-t-butyl-4-hydroxyphenylthio)acetic acid to give N-((3,5-di-t-butyl-4-hydroxyphenylthio)acetyl)-3-dimethylaminosydnonimine (m.p.: 173 - 174°C).

270 MHz-NMR ($CDCl_3$) δ : 1.40 (18H, s), 3.24 (6H, s), 3.81 (2H, s), 5.15 (1H, s), 7.32 (2H, s), 8.00 (1H, s)
MS m/Z : 406 ($M^+$)

[Example 8]

N-((Acetylthio)acetyl)-3-dimethylaminosydnonimine

The procedure of Example 5 was repeated but starting with (acetylthio)acetic acid to give N-((acetylthio)acetyl-3-dimethylaminosydnonimine (m.p.: 99 - 101°C).

60 MHz-NMR ($CDCl_3$) δ : 2.34 (3H, s), 3.23 (6H, s), 3.80 (2H, s), 7.91 (1H, s)

[Example 9]

N-((Benzoylthio)acetyl)-3-dimethylaminosydnonimine

The procedure of Example 5 was repeated but starting with (benzoylthio)acetic acid to give N-((benzoylthio)acetyl-3-dimethylaminosydnonimine (m.p.: 128 - 129°C).

60 MHz-NMR ($CDCl_3$) δ : 3.20 (6H, s), 4.05 (2H, s), 7.26-7.52 (3H, m), 7.81-8.01 (2H, m), 7.89 (1H, s)

[Example 10]

N-((3,4,5-Trimethoxyphenyl)acetyl)-3-dimethylaminosydnonimine

The procedure of Example 5 was repeated but starting with (3,4,5-trimethoxyphenyl)acetic acid to give N-((3,4,5-trimethoxyphenyl)acetyl-3-dimethylaminosydnonimine (m.p.: 88 - 89°C).

60 MHz-NMR ($CDCl_3$) δ : 3.24 (6H, s), 3.65 (2H, s), 3.74 (3H, s), 3.79 (6H, s), 6.52 (2H, s), 7.90 (1H, s)

[Example 11]

N-((S)-3-(Benzoylthio)isobutyryl)-3-(4-(2-methoxyphenyl)piperazin-1-yl)sydnonimine

A mixture of 0.14 g of (S)-3-(benzoylthio)isobutyric acid, 0.14 g of 1-ethyl-3-(3-dimethylaminopropyl)carbodiimide hydrochloride, 0.11 g of 1-hydroxybenzotriazole hydrate and 6 ml of N,N-dimethylformamide was stirred under ice cooling for 1 hour. Next, 0.23 g of 3-(4-(2-methoxyphenyl)piperazin-1-yl)sydnonimine hydrochloride and 0.35 ml of pyridine were added thereto and the resultant mixture was stirred for 1 hour and then at room temperature for additional 3 days. After adding water, the reaction mixture was extracted with ether. The organic layer was washed with water and dried and the solvent was distilled off. The residue thus obtained was purified by silica gel column chromatography (developing solution: chloroform : methanol : aqueous ammonia = 190 : 9 : 1) to give 0.10 g of N-((S)-3-(benzoylthio)isobutyryl)-3-(4-(2-(methoxyphenyl)piperazin-1-yl)sydnonimine (m.p.: 104 - 106°C).

60 MHz-NMR ($CDCl_3$) δ : 1.33 (3H, d), 2.61-3.10 (1H, m), 3.10-3.50 (6H, m), 3.50-3.80 (4H, m), 3.83 (3H, s), 6.70-7.02 (4H, m), 7.20-7.50 (3H, m), 7.70-7.90 (2H, m), 7.94 (1H, s)

[Example 12]

N-((S)-3-(Acetylthio)isobutyryl)-3-(4-(2-methoxyphenyl)piperazin-1-yl)sydnonimine

The procedure of Example 11 was repeated but starting with (S)-3-(acetylthio)isobutyric acid to give N-((S)-3-(acetylthio)isobutyryl)-3-(4-(2-methoxyphenyl)piperazin-1-yl)sydnonimine (m.p.: 91 - 93°C).

60 MHz-NMR ($CDCl_3$) δ : 1.25 (3H, d), 2.25 (3H, s), 2.50-3.03 (1H, m), 3.03-3.49 (6H, m), 3.49-3.80 (4H, m), 3.83 (3H, s), 6.65-7.03 (4H, m), 7.92 (1H, s)

[Example 13]

N-((3,5-Di-t-butyl-4-hydroxyphenylthio)acetyl)-3-(2,6-dimethylpiperidin-1-yl)sydnonimine

A mixture of 0.26 g of (3,5-di-t-butyl-4-hydroxyphenylthio)acetic acid, 0.18 g of 1-ethyl-3-(3-dimethylaminopropyl)carbodiimide hydrochloride, 0.15 g of 1-hydroxybenzotriazole hydrate and 8 ml of N,N-dimethylformamide was stirred under ice cooling for 10 minutes. Next, 0.2 g of 3-(2,6-dimethylpiperidin-1-yl)sydnonimine hydrochloride and 0.28 ml of pyridine were added thereto and the resultant mixture was stirred for 5 minutes and then at room temperature for additional 13 hours. After adding water, the reaction mixture was extracted with ether. The organic layer was washed with water and dried and the solvent was distilled off. The residue thus obtained was purified by silica gel column chromatography (developing solution: hexane : ethyl acetate = 1 : 1) to give 0.36 g of N-((3,5-di-t-butyl-4-hydroxyphenylthio)acetyl)-3-(2,6-dimethylpiperidin-1-yl)sydnonimine (m.p.: 175 - 176°C).

270 MHz-NMR ($CDCl_3$) δ : 0.96 (6H, d), 1.41 (18H, s), 1.45-1.61 (3H, m), 1.79-2.05 (3H, m), 3.27-3.40 (2H, m), 3.81 (2H, s), 5.19 (1H, s), 7.38 (2H, s), 8.00 (1H, s)

[Example 14]

N-((S)-3-(Benzoylthio)isobutyryl)-3-(2,6-dimethylpiperidin-1-yl)sydnonimine

The procedure of Example 13 was repeated but starting with (S)-3-(benzoylthio)isobutyric acid to give N-((S)-3-(benzoylthio)isobutyryl)-3-(2,6-dimethylpiperidin-1-yl)sydnonimine as an oily substance.

270 MHz-NMR ($CDCl_3$) δ : 0.95 (6H, d), 1.36 (3H, d), 1.46-1.59 (3H, m), 1.78-1.94 (3H, m), 2.97-3.00 (1H, m), 3.22-3.50 (4H, m), 7.38-7.58 (3H, m), 7.94-8.02 (2H, m), 8.00 (1H, s)

[Example 15]

N-((3,4,5-Trimethoxyphenyl)acetyl)-3-(2,6-dimethylpiperidin-1-yl)sydnonimine

The procedure of Example 13 was repeated but starting with 3,4,5-trimethoxyphenylacetic acid to give N-((3,4,5-trimethoxyphenyl)acetyl)-3-(2,6-dimethylpiperidin-1-yl)sydnonimine as an oily substance.

270 MHz-NMR ($CDCl_3$) δ : 0.93 (6H, d), 1.40-1.59 (3H, m), 1.75-1.94 (3H, m), 3.21-3.38 (2H, m), 3.74 (2H, s), 3.82 (3H, s), 3.86 (6H s), 6.60 (2H, s), 7.96 (1H, s)

[Example 16]

N-((Benzoylthio)acetyl)-3-(2,6-dimethylpiperidin-1-yl)sydnonimine

The procedure of Example 13 was repeated but starting with (benzoylthio)acetic acid to give N-((benzoylthio)acetyl)-3-(2,6-dimethylpiperidin-1-yl)sydnonimine as an oily substance.

270 MHz-NMR ($CDCl_3$) δ : 0.92 (6H, d), 1.40-1.60 (3H, m), 1.75-1.92 (3H, m), 3.25-3.37 (2H, m), 4.12 (2H, s), 7.41-7.59 (3H, m), 8.00-8.03 (2H, m), 8.01 (1H, s)

[Example 17]

3-(4-Benzylpiperazin-1-yl)-N-((3,4,5-trimethoxyphenyl)acetyl)sydnonimine

A mixture of 0.32 g of (3,4,5-trimethoxyphenyl)acetic acid, 0.30 g of 1-ethyl-3-(3-dimethylaminopropyl)-carbodiimide hydrochloride, 0.25 g of 1-hydroxybenzotriazole hydrate and 10 ml of N,N-dimethylformamide was stirred under ice cooling for 1.5 hours. Next, 0.47 g of 3-(4-benzylpiperazin-1-yl)sydnonimine hydrochloride and 0.8 ml of pyridine were added thereto and the resultant mixture was stirred for 1 hour and then at room temperature for additional 5 hours. After adding water, the reaction mixture was extracted with ether. The organic layer was washed with water and dried and the solvent was distilled off. The residue thus obtained was purified by silica gel column chromatography (developing solution: chloroform : methanol : aqueous ammonia = 190 : 9 : 1) to give 0.29 g of 3-(4-benzylpiperazin-1-yl)-N-((3,4,5-trimethoxyphenyl)acetyl)sydnonimine as an oily substance.

60 MHz-NMR ($CDCl_3$) δ : 2.50-2.91 (4H, m), 3.28-3.56 (4H, m), 3.50 (2H, s), 3.62 (2H, s), 3.71 (3H, s), 3.75 (6H, s), 6.45 (2H, s), 7.15 (5H, brs), 7.81 (1H, s)

[Example 18]

N-((S)-3-(Benzoylthio)isobutyryl)-3-(4-benzylpiperazin-1-yl)sydnonimine

The procedure of Example 17 was repeated but starting with (S)-3-(benzoylthio)isobutyric acid to give N-((S)-3-(benzoylthio)isobutyryl)-3-(4-benzylpiperazin-1-yl)sydnonimine (m.p.: 126 - 127°C).

60 MHz-NMR ($CDCl_3$) δ : 1.32 (3H, d), 2.56-2.84 (4H, m), 2.84-2.86 (1H, m), 3.35-3.54 (6H, m) 3.54 (2H, s), 7.18 (5H, s), 7.12-7.45 (3H, m), 7.71-7.96 (2H, m), 7.86 (1H, s)

[Example 19-A]

N-((S)-3-(Benzoylthio)isobutyryl)-3-(4-benzyloxycarbonylpiperazin-1-yl)sydnonimine

2.3 ml of benzyloxycarbonyl chloride was dropped at room temperature into a mixture of 0.57 g of N-((S)-3-(benzoylthio)isobutyryl)-3-(4-benzylpiperazin-1-yl)sydnonimine, 1.4 g of sodium hydrogencarbonate and 5 ml of benzene and the resultant mixture was stirred for 2 days. After adding water, the reaction mixture was extracted with ethyl acetate. The organic layer was washed with water and dried and the solvent was distilled off. The residue thus obtained was purified by silica gel column chromatography (developing solution: hexane : ethyl acetate = 1 : 2) to give 0.49 g of

N-((S)-3-(benzoylthio)isobutyryl)-3-(4-benzyloxycarbonylpiperazin-1-yl)sydnonimine.

60 MHz-NMR ($CDCl_3$) δ : 1.32 (3H, d), 2.54-3.01 (1H, m), 3.22-3.55 (6H, m), 3.56-3.87 (4H, m), 5.07 (2H, s), 7.16-7.45 (8H, m), 7.71-7.92 (2H, m), 7.93 (1H, s)

[Example 19-B]

N-((S)-3-(Benzoylthio)isobutyryl)-3-(piperazin-1-yl)sydnonimine

Under ice cooling, 7 ml of a 33% solution of hydrogen bromide-acetic acid was added to a mixture of 0.49 g of N-((S)-3-(benzoylthio)isobutyryl)-3-(4-benzyloxycarbonylpiperazin-1-yl)sydnonimine and 15 ml of dichloromethane and the resultant mixture was stirred at room temperature for 3 hours. Under ice cooling, the mixture was poured into ether and N-((S)-3-(benzoylthio)isobutyryl)-3-(piperazin-1-yl)sydnonimine hydrobromide thus precipitated was taken up by filtration. Then it was alkalified with a saturated aqueous solution of sodium bicarbonate under ice cooling and extracted with dichloromethane. The organic layer was washed with water and dried and the solvent was distilled off to give 0.32 g of N-((S)-3-(benzoylthio)isobutyryl)-3-(piperazin-1-yl)sydnonimine.

60 MHz-NMR ($CDCl_3$) δ : 1.30 (3H, d), 2.62-3.16 (5H, m), 3.17-3.51 (6H, m), 7.15-7.41 (3H, m), 7.65-7.83 (2H, m), 7.83 (1H, s)

[Example 19-C]

N-((S)-3-(Benzoylthio)isobutyryl)-3-(4-(3,4,5-trimethoxyphenyl)acetylpiperazin-1-yl)sydnonimine

Under ice cooling, 0.06 g of 1-ethyl-3-(3-dimethylaminopropyl)carbodiimide hydrochloride and 0.05 g of 1-hydroxybenzotriazole hydrate were added to a mixture of 0.07 g of 3,4,5-trimethoxyphenylacetic acid, 0.13 g of N-((S)-3-(benzoylthio)isobutyryl)-3-(piperazin-1-yl)sydnonimine and 2 ml of N,N-dimethylformamide and the resultant mixture was stirred for 2 hours. After adding water, the reaction mixture was extracted with ethyl acetate. The organic layer was washed with water and dried and the solvent was distilled off. The residue thus obtained was purified by silica gel column chromatography (developing solution: dichloromethane : methanol = 95 : 5) to give 0.19 g of N-((S)-3-(benzoylthio)isobutyryl)-3-(4-(3,4,5-trimethoxyphenyl)acetylpiperazin-1-yl)sydnonimine (m.p.: 139 - 140°C).

60 MHz-NMR ($CDCl_3$) δ : 1.31 (3H, d), 2.70-3.04 (1H, m), 3.22-3.51 (6H, m), 3.54-3.77 (6H, m), 3.77 (9H, s), 6.30 (2H, s), 7.12-7.43 (3H, m), 7.70-7.85 (2H, m), 7.85 (1H, s)

[Example 20]

3-(4-((S)-3-(acetylthio)isobutyryl)piperazin-1-yl)-N-((S)-3-(benzoylthio)isobutyryl)sydnonimine

The procedure of Example 19-C was repeated but starting with (S)-3-(acetylthio)isobutyric acid to give 3-(4-((S)-3-(acetylthio)isobutyryl)piperazin-1-yl)-N-((S)-3-(benzoylthio)isobutyryl)sydnonimine as an oily substance.

60 MHz-NMR ($CDCl_3$) δ : 1.14-1.43 (6H, m), 2.31 (3H, s), 2.77-3.07 (2H, m), 3.26-3.67 (8H, m), 3.70-4.07 (4H, m), 7.19-7.47 (3H, m), 7.76-7.96 (2H, m), 7.97 (1H, s)

[Example 21]

N-((S)-3-(Benzoylthio)isobutyryl)-3-(4-((3,5-di-t-butyl-4-hydroxyphenylthio)acetyl)piperazin-1-yl)sydnonimine

The procedure of Example 19-C was repeated but starting with (3,5-di-t-butyl-4-hydroxyphenylthio)acetic acid to give N-((S)-3-(benzoylthio)isobutyryl)-3-(4-((3,5-di-t-butyl-4-hydroxyphenylthio)acetyl)piperazin-1-yl)sydnonimine as an oily substance.

270 MHz-NMR ($CDCl_3$) δ : 1.34 (3H, d), 1.42 (18H, s), 2.85-2.98 (1H, m), 3.29-3.48 (6H, m), 3.67 (2H, s), 3.68-3.78 (2H, m), 3.84-3.93 (2H, m), 5.35 (1H, s), 7.30 (2H, s), 7.38-7.59 (3H, m), 7.94-8.00 (2H, m), 8.02 (1H, s)
MS m/z : 654 ($M^+$+1)

[Example 22]

N-((S)-3-(Benzoylthio)isobutyryl)-3-(4-(2,2-dimethyl-6-nitro-2H-1-benzopyran-4-carbonyl)piperazin-1-yl)sydnonimine

The procedure of Example 19-C was repeated but starting with 2,2-dimethyl-6-nitro-2H-1-benzopyran-4-carboxylic acid to give N-((S)-3-(benzoylthio)isobutyryl)-3-(4-(2,2-dimethyl-6-nitro-2H-1-benzopyran-4-carbonyl)piperazin-1-yl)sydnonimine as an oily substance.

270 MHz-NMR ($CDCl_3$) $\delta$ : 1.33 (3H, d), 1.54 (6H, s), 2.83-2.96 (1H, m), 3.31 (1H, dd), 3.41 (1H, dd), 3.45-4.16 (8H, m), 5.89 (1H, s), 6.94 (1H, d), 7.38-7.54 (3H, m), 7.93-7.97 (3H, m), 8.06 (1H, s), 8.07-8.13 (1H, m)

[Example 23]

N-((S)-3-(Benzoylthio)isobutyryl)-3-(4-((S)-3-(benzoylthio)isobutyryl)piperazin-1-yl)sydnonimine

The procedure of Example 19-C was repeated but starting with (S)-3-(benzoylthio)isobutyric acid to give N-((S)-3-(benzoylthio)isobutyryl)-3-(4-((S)-3-(benzoylthio)isobutyryl)piperazin-1-yl)sydnonimine as an oily substance.

270 MHz-NMR ($CDCl_3$) $\delta$ : 1.29 (3H, d), 1.34 (3H, d), 2.85-2.97 (1H, m), 3.05-3.16 (2H, m), 3.23-3.60 (7H, m), 3.72-4.20 (4H, m), 7.36-7.63 (6H, m), 7.94-8.05 (4H, m), 8.03 (1H, s)

[Example 24-A]

N-((S)-3-(Acetylthio)isobutyryl)-3-(4-benzylpiperazin-1-yl)sydnonimine

The procedure of Example 17 was repeated but starting with (S)-3-(acetylthio)isobutyric acid to give N-((S)-3-(acetylthio)isobutyryl)-3-(4-benzylpiperazin-1-yl)sydnonimine (m.p.: 45 - 46°C).

60 MHz-NMR ($CDCl_3$) $\delta$ : 1.23 (3H, d), 2.22 (3H, s), 2.51-2.91 (5H, m), 3.01-3.21 (2H, m), 3.36-3.60 (4H, m), 3.60 (2H, s), 7.15 (5H, s), 7.81 (1H, s)

[Example 24-B]

N-((S)-3-(Acetylthio)isobutyryl)-3-(4-benzyloxycarbonylpiperazin-1-yl)sydnonimine

The procedure of Example 19-A was repeated but starting with N-((S)-3-(acetylthio)isobutyryl)-3-(4-benzylpiperazin-1-yl)sydnonimine to give N-((S)-3-(acetylthio)isobutyryl)-3-(4-benzyloxycarbonylpiperazin-1- yl)sydnonimine as an oily substance.

60 MHz-NMR ($CDCl_3$) $\delta$ : 1.23 (3H, d), 2.23 (3H, s), 2.49-2.92 (1H, m), 2.97-3.21 (2H, m), 3.30-3.60 (4H, m), 3.61-3.92 (4H, m), 5.07 (2H, s), 7.26 (5H, s), 7.97 (1H, s)

[Example 24-C]

N-((S)-3-(Acetylthio)isobutyryl)-3-(piperazin-1-yl)sydnonimine hydrochloride

The procedure of Example 19-B was repeated but starting with N-((S)-3-(acetylthio)isobutyryl)-3-(4-benzyloxycarbonylpiperazin-1-yl)sydnonimine to give N-((S)-3-(acetylthio)isobutyryl)-3-(piperazin-1-yl)sydnonimine as an oily substance.

60 MHz-NMR ($CDCl_3$) $\delta$ : 1.21 (3H, d), 2.24 (3H, s), 2.45-2.95 (1H, m), 2.95-3.23 (6H, m), 3.26-3.56 (4H, m), 7.83 (1H, s)

Next, the N-((S)-3-(acetylthio)isobutyryl)-3-(piperazin-1-yl)sydnonimine obtained above was diluted with methanol and conc. hydrochloric acid was added thereto under ice cooling. Then the resultant mixture was concentrated to give N-((S)-3-(acetylthio)isobutyryl)-3-(piperazin-1-yl)sydnonimine hydrochloride.

[Example 24-D]

N-((S)-3-(Acetylthio)isobutyryl)-3-(4-(3,5-di-t-butyl-4-hydroxyphentylthio)acetyl)piperazin-1-yl)sydnonimine

Under ice cooling, 0.08 g of 1-ethyl-3-(3-dimethylaminopropyl)carbodiimide hydrochloride and 0.07 g of 1-hydroxybenzotriazole hydrate were added to a mixture of 0.12 g of (3,5-di-t-butyl-4-hydroxyphenylthio)acetic acid and 2 ml of N,N-dimethylformamide and the resultant mixture was stirred for 30 minutes. Next, 0.15 g of N-((S)-3-(acetylthio)isobutyryl)-3-(piperazin-1-yl)sydnonimine hydrochloride and 0.1 ml of pyridine were added thereto under ice cooling and the resultant mixture was stirred at room temperature for 14 hours. After adding water, the reaction mixture was extracted with ethyl acetate. The organic layer was washed with water and dried and the solvent was distilled off. The residue thus obtained was purified by silica gel column chromatography (developing solution: dichloromethane : methanol = 95 : 5) to give 0.18 g of N-((S)-3-(acetylthio)isobutyryl)-3-(4-(3,5-di-t-butyl-4-hydroxyphentylthio)acetyl)piperazin-1-yl)sydnonimine as an oily substance.

60 MHz-NMR ($CDCl_3$) δ : 1.19-1.50 (21H, m), 2.28 (3H, s), 2.55-2.90 (1H, m), 3.04-3.22 (2H, m), 3.25-3.91 (10H, m), 5.27 (1H, brs), 7.17 (2H, s), 7.88 (1H, s)

[Example 25]

N-((S)-3-(Acetylthio)isobutyryl)-3-(4-(3,4,5-trimethoxyphenyl)acetyl)piperazin-1-yl)sydnonimine

The procedure of Example 24-D was repeated but starting with 3,4,5-trimethoxyphenylacetic acid to give N-((S)-3-(acetylthio)isobutyryl)-3-(4-(3,4,5-trimethoxyphenyl)acetyl)piperazin-1-yl)sydnonimine (m.p.: 122 - 123°C).

60 MHz-NMR ($CDCl_3$) δ : 1.23 (3H, d), 2.24 (3H, s), 2.55-2.79 (1H, m), 2.98-3.53 (6H, m), 3.55-3.85 (6H, m), 3.73 (9H, s), 6.31 (2H, s), 7.84 (1H, s)

[Example 26]

N-((S)-3-(Acetylthio)isobutyryl)-3-(4-(2,2-dimethyl-6-nitro-2H-1-benzopyran-4-carbonyl)piperazin-1-yl)sydnonimine

The procedure of Example 24-D was repeated but starting with 2,2-dimethyl-6-nitro-2H-benzopyran-4-carboxylic acid to give N-((S)-3-(acetylthio)isobutyryl)-3-(4-(2,2-dimethyl-6-nitro-2H-1-benzopyran-4-carbonyl)piperazin-1-yl)sydnonimine as an oily substance.

60 MHz-NMR ($CDCl_3$) δ : 1.28 (3H, d), 1.57 (6H, s), 2.31 (3H, s), 2.56-2.91 (1H, m), 3.06-3.28 (2H, m), 3.42-4.11 (8H, m), 5.87 (1H, s), 6.88 (1H, d), 7.82-8.15 (3H, m)

[Example 27]

N-((S)-3-(Acetylthio)isobutyryl)-3-(4-((S)-3-(acetylthio)isobutyryl)piperazin-1-yl)sydnonimine

The procedure of Example 24-D was repeated but starting with (S)-3-(acetylthio)isobutyric acid to give N-((S)-3-(acetylthio)isobutyryl)-3-(4-((S)-3-(acetylthio)isobutyryl)piperazin-1-yl)sydnonimine as an oily substance.

60 MHz-NMR ($CDCl_3$) δ : 1.11-1.35 (6H, m), 2.24 (3H, s), 2.30 (3H, s), 2.51-3.45 (6H, m), 3.46-4.05 (8H, m), 7.90 (1H, s)

[Example 28]

N-((S)-3-(Acetylthio)isobutyryl)-3-(4-((R)-2-oxo-4-thiazolidinecarbonyl)piperazin-1-yl)sydnonimine

The procedure of Example 24-D was repeated but starting with (-)-2-oxo-4-thiazolidinecarboxylic acid to give N-((S)-3-(acetylthio)isobutyryl)-3-(4-((R)-2-oxo-4-thiazolidinecarbonyl)piperazin-1-yl)sydnonimine (m.p.: 195 - 196°C).

270 MHz-NMR ($CDCl_3$) δ : 1.25 (3H, d), 2.31 (3H, s), 2.73-2.82 (1H, m), 3.05-3.23 (2H, m), 3.41-4.09 (11H, m), 4.75-4.82 (1H, m), 8.13 (1H, s)

[Example 29]

N-((S)-3-(Acetylthio)isobutyryl)-3-(4-((S)-3-(benzoylthio)isobutyryl)piperazin-1-yl)sydnonimine

The procedure of Example 24-D was repeated but starting with (S)-3-(benzoylthio)isobutyric acid to give N-((S)-3-(acetylthio)isobutyryl)-3-(4-((S)-3-(benzoylthio)isobutyryl)piperazin-1-yl)sydnonimine as an oily substance.

60 MHz-NMR ($CDCl_3$) δ : 1.24-1.40 (6H, m), 2.26 (3H, s), 2.47-2.97 (2H, m), 2.98-3.28 (4H, m) 3.30-4.06 (8H, m), 7.16-7.49 (3H, m), 7.71-7.89 (2H, m), 7.90 (1H, s)

[Example 30-A]

N-((Benzoylthio)acetyl)-3-(4-benzylpiperazin-1-yl)sydnonimine

The procedure of Example 17 was repeated but starting with (benzoylthio)acetic acid to give N-((benzoylthio)acetyl)-3-(4-benzylpiperazin-1-yl)sydnonimine.

60 MHz-NMR ($CDCl_3$) δ : 2.58-2.85 (4H, m), 3.37-3.54 (6H, m), 4.04 (2H, s), 7.18-7.45 (8H, m), 7.77-7.98 (2H, m), 7.85 (1H, s)

[Example 30-B]

N-((Benzoylthio)acetyl)-3-(4-benzyloxycarbonylpiperazin-1-yl)sydnonimine

The procedure of Example 19-A was repeated but starting with N-((benzoylthio)acetyl-3-(4-benzylpiperazin-1-yl)sydnonimine to give N-((benzoylthio)acetyl)-3-(4-benzyloxycarbonylpiperazin-1-yl)sydnonimine.

60 MHz-NMR ($CDCl_3$) δ : 3.30-3.50 (4H, m), 3.60-3.83 (4H, m), 3.95 (2H, s), 5.01 (2H, s), 7.10-7.44 (8H, m), 7.74-7.90 (2H, m), 7.88 (1H, s)

[Example 30-C]

N-((Benzoylthio)acetyl)-3-(piperazin-1-yl)sydnonimine hydrochloride

The procedure of Example 24-C was repeated but starting with N-((benzoylthio)acetyl-3-(4-benzyloxycarbonylpiperazin-1-yl)sydnonimine to give N-((benzoylthio)acetyl)-3-(piperazin-1-yl)sydnonimine hydrochloride (m.p.: 158 - 159.5°C).

270 MHz-NMR (DMSO-$d_6$) δ : 3.38-3.45 (4H, m), 3.95-4.03 (4H, m), 4.16 (2H, s), 7.55-7.74 (3H, m), 7.94 (2H, d), 9.04 (1H, s) 9.81 (1H, brs)

[Example 30-D]

3-(4-(Acetylthio)acetyl)piperazin-1-yl)-N-((benzoylthio)acetylsydnonimine

Under ice cooling, a mixture of 0.1 g of (acetylthio)acetic acid, 0.16 g of 1-ethyl-3-(3-dimethylaminopropyl)carbodiimide hydrochloride, 0.13 g of 1-hydroxybenzotriazole hydrate and 5 ml of N,N-dimethylformamide was stirred for 20 minutes. Next, 0.3 g of N-((benzoylthio)acetyl)-3-(piperazin-1-yl)sydnonimine hydrochloride and 0.13 ml of pyridine were added thereto and the resultant mixture was stirred for 5 minutes and then at room temperature for 20 hours. After adding water, the reaction mixture was extracted with a solvent mixture of ethyl acetate with ether. The organic layer was washed with a saturated aqueous solution of sodium chloride and dried and the solvent was distilled off. The residue thus obtained was purified by silica gel column chromatography (developing solution: ethyl acetate) to give 0.18 g of 3-(4-(acetylthio)acetyl)piperazin-1-yl)-N-(benzoylthio)acetyl)sydnonimine as an oily substance.

270 MHz-NMR ($CDCl_3$) δ : 2.40 (3H, s), 3.47-3.62 (4H, m), 3.81 (2H, s), 3.86-4.00 (4H, m), 4.08 (2H, s), 7.44 (2H, t), 7.56 (1H, t), 8.00 (2H, d), 8.06 (1H, s)

[Example 31]

N-((Benzoylthio)acetyl)-3-(4-(benzoylthio)acetyl)piperazin-1-yl)sydnonimine

The procedure of Example 30-D was repeated but starting with (benzoylthio)acetic acid to give N-((benzoylthio)acetyl)-3-(4-(benzoylthio)acetyl)piperazin-1-yl)sydnonimine (m.p.: 103.5 - 105°C).

270 MHz-NMR ($CDCl_3$) δ : 3.56 (4H, brd), 3.97 (4H, t), 4.00 (2H, s), 4.08 (2H, s), 7.41-7.68 (6H, m), 7.95-8.03 (4H, m), 8.05 (1H, s)

[Example 32]

N-((Benzoylthio)acetyl)-3-(4-(3,4,5-trimethoxyphenyl)acetyl)piperazin-1-yl)sydnonimine

The procedure of Example 30-D was repeated but starting with 3,4,5-trimethoxyphenylacetic acid to give N-((benzoylthio)acetyl)-3-(4-(3,4,5-trimethoxyphenyl)acetyl)piperazin-1-yl)sydnonimine (m.p.: 191.5 - 193°C).

270 MHz-NMR ($CDCl_3$) δ : 3.31-3.51 (4H, m), 3.71 (2H, s), 3.72-3.82 (2H, m), 3.83 (3H, s), 3.84 (6H, s), 3.90-3.99 (2H, m), 4.07 (2H, s), 6.44 (2H, s), 7.41-7.57 (3H, m), 7.98-8.02 (2H, m), 8.00 (1H, s)

[Example 33]

N-((Benzoylthio)acetyl)-3-(4-(3,5-di-t-butyl-4-hydroxyphenylthio)acetylpiperazin-1-yl)sydnonimine

The procedure of Example 30-D was repeated but starting with (3,5-di-t-butyl-4-hydroxyphenylthio)acetic acid to give N-((benzoylthio)acetyl)-3-(4-(3,5-di-t-butyl-4-hydroxyphenylthio)acetylpiperazin-1-yl)sydnonimine as an oily substance.

270 MHz-NMR ($CDCl_3$) δ : 1.42 (18H, s), 3.44-3.48 (4H, m), 3.67 (2H, s), 3.70-3.79 (2H, m), 3.86-3.93 (2H, m), 4.08 (2H, s), 5.34 (1H, s), 7.29 (2H, s), 7.41-7.62 (3H, m), 7.99-8.01 (2H, m), 8.02 (1H, s)
MS m/z : 625 ($M^+$)

[Example 34-A]

3-(4-Benzyloxycarbonylpiperazin-1-yl)-N-((3,4,5-trimethoxyphenyl)acetyl)sydnonimine

The procedure of Example 19-A was repeated but starting with 3-(4-benzylpiperazin-1-yl)-N-((3,4,5-trimethoxyphenyl)acetyl)sydnonimine to give 1.8 g of 3-(4-benzyloxycarbonylpiperazin-1-yl)-N-((3,4,5-trimethoxyphenyl)acetyl)sydnonimine as an oily substance.

60 MHz-NMR ($CDCl_3$) δ : 3.10-3.41 (4H, m), 3.47-3.78 (15H, m), 4.99 (2H, s), 6.40 (2H, s), 7.15 (5H, m), 7.78 (1H, s)
MS m/z : 511 ($M^+$)

[Example 34-B]

N-((3,4,5-Trimethoxyphenyl)acetyl)-3-(piperazin-1-yl)sydnonimine

The procedure of Example 19-B was repeated but starting with 3-(4-benzyloxycarbonylpiperazin-1-yl)-N-((3,4,5-trimethoxyphenyl)acetyl)sydnonimine to give 0.8 g of N-((3,4,5-trimethoxyphenyl)acetyl)-3-(piperazin-1-yl)sydnonimine as an oily substance.

60 MHz-NMR ($CDCl_3$) δ : 2.92-3.23 (4H, m), 3.26-3.57 (4H, m), 3.67 (2H, s), 3.77 (3H, s), 3.81 (6H, s), 6.55 (2H, s), 7.91 (1H, s)

[Example 34-C]

3-(4-(Acetylthio)acetylpiperazin-1-yl)-N-((3,4,5-trimethoxyphenyl)acetyl)sydnonimine

Under ice cooling, a mixture of 0.03 g of (acetylthio)acetic acid, 0.06 g of 1-ethyl-3-(3-dimethylaminopropyl)carbodiimide hydrochloride, 0.05 g of 1-hydroxybenzotriazole hydrate and 2 ml of N,N-dimethylformamide was stirred for 15 minutes. Next, 0.1 g of N-((3,4,5-trimethoxyphenyl)acetyl)-3-(piperazin-1-yl)-sydnonimine was added thereto and the resultant mixture was stirred for 5 minutes and then at room temperature for 20 hours. After adding water, the reaction mixture was extracted with ethyl acetate. The organic layer was washed with a saturated aqueous solution of sodium chloride and dried and the solvent was distilled off. The residue thus obtained was purified by silica gel column chromatography (developing solution: dichloromethane : methanol = 95 : 5) to give 0.03 g of 3-(4-(acetylthio)acetylpiperazin-1-yl)-N-((3,4,5-trimethoxyphenyl)acetyl)sydnonimine as an oily substance.

270 MHz-NMR ($CDCl_3$) δ : 2.39 (3H, s), 3.42-3.59 (4H, m), 3.71 (2H, s), 3.81 (3H, s), 3.84 (6H, s), 3.82-3.95 (6H, m), 6.58 (2H, s), 8.02 (1H, s)

[Example 35-A]

3-(4-Benzylpiperazin-1-yl)-N-((3,5-di-t-butyl-4-hydroxyphenylthio)acetyl)sydnonimine

The procedure of Example 17 was repeated but starting with (3,5-di-t-butyl-4-hydroxyphenylthio)acetic acid to give 3-(4-benzylpiperazin-1-yl)-N-((3,5-di-t-butyl-4-hydroxyphenylthio)acetyl)sydnonimine.

60 MHz-NMR ($CDCl_3$) δ : 1.36 (18H, s), 2.61-2.81 (4H, m), 3.40-3.62 (4H, m), 3.55 (2H, s), 3.73 (2H, s), 5.05 (1H, s), 7.21 (7H, m), 7.85 (1H, s)

[Example 35-B]

N-((3,5-Di-t-butyl-4-hydroxyphenylthio)acetyl)-3-(piperazin-1-yl)sydnonimine

At room temperature, a mixture of 4.9 g of 3-(4-benzylpiperazin-1-yl)-N-((3,5-di-t-butyl-4-hydroxyphenylthio)acetyl)sydnonimine, 1.8 ml of 1-chloroethyl chloroformate, 2.74 g of sodium iodide and 150 ml of acetone was stirred for 50 minutes. Then the reaction mixture was concentrated under reduced pressure and the solid matter thus precipitated was filtered off. After concentrating the filtrate under reduced pressure, the residue thus obtained was mixed with 100 ml of methanol and 100 ml of 1,2-dichloroethane. The resultant mixture was heated under reflux for 1.5 hours. After adding a saturated aqueous solution of sodium bicarbonate, the mixture was extracted with ethyl acetate. The organic layer was washed with a saturated aqueous solution of sodium chloride and dried and the solvent was distilled off. The residue thus obtained was purified by silica gel column chromatography (developing solution: chloroform : methanol : aqueous ammonia = 100 : 10 : 1) to give 1.1 g of N-((3,5-di-t-butyl-4-hydroxyphenylthio)acetyl)-3-(piperazin-1-yl)sydnonimine as an oily substance.

60 MHz-NMR ($CDCl_3$) δ : 1.39 (18H s), 3.00-3.22 (4H, m), 3.33-3.57 (4H, m), 3.75 (2H, s), 7.20 (2H, s), 7.89 (1H, s)

[Example 35-C]

N-((3,5-Di-t-butyl-4-hydroxyphenylthio)acetyl)-3-(4-(R)-2-oxo-4-thiazolidinecarbonyl)piperazin-1-yl)sydnonimine

Under ice cooling, a mixture of 100 mg of (-)-2-oxo-4-thiazolidinecarboxylic acid, 140 mg of 1-ethyl-3-(3-dimethylaminopropyl)carbodiimide hydrochloride, 110 mg of 1-hydroxybenzotriazole hydrate and 5 ml of N,N-dimethylformamide was stirred for 20 minutes. Next, a mixture of 0.30 g of N-((3,5-di-t-butyl-4-hydroxyphenylthio)acetyl)-3-(piperazin-1-yl)sydnonimine and 1 ml of N,N-dimethylformamide was added thereto and the resultant mixture was stirred for 3 minutes and then at room temperature for 24 hours. After adding water, the reaction mixture was extracted with ethyl acetate. The organic layer was washed with a saturated aqueous solution of sodium chloride and dried and the solvent was distilled off. The residue thus obtained was purified by silica gel column chromatography (developing solution: chloroform : methanol : aqueous ammonia = 100 : 10 : 1) to give 0.16 g of N-((3,5-di-t-butyl-4-hydroxyphenylthio)acetyl)-3-(4-(R)-2-oxo-4-thiazolidinecarbonyl)piperazin-1-yl)sydnonimine as an oily substance.

270 MHz-NMR ($CDCl_3$) δ : 1.39 (18H, s), 3.48-4.22 (10H, m), 3.81 (2H, s), 4.76-4.81 (1H, m), 5.81 (1H, s), 7.26

(2H, s), 8.20 (1H, s)

[Example 36]

3-(4-((S)-3-(Acetylthio)isobutyryl)piperazin-1-yl)-N-((3,5-di-t-butyl-4-hydroxyphenylthio)acetyl)sydnonimine

The procedure of Example 35-C was repeated but starting with (S)-3-(acetylthio)isobutyric acid to give 3-(4-((S)-3-(acetylthio)isobutyryl)piperazin-1-yl)-N-((3,5-di-t-butyl-4-hydroxyphenylthio)acetyl)sydnonimine as an oily substance.

270 MHz-NMR ($CDCl_3$) δ : 1.23 (3H, d), 1.41 (18H, s), 2.35 (3H, s), 2.83-2.99 (2H, m), 3.07-3.21 (1H, m), 3.41-3.57 (4H, m), 3.72-4.13 (4H, m), 3.81 (2H, s), 5.16 (1H, s), 7.32 (2H, s), 8.05 (1H, s)

[Example 37]

3-(4-(Acetylthio)acetylpiperazin-1-yl)-N-((3,5-di-t-butyl-4-hydroxyphenylthio)acetyl)sydnonimine

The procedure of Example 35-C was repeated but starting with (acetylthio)acetic acid to give 3-(4-(acetylthio)acetylpiperazin-1-yl)-N-((3,5-di-t-butyl-4-hydroxyphenylthio)acetyl)sydnonimine as an oily substance.

270 MHz-NMR ($CDCl_3$) δ : 1.40 (18H s), 2.40 (3H, s), 3.46-3.63 (4H, m), 3.80 (2H, s), 3.82 (2H, s), 3.87-3.98 (4H, m), 5.16 (1H, s), 7.32 (2H, s), 8.05 (1H, s)

[Example 38-A]

N-(Acetylthio)acetyl)-3-(4-benzylpiperazin-1-yl)sydnonimine

The procedure of Example 17 was repeated but starting with (acetylthio)acetic acid to give N-(acetylthio)acetyl)-3-(4-benzylpiperazin-1-yl)sydnonimine.

60 MHz-NMR ($CDCl_3$) δ : 2.36 (3H, s), 2.60-2.85 (4H, m), 3.40-3.66 (4H, m), 3.66 (2H, s), 3.87 (2H, s), 7.27 (5H, s), 7.89 (1H, s)

[Example 38-B]

N-((Acetylthio)acetyl)-3-(4-benzyloxycarbonylpiperazin-1-yl)sydnonimine

The procedure of Example 19-A was repeated but starting with N-((acetylthio)acetyl)-3-(4-benzylpiperazin-1-yl)sydnonimine to give N-((acetylthio)acetyl)-3-(4-benzyloxycarbonylpiperazin-1-yl)sydnonimine.

60 MHz-NMR ($CDCl_3$) δ : 2.24 (3H, s), 3.30-3.56 (4H, m), 3.57-3.84 (4H, m), 3.75 (2H, s), 5.03 (2H, s), 7.16 (5H, s), 7.89 (1H, s)

[Example 38-C]

N-((Acetylthio)acetyl)-3-(4-((acetylthio)acetyl)piperazin-1-yl)sydnonimine

Under ice cooling, 4 ml of a 33% solution of hydrogen bromide-acetic acid was added to a mixture of 1.5 g of N-((acetylthio)acetyl)-3-(4-benzyloxycarbonylpiperazin-1-yl)sydnonimine and 10 ml of dichloromethane and the resultant mixture was stirred at room temperature for 3 hours. Next, it was poured into ether under ice cooling and the crystals thus precipitated were taken up by filtration to give 1.5 g of N-((acetylthio)acetyl)-3-(piperazin-1-yl)sydnonimine hydrobromide.

Under ice cooling, 0.09 g of 1-ethyl-3-(3-dimethylaminopropyl)carbodiimide hydrochloride and 0.07 g of 1-hydroxybenzotriazole hydrate were added to a mixture of 0.06 g of (acetylthio)acetic acid and 3 ml of N,N-dimethylformamide and the resultant mixture was stirred for 30 minutes. Then 0.15 g of the N-((acetylthio)acetyl)-3-(piperazin-1-yl)sydnonimine hydrobromide obtained above and 0.05 ml of pyridine were added thereto under ice cooling followed by stirring at room temperature for 18 hours. After adding water, the reaction mixture was extracted with ethyl acetate. The organic layer was washed with water and dried and the solvent was distilled off. The residue thus obtained was purified

by silica gel column chromatography (developing solution: dichloromethane : methanol = 95 : 5) to give 0.08 g of N-((acetylthio)acetyl)-3-(4-((acetylthio)acetyl)piperazin-1-yl)sydnonimine as an oily substance.

60 MHz-NMR ($CDCl_3$) δ : 2.35 (3H, s), 2.36 (3H, s), 3.43-3.73 (4H, m), 3.74-4.06 (8H, m), 7.99 (1H, s)

[Example 39]

N-((Acetylthio)acetyl)-3-(4-((benzoylthio)acetyl)piperazin-1-yl)sydnonimine

The procedure of Example 38-C was repeated but replacing (acetylthio)acetic acid by (benzoylthio)acetic acid to give N-((acetylthio)acetyl)-3-(4-((benzoylthio)acetyl)piperazin-1-yl)sydnonimine (m.p.: 158 - 159°C).

60 MHz-NMR ($CDCl_3$) δ : 2.37 (3H, s), 3.51-3.79 (4H, m), 3.80-4.17 (4H, m), 3.86 (2H, s), 4.03 (2H, s), 7.31-7.68 (3H, m), 7.87-8.05 (2H, m), 8.06 (1H, s)

[Example 40]

N-((Acetylthio)acetyl)-3-(4-((3,4,5-trimethoxyphenyl)acetyl)piperazin-1-yl)sydnonimine

The procedure of Example 38-C was repeated but replacing (acetylthio)acetic acid by (3,4,5-trimethoxyphenyl)acetic acid to give N-((acetylthio)acetyl)-3-(4-((3,4,5-trimethoxyphenyl)acetyl)piperazin-1-yl)sydnonimine (m.p.: 159 - 161°C).

60 MHz-NMR ($CDCl_3$) δ : 2.34 (3H, s), 3.24-3.60 (4H, m), 3.62-4.05 (8H, m), 3.82 (9H, s), 6.43 (2H, s), 7.99 (1H, s)

[Example 41]

N-((Acetylthio)acetyl)-3-(4-methylpiperazin-1-yl)sydnonimine

Under ice cooling, 0.12 g of sodium borohydride was added to a mixture of 0.4 g of N-((acetylthio)acetyl)-3-(piperazin-1-yl)sydnonimine hydrobromide obtained in Example 38-C, 0.38 ml of formalin and 5 ml of methanol and the resultant mixture was stirred under ice cooling for 1 hour. After adding water, the reaction mixture was extracted with ethyl acetate. The organic layer was washed with water and dried and the solvent was distilled off. The residue thus obtained was purified by silica gel column chromatography (developing solution: chloroform : methanol : aqueous ammonia = 500 : 10 : 1) to give 0.02 g of N-((acetylthio)acetyl)-3-(4-methylpiperazin-1-yl)sydnonimine (m.p.: 138 - 139°C).

270 MHz-NMR ($CDCl_3$) δ : 2.38 (6H, s), 2.69-2.73 (4H, m), 3.52-3.57 (4H, m), 3.90 (2H, s), 7.98 (1H, s)

[Example 42]

N-((3,4,5-Trimethoxyphenyl)acetyl)-3-(4-methylpiperazin-1-yl)sydnonimine

A mixture of 0.1 g of N-((3,4,5-trimethoxyphenyl)acetyl)-3-(piperazin-1-yl)sydnonimine obtained in Example 34-B, 0.03 g of paraformaldehyde, 67 mg of sodium cyanoborohydride and 5 ml of methanol was stirred at room temperature for 2 hours. After adding water, the reaction mixture was extracted with ethyl acetate. The organic layer was washed with a saturated aqueous solution of sodium chloride and dried and the solvent was distilled off. The residue thus obtained was purified by silica gel column chromatography (developing solution: chloroform : methanol : aqueous ammonia = 100 : 10 : 1) to give 0.05 g of N-((3,4,5-trimethoxyphenyl)acetyl)-3-(4-methylpiperazin-1-yl)sydnonimine as an oily substance.

270 MHz-NMR ($CDCl_3$) δ : 2.36 (3H, s), 2.69 (4H, t), 3.52 (4H, t), 3.71 (2H, s), 3.81 (3H, s), 3.85 (6H, s), 6.59 (2H, s), 7.98 (1H, s)
MS m/z : 391 ($m^+$)

[Example 43]

N-((S)-3-(Acetylthio)isobutyryl)-3-(4-(2-(3,4,5-trimethoxyphenyl)ethyl)piperazin-1-yl)sydnonimine

0.36 g of N-((S)-3-(acetylthio)isobutyryl)-3-(piperazin-1-yl)sydnonimine hydrochloride obtained in Example 24-C was added at room temperature to a mixture of 0.6 g of (3,4,5-trimethoxyphenyl)acetaldehyde and 10 ml of methanol. Then 0.18 g of sodium cyanoborohydride was further added thereto followed by stirring for 18 hours. After adding water, the reaction mixture was extracted with ethyl acetate. The organic layer was washed with water and dried and the solvent was distilled off. The residue thus obtained was purified by silica gel column chromatography (developing solution: dichloromethane : methanol = 95 : 5) to give 0.33 g of N-((S)-3-(acetylthio)isobutyryl)-3-(4-(2-(3,4,5-trimethoxyphenyl)ethyl)piperazin-1-yl)sydnonimine (m.p.: 66 - 67°C).

60 MHz-NMR ($CDCl_3$) δ : 1.23 (3H, d), 2.24 (3H, s), 2.52-2.94 (9H, m), 3.02-3.26 (2H, m), 3.36-3.64 (4H, m), 3.74 (3H, s), 3.77 (6H, s), 6.32 (2H, s), 7.87 (1H, s)

[Example 44]

N-((S)-3-(Acetylthio)isobutyryl)-3-(4-(2-(3,5-di-t-butyl-4-hydroxyphenylthio)ethyl)piperazin-1-yl)sydnonimine.

(1) Under cooling at - 78°C, 3.7 ml of a solution (1.01 mol/l) of diisobutylaluminum hydride in toluene was dropped into a mixture of 1.2 g of ethyl (3,5-di-t-butyl-4-hydroxyphenylthio)acetate and 40 ml of toluene and the resultant mixture was stirred for 10 minutes. Then the mixture was diluted with ether. After adding a saturated aqueous solution of sodium chloride, the mixture was stirred at room temperature for 1 hour. The organic layer was dried and the solvent was distilled off. The residue thus obtained was purified by silica gel column chromatography (developing solution: hexane : dichloromethane = 2 : 1) to give 0.8 g of (3,5-di-t-butyl-4-hydroxyphenylthio)acetaldehyde as an oily substance.

60 MHz-NMR ($CDCl_3$) δ : 1.33 (18H s), 3.40 (2H, d), 5.16 (1H, s), 7.06 (2H, s), 9.36 (1H, t)

(2) The procedure of Example 43 was repeated but starting with (3,5-di-t-butyl-4-hydroxyphenylthio)acetaldehyde to give N-((S)-3-(acetylthio)isobutyryl)-3-(4-(2-(3,5-di-t-butyl-4-hydroxyphenylthio)ethyl)piperazin-1-yl)sydnonimine as an oily substance.

60 MHz-NMR ($CDCl_3$) δ : 1.25 (3H, d), 1.50 (18H, s), 2.28 (3H, s), 2.57-3.05 (9H, m), 3.06-3.25 (2H, m), 3.37-3.63 (4H, m), 5.24 (1H, s), 7.18 (2H, s), 7.97 (1H, s)

[Example 45]

N-((S)-3-(Benzoylthio)isobutyryl)-3-(4-(2-(3,4,5-trimethoxyphenyl)ethyl)piperazin-1-yl)sydnonimine

The procedure of Example 43 was repeated but using, as the sydnonimine derivative, N-((S)-3-(benzoylthio)isobutyryl)-3-piperazin-1-yl)sydnonimine obtained in Example 19-B to give N-((S)-3-(benzoylthio)isobutyryl)-3-(4-(2-(3,4,5-trimethoxyphenyl)ethyl)piperazin-1-yl)sydnonimine as an oily substance.

270 MHz-NMR ($CDCl_3$) δ : 1.34 (3H, d), 2.66-2.84 (8H, m), 2.85-2.98 (1H, m), 3.21-3.47 (2H, m), 3.51-3.60 (4H, m), 3.83 (3H, s), 3.85 (6H, s), 6.42 (2H, s), 7.33-7.44 (2H, m) 7.50-7.53 (1H, m), 7.95-7.98 (2H, m), 8.07 (1H, s)

[Example 46]

N-((S)-3-(Benzoylthio)isobutyryl)-3-(4-(2-(3,5-di-t-butyl-4-hydroxyphenylthio)ethyl)piperazin-1-yl)sydnonimine

At room temperature, a mixture of 0.38 g of N-((S)-3-(benzoylthio)isobutyryl)-3-piperazin-1-yl)sydnonimine, 0.4 g of (3,5-di-t-butyl-4-hydroxyphenylthio)acetaldehyde, 0.09 g of sodium cyanoborohydride and 10 ml of methanol was stirred for 5 hours. After adding water, the reaction mixture was extracted with ethyl acetate. The organic layer was washed with a saturated aqueous solution of sodium chloride and dried. Then the solvent was distilled off and the residue thus obtained was purified by silica gel column chromatography (developing solution: hexane : ethyl acetate = 1 :

1) to give 0.08 g of N-((S)-3-(benzoylthio)isobutyryl)-3-(4-(2-(3,5-di-t-butyl-4-hydroxyphenylthio)ethyl)piperazin-1-yl)sydnonimine as an oily substance.

270 MHz-NMR ($CDCl_3$) δ : 1.34 (3H, d), 1.43 (18H, s), 2.69-2.78 (5H m), 2.85-2.99 (4H, m), 3.29-3.54 (6H, m), 5.25 (1H, s), 7.25 (2H, s), 7.34-7.56 (3H, m), 7.94-7.99 (2H, m), 8.00 (1H, s)

[Example 47]

N-((3,5-Di-t-butyl-4-hydroxyphenylthio)acetyl)-3-(4-(3,4-dimethoxyphenylmethyl)piperazin-1-yl)sydnonimine

At room temperature, a mixture of 0.34 g of N-((3,5-di-t-butyl-4-hydroxyphenylthio)acetyl)-3-(piperazin-1-yl)sydnonimine obtained in Example 35-B, 0.5 g of 3,4-dimethoxybenzaldehyde, 0.2 g of sodium cyanoborohydride and 8 ml of methanol was stirred for 24 hours. After adding water, the reaction mixture was extracted with ethyl acetate. The organic layer was washed with a saturated aqueous solution of sodium chloride and dried. Then the solvent was distilled off and the residue thus obtained was purified by silica gel column chromatography (developing solution: dichloromethane : methanol = 95 : 5) to give 0.24 g of N-((3,5-di-t-butyl-4-hydroxyphenylthio)acetyl)-3-(4-(3,4-dimethoxyphenylmethyl)piperazin-1-yl)sydnonimine as an oily substance.

270 MHz-NMR ($CDCl_3$) δ : 1.40 (18H, s), 2.73 (4H, t), 3.54-3.56 (4H, m), 3.53 (2H, s), 3.80 (2H, s), 3.87 (3H, s), 3.89 (3H, s), 5.17 (1H, s), 6.82 (2H, s), 6.87 (1H, s), 7.32 (2H, s), 8.02 (1H, s)

[Example 48]

N-((Acetylthio)acetyl)-3-(4-(3,4,5-trimethoxyphenylmethyl)piperazin-1-yl)sydnonimine

(1) Under ice cooling, 1.2 ml of conc. sulfuric acid was added to a mixture of 5.84 g of 4-(3,4,5-trimethoxyphenylmethyl)piperazine and 70 ml of water. Further, a mixture of 1.55 g of sodium nitrite and 10 ml of water was added thereto and the resultant mixture was stirred for 30 minutes. Under ice cooling, a saturated aqueous solution of sodium bicarbonate was added to the mixture. After neutralizing, the reaction mixture was extracted with ethyl acetate. The organic layer was washed with water and dried and the solvent was distilled off to give 6.3 g of 1-nitroso-4-(3,4,5-trimethoxyphenylmethyl)piperazine as an oily substance. To a mixture of 6.3 g of the thus obtained 1-nitroso-4-(3,4,5-trimethoxyphenylmethyl)piperazine and 100 ml of teterahydrofuran was added at room temperature 1.62 g of lithium aluminum hydride followed by stirring for 1 hour. Then the mixture was diluted with ethyl acetate. After adding a saturated aqueous solution of sodium chloride, the mixture was stirred at room temperature for 30 minutes. Then the organic layer was dried and the solvent was distilled off to give 3.7 g of 1-amino-4-(3,4,5-trimethoxyphenylmethyl)piperazine as an oily substance.

60 MHz-NMR ($CDCl_3$) δ : 2.35-2.84 (8H, m), 2.98-3.22 (2H, m), 3.41 (2H, s), 3.80 (3H, s), 3.82 (6H, s), 6.59 (2H, s)

(2) At room temperature, a mixture of 3.7 g of 1-amino-4-(3,4,5-trimethoxyphenylmethyl)piperazine, 1.8 g of formaldehyde-sodium bisulfite adduct and 30 ml of water was stirred for 30 minutes. Then 0.87 g of potassium cyanide was added thereto and the resultant mixture was stirred at 50°C for additional 2 hours followed by extraction with ethyl acetate. The organic layer was washed with water and dried and the solvent was distilled off. The residue thus obtained was purified by silica gel column chromatography (developing solution: dichloromethane : methanol = 98 : 2) to give 1.15 g of 1-(cyanomethylamino)-4-(3,4,5-trimethoxyphenylmethyl)-piperazine as an oily substance.

60 MHz-NMR ($CDCl_3$) δ : 2.38-2.95 (9H, m), 3.40 (2H, s), 3.67 (2H, d), 3.80 (3H, s), 3.82 (6H, s), 6.50 (2H, s)

(3) Under ice cooling, 2.5 ml of a 15 % solution of ethyl nitrite in ethanol was dropped into a mixture of 1.15 g of 1-(cyanomethylamino)-4-(3,4,5-trimethoxyphenylmethyl)piperazine, 0.6 ml of conc. hydrochloric acid and 10 ml of ethanol. The resultant mixture was stirred for 1 hour and then at room temperature for 5 hours. The crystals thus precipitated were taken up by filtration to give 0.6 g of 3-(4-(3,4,5-trimethoxyphenyl-methyl)piperazin-1-yl)sydnonimine hydrochloride.

Under ice cooling, 0.06 g of 1-ethyl-3-(3-dimethylaminopropyl)carbodiimide hydrochloride and 0.05 g of 1-hydroxybenzotriazole hydrate were added to a mixture of 0.08 g of (acetylthio)acetic acid and 2 ml of N,N-dimethylformamide and the resultant mixture was stirred for 15 minutes. Next, 0.1 g of 3-(4-(3,4,5-trimethoxyphenylmethyl)piperazin-1-yl)sydnonimine hydrochloride obtained above and 0.1 ml of pyridine were added thereto and the resultant mixture was stirred for 1 hour under ice cooling and then for 6 hours at room temperature. After adding water, the reaction mixture was extracted with ethyl acetate. The organic layer was washed with water and dried. Then the solvent was distilled off and the residue thus obtained was purified by recrystallization from ethyl acetate and ether to give 0.07 g of N-((acetylthio)acetyl-3-(4-(3,4,5-trimethoxyphenylmethyl)piperazin-1-yl)sydnonimine (m.p.: 136 - 137°C).

270 MHz-NMR ($CDCl_3$) $\delta$ : 2.38 (3H, s), 2.73-2.78 (4H, m), 3.52 (2H, s), 3.52-3.60 (4H, m), 3.85 (3H, s), 3.87 (6H, s) 3.90 (2H, s), 6.55 (2H, s), 7.98 (1H, s)

[Example 49]

N-((Benzoylthio)acetyl)-3-(4-(3,4,5-trimethoxyphenylmethyl)piperazin-1-yl)sydnonimine

The procedure of Example 48 (3) was repeated but replacing (acetylthio)acetic acid by (benzoylthio)acetic acid to give N-((benzoylthio)acetyl)-3-(4-(3,4,5-trimethoxyphenylmethyl)piperazin-1-yl)sydnonimine (m.p.: 88 - 89°C).

270 MHz-NMR ($CDCl_3$) $\delta$ : 2.73-2.77 (4H, m), 3.51 (2H, s), 3.51-3.58 (4H, m), 3.85 (3H, s), 3.87 (6H, s), 4.09 (2H, s), 6.55 (2H, s), 7.40-7.47 (2H, m), 7.50-7.57 (1H, m), 7.78-8.03 (2H, m), 8.01 (1H, s)

[Example 50]

N-((Acetylthio)acetyl)-3-(4-(2,3,4-trimethoxyphenylmethyl)piperazin-1-yl)sydnonimine

Under ice cooling, a mixture of 0.04 g of (acetylthio)acetic acid, 0.06 g of 1-ethyl-3-(3-dimethylaminopropyl)carbodiimide hydrochloride, 0.05 g of 1-hydroxybenzotriazole hydrate and 2 ml of N,N-dimethylformamide was stirred for 15 minutes. Then 0.1 g of 3-(4-(2,3,4-trimethoxyphenylmethyl)piperazin-1-yl)sydnonimine hydrochloride and 0.1 ml of pyridine were added thereto and the resultant mixture was stirred for 30 minutes and then for 3 hours at room temperature. After adding water, the reaction mixture was extracted with ethyl acetate. The organic layer was washed with water and dried. Then the solvent was distilled off and the residue thus obtained was purified by silica gel column chromatography (developing solution: dichloromethane : methanol = 97 : 3) to give 0.06 g of N-((acetylthio)acetyl)-3-(4-(2,3,4-trimethoxyphenylmethyl)piperazin-1-yl)sydnonimine as an oily substance.

60 MHz-NMR ($CDCl_3$) $\delta$ : 2.36 (3H, s), 2.65-2.94 (4H, m), 3.41-3.71 (4H, m), 3.55 (2H, s), 3.80-4.01 (2H, m), 3.85 (9H, s), 6.61 (1H, d), 6.95 (1H, d), 7.94 (1H, s)

[Example 51]

N-((Benzoylthio)acetyl)-3-(4-(2,3,4-trimethoxyphenylmethyl)piperazin-1-yl)sydnonimine

The procedure of Example 50 was repeated but starting with (benzoylthio)acetic acid to give N-((benzoylthio)acetyl)-3-(4-(2,3,4-trimethoxyphenylmethyl)piperazin-1-yl)sydnonimine as an oily substance.

60 MHz-NMR ($CDCl_3$) $\delta$ : 2.58-2.93 (4H, m), 3.48-3.68 (4H, m), 3.52 (2H, s), 3.84 (3H, s), 3.85 (6H, s), 4.05 (2H, s), 6.59 (1H, d), 6.92 (1H, d), 7.28-7.56 (3H, m), 7.81-8.05 (2H, m) 7.92 (1H, s)

[Example 52-A]

N-((S)-3-(Benzoylthio)isobutyryl)-3-(4-(2-hydroxyethyl)piperazin-1-yl)sydnonimine

At room temperature, 0.12 g of N,N-carbonyldiimidazole was added to a mixture of 0.16 g of (S)-3-(benzoylthio)isobutyric acid and 4 ml of dimethylsulfoxide and the resultant mixture was stirred for 1 hour. Next, 0.2 g of 3-(4-(2-hydroxyethyl)piperazin-1-yl)sydnonimine hydrochloride and 0.4 ml of pyridine were added thereto and the resultant mixture was stirred for 7 hours at room temperature. After adding water, the reaction mixture was extracted with ethyl acetate. The organic layer was washed with water and dried. Then the solvent was distilled off and the residue thus obtained was purified by silica gel column chromatography (developing solution chloroform : methanol : aqueous ammonia = 200 : 10 : 1) to give 0.08 g of N-((S)-3-(benzoylthio)isobutyryl)-3-(4-(2-hydroxyethyl)piperazin-1-yl)sydnonimine as an oily substance.

270 MHz-NMR ($CDCl_3$) δ : 1.34 (3H, d) 2.77-2.98 (5H, m), 3.24-3.49 (6H, m), 3.53-3.60 (4H, m), 7.38-7,58 (3H, m), 7.93-7.98 (2H, m), 8.03 (1H, s)

[Example 52-B]

N-((S)-3-(Benzoylthio)isobutyryl)-3-(4-(2-(benzoylthio)ethyl)piperazin-1-yl)sydnonimine

Under ice cooling, 0.07 g of triphenylphosphine and 0.04 ml of diethyl azodicarboxylate were added to a mixture of 0.06 ml of thiobenzoic acid, 0.1 g of N-((S)-3-(benzoylthio)isobutyryl)-3-(4-(2-hydroxyethyl)piperazin-1-yl)sydnonimine and 2 ml of teterahydrofuran and the resultant mixture was stirred for 1 hour under ice cooling and then for 1 hour at room temperature. After adding water, the reaction mixture was extracted with ethyl acetate. The organic layer was washed with water and dried. Then the solvent was distilled off and the residue thus obtained was purified by silica gel column chromatography (developing solution: hexane : ethyl acetate = 1 : 2) to give 0.03 g of N-((S)-3-(benzoylthio)isobutyryl)-3-(4-(2-(benzoylthio)ethyl)piperazin-1-yl)sydnonimine as an oily substance.

270 MHz-NMR ($CDCl_3$) δ : 1.34 (3H, d), 2.74 (2H, dd), 2.84-2.98 (5H, m), 3.22 (2H, dd), 3.29-3.46 (2H, m), 3.53-3.58 (4H, m), 7.37-7.72 (6H, m), 7.94-7.99 (4H, m), 8.01 (1H, s)

[Example 53-A]

N-((Benzoylthio)acetyl)-3-(4-(2-chloroethyl)piperazin-1-yl)sydnonimine

A mixture of 0.5 g of 3-(4-(2-hydroxyethyl)piperazin-1-yl)sydnonimine hydrochloride, 5 ml of thionyl chloride and a drop of pyridine was stirred at 60°C for 4 hours. Then the reaction mixture was diluted with ether and the crystals thus formed were taken up by filtration to give 0.5 g of 3-(4-(2-chloroethyl)piperazin-1-yl)sydnonimine hydrochloride. Under ice cooling, 0.34 g of 1-ethyl-3-(3-dimethylaminopropyl)carbodiimide hydrochloride and 0.27 g of 1-hydroxybenzotriazole hydrate were added to a mixture of 0.34 g of (benzoylthio)acetic acid and 10 ml of N,N-dimethylformamide and the resultant mixture was stirred for 30 minutes. Next, 0.5 g of 3-(4-(2-chloroethyl)piperazin-1-yl)sydnonimine hydrochloride and 0.6 ml of pyridine were added thereto and the resultant mixture was stirred for 30 minutes under ice cooling and then for 15 hours at room temperature. After adding water, the reaction mixture was extracted with ethyl acetate. The organic layer was washed with water and dried. Then the solvent was distilled off and the residue thus obtained was purified by silica gel column chromatography (developing solution: dichloromethane : methanol = 95 : 5) to give 0.3 g of N-((benzoylthio)acetyl)-3-(4-(2-chloroethyl)piperazin-1-yl)sydnonimine.

270 MHz-NMR ($CDCl_3$) δ : 2.80-2.86 (6H, m), 3.54-3.62 (6H, m), 4.09 (2H, s), 7.40-7.47 (2H, m), 7.53-7.60 (1H, m), 7.98-8.01 (2H, m), 8.02 (1H, s)

[Example 53-B]

3-(4-(2-Acetylthio)ethyl)piperazin-1-yl)-N-((benzoylthio)acetyl)sydnonimine

A mixture of 0.07 g of N-((benzoylthio)acetyl)-3-(4-(2-chloroethyl)piperazin-1-yl)sydnonimine, 0.04 g of potassium thioacetate, 0.03 g of potassium iodide and 1 ml of N,N-dimethylformamide was stirred at 60°C for 4 hours. After adding water, the reaction mixture was extracted with ethyl acetate. The organic layer was washed with water and dried. Then the solvent was distilled off and the residue thus obtained was purified by silica gel column chromatography (developing solution: dichloromethane : methanol = 95 : 5) to give 0.02 g of 3-(4-(2-acetylthio)ethyl)piperazin-1-yl)-N-((benzoylthio)acetyl)sydnonimine (m.p.: 106 - 107°C).

270 MHz-NMR ($CDCl_3$) δ : 2.35 (3H, s), 2.61-2.66 (2H, m), 2.73-2.83 (4H, m), 2.97-3.04 (2H, m), 3.47-3.58 (4H, m), 4.09 (2H, s), 7.41-7.56 (3H, m), 7.98-8.02 (2H, m), 8.00 (1H, s)

[Example 54-A]

N-((S)-3-(Benzoylthio)isobutyryl)-3-(4-(2-chloroethyl)piperazin-1-yl)sydnonimine

The procedure of Example 53-A was repeated but replacing (benzoylthio)acetic acid by (S)-3-(benzoylthio)isobutyric acid to give N-((S)-3-(benzoylthio)isobutyryl)-3-(4-(2-chloroethyl)piperazin-1-yl)sydnonimine.

270 MHz-NMR ($CDCl_3$) δ : 1.34 (3H, d), 2.81-2.96 (7H, m), 3.29-3.45 (2H, m), 3.54-3.63 (6H, m), 7.38-7.45 (2H, m), 7.51-7.57 (1H, m) 7.94-7.98 (2H, m), 8.02 (1H, s)

[Example 54-B]

3-(4-(2-(Acetylthio)ethyl)piperazin-1-yl)-N-((S)-3-(benzoylthio)isobutyryl)sydnonimine

The procedure of Example 53-B was repeated but starting with N-((S)-3-(benzoylthio)isobutyryl)-3-(4-(2-chloroethyl)piperazin-1-yl)sydnonimine to give 3-(4-(2-(acetylthio)ethyl)piperazin-1-yl)-N-((S)-3-(benzoylthio)isobutyryl)sydnonimine as an oily substance.

270 MHz-NMR ($CDCl_3$) δ : 1.34 (3H, d), 2.35 (3H, s), 2.63 (2H, dd), 2.80-2.99 (1H, m), 3.02 (2H, dd), 3.29-3.46 (2H, m), 3.51-3.56 (4H, m), 7.38-7.45 (2H, m), 7.50-7.57 (1H, m), 7.94-7.99 (2H, m), 8.02 (1H, s)

[Example 55-A]

N-((3,5-Di-t-butyl-4-hydroxyphenylthio)acetyl)-3-(4-(2-chloroethyl)piperazin-1-yl)sydnonimine

The procedure of Example 53-A was repeated but replacing (benzoylthio)acetic acid by (3,5-di-t-butyl-4-hydroxyphenylthio)acetic acid to give N-((3,5-di-t-butyl-4-hydroxyphenylthio)acetyl)-3-(4-(2-chloroethyl)piperazin-1-yl)sydnonimine.

60 MHz-NMR ($CDCl_3$) δ : 1.41 (18H, s), 2.70-2.98 (8H, m), 3.46-3.75 (4H, m), 3.78 (2H, s), 5.12 (1H, s), 7.27 (2H, s), 7.96 (1H, s)

[Example 55-B]

3-(4-(2-(Acetylthio)ethyl)piperazin-1-yl)-N-((3,5-di-t-butyl-4-hydroxyphenylthio)acetyl)sydnonimine

The procedure of Example 53-B was repeated but starting with N-((3,5-di-t-butyl-4-hydroxyphenylthio)acetyl)-3-(4-(2-chloroethyl)piperazin-1-yl)sydnonimine to give 3-(4-(2-(acetylthio)ethyl)piperazin-1-yl)-N-((3,5-di-t-butyl-4-hydroxyphenylthio)acetyl)sydnonimine (m.p.: 123 - 125°C).

270 MHz-NMR ($CDCl_3$) δ : 1.40 (18H, s), 2.35 (3H, s), 2.61-2.67 (2H, m), 2.79-2.83 (4H, m), 2.99-3.05 (2H, m), 3.52-3.56 (4H, m), 3.81 (2H, s), 5.14 (1H, s), 7.32 (2H, s), 8.00 (1H, s)

[Example 56-A]

N-((S)-3-(Acetylthio)isobutyryl)-3-(N'-allyl-N'-methylamino)sydnonimine

Under ice cooling, 2.1 g of 1-ethyl-3-(3-dimethylaminopropyl)carbodiimide hydrochloride and 1.65 g of 1-hydroxybenzotriazole hydrate were added to a mixture of 1.5 ml of (S)-3-(acetylthio)isobutyric acid and 60 ml of N,N-dimethylformamide and the resultant mixture was stirred for 30 minutes. Next, 2 g of 3-(N-allyl-N-methylamino)sydnonimine hydrochloride and 3.4 ml of pyridine were added thereto and the resultant mixture was stirred for 1 hour under ice cooling and then for 4 hours at room temperature. After adding water, the reaction mixture was extracted with ethyl acetate. The organic layer was washed with water and dried. Then the solvent was distilled off and the residue thus obtained was purified by silica gel column chromatography (developing solution: hexane : ethyl acetate = 1 : 1) to give 2 g of N-((S)-3-(acetylthio)isobutyryl)-3-(N'-allyl-N'-methylamino)sydnonimine as an oily substance.

60 MHz-NMR ($CDCl_3$) δ : 1.24 (3H, d), 2.24 (3H, s), 2.50-2.93 (1H, m), 3.00-3.31 (5H, m), 3.95 (2H, d) 5.04-6.07 (3H, m), 7.82 (1H, s)

[Example 56-B]

N-((S)-3-(Acetylthio)isobutyryl)-3-methylaminosydnonimine

At room temperature, a mixture of 1.14 g of thiosalicylic acid, 0.35 g of tris(dibenzylideneacetone) (chloroform) dipalladium, 0.14 g of 1,4-bis(diphenylphosphino)butane and 5 ml of tetrahydrofuran was added to another mixture of 2 g of N-((S)-3-(acetylthio)isobutyryl)-3-(N'-allyl-N'-methylamino)sydnonimine and 45 ml of tetrahydrofuran and the resultant mixture was stirred for 5 hours. After adding water, the reaction mixture was extracted with ethyl acetate. The organic layer was washed with water and dried. Then the solvent was distilled off and the residue thus obtained was purified by silica gel column chromatography (developing solution: dichloromethane : methanol = 97 : 3) to give 1.3 g of N-((S)-3-(acetylthio)isobutyryl)-3-methylaminosydnonimine as an oily substance.

60 MHz-NMR ($CDCl_3$) δ : 1.27 (3H, d), 2.28 (3H, s), 2.51-2.97 (1H, m), 2.99-3.19 (2H, m), 3.26 (3H, s), 7.79 (1H, s), 9.54 (1H, brs)

[Example 56-C]

N-((S)-3-(Acetylthio)isobutyryl)-3-(N'-((S)-3-(acetylthio)isobutyryl)-N'-methylamino)sydnonimine

Under ice cooling, 0.09 g of 1-ethyl-3-(3-dimethylaminopropyl)carbodiimide hydrochloride and 0.07 g of 1-hydroxybenzotriazole hydrate were added to a mixture of 0.06 ml of (S)-3-(acetylthio)isobutyric acid, 0.1 g of N-((S)-3-(acetylthio)isobutyryl)-3-methylaminosydnonimine and 3 ml of N,N-dimethylformamide and the resultant mixture was stirred for 1 hour and then at room temperature for 12 hours. After adding water, the reaction mixture was extracted with ethyl acetate. The organic layer was washed with water and dried. Then the solvent was distilled off and the residue thus obtained was purified by silica gel column chromatography (developing solution: hexane : ethyl acetate = 1 : 1) to give 0.08 g of N-((S)-3-(acetylthio)isobutyryl)-3-(N'-((S)-3-(acetylthio)isobutyryl)-N'-methylamino)sydnonimine as an oily substance.

270 MHz-NMR ($CDCl_3$) δ : 1.28 (6H, d), 2.32 (3H, s), 2.35 (3H, s), 2.52-2.72 (1H, m), 2.75-2.86 (1H, m), 2.96-3.24 (4H, m), 3.63 (3H, s), 8.16 (1H, s)

[Example 57]

N-((S)-3-(Acetylthio)isobutyryl)-3-(N'-(S)-3-(benzoylthio)isobutyryl)-N'-methylamino)sydnonimine

The procedure of Example 56-C was repeated but replacing (S)-3-(acetylthio)isobutyric acid by (S)-3-(benzoylthio)isobutyric acid to give N-((S)-3-(acetylthio)isobutyryl)-3-(N'-(S)-3-(benzoylthio)isobutyryl)-N'-methylamino)sydnonimine as an oily substance.

270 MHz-NMR ($CDCl_3$) δ : 1.26 (3H, d), 1.35 (3H, d), 2.31 (3H, s), 2.74-2.87 (1H, m), 3.08-3.28 (2H, m), 3.65 (3H, s), 7.43-7.58 (2H, m), 7.59-7.64 (1H, m), 7.90-7.98 (2H, m), 8.17 (1H, s)

[Example 58]

N-((S)-3-(Acetylthio)isobutyryl)-3-(N'-((3,4,5-trimethoxyphenyl)acetyl)-N'-methylamino)sydnonimine

The procedure of Example 56-C was repeated but replacing (S)-3-(acetylthio)isobutyric acid by 3,4,5-trimethoxyphenylacetic acid to give N-((S)-3-(acetylthio)isobutyryl)-3-(N'-((3,4,5-trimethoxyphenyl)acetyl)-N'-methylamino)sydnonimine as an oily substance.

270 MHz-NMR ($CDCl_3$) δ : 1.27 (3H, d), 2.31 (3H, s), 2.75-2.86 (1H, m), 3.05-3.24 (2H, m), 3.60 (3H, s), 3.66 (2H, s), 3.83 (9H, s), 6.31 (2H, s), 8.00 (1H, s)

[Example 59]

N-((S)-3-(Acetylthio)isobutyryl)-3-(N'-((3,5-di-t-butyl-4-hydroxyphenylthio)acetyl)-N'-methylamino)sydnonimine

The procedure of Example 56-C was repeated but replacing (S)-3-(acetylthio)isobutyric acid by (3,5-di-t-butyl-4-hydroxyphenylthio)acetic acid to give N-((S)-3-(acetylthio)isobutyryl)-3-(N'-((3,5-di-t-butyl-4-hydroxyphenylthio)acetyl)-N'-methylamino)sydnonimine as an oily substance.

60 MHz-NMR ($CDCl_3$) δ : 1.26 (3H, d), 1.41 (18H, s), 2.28 (3H, s), 2.72-2.94 (1H, m), 3.04-3.22 (2H, m), 3.57 (2H, s), 3.58 (3H, s), 5.38 (1H, brs), 7.17 (2H, s), 7.78 (1H, s)

[Example 60-A]

3-(N'-Allyl-N'-methylamino)-N-((S)-3-benzoylthio)isobutyryl)sydnonimine

The procedure of Example 56-A was repeated but replacing (S)-3-(acetylthio)isobutyric acid by (S)-3-(benzoylthio)isobutyric acid to give 3-(N'-allyl-N'-methylamino)-N-((S)-3-benzoylthio)isobutyryl)sydnonimine.

60 MHz-NMR ($CDCl_3$) δ : 1.32 (3H, d), 2.65-3.09 (1H, m), 3.14 (3H, s), 3.25-3.46 (2H, m), 3.98 (2H, d), 5.10-5.90 (3H, m), 7.19-7.51 (3H, m), 7.78-7.97 (2H, m), 7.90 (1H, s)

[Example 60-B]

N-((S)-3-(Benzoylthio)isobutyryl)-3-methylaminosydnonimine

The procedure of Example 56-B was repeated but starting with 3-(N'-allyl-N'-methylamino)-N-((S)-3-benzoylthio)isobutyryl)sydnonimine to give N-((S)-3-(benzoylthio)isobutyryl)-3-methylaminosydnonimine.

60 MHz-NMR ($CDCl_3$) δ : 1.30 (3H, d), 2.62-3.03 (1H, m), 3.24 (3H, s), 3.24-3.38 (2H, m), 7.09-7.44 (3H, m), 7.66-7.87 (2H, m), 7.81 (1H, s), 8.60-9.40 (1H, m)

[Example 60-C]

3-(N'-((S)-3-(Acetylthio)isobutyryl)-N'-methylamino)-N-((S)-3-(benzoylthio)isobutyryl)sydnonimine

The procedure of Example 56-C was repeated but starting with N-((S)-3-(benzoylthio)isobutyryl)-3-methylaminosydnonimine as the sydnonimine derivative to give 3-(N'-((S)-3-(acetylthio)isobutyryl)-N'-methylamino)-N-((S)-3-(benzoylthio)isobutyryl)sydnonimine as an oily substance.

60 MHz-NMR ($CDCl_3$) δ : 1.26 (3H, d), 1.34 (3H, d), 2.30 (3H, s), 2.50-2.89 (2H, m), 2.89-3.09 (2H, m), 3.23-3.44 (2H, m), 3.58 (3H, s), 7.19-7.46 (3H, m), 7.72-7.93 (2H, m), 8.03 (1H, s)

[Example 61]

N-((S)-3-(Benzoylthio)isobutyryl)-3-(N'-((S)-3-(benzoylthio)isobutyryl)-N'-methylamino)sydnonimine

Under ice cooling, 0.06 g of 1-ethyl-3-(3-dimethylaminopropyl)carbodiimide hydrochloride and 0.05 g of 1-hydroxybenzotriazole hydrate were added to a mixture of 0.07 g of (S)-3-(benzoylthio)isobutyric acid, 0.1 g of N-((S)-3-(benzoylthio)isobutyryl)-3-methylaminosydnonimine and 3 ml of N,N-dimethylformamide and the resultant mixture was stirred for 48 hours at room temperature. After adding water, the reaction mixture was extracted with ethyl acetate. The organic layer was washed with water and dried. Then the solvent was distilled off and the residue thus obtained was purified by silica gel column chromatography (developing solution: hexane : ethyl acetate = 2 : 1) to give 0.07 g of N-((S)-3-(benzoylthio)isobutyryl)-3-(N'-((S)-3-(benzoylthio)isobutyryl)-N'-methylamino)sydnonimine as an oily substance.

270 MHz-NMR ($CDCl_3$) δ : 1.30-1.40 (6H, m), 2.80-2.96 (2H, m), 3.14-3.45 (4H, m), 3.65 (3H, s), 7.38-7.62 (6H, m), 7.90-7.98 (4H, m), 8.21 (1H, s)

[Example 62]

N-((S)-3-(Benzoylthio)isobutyryl)-3-(N'-((3,4,5-trimethoxyphenyl)acetyl)-N'-methylamino)sydnonimine

The procedure of Example 61 was repeated but replacing (S)-3-(benzoylthio)isobutyric acid by 3,4,5-trimethoxyphenylacetic acid to give N-((S)-3-(benzoylthio)isobutyryl)-3-(N'-((3,4,5-trimethoxyphenyl)acetyl)-N'-methylamino)sydnonimine as an oily substance.

270 MHz-NMR ($CDCl_3$) δ : 1.34 (3H, d), 2.83-2.99 (1H, m), 3.26-3.48 (2H, m), 3.59 (3H, s), 3.65 (2H, s), 3.82 (9H, s), 6.30 (2H, s), 7.39-7.45 (2H, m), 7.51-7.55 (1H, m), 7.94-7.98 (2H, m), 7.99 (1H, s)

[Example 63-A]

3-(N'-Allyl-N'-methylamino)-N-((3,5-di-t-butyl-4-hydroxyphenylthio)acetyl)sydnonimine

The procedure of Example 56-A was repeated but replacing (S)-3-(acetylthio)isobutyric acid by (3,5-di-t-butyl-4-hydroxyphenylthio)acetic acid to give 3-(N'-allyl-N'-methylamino)-N-((3,5-di-t-butyl-4-hydroxyphenylthio)acetyl)sydnonimine.

60 MHz-NMR ($CDCl_3$) δ : 1.38 (18H, s), 3.13 (3H, s), 3.72 (2H, s), 3.97 (2H, d), 5.04-5.96 (4H, m), 7.19 (2H, s), 7.96 (1H, s)

[Example 63-B]

N-((3,5-Di-t-butyl-4-hydroxyphenylthio)acetyl)-3-methylaminosydnonimine

The procedure of Example 56-B was repeated but starting with 3-(N'-allyl-N'-methylamino)-N-((3,5-di-t-butyl-4-hydroxyphenylthio)acetyl)sydnonimine to give N-((3,5-di-t-butyl-4-hydroxyphenylthio)acetyl)-3-methylaminosydnonimine.

60 MHz-NMR ($CDCl_3$) δ : 1.36 (18H, s), 3.26 (3H, s), 3.83 (2H, s), 5.12 (1H, brs), 7.15 (2H, s), 8.30 (1H, s), 9.80 (1H, brs)

[Example 63-C]

3-(N'-((S)-3-(Acetylthio)isobutyryl)-N'-methylamino-N-((3,5-di-t-butyl-4-hydroxyphenylthio)acetyl)sydnonimine

The procedure of Example 56-C was repeated but using N-((3,5-di-t-butyl-4-hydroxyphenylthio)acetyl)-3-methylaminosydnonimine as the sydnonimine derivative to give 3-(N'-((S)-3-(acetylthio)isobutyryl)-N'-methylamino-N-((3,5-di-t-butyl-4-hydroxyphenylthio)acetyl)sydnonimine as an oily substance.

60 MHz-NMR ($CDCl_3$) δ : 1.24 (3H, d), 1.35 (18H, s), 2.25 (3H, s), 2.35-2.77 (1H, m), 2.82-3.05 (2H, m), 3.55 (3H, s), 3.72 (2H, s), 5.08 (1H, s), 7.17 (2H, s) 8.07 (1H, s)

[Example 64]

N-((S)-3-(Benzoylthio)isobutyryl)-3-(N'-(2-(3,4,5-trimethoxyphenyl)ethyl)-N'-methylamino)sydnonimine

(1) Under ice cooling, 0.5 ml of conc. hydrochloric acid was added to a mixture of 0.8 g of N-(2-(3,4,5-trimethoxyphenyl)ethyl)-N-methylamine and 8 ml of water. Then a mixture of 0.3 g of sodium nitrite and 2 ml of water was further added thereto and the resultant mixture was stirred for 1 hour. Next, 5 ml of dichloromethane was added thereto and the resultant mixture was stirred for 30 minutes at room temperature. After adding water, the reaction mixture was extracted with ethyl acetate. The organic layer was washed with water and dried. Then the solvent was distilled off and the residue thus obtained was purified by silica gel column chromatography (developing solution: dichloromethane : methanol = 97 : 3) to give 0.8 g of N-(2-(3,4,5-trimethoxyphenyl)ethyl)-N-methyl-N-nitrosoamine as an oily substance. 0.17 g of lithium aluminum hydride was added at room temperature to a mixture of 0.58 g of N-(2-(3,4,5-trimethoxyphenyl)ethyl)-N-methyl-N-nitrosoamine obtained above and 10 ml of tetrahydrofuran and the resultant mixture was heated under reflux for 3 hours. After cooling, the mixture was diluted with ethyl acetate and a saturated aqueous solution of sodium chloride was added thereto followed by stirring at room temperature for 30 minutes. The organic layer was dried and the solvent was distilled off. The residue thus obtained was purified by silica gel column chromatography (developing solution: chloroform : methanol : aqueous ammonia = 200 : 10 : 1) to give 0.33 g of N-amino-N-(2-(3,4,5-trimethoxyphenyl)ethyl)-N-methylamine as an oily substance.

60 MHz-NMR ($CDCl_3$) $\delta$ : 2.48 (3H, s), 2.55-2.80 (2H, m), 2.84-3.12 (2H, m), 3.73 (3H, s), 3.74 (6H, s), 6.31 (2H, s)

(2) At room temperature, a mixture of 0.52 g of N-amino-N-(2-(3,4,5-trimethoxyphenyl)ethyl)-N-methylamine, 0.3 g of formaldehyde sodium bisulfite adduct and 5 ml of water was stirred for 30 minutes. Then 0.15 g of potassium cyanide was added thereto and the resultant mixture was stirred for 18 hours. The reaction mixture was extracted with ethyl acetate. The organic layer was washed with water and dried. Then the solvent was distilled off and the residue thus obtained was purified by silica gel column chromatography (developing solution: dichloromethane : methanol = 99 : 1) to give 0.32 g of N-(cyanomethylamino)-N-(2-(3,4,5-trimethoxyphenyl)ethyl)-N-methylamine as an oily substance.

60 MHz-NMR ($CDCl_3$) $\delta$ : 2.48-2.85 (9H, m), 3.78 (3H, s), 3.80 (6H, s), 6.39 (2H, s)

(3) Under ice cooling, 0.8 ml of a 15% solution of ethyl nitrite in ethanol was dropped into a mixture of 0.32 g of N-(cyanomethylamino)-N-(2-(3,4,5-trimethoxyphenyl)ethyl)-N-methylamine, 0.5 ml of conc. hydrochloric acid and 3 ml of ethanol and the resultant mixture was stirred for 1 hour. The obtained mixture was concentrated to give 0.41 g of 3-(N-(2-(3,4,5-trimethoxyphenyl)ethyl)-N-methylamino)sydnonimine hydrochloride. Under ice cooling, 0.13 g of 1-ethyl-3-(3-dimethylaminopropyl)carbodiimide hydrochloride, 0.1 g of 1-hydroxybenzotriazole hydrate and 0.2 ml of pyridine were added to a mixture of 0.22 g of 3-(N-(2-(3,4,5-trimethoxyphenyl)ethyl)-N-methylamino)sydnonimine hydrochloride thus obtained, 0.16 mg of (S)-3-(benzoylthio)isobutyric acid and 2 ml of N,N-dimethylformamide and the resultant mixture was stirred for 4 hours. After adding water, the mixture was extracted with ethyl acetate. The organic layer was washed with water and dried and the solvent was distilled off. The residue thus obtained was purified by silica gel column chromatography (developing solution: hexane : ethyl acetate = 1 : 4) to give 0.15 g of N-((S)-3-(benzoylthio)isobutyryl)-3-(N'-(2-(3,4,5-trimethoxyphenyl)ethyl)-N'-methylamino)sydnonimine as an oily substance.

60 MHz-NMR ($CDCl_3$) $\delta$ : 1.32 (3H, d), 2.57-3.12 (3H, m), 3.17 (3H, s), 3.25-3.46 (2H, m), 3.47-3.82 (2H, m), 3.74 (3H, s), 3.76 (6H, s), 6.30 (2H, s), 7.20-7.47 (2H, m), 7.72-7.88 (2H, m), 7.89 (1H, s)

[Example 65]

N-((S)-3-(Acetylthio)isobutyryl)-3-(N'-(2-(3,4,5-trimethoxyphenyl)ethyl)-N'-methylamino)sydnonimine

The procedure of Example 64 (3) was repeated but replacing (S)-3-(benzoylthio)isobutyric acid by (S)-3-(acetylthio)isobutyric acid to give N-((S)-3-(acetylthio)isobutyryl)-3-(N'-(2-(3,4,5-trimethoxyphenyl)ethyl)-N'-methylamino)sydnonimine as an oily substance.

60 MHz-NMR ($CDCl_3$) $\delta$ : 1.24 (3H, d), 2.25 (3H, s), 2.56-3.16 (5H, m), 3.17 (3H, s), 3.45-3.76 (2H, m), 3.75 (3H, s), 3.76 (6H, s), 6.24 (2H, s), 7.84 (1H, s)

[Example 66]

N-((3,5-Di-t-butyl-4-hydroxyphenylthio)acetyl)-3-morpholinosydnonimine

The procedure of Example 4 was repeated but starting with (3,5-di-t-butyl-4-hydroxyphenylthio)acetic acid to give N-((3,5-di-t-butyl-4-hydroxyphenylthio)acetyl)-3-morpholinosydnonimine (m.p.: 178 - 180°C).

60 MHz-NMR ($CDCl_3$) $\delta$ : 1.36 (18H, s), 3.36-3.61 (4H, m), 3.67 (2H, s), 3.67-4.00 (4H, m), 7.16 (2H, s), 8.00 (1H, s)
MS m/z : 449($M^+$+1)

[Example 67]

N-((S)-3-(Acetylthio)isobutyryl)-3-morpholinosydnonimine

The procedure of Example 4 was repeated but starting with (S)-3-(acethythio)isobutyric acid to give N-((S)-3-(acetylthio)isobutyryl)-3-morpholinosydnonimine (m.p.: 82 - 83°C).

60 MHz-NMR ($CDCl_3$) δ : 1.21 (3H, d), 2.27 (3H, s), 2.49-2.92 (1H, m), 3.02-3.24 (2H, m), 3.50-3.66 (4H, m), 3.80-4.06 (4H, m), 7.93 (1H, s)

[Example 68]

N-((S)-3-(Benzoylthio)isobutyryl)-3-(4-ethylpiperazin-1-yl)sydnonimine

A mixture of 0.07 g of N-((S)-3-(benzoylthio)isobutyryl)-3-(piperazin-1-yl)sydnonimine, 0.02 ml of ethyl iodide, 26 mg of potassium carbonate and 2 ml of N,N-dimethylformamide was stirred at 40°C for 1.5 hours. After adding water, the mixture was extracted with ethyl acetate. The organic layer was washed with a saturated aqueous solution of sodium chloride and dried and the solvent was distilled off. The residue thus obtained was purified by silica gel column chromatography (developing solution: dichloromethane : methanol = 95 : 5) to give 56.4 mg of N-((S)-3-(benzoylthio)isobutyryl)-3-(4-ethylpiperazin-1-yl)sydnonimine (m.p.: 120 - 122°C).

270 MHz-NMR ($CDCl_3$) δ : 1.12 (3H, t), 1.34 (3H, d), 2.51 (2H, q), 2.73-2.76 (4H, m), 2.83-2.98 (1H, m), 3.30-3.46 (2H, m), 3.54-3.57 (4H, m), 7.38-7.44 (2H, m), 7.51-7.56 (1H, m), 7.94-7.98 (2H, m), 8.01 (1H, s)

[Example 69]

N-((S)-3-(Benzoylthio)isobutyryl)-3-(4-isopropylpiperazin-1-yl)sydnonimine

A mixture of 0.43 g of N-((S)-3-(benzoylthio)-isobutyryl)-3-(piperazin-1-yl)sydnonimine, 0.16 ml of isopropyl iodide, 0.29 g of potassium carbonate and 10 ml of N,N-dimethylformamide was stirred at 40°C for 8 hours. After adding water, the mixture was extracted with ether. The organic layer was washed with a saturated aqueous solution of sodium chloride and dried and the solvent was distilled off. The residue thus obtained was purified by silica gel column chromatography (developing solution: dichloromethane : methanol = 95 : 5) to give 0.17 g of N-((S)-3-(benzoylthio)isobutyryl)-3-(4-isopropylpiperazin-1-yl)sydnonimine as an oily substance.

270 MHz-NMR ($CDCl_3$) δ : 1.08 (6H, d) 1.34 (3H, d), 2.76-2.98 (6H, m), 3.32-3.46 (2H, m), 3.51-3.55 (4H, m), 7.38-7.44 (2H, m), 7.51-7.56 (1H, m), 7.94-7.98 (2H, m), 8.01 (1H, s)

[Example 70]

N-((S)-3-(Benzoylthio)isobutyryl)-3-(4-(2,2,2-trifluoroethyl)piperazin-1-yl)sydnonimine

A mixture of 0.2 g of N-((S)-3-(benzoylthio)isobutyryl)-3-(piperazin-1-yl)sydnonimine, 1.1 ml of 2,2,2-trifluoroethyl trifluoromethanesulfonate (prepared from 1.1 ml of 2,2,2-trifluoroethanol, 1.2 ml of pyridine and 2.5 ml of trifluoromethanesulfonic anhydride), 0.74 ml of N,N-diisopropylethylamine and 5 ml of acetonitrile was stirred at 50°C for 17 hours. After adding water, the mixture was extracted with ethyl acetate. The organic layer was dried and the solvent was distilled off. The residue thus obtained was purified by silica gel column chromatography (developing solution: hexane : ethyl acetate = 1 : 2) to give 0.12 g of N-((S)-3-(benzoylthio)isobutyryl)-3-(4-(2,2,2-trifluoroethyl)piperazin-1-yl)sydnonimine (m.p.: 107 - 109°C).

270 MHz-NMR ($CDCl_3$) δ : 1.34 (3H, d), 2.85-2.95 (1H, m), 3.00-3.03 (4H, m), 3.07 (1H, d), 3.14 (1H, d), 3.30-3.46 (2H, m), 3.54-3.58 (4H, m), 7.38-7.44 (2H, m), 7.51-7.56 (1H, m), 7.95-7.97 (2H, m), 8.01 (1H, s)

[Example 71]

N-((S)-3-(Benzoylthio)isobutyryl)-3-(4-cyclobutylpiperazin-1-yl)sydnonimine

Under ice cooling, 35 mg of sodium cyanoborohydride was added to a mixture of 0.11 g of N-((S)-3-(benzoylthio)-isobutyryl)-3-(piperazin-1-yl)sydnonimine hydrochloride, 0.2 ml of cyclobutanone and 3 ml of methanol and the resultant mixture was stirred for 1.5 hours at 0°C. After adding water, the mixture was extracted with ethyl acetate. The organic layer was washed with water and dried and the solvent was distilled off. The residue thus obtained was purified by silica gel column chromatography (developing solution: dichloromethane : methanol : aqueous ammonia = 1,000 : 10 : 1) to give 0.1 g of N-((S)-3-(benzoylthio)isobutyryl)-3-(4-cyclobutylpiperazin-1-yl)sydnonimine as an oily substance.

270 MHz-NMR ($CDCl_3$) δ : 1.34 (3H, d), 1.67-2.18 (6H, m), 2.60-2.63 (4H, m), 2.80-3.00 (2H, m), 3.31-3.48 (2H, m), 3.52-3.55 (4H, m), 7.38-7.44 (2H, m), 7.51-7.56 (1H, m), 7.95-7.97 (2H, m), 8.01 (1H, s)

[Example 72]

N-((S)-3-(Benzoylthio)isobutyryl)-3-(4-cyclopentylpiperazin-1-yl)sydnonimine

Under ice cooling, 61 mg of sodium cyanoborohydride was added to a mixture of 0.2 g of N-((S)-3-(benzoylthio)isobutyryl)-3-(piperazin-1-yl)sydnonimine hydrochloride, 0.43 ml of cyclopentanone and 5 ml of methanol and the resultant mixture was stirred for 3.5 hours at 0°C. After adding water, the mixture was extracted with ethyl acetate. The organic layer was washed with water and dried and the solvent was distilled off. The residue thus obtained was purified by silica gel column chromatography (developing solution: dichloromethane : methanol : aqueous ammonia = 100 : 2 : 1) to give 0.16 g of N-((S)-3-(benzoylthio)isobutyryl)-3-(4-cyclopentylpiperazin-1-yl)sydnonimine as an oily substance.

270 MHz-NMR ($CDCl_3$) δ : 1.34 (3H, d), 1.57-1.95 (10H, m), 2.58-2.70 (1H, m), 2.77-2.80 (4H, m), 2.90-3.00 (1H, m), 3.33-3.48 (2H, m), 3.53-3.56 (4H, m), 7.38-7.44 (2H, m), 7.51-7.56 (1H, m), 7.95-7.97 (2H, m), 8.01 (1H, s)

[Example 73]

N-((S)-3-(Benzoylthio)isobutyryl)-3-(4-cyclohexylpiperazin-1-yl)sydnonimine

Under ice cooling, 61 mg of sodium cyanoborohydride was added to a mixture of 0.2 g of N-((S)-3-(benzoylthio)isobutyryl)-3-(piperazin-1-yl)sydnonimine hydrochloride, 0.5 ml of cyclohexanone and 5 ml of methanol and the resultant mixture was stirred for 3.5 hours at 0°C. After adding water, the mixture was extracted with ethyl acetate. The organic layer was washed with water and dried and the solvent was distilled off. The residue thus obtained was purified by silica gel column chromatography (developing solution: dichloromethane : methanol : aqueous ammonia = 100 : 2 : 1) to give 0.12 g of N-((S)-3-(benzoylthio)isobutyryl)-3-(4-cyclohexylpiperazin-1-yl)sydnonimine (m.p.: 101 - 102°C).

270 MHz-NMR ($CDCl_3$) δ : 1.09-1.33 (2H, m), 1.34 (3H, d), 1.60-1.94 (8H, m), 2.31-2.44 (1H, m), 2.83-3.00 (5H, m), 3.31-3.54 (6H, m), 7.38-7.44 (2H, m), 7.51-7.56 (1H, m), 7.94-7.97 (2H, m), 8.00 (1H, s)

[Example 74]

N-((S)-3-(Benzoylthio)isobutyryl)-3-(4-phenylpiperazin-1-yl)sydnonimine

Under ice cooling, a mixture of 0.31 g of (S)-3-(benzoylthio)isobutyric acid, 0.3 g of 1-ethyl-3-(3-dimethylaminopropyl)carbodiimide hydrochloride, 0.25 g of 1-hydroxybenzotriazole hydrate and 10 ml of N,N-dimethylformamide was stirred for 30 minutes. Next, 0.47 g of 3-(4-phenylpiperazin-1-yl)sydnonimine hydrochloride and 0.8 ml of pyridine were added thereto and the resultant mixture was stirred for 30 minutes and then at room temperature for 19 hours. After adding water, the mixture was extracted with ether. The organic layer was washed with water and dried and the solvent was distilled off. The residue thus obtained was purified by silica gel column chromatography (developing solution: chloroform : methanol : aqueous ammonia = 200 : 10 : 1) to give 0.22 g of N-((S)-3-(benzoylthio)isobutyryl)-3-(4-phenylpiperazin-1-yl)sydnonimine as an oily substance.

60 MHz-NMR ($CDCl_3$) δ : 1.34 (3H, d), 2.68-3.29 (1H, m), 3.29-3.87 (10H, m), 6.70-7.08 (3H, m), 7.08-7.69 (5H, m), 7.80-7.98 (2H, m), 8.00 (1H, s)

[Example 75]

N-((S)-3-(Benzoylthio)isobutyryl)-3-(4-((4-methoxyphenyl)methylthio)piperizin-1-yl)sydnonimine

Under ice cooling, a mixture of 0.3 g of (S)-3-(benzoylthio)isobutyric acid, 0.28 g of 1-ethyl-3-(3-dimethylaminopropyl)carbodiimide hydrochloride, 0.22 g of 1-hydroxybenzotriazole hydrate and 3 ml of N,N-dimethylformamide was stirred for 30 minutes. Next, 0.52 g of 3-(4-((4-methoxyphenyl)methylthio)piperizin-1-yl)sydnonimine hydrochloride and 0.5 ml of pyridine were added thereto and the resultant mixture was stirred for 30 minutes and then at room temperature for 48 hours. After adding water, the mixture was extracted with ethyl acetate. The organic layer was washed with water and dried and the solvent was distilled off. The residue thus obtained was purified by silica gel column chromatography

(developing solution: dichloromethane : methanol = 100 : 3) to give 0.46 g of N-((S)-3-(benzoylthio)isobutyryl)-3-(4-((4-methoxyphenyl)methylthio)piperizin-1-yl)sydnonimine as an oily substance.

270 MHz-NMR ($CDCl_3$) δ : 1.34 (3H, d), 1.85-1.94 (2H, m), 2.07-2.19 (2H, m), 2.73-2.79 (1H, m), 2.86-2.95 (1H, m), 3.24-3.46 (4H, m), 3.66-3.80 (2H, m), 3.74 (2H, s), 3.81 (3H, s), 6.86 (2H, d), 7.24 (2H, d), 7.37-7.44 (2H, m), 7.50-7.58 (1H, m), 7.93-7.98 (2H, m), 7.98 (1H, s)

[Example 76]

N-((S)-3-(Benzoylthio)isobutyryl)-3-(4-mercaptopiperizin-1-yl)sydnonimine

A mixture of 0.46 g of N-((S)-3-(benzoylthio)isobutyryl)-3-(4-((4-methoxyphenyl)methylthio)piperizin-1-yl)sydnonimine and 5 ml of trifluoroacetic acid was heated under reflux for 2 hours. After concentrating the mixture, the residue thus obtained was purified by silica gel column chromatography (developing solution: dichloromethane : methanol : acetic acid = 1,000 : 10 : 1) to give 0.3 g of N-((S)-3-(benzoylthio)isobutyryl)-3-(4-mercaptopiperizin-1- yl)sydnonimine as an oily substance.

270 MHz-NMR ($CDCl_3$) δ : 1.36 (3H, d), 1.66-1.78 (1H, m), 1.86-2.05 (2H, m), 2.22-2.37 (2H, m), 3.06-3.16 (2H, m), 3.24-3.55 (4H, m), 3.91-3.99 (2H, m), 7.39-7.47 (2H, m), 7.53-7.60 (1H, m), 7.90-7.95 (2H, m), 8.50 (1H, s)

[Example 77]

3-(4-(Acetylthio)piperizin-1-yl)-N-((S)-3-(benzoylthio)isobutyryl)sydnonimine

A mixture of 0.15 g of N-((S)-3-(benzoylthio)isobutyryl)-3-(4-mercaptopiperizin-1-yl)sydnonimine, 1 ml of pyridine and 1 ml of acetic anhydride was stirred for 3 hours at room temperature. After concentrating the mixture, the residue thus obtained was purified by silica gel column chromatography (developing solution: dichloromethane : methanol = 100 : 3) to give 0.1 g of 3-(4-(acetylthio)piperizin-1-yl)-N-((S)-3-(benzoylthio)isobutyryl)sydnonimine (m.p.: 133 - 134°C).

270 MHz-NMR ($CDCl_3$) δ : 1.35 (3H, d), 1.89-2.18 (2H, m), 2.19-2.37 (2H, m), 2.36 (3H, s), 2.97-3.08 (1H, m), 3.24-3.55 (4H, m), 3.65-3.79 (3H, m), 7.35-7.49 (2H, m), 7.50-7.58 (1H, m), 7.92-7.98 (2H, m), 8.22 (1H, s)

[Example 78]

N-((S)-3-(Acetylthio)isobutyryl)-3-(4-methylpiperazin-1-yl)sydnonimine

At room temperature, 0.08 g of sodium cyanoborohydride was added to a mixture of 0.2 g of N-((S)-3-(acetylthio)isobutyryl-3-(piperazin-1-yl)sydnonimine hydrobromide, 0.1 ml of formalin and 2 ml of methanol and the resultant mixture was stirred for 15 minutes. After adding water, the mixture was extracted with dichloromethane. The organic layer was washed with water and dried and the solvent was distilled off. The residue thus obtained was purified by silica gel column chromatography (developing solution: chloroform : methanol : aqueous ammonia = 500 : 10 : 1) to give 0.00 g of N-((S)-3-(acetylthio)isobutyryl)-3-(4-methylpiperazin-1-yl)sydnonimine as an oily substance.

270 MHz-NMR ($CDCl_3$) δ : 1.26 (3H, d), 2.30 (3H, s), 2.38 (3H, s), 2.68-2.84 (5H, m), 3.08-3.24 (2H, m), 3.53-3.58 (4H, m), 8.00 (1H, s)

[Example 79]

N-((S)-3-(Acetylthio)isobutyryl)-3-(4-ethylpiperazin-1-yl)sydnonimine

The procedure of Example 68 was repeated but starting with N-((S)-3-(acethythio)isobutyryl-3-(piperazin-1-yl)sydnonimine hydrochloride to give N-((S)-3-(acetylthio)isobutyryl)-3-(4-ethylpiperazin-1-yl)sydnonimine as an oily substance.

270 MHz-NMR ($CDCl_3$) δ : 1.12 (3H, t), 1.27 (3H, d), 2.30 (3H, s), 2.51 (2H, q), 2.71-2.89 (5H, m), 3.09-3.24 (2H, m), 3.52-3.57 (4H, m), 7.99 (1H, s)

[Example 80]

N-((S)-3-(Acetylthio)isobutyryl)-3-(4-isopropylpiperazin-1-yl)sydnonimine

The procedure of Example 69 was repeated but starting with N-((S)-3-(acethythio)isobutyryl-3-(piperazin-1-yl)sydnonimine hydrochloride to give N-((S)-3-(acetylthio)isobutyryl)-3-(4-isopropylpiperazin-1-yl)sydnonimine as an oily substance.

270 MHz-NMR ($CDCl_3$) δ : 1.08 (6H, d), 1.28 (3H, d), 2.30 (3H, s), 2.73-2.96 (6H, m), 3.09-3.24 (2H, m), 3.50-3.55 (4H, m), 7.98 (1H, s)

[Example 81]

N-((3,5-Di-t-butyl-4-hydroxyphenylthio)acetyl]-3-(4-((4-methoxyphenyl)methylthio)piperizin-1-yl)sydnonimine

The procedure of Example 75 was repeated but starting with (3,5-di-t-butyl-4-hydroxyphenylthio)acetic acid to give N-((3,5-di-t-butyl-4-hydroxyphenylthio)acetyl)-3-(4-((4-methoxyphenyl)methylthio)piperizin-1-yl)sydnonimine as an oily substance.

270 MHz-NMR ($CDCl_3$) δ : 1.40 (18, s), 1.82-1.97 (2H, m), 2.11-2.18 (2H, m), 2.73-2.81 (1H, m), 3.25-3.35 (2H, m), 3.68-3.80 (2H, m), 3.74 (2H, s), 3.80 (3H, s), 3.80 (2H, s), 5.15 (1H, s), 6.86 (2H, d), 7.24 (2H, d), 7.31 (2H, s), 7.98 (1H, s)

[Example 82]

N-((3,5-Di-t-butyl-4-hydroxyphenylthio)acetyl)-3-(4-mercaptopiperizin-1-yl)sydnonimine

The procedure of Example 76 was repeated but starting with N-((3,5-di-t-butyl-4-hydroxyphenylthio)acetyl)-3-(4-((4-methoxyphenyl)methylthio)piperizin-1-yl)sydnonimine to give N-((3,5-di-t-butyl-4-hydroxyphenylthio)acetyl)-3-(4-mercaptopiperizin-1-yl)sydnonimine as an oily substance.

270 MHz-NMR ($CDCl_3$) δ : 1.40 (18H, s), 1.64-1.72 (1H, brd), 1.88-2.03 (2H, m), 2.28-2.36 (2H, m), 3.05-3.19 (1H, m), 3.39-3.50 (2H, m), 3.75-3.97 (2H, m), 3.85 (2H, s), 5.24 (1H, brs), 7.30 (2H, s), 8.40 (1H, s)

[Example 83]

3-(4-Acetylthiopiperizin-1-yl)-N-((3,5-di-t-butyl-4-hydroxyphenylthio)acetyl)sydnonimine

The procedure of Example 77 was repeated but starting with N-((3,5-di-t-butyl-4-hydroxyphenylthio)acetyl)-3-(4-mercaptopiperizin-1-yl)sydnonimine to give 3-(4-acetylthiopiperizin-1-yl)-N-((3,5-di-t-butyl-4-hydroxyphenylthio)acetyl)sydnonimine (m.p.: 141 - 143°C).

270 MHz-NMR ($CDCl_3$) δ : 1.40 (18H, s), 1.93-2.04 (2H, m), 2.21-2.33 (2H, m), 2.36 (3H, s), 3.39-3.50 (2H, m), 3.65-3.79 (3H, m), 3.80 (2H, s), 5.15 (1H, s), 7.32 (2H, s), 8.00 (1H, s)

[Example 84]

3-(4-(Acetylthio)piperizin-1-yl)-N-((3,4,5-trimethoxyphenyl)acetyl)sydnonimine

A mixture of 0.29 g of 3-(4-((4-methoxyphenyl)methylthio)piperizin-1-yl)sydnonimine hydrochloride and 3 ml of trifluoroacetic acid was heated under reflux for 3 hours. After concentrating the mixture, 1 ml of acetyl chloride was added thereto and the resultant mixture was stirred for 3 hours at room temperature. Then the mixture was concentrated and dissolved in 3 ml of N,N-dimethyl-formamide followed by the addition of 0.19 g of (3,4,5-trimethoxyphenyl)acetic acid, 0.3 ml of pyridine, 0.17 g of 1-ethyl-3-(3-dimethylaminopropyl)carbodiimide hydrochloride and 0.13 g of 1-hydroxybenzotriazole hydrate at 0°C. The mixture thus obtained was stirred for 13 hours at room temperature. After adding water, the mixture was extracted with ethyl acetate. The organic layer was washed with water and dried. Then the solvent was distilled off and the residue thus obtained was purified by silica gel column chromatography (developing solution: dichloromethane : methanol = 100 : 2) to give 0.1 g of 3-(4-(acetylthio)piperizin-1-yl)-N-((3,4,5-trimethoxyphenyl)acetyl)sydnonimine as an oily substance.

270 MHz-NMR ($CDCl_3$) δ : 1.88-2.08 (2H, m), 2.18-2.33 (2H, m), 2.35 (3H, s), 3.36-3.48 (2H, m), 3.60-3.75 (3H, m), 3.71 (2H, s), 3.81 (3H, s), 3.85 (6H, s), 6.59 (2H, s), 7.98 (1H, s)

[Example 85]

N-(Acetylthioacetyl)-3-(4-ethylpiperazin-1-yl)sydnonimine

The procedure of Example 68 was repeated but starting with N-(acetylthioacetyl)-3-(piperazin-1-yl)sydnonimine hydrobromide to give N-(acetylthioacetyl)-3-(4-ethylpiperazin-1-yl)sydnonimine (m.p.: 111 - 112°C).

270 MHz-NMR ($CDCl_3$) δ : 1.12 (3H, t), 2.38 (3H, s), 2.51 (2H, q), 2.72-2.76 (4H, m), 3.53-3.57 (4H, m), 3.90 (2H, s), 7.97 (1H, s)

[Example 86]

N-(Acetylthioacetyl)-3-(4-isopropylpiperazin-1-yl)sydnonimine

The procedure of Example 69 was repeated but starting with N-(acetylthioacetyl)-3-(piperazin-1-yl)sydnonimine hydrobromide to give N-(acetylthioacetyl)-3-(4-isopropylpiperazin-1-yl)sydnonimine (m.p.: 111 - 113°C).

270 MHz-NMR ($CDCl_3$) δ : 1.08 (6H, d), 2.38 (3H, s), 2.76-2.86 (5H, m), 3.51-3.55 (4H, m), 3.90 (2H, s), 7.96 (1H, s)

[Example 87]

N-(Benzoylthioacetyl)-3-(4-ethylpiperazin-1-yl)sydnonimine

The procedure of Example 68 was repeated but starting with N-(benzoylthioacetyl)-3-(piperazin-1-yl)sydnonimine hydrochloride to give N-(benzoylthioacetyl)-3-(4-ethylpiperazin-1-yl)sydnonimine (m.p.: 143 - 144°C).

270 MHz-NMR ($CDCl_3$) δ : 1.11 (3H, t), 2.51 (2H, q), 2.72-2.76 (4H, m), 3.53-3.57 (4H, m), 4.09 (2H, s), 7.41-7.47 (2H, m), 7.53-7.59 (1H, m), 8.00 (1H, s), 8.00-8.03 (2H, m)

[Example 88]

N-(Benzoylthioacetyl)-3-(4-isopropylpiperazin-1-yl)sydnonimine

The procedure of Example 69 was repeated but starting with N-(benzoylthioacetyl)-3-(piperazin-1-yl)sydnonimine hydrochloride to give N-(benzoylthioacetyl)-3-(4-isopropylpiperazin-1-yl)sydnonimine (m.p.: 132 - 134°C).

270 MHz-NMR ($CDCl_3$) δ : 1.07 (6H, d), 2.76-2.87 (5H, m), 3.51-3.55 (4H, m), 4.09 (2H, s), 7.41-7.46 (2H, m), 7.53-7.59 (1H, m), 7.99 (1H, s), 7.99-8.03 (2H, m)

[Example 89]

N-(Benzoylthioacetyl)-3-(4-(2,2,2-trifluoroethyl)piperazin-1-yl)sydnonimine

The procedure of Example 70 was repeated but starting with N-(benzoylthioacetyl)-3-(piperazin-1-yl)sydnonimine hydrochloride to give N-(benzoylthioacetyl)-3-(4-(2,2,2-trifluoroethyl)piperazin-1-yl)sydnonimine (m.p.: 146 - 147°C).

270 MHz-NMR ($CDCl_3$) δ : 3.00-3.03 (4H, m), 3.07 (1H, d), 3.14 (1H, d), 3.54-3.58 (4H, m), 4.09 (2H, s), 7.41-7.47 (2H, m), 7.53-7.59 (1H, m), 8.00 (1H, s), 7.99-8.02 (2H, m)

[Example 90]

N-(Benzoylthioacetyl)-3-(4-cyclobutylpiperazin-1-yl)sydnonimine

The procedure of Example 71 was repeated but starting with N-(benzoylthioacetyl)-3-(piperazin-1-yl)sydnonimine hydrochloride to give N-(benzoylthioacetyl)-3-(4-cyclobutylpiperazin-1-yl)sydnonimine (m.p.: 151 - 153°C).

270 MHz-NMR ($CDCl_3$) δ : 1.69-2.15 (6H, m), 2.60-2.63 (4H, m), 2.81-2.93 (1H, m), 3.52-3.55 (4H, m), 4.09 (2H, s), 7.41-7.46 (2H, m), 7.53-7.58 (1H, m), 8.00 (1H, s), 8.00-8.02 (2H, m)

[Example 91]

N-(Benzoylthioacetyl)-3-(4-cyclopentylpiperazin-1-yl)sydnonimine

The procedure of Example 72 was repeated but starting with N-(benzoylthioacetyl)-3-(piperazin-1-yl)sydnonimine hydrochloride to give N-(benzoylthioacetyl)-3-(4-cyclopentylpiperazin-1-yl)sydnonimine (m.p.: 136 - 138°C).

270 MHz-NMR ($CDCl_3$) δ : 1.35-1.98 (8H, m), 2.57-2.69 (1H, m), 2.76-2.80 (4H, m), 3.52-3.56 (4H, m), 4.09 (2H, s), 7.41-7.46 (2H, m), 7.53-7.58 (1H, m), 8.00 (1H, s), 8.00-8.02 (2H, m)

[Example 92]

N-(Benzoylthioacetyl)-3-(4-cyclohexylpiperazin-1-yl)sydnonimine

The procedure of Example 73 was repeated but starting with N-(benzoylthioacetyl)-3-(piperazin-1-yl)sydnonimine hydrochloride to give N-(benzoylthioacetyl)-3-(4-cyclohexylpiperazin-1-yl)sydnonimine (m.p.: 141 - 143°C).

270 MHz-NMR ($CDCl_3$) δ : 1.09-1.93 (10H, m), 2.32-2.44 (1H, m), 2.83-2.87 (4H, m), 3.50-3.54 (4H, m), 4.09 (2H, s), 7.41-7.46 (2H, m), 7.53-7.58 (1H, m), 7.99 (1H, s), 7.99-8.03 (2H, m)

[Example 93]

N-(Benzoylthioacetyl)-3-(4-phenylpiperazin-1-yl)sydnonimine

The procedure of Example 74 was repeated but starting with (benzoylthio)acetic acid to give N-(benzoylthioacetyl)-3-(4-phenylpiperazin-1-yl)sydnonimine (m.p.: 160 - 162°C).

270 MHz-NMR ($CDCl_3$) δ : 3.46-3.49 (4H, m), 3.66-3.70 (4H, m), 4.10 (2H, s), 6.95-7.00 (3H, m), 7.26-7.60 (5H, m), 8.00-8.04 (2H, m), 8.07 (1H, s)

[Example 94]

N-(2-(Acetylthio)isobutyryl)-3-(4-methylpiperazin-1-yl)sydnonimine

The procedure of Example 1 was repeated but starting with 2-(acetylthio)isobutyric acid to give N-(2-(acetylthio)isobutyryl)-3-(4-methylpiperazin-1-yl)sydnonimine (m.p.: 128 - 130°C).

270 MHz-NMR ($CDCl_3$) δ : 1.66 (6H, s), 2.25 (3H, s), 2.37 (3H, s), 2.67-2.75 (4H, m), 3.49-3.57 (4H, m), 7.99 (1H, s)

[Example 95]

N-(2-(Acetylthio)isobutyryl)-3-(4-benzylpiperazin-1-yl)sydnonimine

The procedure of Example 17 was repeated but starting with 2-(acetylthio)isobutyric acid to give N-(2-(acetylthio)isobutyryl)-3-(4-benzylpiperazin-1-yl)sydnonimine as an oily substance.

60 MHz-NMR ($CDCl_3$) δ : 1.66 (6H, s), 2.23 (3H, s), 2.59-2.88 (4H, m), 3.35-3.66 (4H, m), 3.56 (2H, s), 7.25 (5H,

s), 7.93 (1H, s)

[Example 96]

N-(2-(Acetylthio)isobutyryl)-3-(4-benzyloxycarbonylpiperazin-1-yl)sydnonimine

The procedure of Example 19-A was repeated but starting with N-(2-(acetylthio)isobutyryl)-3-(4-benzylpiperazin-1-yl)sydnonimine to give N-(2-(acetylthio)isobutyryl)-3-(4-benzyloxycarbonylpiperazin-1-yl)sydnonimine as an oily substance.

270 MHz-NMR ($CDCl_3$) δ : 1.65 (6H, s), 2.24 (3H, s), 3.44-3.50 (4H, m), 3.78-3.83 (4H, m), 5.17 (2H, s), 7.37 (5H, s), 8.00 (1H, s)

[Example 97]

N-(2-(Acetylthio)isobutyryl)-3-(piperazin-1-yl)sydnonimine hydrochloride

The procedure of Example 24-C was repeated but starting with N-(2-(acetylthio)isobutyryl)-3-(4-benzyloxycarbonylpiperazin-1-yl)sydnonimine to give N-(2-(acetylthio)isobutyryl)-3-(piperazin-1-yl)sydnonimine hydrochloride as an amorphous substance.

270 MHz-NMR (DMSO-$d_6$) δ : 1.58 (6H, s), 2.24 (3H, s), 3.34-3.48 (4H, m), 3.86-3.97 (4H, m), 8.84 (1H, s)

[Example 98]

N-(2-(Acetylthio)isobutyryl)-3-(4-ethylpiperazin-1-yl)sydnonimine

The procedure of Example 68 was repeated but starting with N-(2-(acetylthio)isobutyryl)-3-(piperazin-1-yl)sydnonimine hydrochloride to give N-(2-(acetylthio)isobutyryl)-3-(4-ethylpiperazin-1-yl)sydnonimine as an oily substance.

270 MHz-NMR ($CDCl_3$) δ : 1.12 (3H, t), 1.66 (6H, s), 2.25 (3H, s), 2.51 (2H, q), 2.71-2.76 (4H, m), 3.52-3.56 (4H, m), 8.00 (1H, s)

[Example 99]

N-(2-(Acetylthio)isobutyryl)-3-(4-isopropylpiperazin-1-yl)sydnonimine

The procedure of Example 69 was repeated but starting with N-(2-(acetylthio)isobutyryl)-3-(piperazin-1-yl)sydnonimine hydrochloride to give N-(2-(acetylthio)isobutyryl)-3-(4-isopropylpiperazin-1-yl)sydnonimine as an oily substance.

270 MHz-NMR ($CDCl_3$) δ : 1.08 (6H, d), 1.66 (6H, s), 2.25 (3H, s), 2.75-2.88 (5H, m), 3.49-3.54 (4H, m), 7.99 (1H, s)

[Example 100]

N-(2-(Benzoylthio)isobutyryl)-3-(4-methylpiperazin-1-yl)sydnonimine

(1) Under a nitrogen atmosphere, a mixture of 4 g of 2-(acetylthio)isobutyric acid, 4 g of sodium hydroxide and 40 ml of water was stirred at 0°C for 30 minutes. Then 3 ml of benzoyl chloride was added thereto and the resultant mixture was stirred for additional 30 minutes. After adding ice water, the reaction mixture was acidified with hydrochloric acid. The crystals thus formed were taken up by filtration and dissolved in dichloromethane. The organic layer was washed with water and dried. After distilling off the solvent, the obtained residue was purified by silica gel column chromatography (developing solution: chloroform : methanol : acetic acid = 100 : 2 : 1) to give 1.6 g of 2-(benzoylthio)isobutyric acid.

270 MHz-NMR ($CDCl_3$) δ : 1.74 (6H, s), 7.25-7.61 (3H, m), 7.78-7.99 (2H, m)

(2) The procedure of Example 1 was repeated but starting with 2-(benzoylthio)isobutyric acid to give N-(2-(benzoylthio)isobutyryl)-3-(4-methylpiperazin-1-yl)sydnonimine (m.p.: 155 - 157°C).

270 MHz-NMR ($CDCl_3$) δ : 1.76 (6H, s), 2.37 (3H, s), 2.65-2.74 (4H, m), 3.47-3.58 (4H, m), 7.35-7.41 (2H, m), 7.42-7.56 (1H, m), 7.86-7.97 (2H, m), 8.04 (1H, s)

[Example 101]

N-(2-(Benzoylthio)isobutyryl)-3-(4-benzylpiperazin-1-yl)sydnonimine

The procedure of Example 17 was repeated but starting with 2-(benzoylthio)isobutyric acid to give N-(2-(benzoylthio)isobutyryl)-3-(4-benzylpiperazin-1-yl)sydnonimine as an oily substance.

270 MHz-NMR ($CDCl_3$) δ : 1.76 (6H, s), 2.68-2.77 (4H, m), 3.46-3.55 (4H, m), 3.58 (2H, s), 7.28 (5H, s), 7.30-7.43 (2H, m), 7.44-7.57 (1H, m), 7.86-7.92 (2H, m), 8.02 (1H, s)

[Example 102]

N-(2-(Benzoylthio)isobutyryl)-3-(4-benzyloxycarbonylpiperazin-1-yl)sydnonimine

The procedure of Example 19-A was repeated but starting with N-(2-(benzoylthio)isobutyryl)-3-(4-benzylpiperazin-1-yl)sydnonimine to give N-(2-(benzoylthio)isobutyryl)-3-(4-benzyloxycarbonylpiperazin-1-yl)sydnonimine as an oily substance.

270 MHz-NMR ($CDCl_3$) δ : 1.75 (6H, s), 3.43-3.49 (4H, m), 3.78-3.82 (4H, m), 5.16 (2H, s), 7.36-7.42 (2H, m), 7.37 (5H, s), 7.45-7.58 (1H, m), 7.92 (2H, dd), 8.05 (1H, s)

[Example 103]

N-(2-(Benzoylthio)isobutyryl)-3-(piperazin-1-yl)sydnonimine hydrobromide

The procedure of Example 19-B was repeated but starting with N-(2-(benzoylthio)isobutyryl)-3-(4-benzyloxycarbonylpiperazin-1-yl)sydnonimine to give N-(2-(benzoylthio)isobutyryl)-3-(piperazin-1-yl)sydnonimine hydrobromide as an amorphous substance.

270 MHz-NMR (DMSO-$d_6$) δ : 1.71 (6H, s), 3.48 (4H, brs), 3.95 (4H, brs), 7.54-7.60 (2H, m), 7.64-7.72 (1H, m), 7.88 (2H, d), 9.14 (1H, s)

[Example 104]

N-(2-(Benzoylthio)isobutyryl)-3-(4-ethylpiperazin-1-yl)sydnonimine

The procedure of Example 68 was repeated but starting with N-(2-(benzoylthio)isobutyryl)-3-(piperazin-1-yl)sydnonimine hydrobromide to give N-(2-(benzoylthio)isobutyryl)-3-(4-ethylpiperazin-1-yl)sydnonimine as an oily substance.

270 MHz-NMR ($CDCl_3$) δ : 1.11 (3H, t), 1.76 (6H, s), 2.50 (2H, q), 2.64-2.77 (4H, m), 3.49-3.58 (4H, m), 7.33-7.42 (2H, m), 7.44-7.56 (1H, m), 7.90-7.95 (2H, m), 8.03 (1H, s)

[Example 105]

N-(2-(Benzoylthio)isobutyryl)-3-(4-isopropylpiperazin-1-yl)sydnonimine

The procedure of Example 69 was repeated but starting with N-(2-(benzoylthio)isobutyryl)-3-(piperazin-1-yl)sydnonimine hydrobromide to give N-(2-(benzoylthio)isobutyryl)-3-(4-isopropylpiperazin-1-yl)sydnonimine as an oily substance.

270 MHz-NMR ($CDCl_3$) δ : 1.07 (6H, d), 1.77 (6H, s), 2.74-2.83 (5H, m), 3.48-3.54 (4H, m), 7.35-7.41 (2H, m), 7.48-7.54 (1H, m), 7.90-7.95 (2H, m), 8.03 (1H, s)

[Example 106]

N-(3-Acetylthio-2,2-dimethylpropionyl)-3-(4-methylpiperazin-1-yl)sydnonimine

The procedure of Example 1 was repeated but starting with 3-acetylthio-2,2-dimethylpropionic acid to give N-(3-acetylthio-2,2-dimethylpropionyl)-3-(4-methylpiperazin-1-yl)sydnonimine as an oily substance.

270 MHz-NMR ($CDCl_3$) δ : 1.27 (6H, s), 2.31 (3H, s), 2.37 (3H, s), 2.68-2.73 (4H, m), 3.25 (2H, s), 3.50-3.56 (4H, m), 7.97 (1H, s)

[Example 107]

N-(3-Acetylthio-2,2-dimethylpropionyl)-3-(4-benzylpiperazin-1-yl)sydnonimine

The procedure of Example 17 was repeated but starting with 3-acetylthio-2,2-dimethylpropionic acid to give N-(3-acetylthio-2,2-dimethylpropionyl)-3-(4-benzylpiperazin-1-yl)sydnonimine as an oily substance.

270 MHz-NMR ($CDCl_3$) δ : 1.27 (6H, s), 2.30 (3H, s), 2.72-2.75 (4H, m), 3.25 (2H, s), 3.49-3.55 (4H, m), 3.60 (2H, s), 7.29-7.38 (5H, m), 7.97 (1H, s)

[Example 108]

N-(3-Acetylthio-2,2-dimethylpropionyl)-3-(4-benzyloxycarbonylpiperazin-1-yl)sydnonimine

The procedure of Example 19-A was repeated but starting with N-(3-acetylthio-2,2-dimethylpropionyl)-3-(4-benzylpiperazin-1-yl)sydnonimine to give N-(3-acetylthio-2,2-dimethylpropionyl)-3-(4-benzyloxycarbonylpiperazin-1-yl)sydnonimine as an oily substance.

270 MHz-NMR ($CDCl_3$) δ : 1.27 (6H, s), 2.31 (3H, s), 3.25 (2H, s), 3.43-3.51 (4H, m), 3.78-3.84 (4H, m), 5.17 (2H, s), 7.37 (5H, s), 7.99 (1H, s)

[Example 109]

N-(3-Acetylthio-2,2-dimethylpropionyl)-3-(piperazin-1-yl)sydnonimine hydrochloride

The procedure of Example 24-C was repeated but starting with N-(3-acetylthio-2,2-dimethylpropionyl)-3-(4-benzyloxycarbonylpiperazin-1-yl)sydnonimine to give N-(3-acetylthio-2,2-dimethylpropionyl)-3-(piperazin-1-yl)sydnonimine hydrochloride as an amorphous substance.

270 MHz-NMR (DMSO-$d_6$) δ : 1.27 (6H, s), 2.50 (3H, s), 3.33-3.48 (6H, m), 3.91-4.05 (4H, m), 9.09 (1H, brs), 9.79 (1H, brs)

[Example 110]

N-(3-Acetylthio-2,2-dimethylpropionyl)-3-(4-ethylpiperazin-1-yl)sydnonimine

The procedure of Example 68 was repeated but starting with N-(3-acetylthio-2,2-dimethylpropionyl)-3-(piperazin-1-yl)sydnonimine hydrochloride to give N-(3-acetylthio-2,2-dimethylpropionyl)-3-(4-ethylpiperazin-1-yl)sydnonimine as an oily substance.

270 MHz-NMR ($CDCl_3$) δ : 1.12 (3H, t), 1.27 (6H, s), 2.31 (3H, s), 2.51 (2H, q), 2.71-2.83 (4H, m), 3.25 (2H, s), 3.51-3.57 (4H, m), 7.97 (1H, s)

[Example 111]

N-(3-Acetylthio-2,2-dimethylpropionyl)-3-(4-isopropylpiperazin-1-yl)sydnonimine

The procedure of Example 69 was repeated but starting with N-(3-acetylthio-2,2-dimethylpropionyl)-3-(piperazin-1-yl)sydnonimine hydrochloride to give N-(3-acetylthio-2,2-dimethylpropionyl)-3-(4-isopropylpiperazin-1-yl)sydnonimine as an oily substance.

270 MHz-NMR ($CDCl_3$) δ : 1.08 (6H, d), 1.27 (6H, s), 2.31 (3H, s), 2.76-2.86 (4H, m), 3.25 (2H, s), 3.47-3.55 (4H, m), 7.98 (1H, s)

[Example 112]

N-(3-Benzoylthio-2,2-dimethylpropionyl)-3-(4-methylpiperazin-1-yl)sydnonimine

The procedure of Example 1 was repeated but starting with 3-benzoylthio-2,2-dimethylpropionic acid to give N-(3-benzoylthio-2,2-dimethylpropionyl)-3-(4-methylpiperazin-1-yl)sydnonimine (m.p.: 102 - 103°C).

60 MHz-NMR ($CDCl_3$) δ : 1.37 (6H, s), 2.36 (3H, s), 2.58-2.88 (4H, m), 3.38-3.69 (4H, m), 3.47 (2H, s), 7.18-7.60 (3H, m), 7.86-8.17 (2H, m), 7.99 (1H, s)

[Example 113]

N-(3-Benzoylthio-2,2-dimethylpropionyl)-3-(4-benzylpiperazin-1-yl)sydnonimine

The procedure of Example 17 was repeated but starting with 3-benzoylthio-2,2-dimethylpropionic acid to give N-(3-benzoylthio-2,2-dimethylpropionyl)-3-(4-benzylpiperazin-1-yl)sydnonimine as an oily substance.

60 MHz-NMR ($CDCl_3$) δ : 1.46 (6H, s), 2.57-2.92 (4H, m), 3.47-3.70 (4H, m), 3.50 (2H, s), 3.58 (2H, s), 7.16-7.55 (3H, m), 7.28 (5H, s), 7.84-8.17 (2H, m) 7.93 (1H, s)

[Example 114]

N-(3-Benzoylthio-2,2-dimethylpropionyl)-3-(4-benzyloxycarbonylpiperazin-1-yl)sydnonimine

The procedure of Example 19-A was repeated but starting with N-(3-benzoylthio-2,2-dimethylpropionyl)-3-(4-benzylpiperazin-1-yl)sydnonimine to give N-(3-benzoylthio-2,2-dimethylpropionyl)-3-(4-benzyloxycarbonylpiperazin-1-yl)sydnonimine as an oily substance.

270 MHz-NMR ($CDCl_3$) δ : 1.34 (6H, s), 3.43-3.50 (4H, m), 3.47 (2H, s), 3.78-3.83 (4H, m), 5.17 (2H, s), 7.37 (5H, s), 7.36-7.44 (2H, m), 7.48-7.57 (1H, m), 7.98 (2H, d), 8.00 (1H, s)

[Example 115]

N-(3-Benzoylthio-2,2-dimethylpropionyl)-3-(piperazin-1-yl)sydnonimine hydrobromide

The procedure of Example 19-B was repeated but starting with N-(3-benzoylthio-2,2-dimethylpropionyl)-3-(4-benzyloxycarbonylpiperazin-1-yl)sydnonimine to give N-(3-benzoylthio-2,2-dimethylpropionyl)-3-(piperazin-1-yl)sydnonimine hydrobromide as an amorphous substance.

270 MHz-NMR (DMSO-$d_6$) δ : 1.35 (6H, s), 3.47 (6H, brs), 4.01 (4H, brs), 7.52-7.58 (2H, m), 7.66-7.72 (1H, m), 7.90 (2H, d), 9.29 (1H, s)

[Example 116]

N-(3-Benzoylthio-2,2-dimethylpropionyl)-3-(4-ethylpiperazin-1-yl)sydnonimine

The procedure of Example 68 was repeated but starting with N-(3-benzoylthio-2,2-dimethylpropionyl)-3-(piperazin-1-yl)sydnonimine hydrobromide to give N-(3-benzoylthio-2,2-dimethylpropionyl)-3-(4-ethylpiperazin-1-yl)sydnonimine as an oily substance.

270 MHz-NMR ($CDCl_3$) δ : 1.11 (3H, t), 1.34 (6H, s), 2.50 (2H, q), 2.70-2.78 (4H, m), 3.48 (2H, s), 3.52-3.57 (4H, m), 7.32-7.44 (2H, m), 7.47-7.57 (1H, m), 7.95-8.01 (2H, m), 7.99 (1H, s)

[Example 117]

N-(3-Benzoylthio-2,2-dimethylpropionyl)-3-(4-isopropylpiperazin-1-yl)sydnonimine

The procedure of Example 69 was repeated but starting with N-(3-benzoylthio-2,2-dimethylpropionyl)-3-(piperazin-1-yl)sydnonimine hydrobromide to give N-(3-benzoylthio-2,2-dimethylpropionyl)-3-(4-isopropylpiperazin-1-yl)sydnonimine as an oily substance.

270 MHz-NMR ($CDCl_3$) δ : 1.08 (6H, d), 1.34 (6H, s), 2.75-2.84 (5H, m), 3.48 (2H, s), 3.49-3.56 (4H, m), 7.37-7.45 (2H, m), 7.48-7.58 (1H, m), 7.95-8.01 (2H, m), 7.99 (1H, s)

[Example 118]

N-(3-(Acetylthio)propionyl)-3-(4-methylpiperazin-1-yl)sydnonimine

The procedure of Example 1 was repeated but starting with 3-(acetylthio)propionic acid to give N-(3-(acetylthio)propionyl)-3-(4-methylpiperazin-1-yl)sydnonimine (m.p.: 134 - 136°C).

270 MHz-NMR ($CDCl_3$) δ : 2.31 (3H, s), 2.38 (3H, s), 2.70-2.73 (4H, m), 2.79 (2H, t), 3.20 (2H, t), 3.53-3.56 (4H, m), 7.98 (1H, s)

[Example 119]

N-(3-(Acetylthio)propionyl)-3-(4-benzylpiperazin-1-yl)sydnonimine

The procedure of Example 17 was repeated but starting with 3-(acetylthio)propionic acid to give N-(3-(acetylthio)propionyl)-3-(4-benzylpiperazin-1-yl)sydnonimine (m.p.: 128°C).

270 MHz-NMR ($CDCl_3$) δ : 2.30 (3H, s), 2.73-2.81 (6H, m), 3.20 (2H, t), 3.52-3.56 (4H, m), 3.60 (2H, s), 7.26-7.40 (5H, m), 7.97 (1H, s)

[Example 120]

N-(3-(Acetylthio)propionyl)-3-(4-benzyloxycarbonylpiperazin-1-yl)sydnonimine

The procedure of Example 19-A was repeated but starting with N-(3-(acetylthio)propionyl)-3-(4-benzylpiperazin-1-yl)sydnonimine to give N-(3-(acetylthio)propionyl)-3-(4-benzyloxycarbonylpiperazin-1-yl)sydnonimine.

270 MHz-NMR ($CDCl_3$) δ : 2.31 (3H, s), 2.79 (2H, t), 3.20 (2H, t), 3.47-3.50 (4H, m), 3.80-3.84 (4H, m), 5.17 (2H, s), 7.37 (5H, m), 8.00 (1H, s)

[Example 121]

N-(3-(Acetylthio)propionyl)-3-(piperazin-1-yl)sydnonimine hydrobromide

The procedure of Example 19-B was repeated but starting with N-(3-(acetylthio)propionyl)-3-(4-benzyloxycarbonylpiperazin-1-yl)sydnonimine to give 2.23 g of N-(3-(acetylthio)propionyl)-3-(piperazin-1-yl)sydnonimine hydrobromide.

270 MHz-NMR (DMSO-$d_6$) δ : 2.34 (3H, s), 2.88 (2H, t), 3.13 (2H, t), 3.43-3.54 (4H, m), 3.96-4.06 (4H, m), 9.18 (1H, brs), 9.33(1H, s)

[Example 122]

N-(3-(Acetylthio)propionyl)-3-(4-ethylpiperazin-1-yl)sydnonimine

The procedure of Example 68 was repeated but starting with N-(3-(acetylthio)propionyl)-3-(piperazin-1-yl)sydnonimine hydrobromide to give N-(3-(acetylthio)propionyl)-3-(4-ethylpiperazin-1-yl)sydnonimine (m.p.: 103.5 - 105°C).

270 MHz-NMR ($CDCl_3$) δ : 1.12 (3H, t), 2.31 (3H, s), 2.51 (2H, q), 2.70-2.82 (6H, m), 3.20 (2H, t), 3.53-3.57 (4H, m), 7.98 (1H, s)

[Example 123]

N-(3-(Acetylthio)propionyl)-3-(4-isopropylpiperazin-1-yl)sydnonimine

The procedure of Example 69 was repeated but starting with N-(3-(acetylthio)propionyl)-3-(piperazin-1-yl)sydnonimine hydrobromide to give N-(3-(acetylthio)propionyl)-3-(4-isopropylpiperazin-1-yl)sydnonimine (m.p.: 116 - 118°C).

270 MHz-NMR ($CDCl_3$) δ : 1.08 (6H, d), 2.31 (3H, s), 2.77-2.87 (7H, m), 3.21 (2H, t), 3.51-3.55 (4H, m), 7.97 (1H, s)

[Example 124]

N-(3-(Benzoylthio)propionyl)-3-(4-methylpiperazin-1-yl)sydnonimine

The procedure of Example 1 was repeated but starting with 3-(benzoylthio)propionic acid to give N-(3-(benzoylthio)propionyl)-3-(4-methylpiperazin-1-yl)sydnonimine (m.p.: 128 - 131°C).

270 MHz-NMR ($CDCl_3$) δ : 2.38 (3H, s), 2.70-2,73 (4H, m), 2.90 (2H, t), 3.41 (2H, t), 3.53-3.57 (4H, m), 7.39-7.45 (2H, m), 7.51-7.57 (1H, m), 7.94-7.97 (2H, m), 8.00 (1H, s)

[Example 125]

N-(3-(Benzoylthio)propionyl)-3-(4-benzylpiperazin-1-yl)sydnonimine

The procedure of Example 17 was repeated but starting with 3-(benzoylthio)propionic acid to give N-(3-(benzoylthio)propionyl)-3-(4-benzylpiperazin-1-yl)sydnonimine (m.p.: 126 - 127°C).

270 MHz-NMR ($CDCl_3$) δ : 2.74-2.77 (4H, m), 2.90 (2H, t), 3.41 (2H, t), 3.53-3.56 (4H, m), 3.61 (2H, s), 7.29-7.35 (5H, m), 7.39-7.44 (2H, m), 7.51-7.56 (1H, m), 7,94-7.97 (2H, m), 8.00 (1H, s)

[Example 126]

N-(3-(Benzoylthio)propionyl)-3-(4-benzyloxycarbonylpiperazin-1-yl)sydnonimine

The procedure of Example 19-A was repeated but starting with N-(3-(benzoylthio)propionyl)-3-(4-benzylpiperazin-1-yl)sydnonimine to give N-(3-(benzoylthio)propionyl)-3-(4-benzyloxycarbonylpiperazin-1-yl)sydnonimine as an oily substance.

270 MHz-NMR ($CDCl_3$) δ : 2.88 (2H, t), 3.40 (2H, t), 3.47-3.51 (4H, m), 3.78-3.82 (4H, m), 5.16 (2H, s), 7.35-7.43 (8H, m), 7.50-7.56 (1H, m), 7.92-7.96 (2H, m), 8.08 (1H, s)

[Example 127]

N-(3-(Benzoylthio)propionyl)-3-(piperazin-1-yl)sydnonimine hydrochloride

The procedure of Example 24-C was repeated but starting with N-(3-(benzoylthio)propionyl)-3-(4-benzyloxycarbonylpiperazin-1-yl)sydnonimine to give N-(3-(benzoylthio)propionyl)-3-(piperazin-1-yl)sydnonimine hydrochloride.

270 MHz-NMR ($DMSO-d_6$) δ : 2.87 (2H, t), 3.33 (2H, t), 3.37-3.45 (4H, m), 3.92-4.00 (4H, m), 7.53-7.58 (2H, m), 7.67-7.72 (1H, m), 7.89-7.92 (2H, m), 8.94 (1H, s), 9.77 (1H, brs)

[Example 128]

N-(3-(Benzoylthio)propionyl)-3-(4-ethylpiperazin-1-yl)sydnonimine

The procedure of Example 68 was repeated but starting with N-(3-(benzoylthio)propionyl)-3-(piperazin-1-yl)sydnonimine hydrochloride to give N-(3-(benzoylthio)propionyl)-3-(4-ethylpiperazin-1-yl)sydnonimine (m.p.: 119 - 121°C).

270 MHz-NMR ($CDCl_3$) δ : 1.12 (3H, t), 2.52 (2H, q), 2.73-2.76 (4H, m), 2.90 (2H, t), 3.41 (2H, t), 3.54-3.58 (4H, m), 7.39-7.45 (2H, m), 7.52-7.57 (1H, m), 7.94-7.98 (2H, m), 8.00 (1H, s)

[Example 129]

N-(3-(Benzoylthio)propionyl)-3-(4-isopropylpiperazin-1-yl)sydnonimine

The procedure of Example 69 was repeated but starting with N-(3-(benzoylthio)propionyl)-3-(piperazin-1-yl)sydnonimine hydrochloride to give N-(3-(benzoylthio)propionyl)-3-(4-isopropylpiperazin-1-yl)sydnonimine (m.p.: 128 - 130°C).

270 MHz-NMR ($CDCl_3$) δ : 1.08 (6H, d), 2.76-2.86 (5H, m), 2.90 (2H, t), 3.42 (2H, t), 3.52-3.55 (4H, m), 7.39-7.45 (2H, m), 7.52-7.57 (1H, m), 7.94-7.98 (2H, m), 7.99 (1H, s)

[Example 130]

N-(4-(Acetylthio)butyryl)-3-(4-methylpiperazin-1-yl)sydnonimine

The procedure of Example 1 was repeated but starting with 4-(acetylthio)butyric acid to give N-(4-(acetylthio)butyryl)-3-(4-methylpiperazin-1-yl)sydnonimine (m.p.: 92 - 93°C).

270 MHz-NMR ($CDCl_3$) δ : 1.90-2.01 (2H, m), 2.32 (3H, s), 2.38 (3H, s), 2.55 (2H, t), 2.69-2.74 (4H, m), 2.96 (2H, t), 3.52-3.57 (4H, m), 7.96 (1H, s)

[Example 131]

N-(3-(Cyclohexanecarbonylthio)propionyl)-3-(4-methylpiperazin-1-yl)sydnonimine

(1) Under a nitrogen atmosphere, 2.6 ml of cyclohexanecarbonyl chloride was added to a mixture of 2 g of 3-mercaptopropionic acid, 1.5 g of sodium hydroxide and 20 ml of water at 0°C and the resultant mixture was stirred for 4 hours at room temperature. Next, the reaction mixture was acidified with hydrochloric acid and extracted with ethyl acetate. The organic layer was washed with water and dried followed by the distillation of the solvent to give 1.5 g of 3-(cyclohexanecarbonylthio)propionic acid.

270 MHz-NMR ($CDCl_3$) δ : 1.16-1.52 (5H, m), 1.63-1.94 (5H, m), 2.41-2.54 (1H, m), 2.68 (2H, t), 3.09 (2H, t)

(2) The procedure of Example 1 was repeated but starting with 3-(cyclohexanecarbonylthio)propionic acid to give N-(3-(cyclohexanecarbonylthio)propionyl)-3-(4-methylpiperazin-1-yl)sydnonimine (m.p.: 137 - 138°C).

270 MHz-NMR ($CDCl_3$) δ : 1.19-1.38 (4H, m), 1.39-1.53 (2H, m), 1.60-1.94 (4H, m), 2.38 (3H, s), 2.38-2.51 (1H, m), 2.69-2.74 (4H, m), 2.78 (2H, t), 3.18 (2H, t), 3.52-3.57 (4H, m), 7.98 (1H, s)

[Example 132]

N-((2,6-Dimethylbenzoylthio)acetyl)-3-(4-methylpiperazin-1-yl)sydnonimine

(1) A mixture of 2.0 g of 2,6-dimethylbenzoic acid, 10 ml of thionyl chloride and 2 drops of N,N-dimethylformamide was heated under reflux for 2 hours. After distilling off the solvent, the residue was obtained. A mixture of 0.93 ml of mercaptoacetic acid, 3.25 g of 4-dimethylaminopyridine and 14 ml of chloroform was stirred for 5 minutes under ice cooling. Then the residue obtained above was added thereto and the resultant mixture was stirred for 25 minutes and then at room temperature for 1.5 hours. After adding water, the mixture was acidified with dilute hydrochloric acid and extracted with ethyl acetate. The organic layer was washed with a saturated aqueous solution of sodium chloride and dried and the solvent was distilled off to give 1.48 g of (2,6-dimethylbenzoylthio)acetic acid.

270 MHz-NMR ($CDCl_3$) $\delta$ : 2.33 (6H, s), 3.92 (2H, s), 7.02-7.05 (2H, m), 7.18-7.24 (1H, m) MS m/z : 244 (M+)

(2) The procedure of Example 1 was repeated but starting with (2,6-dimethylbenzoylthio)acetic acid to give N-((2,6-dimethylbenzoylthio)acetyl)-3-(4-methylpiperazin-1-yl)sydnonimine (m.p.: 165 - 166°C).

270 MHz-NMR ($CDCl_3$) $\delta$ : 2.38 (9H, s), 2.69-2.73 (4H, m), 3.54-3.58 (4H, m), 4.07 (2H, s), 7.00-7.03 (2H, m), 7.14-7.20 (1H, m), 8.01 (1H, s)

[Example 133]

N-((2,6-Dimethylbenzoylthio)acetyl)-3-(4-ethylpiperazin-1-yl)sydnonimine

Under ice cooling, a mixture of 0.17 g of (2,6-dimethylbenzoylthio)acetic acid, 0.16 g of 1-ethyl-3-(3-dimethylaminopropyl)carbodiimide hydrochloride, 0.13 g of 1-hydroxybenzotriazole hydrate and 8 ml of N,N-dimethylformamide was stirred for 15 minutes. After adding 0.2 g of 3-(4-ethylpiperazin-1-yl)sydnonimine hydrochloride and 0.24 ml of pyridine, the resultant mixture was stirred for 5 minutes and then for 15 hours at room temperature. After adding water and a saturated aqueous solution of sodium bicarbonate, the mixture was extracted with ethyl acetate. The organic layer was dried and the solvent was distilled off. The residue thus obtained was purified by silica gel column chromatography (developing solution: dichloromethane : methanol = 95 : 5) to give 98.9 mg of N-((2,6-dimethylbenzoylthio)acetyl)-3-(4-ethylpiperazin-1-yl)sydnonimine (m.p.: 139 - 140°C).

270 MHz-NMR ($CDCl_3$) $\delta$ : 1.12 (3H, t), 2.37 (6H, s), 2.51 (2H, q), 2.72-2.76 (4H, m), 3.54-3.58 (4H, m), 4.07 (2H, s), 6.99-7.03 (2H, m), 7.14-7.20 (1H, m), 8.01 (1H, s)

[Example 134]

N-((2-6-Dimethylbenzoylthio)acetyl)-3-(4-isopropylpiperazin-1-yl)sydnonimine

Under ice cooling, a mixture of 0.16 g of (2,6-dimethylbenzoylthio)acetic acid, 0.15 g of 1-ethyl-3-(3-dimethylaminopropyl)carbodiimide hydrochloride, 0.12 g of 1-hydroxybenzotriazole hydrate and 7 ml of N,N-dimethylformamide was stirred for 10 minutes. After adding 0.2 g of 3-(4-isopropylpiperazin-1-yl)sydnonimine hydrochloride and 0.23 ml of pyridine, the resultant mixture was stirred for 5 minutes and then for 15 hours at room temperature. After adding water and a saturated aqueous solution of sodium bicarbonate, the mixture was extracted with ethyl acetate. The organic layer was dried and the solvent was distilled off. The residue thus obtained was purified by silica gel column chromatography (developing solution: dichloromethane : methanol = 95 : 5) to give 168.6 mg of N-((2,6-dimethylbenzoylthio)acetyl)-3-(4-isopropylpiperazin-1-yl)sydnonimine (m.p.: 136 - 138°C).

270 MHz-NMR ($CDCl_3$) $\delta$ : 1.08 (6H, d), 2.37 (6H, s), 2.78-2.83 (5H, m), 3.52-3.56 (4H, m), 4.07 (2H, s), 6.99-7.03 (2H, m), 7.14-7.20 (1H, m), 8.00 (1H, s)

[Example 135]

N-(3-(4-Fluorobenzoylthio)propionyl)-3-(4-methylpiperazin-1-yl)sydnonimine

(1) The procedure of Example 131 (1) was repeated but starting with 4-fluorobenzoic acid chloride to give 3-(4-fluorobenzoylthio)propionic acid.

270 MHz-NMR ($CDCl_3$) δ : 2.81 (2H, t), 3.32 (2H, t), 7.09-7.16 (2H, m), 7.95-8.01 (2H, m) MS m/z : 228 (M+)

(2) The procedure of Example 1 was repeated but starting with 3-(4-fluorobenzoylthio)propionic acid to give N-(3-(4-fluorobenzoylthio)propionyl)-3-(4-methylpiperazin-1-yl)sydnonimine (m.p.: 155 - 156°C).

270 MHz-NMR ($CDCl_3$) δ : 2.38 (3H, s), 2.70-2.74 (4H, m), 2.90 (2H, t), 3.41 (2H, t), 3.53-3.57 (4H, m), 7.06-7.13 (2H, m), 7.95-8.01 (2H, m), 8.00 (1H, s)

[Example 136]

N-(3-(4-Fluorobenzoylthio)propionyl)-3-(4-ethylpiperazin-1-yl)sydnonimine

The procedure of Example 133 was repeated but starting with 3-(4-fluorobenzoylthio)propionic acid to give N-(3-(4-fluorobenzoylthio)propionyl)-3-(4-ethylpiperazin-1-yl)sydnonimine (m.p.: 126.5 - 129°C).

270 MHz-NMR ($CDCl_3$) δ : 1.12 (3H, t), 2.52 (2H, q), 2.72-2.77 (4H, m), 2.90 (2H, t), 3.41 (2H, t), 3.54-3.58 (4H, m), 7.06-7.13 (2H, m), 7.95-8.00 (2H, m), 8.00 (1H, s)

[Example 137]

N-(3-(4-Fluorobenzoylthio)propionyl)-3-(4-isopropylpiperazin-1-yl)sydnonimine

The procedure of Example 134 was repeated but starting with 3-(4-fluorobenzoylthio)propionic acid to give N-(3-(4-fluorobenzoylthio)propionyl)-3-(4-isopropylpiperazin-1-yl)sydnonimine (m.p.: 123 - 124°C).

270 MHz-NMR ($CDCl_3$) δ : 1.08 (6H, d), 2.76-2.83 (5H, m), 2.89 (2H, t), 3.41 (2H, t), 3.52-3.56 (4H, m), 7.06-7.13 (2H, m), 7.95-8.01 (2H, m), 7.99 (1H, s)

[Example 138]

N-(3-(4-Trifluoromethylbenzoylthio)propionyl)-3-(4-methylpiperazin-1-yl)sydnonimine

(1) The procedure of Example 131 (1) was repeated but starting with 4-trifluoromethylbenzoyl chloride to give 3-(4-trifluoromethylbenzoylthio)propionic acid.

270 MHz-NMR ($CDCl_3$) δ : 2.83 (2H, t), 3.36 (2H, t), 7.73 (2H, d), 8.06 (2H, d)

(2) The procedure of Example 1 was repeated but starting with 3-(4-trifluoromethylbenzoylthio)propionic acid to give N-(3-(4-trifluoromethylbenzoylthio)propionyl)-3-(4-methylpiperazin-1-yl)sydnonimine (m.p.: 162 - 163°C).

270 MHz-NMR ($CDCl_3$) δ : 2.38 (3H, s), 2.70-2.74 (4H, m), 2.91 (2H, t), 3.44 (2H, t), 3.53-3.57 (4H, m), 7.69 (2H, d), 8.00 (1H, s), 8.06 (2H, d)

[Example 139]

N-(3-(4-Methoxybenzoylthio)propionyl)-3-(4-methylpiperazin-1-yl)sydnonimine

(1) The procedure of Example 131 (1) was repeated but starting with 4-methoxybenzoyl chloride to give 3-(4-methoxybenzoylthio)propionic acid.

270 MHz-NMR ($CDCl_3$) δ : 2.80 (2H, t), 3.30 (2H, t), 3.87 (3H, s), 6.92 (2H, d), 7.93 (2H, d)

(2) The procedure of Example 1 was repeated but starting with 3-(4-methoxybenzoylthio)propionic acid to give N-(3-(4-methoxybenzoylthio)propionyl)-3-(4-methylpiperazin-1-yl)sydnonimine (m.p.: 136 - 137°C).

270 MHz-NMR ($CDCl_3$) δ : 2.38 (3H, s), 2.69-2.73 (4H, m), 2.89 (2H, t), 3.39 (2H, t), 3.52-3.56 (4H, m), 3.85 (3H, s), 6.89 (2H, d), 7.94 (2H, d), 8.00 (1H, s)

[Example 140]

N-(3-(4-Methoxybenzoylthio)propionyl)-3-(4-benzylpiperazin-1-yl)sydnonimine

The procedure of Example 17 was repeated but starting with 3-(4-methoxybenzoylthio)propionic acid to give N-(3-(4-methoxybenzoylthio)propionyl)-3-(4-benzylpiperazin-1-yl)sydnonimine (m.p.: 145 - 147°C).

270 MHz-NMR ($CDCl_3$) δ : 2.72-2.77 (4H, m), 2.89 (2H, t), 3.39 (2H, t), 3.52-3.56 (4H, m), 3.60 (2H, s), 3.85 (3H, s), 6.89 (2H, d), 7.28-7.38 (5H, m), 7.94 (2H, d), 7.99 (1H, s)

[Example 141]

N-(3-(2,6-Dimethylbenzoylthio)propionyl)-3-(4-methylpiperazin-1-yl)sydnonimine

(1) The procedure of Example 132 (1) was repeated but replacing mercaptoacetic acid by 3-mercaptopropionic acid to give 3-(2,6-dimethylbenzoylthio)propionic acid.

270 MHz-NMR ($CDCl_3$) δ : 2.31 (6H, s), 2.84 (2H, t), 3.32 (2H, t), 7.00-7.07 (2H, m), 7.15-7.21 (1H, m)

(2) The procedure of Example 1 was repeated but starting with 3-(2,6-dimethylbenzoylthio)propionic acid to give N-(3-(2,6-dimethylbenzoylthio)propionyl)-3-(4-methylpiperazin-1-yl)sydnonimine (m.p.: 155 - 157°C).

270 MHz-NMR ($CDCl_3$) δ : 2.30 (6H, s), 2.38 (3H, s), 2.69-2.73 (4H, m), 2.92 (2H, t), 3.40 (2H, t), 3.53-3.57 (4H, m), 6.97-7.00 (2H, m), 7.12-7.18 (1H, m), 7.98 (1H, s)

[Example 142]

N-(3-(2,6-Dimethylbenzoylthio)propionyl)-3-(4-ethylpiperazin-1-yl)sydnonimine

The procedure of Example 133 was repeated but starting with 3-(2,6-dimethylbenzoylthio)propionic acid to give N-(3-(2,6-dimethylbenzoylthio)propionyl)-3-(4-ethylpiperazin-1-yl)sydnonimine (m.p.: 120 - 121°C).

270 MHz-NMR ($CDCl_3$) δ : 1.12 (3H, t), 2.31 (6H, s), 2.52 (2H, q), 2.72-2.76 (4H, m), 2.92 (2H, t), 3.40 (2H, t), 3.53-3.58 (4H, m), 6.97-7.00 (2H, m), 7.12-7.18 (1H, m), 7.98 (1H, s)

[Example 143]

N-(3-(2,6-Dimethylbenzoylthio)propionyl)-3-(4-isopropylpiperazin-1-yl)sydnonimine

The procedure of Example 134 was repeated but starting with 3-(2,6-dimethylbenzoylthio)propionic acid to give N-(3-(2,6-dimethylbenzoylthio)propionyl)-3-(4-isopropylpiperazin-1-yl)sydnonimine as an oily substance.

270 MHz-NMR ($CDCl_3$) δ : 1.07 (6H, d), 2.30 (6H, s), 2.78-2.82 (5H, m), 2.92 (2H, t), 3.40 (2H, t), 3.51-3.55 (4H, m), 6.97-7.00 (2H, m), 7.12-7.18 (1H, m), 7.99 (1H, s)

[Example 144]

(3-(2,6-Dichlorobenzoylthio)propionyl)-3-(4-methylpiperazin-1-yl)sydnonimine

(1) The procedure of Example 131 (1) was repeated but starting with 2,6-dichlorobenzoyl chloride to give 3-(2,6-dichlorobenzoylthio)propionic acid.

270 MHz-NMR ($CDCl_3$) δ : 2.88 (2H, t), 3.37(2H, t), 7.29-7.36 (3H, m) MS m/z : 279 (M+)

(2) The procedure of Example 1 was repeated but starting with 3-(2,6-dichlorobenzoylthio)propionic acid to give N-(3-(2,6-dichlorobenzoylthio)propionyl)-3-(4-methylpiperazin-1-yl)sydnonimine (m.p.: 164 - 166°C).

270 MHz-NMR ($CDCl_3$) δ : 2.38 (3H, s), 2.69-2.73 (4H, m), 2.95 (2H, t), 3.46 (2H, t), 3.53-3.57 (4H, m), 7.21-7.32 (3H, m), 7.99 (1H, s)

[Example 145]

N-(3-(2,6-Difluorobenzoylthio)propionyl)-3-(4-methylpiperazin-1-yl)sydnonimine

(1) The procedure of Example 131 (1) was repeated but starting with 2,6-difluorobenzoyl chloride to give 4.6 g of 3-(2,6-difluorobenzoylthio)propionic acid.

270 MHz-NMR ($CDCl_3$) δ : 2.85 (2H, t), 3.35 (2H, t), 6.92-6.99 (2H, m), 7.35-7.46 (1H, m)

(2) The procedure of Example 1 was repeated but starting with 3-(2,6-difluorobenzoylthio)propionic acid to give N-(3-(2,6-difluorobenzoylthio)propionyl)-3-(4-methylpiperazin-1-yl)sydnonimine (m.p.: 139 - 141°C).

270 MHz-NMR ($CDCl_3$) δ : 2.37 (3H, s), 2.62-2.73 (4H, m), 2.92 (2H, t), 3.44 (2H, t), 3.52-3.56 (4H, m), 6.88-6.95 (2H, m), 7.30-7.42 (1H, m), 7.98 (1H, s)

[Example 146]

N-(3-(2,6-Difluorobenzoylthio)propionyl)-3-(4-ethylpiperazin-1-yl)sydnonimine

The procedure of Example 133 was repeated but starting with 3-(2,6-difluorobenzoylthio)propionic acid to give N-(3-(2,6-difluorobenzoylthio)propionyl)-3-(4-ethylpiperazin-1-yl)sydnonimine (m.p.: 120 - 121.5°C).

270 MHz-NMR ($CDCl_3$) δ : 1.12 (3H, t), 2.52 (2H, q), 2.72-2.76 (4H, m), 2.92 (2H, t), 3.44 (2H, t), 3.54-3.58 (4H, m), 6.89-6.95 (2H, m), 7.30-7.42 (1H, m), 7.99 (1H, s)

[Example 147]

N-(3-(2,6-Difluorobenzoylthio)propionyl)-3-(4-isopropylpiperazin-1-yl)sydnonimine

The procedure of Example 134 was repeated but starting with 3-(2,6-difluorobenzoylthio)propionic acid to give N-(3-(2,6-difluorobenzoylthio)propionyl)-3-(4-isopropylpiperazin-1-yl)sydnonimine (m.p.: 124 - 125°C).

270 MHz-NMR ($CDCl_3$) δ : 1.08 (6H, d), 2.76-2.83 (5H, m), 2.92 (2H, t), 3.44 (2H, t), 3.51-3.55 (4H, m), 6.88-6.95 (2H, m), 7.30-7.42 (1H, m), 7.98 (1H, s)

[Example 148]

N-(3-(2,6-Dimethoxybenzoylthio)propionyl)-3-(4-methylpiperazin-1-yl)sydnonimine

(1) Under a nitrogen atmosphere, 1.2 g of 4-dimethylaminopyridine was added to a mixture of 0.44 ml of 3-mercaptopropionic acid, 1 g of 2,6-dimethoxybenzoyl chloride and 10 ml of chloroform and the resultant mixture was stirred for 2 hours at room temperature. The organic layer was washed with water and dried and the solvent was distilled off to give 0.64 g of 3-(2,6-dimethoxybenzoylthio)propionic acid as an oily substance.

270 MHz-NMR ($CDCl_3$) δ : 2.82 (2H, t), 3.29 (2H, t), 3.80 (6H, s), 6.55 (2H, d), 7.29 (1H, t)

(2) The procedure of Example 1 was repeated but starting with 3-(2,6-dimethoxybenzoylthio)propionic acid to give N-(3-(2,6-dimethoxybenzoylthio)propionyl)-3-(4-methylpiperazin-1-yl)sydnonimine as an oily substance.

270 MHz-NMR ($CDCl_3$) δ : 2.38 (3H, s), 2.69-2.74 (4H, m), 2.90-2.99 (2H, m), 3.24-3.40 (2H, m), 3.52-3.57 (4H, m), 3.79 (6H, s), 6.50-6.56 (2H, m), 7.20-7.27 (1H, m), 7.99 (1H, s)

[Example 149]

N-(3-(2,6-Dimethoxybenzoylthio)propionyl)-3-(4-ethylpiperazin-1-yl)sydnonimine

The procedure of Example 133 was repeated but starting with 3-(2,6-dimethoxybenzoylthio)propionic acid to give N-(3-(2,6-dimethoxybenzoylthio)propionyl)-3-(4-ethylpiperazin-1-yl)sydnonimine (m.p.: 136 - 138°C).

270 MHz-NMR ($CDCl_3$) δ : 1.12 (3H, t), 2.51 (2H, q), 2.72-2.76 (4H, m), 2.91 (2H, t), 3.39 (2H, t), 3.52-3.57 (4H, m), 3.79 (6H, s), 6.52 (2H, d), 7.22-7.28 (1H, m), 7.98 (1H, s)

[Example 150]

N-(3-(2,6-Dimethoxybenzoylthio)propionyl)-3-(4-isopropylpiperazin-1-yl)sydnonimine

The procedure of Example 134 was repeated but starting with 3-(2,6-dimethoxybenzoylthio)propionic acid to give N-(3-(2,6-dimethoxybenzoylthio)propionyl)-3-(4-isopropylpiperazin-1-yl)sydnonimine (m.p.: 121 - 122°C).

270 MHz-NMR ($CDCl_3$) δ : 1.08 (6H, d), 2.77-2.83 (5H, m), 2.91 (2H, t), 3.39 (2H, t), 3.50-3.55 (4H, m), 3.79 (6H, s), 6.52 (2H, d), 7.21-7.29 (1H, m), 7.94 (1H, s)

[Example 151]

N-(Pivaloylthioacetyl)-3-(4-methylpiperazin-1-yl)sydnonimine

(1) Under a nitrogen atmosphere, 2 g of 4-dimethylaminopyridine was added at 0°C to a mixture of 1.45 ml of methyl mercaptoacetate, 2 ml of pivaloyl chloride and 20 ml of chloroform and the resultant mixture was stirred for 15 hours at room temperature. Then the reaction mixture was acidified with hydrochloric acid and extracted with

chloroform. The organic layer was washed with water and dried and the solvent was distilled off to give 3.9 g of methyl 2-(pivaloylthio)acetate as an oily substance. Next, 8 ml of acetic acid and 4 ml of conc. hydrochloric acid were added to the obtained methyl 2-(pivaloylthio)acetate and the resultant mixture was heated under reflux for 1 hour. After adding water, the mixture was extracted with ethyl acetate. The organic layer was washed with water and dried. After distilling off the solvent, the obtained residue was purified by silica gel column chromatography (developing solution: chloroform : methanol : acetic acid = 200 : 10 : 1) to give 2 g of (pivaloylthio)acetic acid. 270 MHz-NMR ($CDCl_3$) $\delta$ : 1.27 (9H, s), 3.69 (2H, s)

(2) The procedure of Example 1 was repeated but starting with (pivaloylthio)acetic acid to give N-(pivaloylthioacetyl)-3-(4-methylpiperazin-1-yl)sydnonimine (m.p.: 129 - 130°C).

270 MHz-NMR ($CDCl_3$) $\delta$ : 1.27 (9H, s), 2.38 (3H, s), 2.69-2.74 (4H, m), 3.51-3.57 (4H, m), 3.86 (2H, s), 7.98 (1H, s)

[Example 152]

N-(Pivaloylthioacetyl)-3-(4-ethylpiperazin-1-yl)sydnonimine

The procedure of Example 133 was repeated but starting with (pivaloylthio)acetic acid to give N-(pivaloylthioacetyl)-3-(4-ethylpiperazin-1-yl)sydnonimine (m.p.: 111 - 112°C).

270 MHz-NMR ($CDCl_3$) $\delta$ : 1.12 (3H, t), 1.27 (9H, s), 2.51 (2H, q), 2.72-2.77 (4H, m), 3.52-3.57 (4H, m), 3.86 (2H, s), 7.98 (1H, s)

[Example 153]

N-(Pivaloylthioacetyl)-3-(4-isopropylpiperazin-1-yl)sydnonimine

The procedure of Example 134 was repeated but starting with (pivaloylthio)acetic acid to give N-(pivaloylthioacetyl)-3-(4-isopropylpiperazin-1-yl)sydnonimine (m.p.: 107 - 108°C).

270 MHz-NMR ($CDCl_3$) $\delta$ : 1.08 (6H, d), 1.27 (9H, s), 2.78-2.84 (5H, m), 3.51-3.55 (4H, m), 3.86 (2H, s), 7.98 (1H, s)

[Example 154]

N-(3-(Pivaloylthio)propionyl)-3-(4-methylpiperazin-1-yl)sydnonimine

(1) The procedure of Example 131 (1) was repeated but starting with pivaloyl chloride to give 3-(pivaloylthio)propionic acid as an oily substance.

270 MHz-NMR ($CDCl_3$) $\delta$ : 1.23 (9H, s), 2.67 (2H, t), 3.08 (2H, t)

(2) The procedure of Example 1 was repeated but starting with 3-(pivaloylthio)propionic acid to give N-(3-(pivaloylthio)propionyl)-3-(4-methylpiperazin-1-yl)sydnonimine (m.p.: 136 - 137°C).

270 MHz-NMR ($CDCl_3$) $\delta$ : 1.22 (9H, s), 2.38 (3H, s), 2.70-2.81 (6H, m), 3.17 (2H, t), 3.53-3.58 (4H, m), 7.99 (1H, s)

[Example 155]

N-(3-(Pivaloylthio)propionyl)-3-(4-ethylpiperazin-1-yl)sydnonimine

The procedure of Example 133 was repeated but starting with 3-(pivaloylthio)propionic acid to give N-(3-(pivaloylthio)propionyl)-3-(4-ethylpiperazin-1-yl)sydnonimine (m.p.: 98 - 99°C).

270 MHz-NMR ($CDCl_3$) δ : 1.12 (3H, t), 1.20 (9H, s), 2.52 (2H, q), 2.73-2.81 (6H, m), 3.17 (2H, t), 3.54-3.58 (4H, m), 8.01 (1H, s)

[Example 156]

N-(3-(Pivaloylthio)propionyl)-3-(4-isopropylpiperazin-1-yl)sydnonimine

The procedure of Example 134 was repeated but starting with 3-(pivaloylthio)propionic acid to give N-(3-(pivaloylthio)propionyl)-3-(4-isopropylpiperazin-1-yl)sydnonimine (m.p.: 117 - 118°C).

270 MHz-NMR ($CDCl_3$) δ : 1.16 (6H, d), 1.30 (9H, s), 2.83-2.93 (7H, m), 3.25(2H, t), 3.60-3.64 (4H, m), 8.08 (1H, s)

[Example 157]

N-(Pivaloylthiopivaloyl)-3-(4-methylpiperazin-1-yl)sydnonimine

(1) Under ice cooling, a mixture of 1.5 g of mercaptopivalic acid, 1.5 ml of pivaloyl chloride, 2.74 g of 4-dimethylaminopyridine and 11 ml of chloroform was stirred for 6 hours. After adding water, the reaction mixture was acidified with dilute hydrochloric acid and extracted with ethyl acetate. The organic layer was dried and the solvent was distilled off to give 0.81 g of (pivaloylthio)pivalic acid.

270 MHz-NMR ($CDCl_3$) δ : 1.24 (3H, s), 1.24 (9H, s), 1.26 (3H, s), 3.15 (2H, s)

(2) The procedure of Example 1 was repeated but starting with (pivaloylthio)pivalic acid to give N-(pivaloylthiopivaloyl)-3-(4-methylpiperazin-1-yl)sydnonimine (m.p.: 115 - 117°C).

270 MHz-NMR ($CDCl_3$) δ : 1.22 (9H, s), 1.26(6H, s), 2.37 (3H, s), 2.68-2.72 (4H, m), 3.24 (2H, s), 3.51-3.55 (4H, m), 7.98 (1H, s)

[Example 158]

N-(Pivaloylthiopivaloyl)-3-(4-ethylpiperazin-1-yl)sydnonimine

The procedure of Example 133 was repeated but starting with (pivaloylthio)pivalic acid to give N-(pivaloylthiopivaloyl)-3-(4-ethylpiperazin-1-yl)sydnonimine (m.p.: 62 - 65°C).

270 MHz-NMR ($CDCl_3$) δ : 1.12 (3H, t), 1.22 (9H, s), 1.26 (6H, s), 2.51 (2H, q), 2.71-2.76 (4H, m), 3.24 (2H, s), 3.52-3.56 (4H, m), 7.98 (1H, s)

[Example 159]

N-(Pivaloylthiopivaloyl)-3-(4-isopropylpiperazin-1-yl)sydnonimine

The procedure of Example 134 was repeated but starting with (pivaloylthio)pivalic acid to give N-(pivaloylthiopivaloyl)-3-(4-isopropylpiperazin-1-yl)sydnonimine as an oily substance.

270 MHz-NMR ($CDCl_3$) δ : 1.08 (6H, d), 1.22 (9H, s), 1.26 (6H, s), 2.75-2.86 (5H, m), 3.24 (2H, s), 3.50-3.54 (4H, m), 7.98 (1H, s)

[Example 160]

N-(3-(Benzyloxycarbonylthio)propionyl)-3-(4-methylpiperazin-1-yl)sydnonimine

Under a nitrogen atmosphere, 2.7 ml of benzyloxycarbonyl chloride was added at 0°C to a mixture of 2 g of 3-mercaptopropionic acid, 1.51 g of sodium hydroxide and 20 ml of water and the resultant mixture was stirred for 1 hour at 0°C. Next, the reaction mixture was acidified with hydrochloric acid and extracted with ethyl acetate. The organic

layer was washed with water and dried and the solvent was distilled off to give 2 g of 3-(benzyloxycarbonylthio)propionic acid as an oily substance. Then the procedure of Example 1 was repeated but starting with the thus obtained 3-(benzyloxycarbonylthio)propionic acid to give N-(3-(benzyloxycarbonylthio)propionyl)-3-(4-methylpiperazin-1-yl)sydnonimine (m.p.: 125 - 126°C).

270 MHz-NMR ($CDCl_3$) δ : 2.38 (3H, s), 2.69-2,74 (4H, m), 2.87 (2H, t), 3.21 (2H, t), 3.52-3.57 (4H, m), 5.23 (2H, s), 7.35 (5H, s), 7.97 (1H, s)

[Example 161]

N-(Ethylcarbamoylthioacetyl)-3-(4-methylpiperazin-1-yl)sydnonimine

Under a nitrogen atmosphere, 2.82 ml of triethylamine was added at 0°C to a mixture of 1.4 ml of mercaptoacetic acid, 1.6 ml of ethyl isocyanate and 20 ml of chloroform and the resultant mixture was stirred for 1 hour at 0°C. Next, the reaction mixture was acidified with hydrochloric acid and extracted with chloroform. The organic layer was washed with water and dried and the solvent was distilled off. The obtained residue was purified by silica gel column chromatography (developing solution: chloroform : methanol : acetic acid = 200 : 10 : 1) to give 1.2 g of (ethylcarbamoylthio)acetic acid as an oily substance.

270 MHz-NMR ($CDCl_3$) δ : 1.19 (3H, t), 3.24-3.41 (2H, m), 3.75 (2H, s), 5.62 (1H, brs)

(2) The procedure of Example 1 was repeated but starting with (ethylcarbamoylthio)acetic acid to give N-(ethylcarbamoylthioacetyl)-3-(4-methylpiperazin-1-yl)sydnonimine (m.p.: 139 - 140°C).

270 MHz-NMR ($CDCl_3$) δ : 1.18 (3H, t), 2.38 (3H, s), 2.69-2.74 (4H, m), 3.30-3.41 (2H, m), 3.52-3.57 (4H, m), 3.98 (2H, s), 5.41 (1H, brs), 7.97 (1H, s)

[Example 162]

N-(3-(Ethylcarbamoylthio)propionyl)-3-(4-methylpiperazin-1-yl)sydnonimine

Under a nitrogen atmosphere, 2.63 ml of triethylamine was added at 0°C to a mixture of 2 g of 3-mercaptopropionic acid, 1.5 ml of ethyl isocyanate and 20 ml of chloroform and the resultant mixture was stirred for 13 hours at room temperature. Next, the reaction mixture was acidified with hydrochloric acid and extracted with chloroform. The organic layer was washed with water and dried and the solvent was distilled off. The obtained residue was purified by silica gel column chromatography (developing solution: chloroform : methanol : acetic acid = 1,000 : 10 : 1) to give 1 g of 3-(ethylcarbamoylthio)propionic acid.

270 MHz-NMR ($CDCl_3$) δ : 1.17 (3H, t), 2.76 (2H, t), 3.14 (2H, t), 3.26-3.40 (2H, m), 5.36 (1H, brs)

(2) The procedure of Example 1 was repeated but starting with 3-(ethylcarbamoylthio)propionic acid to give N-(3-(ethylcarbamoylthio)propionyl)-3-(4-methylpiperazin-1-yl)sydnonimine (m.p.: 161 - 162°C).

270 MHz-NMR ($CDCl_3$) δ : 1.15 (3H, t), 2.38 (3H, s), 2.69-2.74 (4H, m), 2.84 (2H, t), 3.24 (2H, t), 3.24-3.38 (2H, m), 3.52-3.57 (4H, m), 5.42 (1H, brs), 7.99 (1H, s)

[Example 163]

N-(3-(Ethylcarbamoylthio)propionyl)-3-(4-ethylpiperazin-1-yl)sydnonimine

The procedure of Example 133 was repeated but starting with 3-(ethylcarbamoylthio)propionic acid to give N-(3-(ethylcarbamoylthio)propionyl)-3-(4-ethylpiperazin-1-yl)sydnonimine (m.p.: 148 - 149°C).

270 MHz-NMR ($CDCl_3$) δ : 1.12 (3H, t), 1.15 (3H, t), 2.51 (2H, q), 2.72-2.77 (4H, m), 2.85 (2H, t), 3.24 (2H, t), 3.24-3.35 (2H, m), 3.52-3.57 (4H, m), 5.34 (1H, brs), 7.98 (1H, s)

[Example 164]

N-(3-(Ethylcarbamoylthio)propionyl)-3-(4-isopropylpiperazin-1-yl)sydnonimine

The procedure of Example 134 was repeated but starting with 3-(ethylcarbamoylthio)propionic acid to give N-(3-(ethylcarbamoylthio)propionyl)-3-(4-isopropylpiperazin-1-yl)sydnonimine (m.p.: 135 - 136°C).

270 MHz-NMR ($CDCl_3$) δ : 1.05 (6H, d), 1.15 (3H, t), 2.76-2.89 (6H, m), 3.24 (2H, t), 3.24-3.35 (1H, m), 3.51-3.55 (4H, m), 5.38 (1H, brs), 7.98 (1H, s)

[Example 165]

N-(Ethylcarbamoylthiopivaloyl)-3-(4-methylpiperazin-1-yl)sydnonimine

(1) The procedure of Example 162 (1) was repeated but starting with mercaptopivalic acid to give (ethylcarbamoylthio)pivalic acid.

270 MHz-NMR ($CDCl_3$) δ : 1.16 (3H, t), 1.29 (6H, s), 3.22 (2H, s), 3.28-3.38 (2H, m), 5.28-5.38 (1H, m)

(2) The procedure of Example 1 was repeated but starting with (ethylcarbamoylthio)pivalic acid to give N-(ethylcarbamoylthiopivaloyl)-3-(4-methylpiperazin-1-yl)sydnonimine (m.p.: 117 - 119°C).

270 MHz-NMR ($CDCl_3$) δ : 1.14 (3H, t), 1.29 (6H, s), 2.38 (3H, s), 2.69-2.73 (4H, m), 3.29 (2H, s), 3.26-3.36 (2H, m), 3.51-3.55 (4H, m), 5.40-5.49 (1H, m), 7.98 (1H, s)

[Example 166]

N-(Ethylcarbamoylthiopivaloyl)-3-(4-ethylpiperazin-1-yl)sydnonimine

The procedure of Example 133 was repeated but starting with (ethylcarbamoylthio)pivalic acid to give N-(ethylcarbamoylthiopivaloyl)-3-(4-ethylpiperazin-1-yl)sydnonimine as an oily substance.

270 MHz-NMR ($CDCl_3$) δ : 1.12 (3H, t), 1.14 (3H, t), 1.29 (6H, s), 2.51 (2H, q), 2.72-2.76 (4H, m), 3.29 (2H, s), 3.26-3.36 (2H, m), 3.52-3.56 (4H, m), 5.45-5.52 (1H, m), 7.98 (1H, s)

[Example 167]

N-(Ethylcarbamoylthiopivaloyl)-3-(4-isopropylpiperazin-1-yl)sydnonimine

The procedure of Example 134 was repeated but starting with (ethylcarbamoylthio)pivalic acid to give N-(ethylcarbamoylthiopivaloyl)-3-(4-isopropylpiperazin-1-yl)sydnonimine (m.p.: 114 - 116°C).

270 MHz-NMR ($CDCl_3$) δ : 1.08 (6H, d), 1.13 (3H, t), 1.30 (6H, s), 2.76-2.86 (5H, m), 3.30 (2H, s), 3.26-3.36 (2H, m), 3.50-3.54 (4H,m), 5,37-5.45 (1H, m), 7.97 (1H, s)

[Example 168]

N-(3-(t-Butylcarbamoylthio)propionyl)-3-(4-methylpiperazin-1-yl)sydnonimine

(1) Under ice cooling, a mixture of 2.0 g of 3-mercaptopropionic acid, 2.2 ml of t-butyl isocyanate, 2.8 ml of triethylamine and 20 ml of chloroform was stirred for 1 hour and then at room temperature for 22 hours. After adding water, the mixture was acidified with dilute hydrochloric acid and then extracted with ethyl acetate. The organic layer was dried and the solvent was distilled off to give 2.25 g of 3-(t-butylcarbamoylthio)propionic acid.

270 MHz-NMR ($CDCl_3$) δ : 1.35 (9H, s), 2.74 (2H, t), 3.09 (2H, t), 5.17 (1H, brs)

(2) The procedure of Example 1 was repeated but starting with 3-(t-butylcarbamoylthio)propionic acid to give N-(3-(t-butylcarbamoylthio)propionyl)-3-(4-methylpiperazin-1-yl)sydnonimine (m.p.: 171 - 172°C).

270 MHz-NMR ($CDCl_3$) δ : 1.34 (9H, s), 2.38 (3H, s), 2.69-2.73 (4H m), 2.83 (2H, t), 3.19 (2H, t), 3.52-3 .56 (4H, m), 5.16 (1H, brs), 7.98 (1H, s)

[Example 169]

N-(3-(t-Butylcarbamoylthio)propionyl)-3-(4-ethylpiperazin-1-yl)sydnonimine

The procedure of Example 133 was repeated but starting with 3-(t-butylcarbamoylthio)propionic acid to give N-(3-(t-butylcarbamoylthio)propionyl)-3-(4-ethylpiperazin-1-yl)sydnonimine (m.p.: 138 - 141°C).

270 MHz-NMR ($CDCl_3$) δ : 1.12 (3H, t), 1.34 (9H, s), 2.52 (2H, q), 2.72-2.76 (4H, m), 2.83 (2H, t), 3.19 (2H, t), 3.16-3.21 (4H, m), 5.14 (1H, brs), 7.98 (1H, s)

[Example 170]

N-(3-(t-Butylcarbamoylthio)propionyl)-3-(4-isopropylpiperazin-1-yl)sydnonimine

The procedure of Example 134 was repeated but starting with 3-(t-butylcarbamoylthio)propionic acid to give N-(3-(t-butylcarbamoylthio)propionyl)-3-(4-isopropylpiperazin-1-yl)sydnonimine (m.p.: 172 - 174°C).

270 MHz-NMR ($CDCl_3$) δ : 1.08 (6H, d) 1.34 (9H, s), 2.76-2.86 (7H, m), 3.19 (2H, t), 3.50-3.54 (4H, m), 5.14 (1H, brs), 7.97 (1H, s)

[Example 171]

N-(3-(Phenylcarbamoylthio)propionyl)-3-(4-methylpiperazin-1-yl)sydnonimine

(1) Under a nitrogen atmosphere, 2.7 ml of triethylamine was added at 0°C to a mixture of 2 g of 3-mercaptopropionic acid, 2.1 ml of phenyl isocyanate and 20 ml of chloroform and the resultant mixture was stirred for 13 hours at room temperature. The mixture was acidified with hydrochloric acid and the crystals thus precipitated were taken up by filtration to give 3.6 g of 3-(phenylcarbamoylthio)propionic acid.

270 MHz-NMR ($CDCl_3$) δ : 2.73 (2H, t), 3.20 (2H, t), 5.04 (1H, brs), 7.04-7.11 (1H, m), 7.25-7.33 (2H, m), 7.44-7.50 (2H, m), 8.39 (1H, brs)

(2) The procedure of Example 1 was repeated but starting with 3-(phenylcarbamoylthio)propionic acid to give N-(3-(phenylcarbamoylthio)propionyl)-3-(4-methylpiperazin-1-yl)sydnonimine (m.p.: 154 - 155°C).

270 MHz-NMR ($CDCl_3$) δ : 2.38 (3H, s), 2.68-2.73 (4H, m), 2.90 (2H, t), 3.32 (2H, t), 3.51-3.56 (4H, m), 7.05-7.11 (1H, m), 7.23 (1H, brs), 7.26-7.33 (2H, m), 7.39-7.43 (2H, m), 7.98 (1H, s)

[Example 172]

N-(3-(Phenylcarbamoylthio)propionyl)-3-(4-ethylpiperazin-1-yl)sydnonimine

The procedure of Example 133 was repeated but starting with 3-(phenylcarbamoylthio)propionic acid to give N-(3-(phenylcarbamoylthio)propionyl)-3-(4-ethylpiperazin-1-yl)sydnonimine (m.p.: 143 - 144°C).

270 MHz-NMR ($CDCl_3$) δ : 1.11 (3H, t), 2.50 (2H, q), 2.69-2.74 (4H, m), 2.89 (2H, t), 3.30 (2H, t), 3.49-3.54 (4H, m), 7.03-7.10 (1H, m), 7.24-7.32 (2H, m), 7.42-7.47 (2H, m), 7.83 (1H, brs), 7.98 (1H, s)

[Example 173]

N-(3-(Phenylcarbamoylthio)propionyl)-3-(4-isopropylpiperazin-1-yl)sydnonimine

The procedure of Example 134 was repeated but starting with 3-(phenylcarbamoylthio)propionic acid to give N-(3-(phenylcarbamoylthio)propionyl)-3-(4-isopropylpiperazin-1-yl)sydnonimine (m.p.: 142 - 143°C).

270 MHz-NMR ($CDCl_3$) δ : 1.08 (6H, d), 2.75-2.86 (5H, m), 2.90 (2H, t), 3.31 (2H, t), 3.49-3.54 (4H, m), 7.04-7.11 (1H, m), 7.26-7.33 (2H, m), 7.41-7.46 (3H, m), 7.97 (1H, s)

[Example 174]

N-(4-(Benzoylthio)butyryl)-3-(4-methylpiperazin-1-yl)sydnonimine

The procedure of Example 1 was repeated but starting with 4-(benzoylthio)butyric acid to give N-(4-(benzoylthio)butyryl)-3-(4-methylpiperazin-1-yl)sydnonimine (m.p.: 87.5 - 90°C).

270 MHz-NMR ($CDCl_3$) δ : 2.03-2.15 (2H, m), 2.38 (3H, s), 2.63 (2H, t), 2.72-2.78 (4H, m), 3.16 (2H, t), 3.54-3.60 (4H, m), 7.40-7.60 (3H, m), 7.94-8.00 (2H, m), 7.97 (1H, s)

[Example 175]

N-(4-(Benzoylthio)butyryl)-3-(4-ethylpiperazin-1-yl)sydnonimine

The procedure of Example 133 was repeated but starting with 4-(benzoylthio)butyric acid to give N-(4-(benzoylthio)butyryl)-3-(4-ethylpiperazin-1-yl)sydnonimine as an oily substance.

270 MHz-NMR ($CDCl_3$) δ : 1.17 (3H, t) 2.01-2.14 (2H, m), 2.51-2.59 (2H, m), 2.63 (2H, t), 2.78-2.87 (4H, m), 3.14 (2H, dd), 3.56-3.66 (4H, m), 7.40-7.60 (3H, m), 7.86-8.00 (2H, m), 8.01 (1H, s)

[Example 176]

N-(4-(Benzoylthio)butyryl)-3-(4-isopropylpiperazin-1-yl)sydnonimine

The procedure of Example 134 was repeated but starting with 4-(benzoylthio)butyric acid to give N-(4-(benzoylthio)butyryl)-3-(4-isopropylpiperazin-1-yl)sydnonimine as an oily substance.

270 MHz-NMR ($CDCl_3$) δ : 1.08 (6H, d), 2.04-2.16 (2H, m), 2.63 (2H, t), 2.80-2.89 (5H, m), 3.16 (2H, t), 3.59-3.67 (4H, m), 7.40-7.60 (3H, m), 7.94-8.02 (2H, m), 7.97 (1H, s)

[Example 177]

N-(3-Acetylthio-2-benzylpropionyl)-3-(4-methylpiperazin-1-yl)sydnonimine

The procedure of Example 1 was repeated but starting with 3-acetylthio-2-benzylpropionic acid to give N-(3-acetylthio-2-benzylpropionyl)-3-(4-methylpiperazin-1-yl)sydnonimine as an oily substance.

270 MHz-NMR ($CDCl_3$) δ : 2.27 (3H, s), 2.38 (3H, s), 2.68-2.73 (4H, m), 2.81-2.89 (1H, m), 3.01-3.22 (4H, m), 3.52-3.55 (4H, m), 7.14-7.29 (5H, m), 7.98 (1H, s)

[Example 178]

N-(3-(Methylthio)propionyl)-3-(4-methylpiperazin-1-yl)sydnonimine

The procedure of Example 1 was repeated but starting with 3-(methylthio)propionic acid to give N-(3-(methylthio)propionyl)-3-(4-methylpiperazin-1-yl)sydnonimine (m.p.: 116 - 117°C).

270 MHz-NMR ($CDCl_3$) δ : 2.13 (3H, s), 2.38 (3H, s), 2.69-2.89 (8H, m), 3.52-3.57 (4H, m), 7.99 (1H, s)

[Example 179]

N-(3-(3,5-Di-t-butyl-4-hydroxyphenylthio)propionyl)-3-(4-methylpiperazin-1-yl)sydnonimine

The procedure of Example 1 was repeated but starting with 3-(3,5-di-t-butyl-4-hydroxyphenylthio)propionic acid to give N-(3-(3,5-di-t-butyl-4-hydroxyphenylthio)propionyl)-3-(4-methylpiperazin-1-yl)sydnonimine as an oily substance.

270 MHz-NMR ($CDCl_3$) δ : 1.45 (18H, s), 2.38 (3H, s), 2.69-2.75 (4H, m), 2.78 (2H, dd), 3.15 (2H, dd), 3.54-3.57 (4H, m), 5.18 (1H, s), 7.28 (2H, s), 7.98 (1H, s)

[Example 180]

N-(4-(3,5-Di-t-butyl-4-hydroxyphenylthio)butyryl)-3-(4-methylpiperazin-1-yl)sydnonimine

The procedure of Example 1 was repeated but starting with 4-(3,5-di-t-butyl-4-hydroxyphenylthio)butyric acid to give N-(4-(3,5-Di-t-butyl-4-hydroxyphenylthio)butyryl)-3-(4-methylpiperazin-1-yl)sydnonimine as an oily substance.

270 MHz-NMR ($CDCl_3$) δ : 1.42 (18H, s), 1.95-2.05 (2H, m), 2.37 (3H, s), 2.61 (2H, t), 2.69-2.73 (4H, m), 2.90 (2H, t), 3.51-3.56 (4H, m), 5.16 (1H, s), 7.23 (2H, s), 7.95 (1H, s)

[Example 181]

N-((R)-2-Acetylamino-3-(acetylthio)propionyl)-3-(4-methylpiperazin-1-yl)sydnonimine

(1) Under ice cooling, a mixture of 3.0 g of N-acetyl-L-cysteine, 6.2 g of sodium hydrogencarbonate and 40 ml of water was stirred for 5 minutes. After adding 11 ml of acetic anhydride, the resultant mixture was stirred for 10 minutes and then for 1 hour at room temperature. After adding water, the mixture was acidified with dilute sulfuric acid and then extracted with ethyl acetate. The organic layer was dried and the solvent was distilled off to give 3.24 g of (R)-2-acetylamino-3-(acetylthio)propionic acid.

270 MHz-NMR ($CDCl_3$) δ : 2.05 (3H, s), 2.37 (3H, s), 3.32 (1H, dd), 3.47 (1H, dd), 4.76-4.83 (1H, m), 6.82 (1H, brd)

(2) The procedure of Example 1 was repeated but starting with (R)-2-acetylamino-3-(acetylthio)propionic acid to give N-((R)-2-acetylamino-3-(acetylthio)propionyl)-3-(4-methylpiperazin-1-yl)sydnonimine (m.p.: 155 - 157°C).

270 MHz-NMR ($CDCl_3$) δ : 2.02 (3H, s), 2.31 (3H, s), 2.38 (3H, s), 2.70-2.74 (4H, m), 3.44 (1H, dd), 3.54-3.63 (5H, m), 4.77-4.84 (1H, m), 6.53 (1H, brd), 7.97 (1H, s)

[Example 182]

N-((R)-2-Acetylamino-3-(acetylthio)propionyl)-3-(4-ethylpiperazin-1-yl)sydnonimine

The procedure of Example 133 was repeated but starting with (R)-2-acetylamino-3-(acetylthio)propionic acid to give N-((R)-2-acetylamino-3-(acetylthio)propionyl)-3-(4-ethylpiperazin-1-yl)sydnonimine (m.p.: 132 - 133°C).

270 MHz-NMR ($CDCl_3$) δ : 1.12 (3H, t), 2.02 (3H, s), 2.31 (3H, s), 2.52 (2H, q), 2.73-2.77 (4H, m), 3.44 (1H, dd), 3.55-3.64 (5H, m), 4.77-4.84 (1H, m), 6.53 (1H, brd), 7.97 (1H, s)

[Example 183]

N-((R)-2-Acetylamino-3-(acetylthio)propionyl)-3-(4-isopropylpiperazin-1-yl)sydnonimine

The procedure of Example 134 was repeated but starting with (R)-2-acetylamino-3-(acetylthio)propionic acid to give N-((R)-2-acetylamino-3-(acetylthio)propionyl)-3-(4-isopropylpiperazin-1-yl)sydnonimine (m.p.: 146 - 148°C).

270 MHz-NMR ($CDCl_3$) δ : 1.08 (6H, d), 2.02 (3H, s), 2.31 (3H, s), 2.77-2.87 (5H, m), 3.44 (1H, dd), 3.53-3.64 (5H, m), 4.77-4.84 (1H, m), 6.56 (1H, brd), 7.97 (1H, s)

[Example 184]

N-((R)-2-Acetylamino-3-(benzoylthio)propionyl)-3-(4-methylpiperazin-1-yl)sydnonimine

(1) The procedure of Example 181 (1) was repeated but replacing acetic anhydride by benzoyl chloride to give (R)-2-acetylamino-3-(benzoylthio)propionic acid.

270 MHz-NMR ($CDCl_3$ + DMSO-$d_6$) d : 2.00 (3H, s), 3.54 (1H, dd), 3.70 (1H, dd), 4.80-4.87 (1H,m), 6.64 (1H, brd), 7.42-7.49 (2H, m), 7.56-7.62 (1H, m), 7.94-7.97 (2H, m)

(2) The procedure of Example 1 was repeated but starting with (R)-2-acetylamino-3-(benzoylthio)propionic acid to give N-((R)-2-acetylamino-3-(benzoylthio)propionyl)-3-(4-methylpiperazin-1-yl)sydnonimine (m.p.: 130 - 132°C).

270 MHz-NMR ($CDCl_3$) δ : 2.01 (3H, s), 2.38 (3H, s), 2.70-2.74(4H, m), 3.54-3.58 (4H, m), 3.67 (1H, dd), 3.82 (1H, dd), 4.88-4.95 (1H, m), 6.63 (1H, brd), 7.38-7.45 (2H, m), 7.52-7.58 (1H, m), 7.92-7.95 (2H, m), 7.98 (1H, s)

[Example 185]

N-((R)-2-Acetylamino-3-(4-fluorobenzoylthio)propionyl)-3-(4-methylpiperazin-1-yl)sydnonimine

(1) The procedure of Example 181 (1) was repeated but replacing acetic anhydride by 4-fluorobenzoic acid chloride to give (R)-2-acetylamino-3-(4-fluorobenzoylthio)propionic acid.

270 MHz-NMR ($CDCl_3$) δ : 2.00 (3H, s), 3.53 (1H, dd), 3.70 (1H, dd), 4.80-4.88 (1H, m), 6.53 (1H, brd), 7.09-7.16 (2H, m), 7.96-8.01 (2H, m)

(2) The procedure of Example 1 was repeated but starting with (R)-2-acetylamino-3-(4-fluorobenzoylthio)propionic acid to give N-((R)-2-acetylamino-3-(4-fluorobenzoylthio)propionyl)-3-(4-methylpiperazin-1-yl)sydnonimine (m.p.: 132 - 134°C).

270 MHz-NMR ($CDCl_3$) δ : 2.01 (3H, s), 2.38 (3H, s), 2.70-2.74 (4H, m), 3.54-3.58 (4H, m), 3.65 (1H, dd), 3.82 (1H, dd), 4.87-4.94 (1H, m), 6.60 (1H, brd), 7.05-7.12 (2H, m), 7.93-7.99 (2H, m), 7.98 (1H, s)

[Example 186]

N-((R)-2-Acetylamino-3-(4-methoxybenzoylthio)propionyl)-3-(4-methylpiperazin-1-yl)sydnonimine

(1) At room temperature, 1.1 ml of pyridine and 70 mg of 4-dimethylaminopyridine were added to a mixture of 2 g of 4-methoxybenzoyl chloride, 1.91 g of N-acetyl-L-cysteine and 12 ml of chloroform and the resultant mixture was stirred for 20 hours at room temperature. Next, the mixture was acidified with hydrochloric acid and then extracted with dichloromethane. The organic layer was washed with water and dried and the solvent was distilled off. The residue thus obtained was washed with ethyl acetate to give 0.54 g of (R)-2-acetylamino-3-(4-methoxybenzoylthio)propionic acid.

270 MHz-NMR ($CDCl_3$) δ : 1.99 (3H, s), 3.43-3.68 (2H, m), 3.87 (3H, s), 4.77-4.86 (1H, m), 6.58-6.64 (1H, m), 6.93 (2H, brd), 7.93 (2H, brd)

(2) The procedure of Example 1 was repeated but starting with (R)-2-acetylamino-3-(4-methoxybenzoylthio)propionic acid to give N-((R)-2-acetylamino-3-(4-methoxybenzoylthio)propionyl)-3-(4-methylpiperazin-1-yl)sydnonimine as an oily substance.

270 MHz-NMR ($CDCl_3$) δ : 2.01 (3H, s), 2.38 (3H, s), 2.70-2.74 (4H, m), 3.53-3.58 (4H, m), 3.59-3.87 (2H, m), 3,86 (3H, s), 4.86-4.94 (1H, m), 6.62 (1H, brd), 6.89 (2H, d), 7.92 (2H, d), 7.98 (1H, s)

[Example 187]

N-((R)-2-Acetylamino-3-(2,6-dimethylbenzoylthio)propionyl)-3-(4-methylpiperazin-1-yl)sydnonimine

(1) A mixture of 2 g of 2,6-dimethylbenzoic acid, 5 ml of thionyl chloride and 0.5 ml of N,N-dimethylformamide was heated under reflux for 2 hours. After concentrating the mixture, 2.17 g of N-acetyl-L-cysteine, 1.3 ml of pyridine, 80 mg of 4-dimethylaminopyridine and 15 ml of chloroform were added thereto at room temperature. Then the resultant mixture was stirred for 3 hours at room temperature. Next, the mixture was acidified with hydrochloric acid and then extracted with dichloromethane. The organic layer was washed with water and dried and the solvent was distilled off. The residue thus obtained was purified by silica gel column chromatography (developing solution: chloroform : methanol : acetic acid = 100 : 5 : 1) to give 0.23 g of (R)-2-acetylamino-3-(2,6-dimethylbenzoylthio)propionic acid as an oily substance.

270 MHz-NMR ($CDCl_3$) δ : 2.03 (3H, s), 2.30 (6H, s), 3.51-3.72 (2H, m), 4.80-4.88 (1H, m), 6.51 (1H, brd), 7.02 (2H, d), 7.19 (1H, t)

(2) The procedure of Example 1 was repeated but starting with (R)-2-acetylamino-3-(2,6-dimethylbenzoylthio)propionic acid to give N-((R)-2-acetylamino-3-(2,6-dimethylbenzoylthio)propionyl)-3-(4-methylpiperazin-1-yl)sydnonimine (m.p.: 166 - 167°C).

270 MHz-NMR ($CDCl_3$) δ : 2.04 (3H, s), 2.28 (6H, s), 2.37 (3H, s), 2.68-2.73 (4H, m), 3.53-3.58 (4H, m), 3.62-3.86 (2H, m), 4.87-4.94 (1H, m), 6.74 (1H, brd), 6.98 (2H, d), 7.15 (1H, t), 7.99 (1H, s)

[Example 188]

N-((R)-2-Acetylamino-3-(2,6-dichlorobenzoylthio)propionyl)3-(4-methylpiperazin-1-yl)sydnonimine

(1) The procedure of Example 181 (1) was repeated but starting with 2,6-dichlorobenzoyl chloride to give (R)-2-acetylamino-3-(2,6-dichlorobenzoylthio)propionic acid.

270 MHz-NMR ($CDCl_3$) δ : 2.02 (3H, s), 3.58 (1H, dd), 3.81 (1H, dd), 4.84-4.91 (1H, m), 6.65 (1H, brd), 7.32-7.38 (3H, m)

(2) The procedure of Example 1 was repeated but starting with (R)-2-acetylamino-3-(2,6-dichlorobenzoylthio)propionic acid to give N-((R)-2-acetylamino-3-(2,6-dichlorobenzoylthio)propionyl)-3-(4-methylpiperazin-1-yl)sydnonimine (m.p.: 148.5 - 149.5°C).

270 MHz-NMR ($CDCl_3$) δ : 2.04 (3H, s), 2.38 (3H, s), 2.70-2.74 (4H, m), 3.54-3.58 (4H, m), 3.72 (1H, dd), 3.94 (1H, dd), 4.92-4.99 (1H, m), 6.66 (1H, brd), 7.21-7.32 (3H, m), 7.98 (1H, s)

[Example 189]

N-((R)-2-Acetylamino-3-(2,6-difluorobenzoylthio)propionyl)-3-(4-methylpiperazin-1-yl)sydnonimine

(1) The procedure of Example 181 (1) was repeated but starting with 2,6-difluorobenzoyl chloride to give (R)-2-acetylamino-3-(2,6-difluorobenzoylthio)propionic acid.

270 MHz-NMR ($CDCl_3$) $\delta$ : 2.02 (3H, s), 3.58 (1H, dd) 3,75 (1H, dd), 4.83-4.90 (1H, m), 6.51 (1H, brd), 6.93-6.99 (2H, m), 7.37-7.48 (1H, m)

(2) The procedure of Example 1 was repeated but starting with (R)-2-acetylamino-3-(2,6-difluorobenzoylthio)propionic acid to give N-((R)-2-acetylamino-3-(2,6-difluorobenzoylthio)propionyl)-3-(4-methylpiperazin-1-yl)sydnonimine (m.p.: 134 - 136°C).

270 MHz-NMR ($CDCl_3$) $\delta$ : 2.03 (3H, s), 2.38 (3H, s), 2.70-2.74 (4H, m), 3.54-3.58 (4H, m), 3.71 (1H, dd), 3.87 (1H, dd), 4.89-4.96 (1H, m), 6.64 (1H, brd), 6.87-6.95 (2H, m), 7.31-7.42 (1H, m), 7.98 (1H, s)

[Example 190]

N-((R)-2-Acetylamino-3-(4-trifluoromethylbenzoylthio)propionyl)-3-(4-methylpiperazin-1-yl)sydnonimine

(1) The procedure of Example 181 (1) was repeated but starting with 4-trifluoromethylbenzoyl chloride to give (R)-2-acetylamino-3-(4-trifluoromethylbenzoylthio)propionic acid.

270 MHz-NMR ($CDCl_3$) $\delta$ : 2.01 (3H, s), 3.56 (1H, dd), 3.76 (1H, dd), 4.83-4.90 (1H, m), 6.54 (1H, brd), 7.72 (2H, d), 8.06 (2H, d)

(2) The procedure of Example 1 was repeated but starting with (R)-2-acetylamino-3-(4-trifluoromethylbenzoylthio)propionic acid to give N-((R)-2-acetylamino-3-(4-trifluoromethylbenzoylthio)propionyl)-3-(4-methyl piperazin-1-yl)sydnonimine (m.p.: 101 - 103°C).

270 MHz-NMR ($CDCl_3$) $\delta$ : 2.01 (3H, s), 2.38 (3H, s), 2.70-2.74 (4H, m), 3.54-3.58 (4H, m), 3.65 (1H, dd), 3.82 (1H, dd), 4.87-4.94 (1H, m), 6.61 (1H, brd), 7.69 (2H, d), 7.98 (1H, s), 8.04 (2H, d)

[Example 191]

N-((6-Hydroxy-2,5,7,8-tetramethylchroman-2-carbonylthio)acetyl)-3-(4-methylpiperazin-1-yl)sydnonimine

(1) 0.8 ml of thionyl chloride was added to a mixture of 1.25 g of 6-hydroxy-2,5,7,8-tetramethylchroman-2-carboxylic acid and 10 ml of chloroform and the resultant mixture was heated under reflux for 2 hours. After concentrating, the mixture was dissolved in 10 ml of chloroform and then added at room temperature to a mixture of 0.7 ml of mercaptoacetic acid, 0.8 g of sodium hydroxide and 10 ml of water. Next, the resultant mixture was stirred for 30 minutes at room temperature. Then it was acidified with hydrochloric acid and extracted with chloroform. The organic layer was washed with water and dried and the solvent was distilled off. The residue thus obtained was purified by silica gel column chromatography (developing solution: hexane : ethyl acetate : acetic acid = 200 : 100 : 1) to give 0.4 g of (6-hydroxy-2,5,7,8-tetramethylchroman-2-carbonylthio)acetic acid as an oily substance.

270 MHz-NMR ($CDCl_3$) $\delta$ : 1.55 (3H, s), 1.80-1.95 (1H, m), 2.07 (3H, s), 2.19 (3H, s), 2.25 (3H, s), 2.33-2.69 (3H, m), 3.50 (1H, d), 3.66 (1H, d), 5.30 (1H, s)

(2) The procedure of Example 1 was repeated but starting with (6-hydroxy-2,5,7,8-tetramethylchroman-2-carbonylthio)acetic acid to give N-((6-hydroxy-2,5,7,8-tetramethylchroman-2-carbonylthio)acetyl)-3-(4-methylpiperazin-1-yl)sydnonimine (m.p.: 164 - 165°C).

270 MHz-NMR ($CDCl_3$) $\delta$ : 1.56 (3H, s), 1.77-1.90 (1H, m), 2.07 (3H, s), 2.17 (3H, s), 2.26 (3H, s), 2.37 (3H, s), 2.37-2.60 (3H, m), 2.61-2.73 (4H, m), 3.46-3.55 (4H, m), 3.67 (1H, d), 3.87 (1H, d), 7.96 (1H, s)

[Example 192]

N-((6-Hydroxy-2,5,7,8-tetramethylchroman-2-carbonylthio)acetyl)-3-(4-ethylpiperazin-1-yl)sydnonimine

The procedure of Example 133 was repeated but starting with (6-hydroxy-2,5,7,8-tetramethylchroman-2-carbonylthio)acetic acid to give N-((6-hydroxy-2,5,7,8-tetramethylchroman-2-carbonylthio)acetyl)-3-(4-ethylpiperazin-1-yl)sydnonimine (m.p.: 152 - 153°C).

270 MHz-NMR ($CDCl_3$) δ : 1.11 (3H, t), 1.56 (3H, s), 1.78-1.89 (1H, m), 2.07 (3H, s), 2.18 (3H, s), 2.26 (3H, s), 2.37-2.60 (3H, m), 2.51 (2H, q), 2.71-2.75 (4H, m), 3.51-3.55 (4H, m), 3.67 (1H, d), 3.88 (1H,d), 4.31 (1H, brs), 7.95 (1H, s)

[Example 193]

N-((6-Hydroxy-2,5,7,8-tetramethylchroman-2-carbonylthio)acetyl)-3-(4-isopropylpiperazin-1-yl)sydnonimine

The procedure of Example 134 was repeated but starting with (6-hydroxy-2,5,7,8-tetramethylchroman-2-carbonylthio)acetic acid to give N-((6-hydroxy-2,5,7,8-tetramethylchroman-2-carbonylthio)acetyl)-3-(4-isopropylpiperazin-1-yl)sydnonimine (m.p.: 172 - 173°C).

270 MHz-NMR ($CDCl_3$) δ : 1.08 (6H, d), 1.56 (3H, s), 1.77-1.90 (1H, m), 2.07 (3H, s), 2.18 (3H, s), 2.26 (3H, s), 2.39-2.54 (1H, m), 2.55-2.63 (3H, m), 2.77-2.82 (4H, m), 3.49-3.54 (4H, m), 3.64 (1H, d), 3.88 (1H, d), 4.31 (1H, brs), 7.95 (1H, s)

[Example 194]

N-(6-Hydroxy-2,5,7,8-tetramethylchroman-2-carbonyl)-3-morpholinosydnonimine

0.16 ml of thionyl chloride was added to a mixture of 0.5 g of 6-hydroxy-2,5,7,8-tetramethylchroman-2-carboxylic acid and 3 ml of chloroform and the resultant mixture was heated under reflux for 3 hours. After concentrating the mixture, 5 ml of pyridine and 0.41 g of 3-morpholinosydnonimine hydrochloride were added thereto at room temperature and the resultant mixture was stirred for 18 hours at room temperature. After adding water, the mixture was extracted with dichloromethane. The organic layer was washed with water and dried. After distilling off the solvent, the obtained residue was purified by silica gel column chromatography (developing solution: hexane : ethyl acetate = 1 : 2) to give 0.19 g of N-(6-hydroxy-2,5,7,8-tetramethylchroman-2-carbonyl)-3-morpholinosydnonimine (m.p.: 139 - 141°C).

270 MHz-NMR ($CDCl_3$) δ : 1.66 (3H, s), 1.80-1.96 (1H, m), 2.06-2.11 (3H, m), 2.16-2.19 (3H, m), 2.23 (3H, s), 2.40-2.69 (3H, m), 3.44-3.51 (4H, m), 3.92-3.97 (4H, m), 8.01 (1H, s)

[Example 195]

N-(6-Hydroxy-2,5,7,8-tetramethylchroman-2-carbonyl)-3-(4-methylpiperazin-1-yl)sydnonimine

The procedure of Example 1 was repeated but starting with 6-hydroxy-2,5,7,8-tetramethylchroman-2-carboxylic acid to give N-(6-hydroxy-2,5,7,8-tetramethylchroman-2-carbonyl)-3-(4-methylpiperazin-1-yl)sydnonimine (m.p.: 164 - 166°C).

270 MHz-NMR ($CDCl_3$) δ : 1.65 (3H, s), 1.82-1.92 (1H, m), 2.02 (3H, s), 2.15 (3H, s), 2.26 (3H, s), 2.35 (3H, s), 2.42-2.62 (3H, m), 2.65-2.69 (4H, m), 3.46-3.50 (4H, m), 4.20 (1H, brs), 8.00 (1H, s)

[Example 196]

N-(6-Hydroxy-2,5,7,8-tetramethylchroman-2-carbonyl)-3-(4-benzylpiperazin-1-yl)sydnonimine

The procedure of Example 17 was repeated but starting with 6-hydroxy-2,5,7,8-tetramethylchroman-2-carboxylic acid to give N-(6-hydroxy-2,5,7,8-tetramethylchroman-2-carbonyl)-3-(4-benzylpiperazin-1-yl)sydnonimine (m.p.: 99 - 101°C).

270 MHz-NMR ($CDCl_3$) δ : 1.65 (3H, s), 1.81-1.95 (1H, m), 2.02 (3H, s), 2.15 (3H, s), 2.26 (3H, s), 2.46-2.71 (3H, m), 2.66-2.74 (4H, m), 3.43-3.52 (4H, m), 3.58 (2H, s), 4.17 (1H, s), 7.25-7.34 (5H, m), 7.99 (1H, s)

[Example 197]

N-(6-Methoxymethyloxy-2,5,7,8-tetramethylchroman-2-carbonyl)-3-(4-benzylpiperazin-1-yl)sydnonimine

At room temperature, 0.8 ml of methoxymethyl chloride was added to a mixture of 2.1 g of N-(6-hydroxy-2,5,7,8-tetramethylchroman-2-carbonyl)-3-(4-benzylpiperazin-1-yl)sydnonimine, 2 ml of N,N-diisopropylethylamine and 20 ml of dichloromethane and the resultant mixture was stirred for 15 hours at room temperature and then for 5 hours at 40°C. After adding water, the mixture was extracted with dichloromethane. The organic layer was washed with water and dried. After distilling off the solvent, the obtained residue was purified by silica gel column chromatography (developing solution: hexane : ethyl acetate = 1 : 1) to give 0.88 g of N-(6-methoxymethyloxy-2,5,7,8-tetramethylchroman-2-carbonyl)-3-(4-benzylpiperazin-1-yl)sydnonimine as an oily substance.

270 MHz-NMR ($CDCl_3$) δ : 1.65 (3H, s), 1.80-1.96 (1H, m), 2.07 (3H, s), 2.18 (3H, s), 2.23 (3H, s), 2.39-2.75 (3H, m), 2.69-2.77 (4H, m), 3.45-3.51 (4H, m), 3.57 (2H, s), 3.59 (3H, s), 4.79-4.85 (2H, m), 7.26-7.35 (5H, m), 7.99 (1H, s)

[Example 198]

N-(6-Methoxymethyloxy-2,5,7,8-tetramethylchroman-2-carbonyl)-3-(4-benzyloxycarbonylpiperazin-1-yl)sydnonimine

The procedure of Example 19-A was repeated but starting with N-(6-methoxymethyloxy-2,5,7,8-tetramethylchroman-2-carbonyl)-3-(4-benzylpiperazin-1-yl)sydnonimine to give N-(6-methoxymethyloxy-2,5,7,8-tetramethylchroman-2-carbonyl)-3-(4-benzyloxycarbonylpiperazin-1-yl)sydnonimine as an oily substance.

270 MHz-NMR ($CDCl_3$) δ : 1.65 (3H, s), 1.80-1.92 (1H, m), 2.07 (3H, s), 2.19 (3H, s), 2.23 (3H, s), 2.44-2.66 (3H, m), 3.39-3.46 (4H, m), 3.60 (3H, s), 3.75-3.81 (4H, m), 4.80-4.85 (2H, m), 5.15 (2H, s), 7.36 (5H, s), 8.01 (1H, s)

[Example 199]

N-(6-Hydroxy-2,5,7,8-tetramethylchroman-2-carbonyl)-3-(piperazin-1-yl)sydnonimine hydrochloride

The procedure of Example 24-C was repeated but starting with N-(6-methoxymethyloxy-2,5,7,8-tetramethylchroman-2-carbonyl)-3-(4-benzyloxycarbonyl-piperazin-1-yl)sydnonimine to give N-(6-hydroxy-2,5,7,8-tetramethylchroman-2-carbonyl)-3-(piperazin-1-yl)sydnonimine hydrochloride as an amorphous substance.

270 MHz-NMR (DMSO-$d_6$) δ : 1.56 (3H, s), 1.76-1.85 (1H, m), 1.95 (3H, s), 2.06 (3H, s), 2.10 (3H, s), 2.29-2.62 (3H, m), 3.38 (4H, brs), 3.92 (4H, brs), 8.98 (1H, brs), 9.71 (1H, brs)

[Example 200]

N-(6-Hydroxy-2,5,7,8-tetramethylchroman-2-carbonyl)-3-(4-ethylpiperazin-1-yl)sydnonimine

The procedure of Example 68 was repeated but starting with N-(6-hydroxy-2,5,7,8-tetramethylchroman-2-carbonyl)-3-(piperazin-1-yl)sydnonimine hydrochloride to give N-(6-hydroxy-2,5,7,8-tetramethylchroman-2-carbonyl)-3-(4-ethyl piperazin-1-yl)sydnonimine (m.p.: 137 - 138°C).

270 MHz-NMR ($CDCl_3$) δ : 1.10 (3H, t), 1.65 (3H, s), 1.80-1.92 (1H, m), 2.01 (3H, s), 2.13 (3H, s), 2.25 (3H, s), 2.49 (2H, q), 2.50-2.71 (7H, m), 3.46-3.51 (4H, m), 4.38 (1H, brs), 8.00 (1H, s)

[Example 201]

N-(6-Hydroxy-2,5,7,8-tetramethylchroman-2-carbonyl)-3-(4-isopropylpiperazin-1-yl)sydnonimine

The procedure of Example 69 was repeated but starting with N-(6-hydroxy-2,5,7,8-tetramethylchroman-2-carbonyl)-3-(piperazin-1-yl)sydnonimine hydrochloride to give N-(6-hydroxy-2,5,7,8-tetramethylchroman-2-carbonyl)-3-(4-isopropylpiperazin-1-yl)sydnonimine (m.p.: 180 - 182°C).

270 MHz-NMR ($CDCl_3$) δ : 1.06 (6H, d), 1.65 (3H, s), 1.82-1.91 (1H, m), 2.01 (3H, s), 2.13 (3H, s), 2.25 (3H, s), 2.42-2.71 (3H, m), 2.73-2.84 (5H, m), 3.44-3.49 (4H, m), 4.36 (1H, brs), 7.99 (1H, s)

[Example 202]

N-(2,3-Dihydro-5-hydroxy-2,4,6,7-tetramethylbenzofuran-2-carbonyl)-3-(4-methylpiperazin-1-yl)sydnonimine

The procedure of Example 1 was repeated but starting with 2,3-dihydro-5-hydroxy-2,4,6,7-tetramethylbenzofuran-2-carboxylic acid to give N-(2,3-dihydro-5-hydroxy-2,4,6,7-tetramethylbenzofuran-2-carbonyl)-3-(4-methylpiperazin-1-yl)sydnonimine (m.p.: 120 - 122°C).

270 MHz-NMR ($CDCl_3$) δ : 1.76 (3H, s), 2.10 (3H, s), 2.37 (3H, s), 2.49 (3H, s), 2.68-2.72 (4H, m), 3.11 (1H, d), 3.51-3.56 (4H, m), 3.72 (1H, d), 5.30 (1H, s), 8.02 (1H, s)

[Example 203]

N-((R)-2-Oxo-4-thiazolidinecarbonyl)-3-(4-methylpiperazin-1-yl)sydnonimine

The procedure of Example 1 was repeated but starting with (-)-2-oxo-4-thiazolidinecarboxylic acid to give N-((R)-2-oxo-4-thiazolidinecarbonyl)-3-(4-methylpiperazin-1-yl)sydnonimine.

270 MHz-NMR ($CDCl_3$ + DMSO-$d_6$) δ : 2.38 (3H, s), 2.71-2.75 (4H, m), 3.60-3.64 (4H, m), 3.69 (2H, d), 4.54 (1H, t), 7.04 (1H, brs), 8.35 (1H, s)

[Example 204]

N-(Cyclohexanecarbonyl)-3-(4-methylpiperazin-1-yl)sydnonimine

The procedure of Example 1 was repeated but starting with cyclohexanecarboxylic acid to give N-(cyclohexanecarbonyl)-3-(4-methylpiperazin-1-yl)sydnonimine (m.p.: 143 - 146°C).

270 MHz-NMR ($CDCl_3$) δ : 1.25-1.98 (10H, m), 2.37 (3H, s), 2.36-2.45 (1H, m), 2.69-2.73 (4H, m), 3.51-3.55 (4H, m), 7.97 (1H, s)

[Example 205]

N-(Cyclohexanecarbonyl)-3-(4-benzylpiperazin-1-yl)sydnonimine

The procedure of Example 17 was repeated but starting with cyclohexanecarboxylic acid to give N-(cyclohexanecarbonyl)-3-(4-benzylpiperazin-1-yl)sydnonimine (m.p.: 119 - 121°C).

270 MHz-NMR ($CDCl_3$) δ : 1.23-1.98 (10H, m), 2.35-2.45 (1H, m), 2.72-2.76 (4H, m), 3.50-3.54 (4H, m), 3.60 (2H, s), 7.28-7.38 (5H, m), 7.96 (1H, s)

[Example 206]

N-(Cyclohexanecarbonyl)-3-(4-benzyloxycarbonylpiperazin-1-yl)sydnonimine

The procedure of Example 19-A was repeated but starting with N-(cyclohexanecarbonyl)-3-(4-benzylpiperazin-1-yl)sydnonimine to give N-(cyclohexanecarbonyl)-3-(4-benzyloxycarbonylpiperazin-1-yl)sydnonimine.

270 MHz-NMR ($CDCl_3$) δ : 1.25-1.98 (10H, m), 2.35-2.45 (1H, m), 3.45-3.49 (4H, m), 3.79-3.83 (4H, m), 5.17 (2H, s), 7.29-7.42 (5H, m), 7.98 (1H, s)

[Example 207]

N-(Cyclohexanecarbonyl)-3-(piperazin-1-yl)sydnonimine hydrobromide

The procedure of Example 19-B was repeated but starting with N-(cyclohexanecarbonyl)-3-(4-benzyloxycarbonylpiperazin-1-yl)sydnonimine to give N-(cyclohexanecarbonyl)-3-(piperazin-1-yl)sydnonimine hydrobromide.

270 MHz-NMR (DMSO-$d_6$) δ : 1.24-1.91 (10H, m), 2.50-2.59 (1H, m), 3.37-3.49 (4H, m), 3.98-4.01 (4H, m), 9.14 (1H, brs), 9.36 (1H, s)

[Example 208]

N-(Cyclohexanecarbonyl)-3-(4-(acetylthioacetyl)piperazin-1-yl)sydnonimine

Under ice cooling, a mixture of 0.08 g of (acetylthio)acetic acid, 0.12 g of 1-ethyl-3-(3-dimethylaminopropyl)carbodiimide hydrochloride, 0.09 g of 1-hydroxybenzotriazole and 6 ml of N,N-dimethylformamide was stirred for 25 minutes. Next, 0.2 g of N-(cyclohexanecarbonyl)-3-(piperazin-1-yl)sydnonimine hydrobromide and 0.18 ml of pyridine were added thereto and the resultant mixture was stirred for 10 minutes and then for 16 hours at room temperature. After adding water and a saturated aqueous solution of sodium bicarbonate, the mixture was extracted with ether. The organic layer was dried and the solvent was distilled off. The residue thus obtained was purified by silica gel column chromatography (developing solution: dichloromethane : methanol = 95 : 5) to give 0.12 g of N-(cyclohexanecarbonyl)-3-(4-(acetylthioacetyl)piperazin-1-yl)sydnonimine as an oily substance.

270 MHz-NMR ($CDCl_3$) δ : 1.23-1.97 (10H, m), 2.34-2.44 (1H, m), 2.40 (3H, s), 3.48-3.61 (4H, m), 3.83 (2H, s), 3.84-3.90 (4H, m), 8.02 (1H, s)

[Example 209]

N-(Cyclohexanecarbonyl)-3-(4-(benzoylthioacetyl)piperazin-1-yl)sydnonimine

The procedure of Example 208 was repeated but starting with (benzoylthio)acetic acid to give N-(cyclohexanecarbonyl)-3-(4-(benzoylthioacetyl)piperazin-1-yl)sydnonimine (m.p.: 156 - 157.5°C).

270 MHz-NMR ($CDCl_3$) δ : 1.19-1.98 (10H, m), 2.35-2.45 (1H, m), 3.49-3.62 (4H, m), 3.94-3.99 (4H, m), 4.01 (2H, s), 7.45-7.51 (2H, m), 7,59-7.66 (1H, m), 7.95-7.99 (2H, m), 8.01 (1H, s)

[Example 210]

N-(Cyclohexanecarbonyl)-3-(4-(3,4,5-trimethoxyphenyl)acetylpiperazin-1-yl)sydnonimine

The procedure of Example 208 was repeated but starting with 3,4,5-trimethoxyphenylacetic acid to give N-(cyclohexanecarbonyl)-3-(4-(3,4,5-trimethoxyphenyl)acetylpiperazin-1-yl)sydnonimine (m.p.: 214 - 216°C).

270 MHz-NMR ($CDCl_3$) δ : 1.25-1.97 (10H, m), 2.34-2.45 (1H, m), 3.32-3.38 (2H, m), 3.42-3.50 (2H, m), 3.70-3.80 (2H, m), 3.72 (2H, s), 3.84 (3H, s), 3.85 (6H, s), 3.91-3.98 (2H, m), 6.44 (2H, s), 7.96 (1H, s)

[Example 211]

N-(Cyclohexanecarbonyl)-3-(4-(3,5-di-t-butyl-4-hydroxyphenylthio)acetylpiperazin-1-yl)sydnonimine

The procedure of Example 208 was repeated but starting with (3,5-di-t-butyl-4-hydroxyphenylthio)acetic acid to give N-(cyclohexanecarbonyl)-3-(4-(3,5-di-t-butyl-4-hydroxyphenylthio)acetylpiperazin-1-yl)sydnonimine as an oily substance.

270 MHz-NMR ($CDCl_3$) δ : 1.18-1.97 (10H, m), 1.42 (18H, s), 2.35-2.45 (1H, m), 3.42-3.46 (4H, m), 3.67 (2H, s), 3.69-3.77 (2H, m), 3.87-3.92 (2H, m), 5.35 (1H, s), 7.30 (2H, s), 7.98 (1H, s)

[Example 212]

N-(Cyclohexanecarbonyl)-3-(4-(2-acetylthioethyl)piperazin-1-yl)sydnonimine

The procedure of Example 53-A was repeated but replacing (benzoylthio)acetic acid by cyclohexanecarboxylic acid to give N-(cyclohexanecarbonyl)-3-(4-(2-chloroethylpiperazin-1-yl)sydnonimine. Next, the procedure of Example 53-B was repeated but starting with N-(cyclohexanecarbonyl)-3-(4-(2-chloroethylpiperazin-1-yl)sydnonimine to give N-(cyclohexanecarbonyl)-3-(4-(2-acetylthioethyl)piperazin-1-yl)sydnonimine (m.p.: 121 - 122°C).

270 MHz-NMR ($CDCl_3$) $\delta$ : 1.20-1.57 (4H, m), 1.58-1.84 (4H, m), 1.91-2.01 (2H, m), 2.35 (3H, s), 2.35-2.45 (1H, m), 2.63 (2H, dd), 2.77-2.83 (4H, m), 3.02 (2H, dd), 3.48-3.54 (4H, m), 7.96 (1H, s)

[Example 213]

N-(Cyclohexanecarbonyl)-3-(4-(3,4,5-trimethoxyphenylmethyl)piperazin-1-yl)sydnonimine

The procedure of Example 48 (3) was repeated but replacing (acetylthio)acetic acid by cyclohexanecarboxylic acid to give N-(cyclohexanecarbonyl)-3-(4-(3,4,5-trimethoxyphenylmethyl)piperazin-1-yl)sydnonimine as an oily substance.

270 MHz-NMR ($CDCl_3$) $\delta$ : 1.20-1.57 (4H, m), 1.60-1.83 (4H, m), 1.91-2.04 (2H, m), 2.36-2.48 (1H, m), 2.72-2.77 (4H, m), 3.52 (2H, s), 3.52-3.57 (4H, m), 3.85 (3H, s), 3.87 (6H, s), 6.55 (2H,s), 7.98 (1H, s)

[Example 214]

N-(Cyclohexanecarbonyl)-3-(4-(2,3,4-trimethoxyphenylmethyl)piperazin-1-yl)sydnonimine

The procedure of Example 50 was repeated but replacing (acetylthio)acetic acid by cyclohexanecarboxylic acid to give N-(cyclohexanecarbonyl)-3-(4-(2,3,4-trimethoxyphenylmethyl)piperazin-1-yl)sydnonimine (m.p.: 163 - 164°C).

270 MHz-NMR ($CDCl_3$) $\delta$ : 1.19-1.51 (4H, m), 1.60-1.83 (4H, m), 1.93-2.01 (2H, m), 2.35-2.45 (1H, m), 2.73-2.78 (4H, m), 3.48-3.53 (4H, m), 3.56 (2H, s), 3.87 (3H, s), 3.88 (3H, s), 3.89 (3H, s), 6.66 (1H, d), 6.96 (1H, d), 7.95 (1H, s)

[Example 215]

N-(Cyclohexanecarbonyl)-3-(N'-allyl-N'-methylamino)sydnonimine

The procedure of Example 56-A was repeated but starting with cyclohexanecarboxylic acid to give N-(cyclohexanecarbonyl)-3-(N'-allyl-N'-methylamino)sydnonimine.

270 MHz-NMR ($CDCl_3$) $\delta$ : 1.20-1.52 (4H, m), 1.63-1.82 (4H, m), 1.93-1.99 (2H, m), 2.36-2.47 (1H, m), 3.17 (3H, s), 4.01 (2H, d), 5.34-5.41 (2H, m), 5.77-5.90 (1H, m), 7.95 (1H, s)

[Example 216]

N-(Cyclohexanecarbonyl)-3-methylaminosydnonimine

The procedure of Example 56-B was repeated but starting with N-(cyclohexanecarbonyl)-3-(N'-allyl-N'-methylamino)sydnonimine to give N-(cyclohexanecarbonyl)-3-methylaminosydnonimine.

270 MHz-NMR ($CDCl_3$) $\delta$ : 1.18-1.50 (4H, m), 1.51-2.01 (6H, m), 2.30-2.43 (1H, m), 3.22 (3H, s), 7.29-7.75 (2H, m)

[Example 217]

N-(Cyclohexanecarbonyl)-3-(N'-acetylthioacetyl-N'-methylamino)sydnonimine

Under ice cooling, a mixture of 0.06 g of (acetylthio)acetic acid, 0.09 g of 1-ethyl-3-(3-dimethylaminopropyl)carbodiimide hydrochloride, 0.07 g of 1-hydroxybenzotriazole hydrate and 2 ml of tetrahydrofuran was stirred for 30 minutes. Next, 0.1 g of N-(cyclohexanecarbonyl)-3-methylaminosydnonimine was added thereto and the resultant mixture was stirred for 30 minutes and then for additional 30 minutes at room temperature. After adding water, the mixture was extracted with ethyl acetate. The organic layer was washed with water and dried, and the solvent was distilled off. The residue thus obtained was purified by silica gel column chromatography (developing solution: hexane : ethyl acetate = 1 : 2) to give 0.08 g of N-(cyclohexanecarbonyl)-3-(N'-acetylthioacetyl-N'-methylamino)sydnonimine (m.p.: 179 - 180°C).

270 MHz-NMR ($CDCl_3$) δ : 1.20-1.58 (4H, m), 1.60-1.91 (4H, m), 1.92-2.03 (2H, m), 2.38 (3H, s), 2.38-2.52 (1H, m), 3.66 (5H, s), 8.25 (1H, s)

[Referential Example 1]

Synthesis of 3-(4-(2-methoxyphenyl)piperazin-1-yl)sydnonimine hydrochloride

At room temperature, a mixture of 2.48 g of 1-amino-4-(2-methoxyphenyl)piperazine, 1.69 g of formaldehyde sodium bisulfite adduct and 23 ml of water was stirred for 30 minutes. After adding 0.62 g of sodium cyanide, the resultant mixture was stirred for 5 hours at 60°C. Then the mixture was extracted with ether. The organic layer was washed with water and dried, and the solvent was distilled off. The residue thus obtained was purified by silica gel column chromatography (developing solution: hexane : ethyl acetate = 1 : 1) to give 0.9 g of 1-(cyanomethylamino)-4-(2-methoxyphenyl)piperazine as an oily substance. Under ice cooling, a mixture of 0.89 g of the obtained 1-(cyanomethylamino)-4-(2-methoxyphenyl)piperazine, 0.82 g of conc. sulfuric acid, sodium nitrite and 4 ml of water was stirred for 30 minutes. After neutralizing with 2 N sodium hydroxide, the mixture was extracted with ether. The organic layer was washed with water and dried. After distilling off the solvent, the obtained residue was diluted with 10 ml of methanol. After adding 1 ml of conc. hydrochloric acid, the mixture was concentrated and the crystals thus obtained were taken up by filtration to give 0.9 g of 3-(4-(2-methoxyphenyl)piperazin-1-yl)sydnonimine hydrochloride.

270 MHz-NMR (DMSO-$d_6$) δ : 3.24-3.30 (4H, m), 3.70-3.77 (4H, m), 3.81 (3H, s), 6.86-7.86 (4H, m), 8.30 (1H, s), 9.49 (1H, brs)

[Referential Example 2]

Synthesis of 3-(4-benzylpiperazin-1-yl)sydnonimine hydrochloride

At room temperature, a mixture of 20 g of 1-amino-4-benzylpiperazine, 16 g of formaldehyde sodium bisulfite adduct and 70 ml of water was stirred for 1 hour. After adding 7 g of potassium cyanide, the resultant mixture was stirred for additional 16 hours. Then the mixture was extracted with ethyl acetate. The organic layer was washed with water and dried, and the solvent was distilled off. The residue thus obtained was purified by silica gel column chromatography (developing solution: chloroform : methanol : aqueous ammonia = 200 : 10 : 1) to give 13 g of 4-benzyl-1-(cyanomethylamino)piperazine as an oily substance. Under ice cooling, 35 ml of a 15% solution of ethyl nitrite in ethanol was dropped into a mixture of 13 g of the obtained 4-benzyl-1-(cyanomethylamino)piperazine, 24 ml of conc. hydrochloric acid and 120 ml of ethanol and the obtained mixture was allowed to stand at 5°C for 3 days. Then, the mixture was concentrated and the crystals thus obtained were taken up by filtration to give 15 g of 3-(4-benzylpiperazin-1-yl)sydnonimine hydrochloride.

270 MHz-NMR (DMSO-$d_6$) δ : 3.28-3.59 (4H, m), 3.79-4.21 (4H, m), 4.40 (2H, s), 7.39-7.48 (3H, m), 7.60-7.74 (2H, m), 8.36 (1H, s), 9.73 (1H, brs)

[Referential Example 3]

Synthesis of 3-(4-ethylpiperazin-1-yl)sydnonimine hydrochloride

At room temperature, a mixture of 3.5 g of 1-amino-4-ethylpiperazine, 3.64 g of formaldehyde sodium bisulfite

adduct and 20 ml of water was stirred for 1 hour. After adding 1.77 g of potassium cyanide, the resultant mixture was stirred for 5 hours at 60°C. Then the mixture was extracted with dichloromethane. The organic layer was dried and the solvent was distilled off. The residue thus obtained was purified by silica gel column chromatography (developing solution: chloroform : methanol : aqueous ammonia = 100 : 10 : 1) to give 1.7 g of 1-(cyanomethylamino)-4-ethylpiperazine as an oily substance. Under ice cooling, 7.0 ml of a 15% solution of ethyl nitrite in ethanol was dropped into a mixture of 1.7 g of the obtained 1-(cyanomethylamino)-4-ethylpiperazine, 4.3 ml of conc. hydrochloric acid and 18 ml of ethanol and the obtained mixture was allowed to stand at room temperature overnight. Then, the mixture was concentrated and the crystals thus obtained were recrystallized from water/ethanol/ether to give 0.45 g of 3-(4-ethylpiperazin-1-yl)sydnonimine hydrochloride (m.p.:210-212°C).

270 MHz-NMR (DMSO-$d_6$) $\delta$ : 1.30 (3H, t), 3.13-3.51 (4H, m), 3.63-3.94 (4H, m), 4.12-4.26 (2H, m), 8.38 (1H, s), 9.73 (1H, brs)

[Referential Example 4]

Synthesis of 3-(4-isopropylpiperazin-1-yl)sydnonimine hydrochloride

At room temperature, a mixture of 6 g of 1-amino-4-isopropylpiperazine, 6.2 g of formaldehyde sodium bisulfite adduct and 40 ml of water was stirred for 1 hour. After adding 2.8 g of potassium cyanide, the resultant mixture was stirred for 2.5 hours at 60°C. Then the mixture was extracted with ethyl acetate. The organic layer was dried and the solvent was distilled off. The residue thus obtained was purified by silica gel column chromatography (developing solution: chloroform : methanol : aqueous ammonia = 100 : 5 : 1) to give 2.2 g of 1-(cyanomethylamino)-4-isopropylpiperazine as an oily substance. Under ice cooling, 8 ml of a 15% solution of ethyl nitrite in ethanol was dropped into a mixture of 2.2 g of the obtained 1-(cyanomethylamino)-4-isopropylpiperazine, 5 ml of conc. hydrochloric acid and 15 ml of ethanol and the obtained mixture was stirred at room temperature for 15 hours. Then, the mixture was concentrated and the crystals thus obtained were washed with isopropanol and taken up by filtration to give 1.7 g of 3-(4-isopropylpiperazin-1-yl)sydnonimine hydrochloride.

270 MHz-NMR (DMSO-$d_6$) $\delta$ : 1.33 (6H, d), 3.22-3.72 (5H, m), 3.90-4.01 (2H, m), 4.18-4.25 (2H, m), 8.40 (1H, s)

[Referential Example 5]

Synthesis of 3-(4-((4-methoxyphenyl)methylthio)piperidin-1-yl)sydnonimine hydrochloride

Under ice cooling, a mixture of 2.5 g of sodium nitrite and 10 ml of water was dropped into another mixture of 7 g of 4-((4-methoxyphenyl)methylthio)piperidine, 2 ml of conc. sulfuric acid and 30 ml of water and the resultant mixture was stirred for 1 hour at 0°C. Then the mixture was extracted with ethyl acetate. The organic layer was washed with water and dried and the solvent was distilled off. The residue thus obtained was purified by silica gel column chromatography (developing solution: chloroform : methanol = 100 : 1) to give 6.2 g of 4-((4-methoxyphenyl)methylthio)-N-nitrosopiperidine as an oily substance. Under ice cooling, 1.8 g of lithium aluminum hydride was added to a mixture of 6.2 g of the obtained 4-((4-methoxyphenyl)methylthio)-1-nitrosopiperidine and 100 ml of tetrahydrofuran and the resultant mixture was stirred for 18 hours at room temperature. After heating under reflux for 4 hours, the mixture was diluted with ethyl acetate. Then a saturated aqueous solution of sodium chloride was added thereto and the resultant mixture was stirred for 1 hour. The organic layer was dried and the solvent was distilled off to give 6 g of 1-amino-4-((4-methoxyphenyl)methylthio)piperidine as an oily substance. A mixture of 6 g of the obtained 1-amino-4-((4-methoxyphenyl)methylthio)piperidine, 5 g of formaldehyde sodium bisulfite adduct and 25 ml of water was stirred at room temperature for 1 hour. After adding 2.3 g of potassium cyanide, the resultant mixture was stirred for 3 hours at 60°C. Then the mixture was extracted with ethyl acetate and the organic layer was washed with water and dried. After distilling off the solvent, the obtained residue was purified by silica gel column chromatography (developing solution: chloroform : methanol : aqueous ammonia = 1,000 : 10 : 1) to give 5.5 g of 1-(cyanomethylamino)-4-((4-methoxyphenyl)methylthio)piperidine as an oily substance. Under ice cooling, 11 ml of a 15% solution of ethyl nitrite in ethanol was added to a mixture of 5.5 g of 1-(cyanomethylamino)-4-((4-methoxy-phenyl)methylthio)piperidine obtained above, 1.8 ml of conc. hydrochloric acid and 20 ml of ethanol and the resultant mixture was stirred for 16 hours at room temperature. The crystals thus precipitated were taken up by filtration to give 2.9 g of 3-(4-((4-methoxyphenyl)methylthio)piperidin-1-yl)sydnonimine hydrochloride.

270 MHz-NMR (DMSO-$d_6$) $\delta$ : 1.68-1.80 (2H, m), 2.07-2.17 (2H, m), 2.78-2.86 (1H, m), 3.30-3.42 (2H, m), 3.74 (3H, s), 3.78 (2H, s), 3.78-3.89 (2H, m), 6.88 (2H, d), 7.26 (2H, d), 8.20 (1H, s), 9.42 (1H, brs)

[Referential Example 6]

Synthesis of 3-(4-phenylpiperazin-1-yl)sydnonimine hydrochloride

At room temperature, a mixture of 13.4 g of 1-amino-4-phenylpiperazine, 10.3 g of formaldehyde sodium bisulfite adduct and 75 ml of water was stirred for 1.5 hours. After adding 5.1 g of potassium cyanide, the resultant mixture was stirred for 5 hours at 60°C. Then the mixture was extracted with ethyl acetate and the organic layer was dried. After distilling off the solvent, the obtained residue was purified by silica gel column chromatography (developing solution: chloroform : methanol : aqueous ammonia = 100 : 5 : 1) to give 6.4 g of 1-(cyanomethylamino)-4-phenylpiperazine. Under ice cooling, 16.3 ml of a 15% solution of ethyl nitrite in ethanol was added to a mixture of 6.4 g of 1-(cyanomethylamino)-4-phenylpiperazine obtained above, 12.5 ml of conc. hydrochloric acid and 60 ml of ethanol and the resultant mixture was stirred for 6 days at room temperature. The crystals thus precipitated were taken up by filtration to give 1.7 g of 3-(4-phenylpiperazin-1-yl)sydnonimine hydrochloride.

270 MHz-NMR (DMSO-$d_6$) $\delta$ : 3.43-3.65 (4H, m), 3.75-3.97 (4H, m), 6.68-7.35 (5H, m), 8.36 (1H, s), 9.64 (1H, s)

The following Test Examples will be given to show the excellent vasodilative, myocardial protective and antiplatelet aggregation effects of typical examples of the compounds of the present invention.

[Test Example 1]

Test with the use of isolated rat aorta

The thoracic aorta was taken out from male Sprague Dawley rats weighing 450 to 600 g and cut into circular samples of 2 mm in width. The samples obtained were hung under a tension of 2 g in an organ bath containing 10 ml of Krebs-Henseleit solution (119 mM of NaCl, 4.8 mM of KCl, 2.53 mM of $CaCl_2 \cdot 2H_2O$, 1.2 mM of $KH_2PO_4$, 1.2 mM of $MgSO_4 \cdot 7H_2O$, 24.8 mM of $NaHCO_3$, 10 mM of glucose, 37°C) aerated with 95% of oxygen and 5% of carbon dioxide. The contraction reactions of the samples were isometrically recorded with FD pick-up. After 1 to 1.5 hours of equilibration, 30 mM potassium chloride was added to contract the tissue. Thus the activity of each test compound of relaxing the continuous contraction due to potassium chloride was evaluated by determining the 50% inhibitory concentration ($IC_{50}$) of the compound against the maximum relaxation effect of the compound.

As the test compounds, the invention compounds obtained in the above Examples were employed. As a control, use was made of 3-morpholinosydnonimine hydrochloride (hereinafter referred to as SIN-1) which is the active segment of molsidomine. Table B summarizes the results.

Table B

| No. | Rat aorta $IC_{50}$ | No. | Rat aorta $IC_{50}$ |
|---|---|---|---|
| Ex. 1 | $6.5 \times 10^{-7}$ | Ex. 2 | $4.3 \times 10^{-8}$ |
| Ex. 3 | $3.0 \times 10^{-8}$ | Ex. 4 | $2.3 \times 10^{-8}$ |
| Ex. 5 | $5.7 \times 10^{-7}$ | Ex. 8 | $1.9 \times 10^{-7}$ |
| Ex. 9 | $8.3 \times 10^{-8}$ | Ex. 11 | $1.6 \times 10^{-7}$ |
| Ex. 13 | $5.5 \times 10^{-7}$ | Ex. 14 | $6.3 \times 10^{-7}$ |
| Ex. 16 | $5.6 \times 10^{-8}$ | Ex. 20 | $6.5 \times 10^{-7}$ |
| Ex. 24-D | $4.7 \times 10^{-7}$ | Ex. 25 | $6.3 \times 10^{-7}$ |
| Ex. 27 | $9.7 \times 10^{-7}$ | Ex. 28 | $7.1 \times 10^{-7}$ |
| Ex. 29 | $6.3 \times 10^{-7}$ | Ex. 30-D | $1.2 \times 10^{-7}$ |
| Ex. 31 | $3.6 \times 10^{-8}$ | Ex. 32 | $4.5 \times 10^{-8}$ |
| Ex. 33 | $1.8 \times 10^{-7}$ | Ex. 38-C | $2.3 \times 10^{-7}$ |
| Ex. 39 | $1.2 \times 10^{-7}$ | Ex. 40 | $2.8 \times 10^{-7}$ |
| Ex. 41 | $1.3 \times 10^{-7}$ | Ex. 44 | $9.8 \times 10^{-7}$ |

Table B (continued)

| No. | Rat aorta $IC_{50}$ | No. | Rat aorta $IC_{50}$ |
|---|---|---|---|
| Ex. 45 | $7.8 \times 10^{-7}$ | Ex. 47 | $9.4 \times 10^{-7}$ |
| Ex. 48 | $1.1 \times 10^{-7}$ | Ex. 49 | $4.6 \times 10^{-8}$ |
| Ex. 50 | $1.4 \times 10^{-7}$ | Ex. 51 | $3.0 \times 10^{-7}$ |
| Ex. 52-B | $4.2 \times 10^{-7}$ | Ex. 64 | $9.1 \times 10^{-7}$ |
| Ex. 67 | $5.4 \times 10^{-7}$ | | |
| SIN-1 | $8.5 \times 10^{-7}$ | | |

[Test Example 2]

Blood pressure-lowering effect in anesthetized rat

Male Sprague Dawley rats weighing 280 to 450 g were anesthetized with sodium pentobarbital (50 mg/kg, i.p.) and fixed in the supine position. A canula was inserted into the trachea of each animal. Then the blood pressure of the animal was measured (AP-621G, Nippon Kohden Corp.) through a polyethylene tube (PE50) inserted into the right femoral artery via a pressure transducer (Life Kit DTS, DX-300, Nippon Kohden Corp.) and the heart rate was determined from the pulse wave (AT-610G, Nippon Kohden Corp.). A canula (PE10) for injecting the maintenance anesthetic drug (sodium pentobarbital 20 mg/kg/h) was inserted into the right femoral vein. The abdominal region was medianly sectioned and a canula (PE50 connected to a injection needle 23G) for duodenal drug administration was inserted thereinto and fixed with Aronalpha for surgical use. After 60-90 minutes of recovery period, the drug was administered into duodenum. After administering the drug, the blood pressure was measured until the average blood pressure returned to the level before the administration.

As the indication of the blood pressure-lowering effect of each drug, the dose causing a decrease in the blood pressure by 30%, based on the level before the administration, was calculated from the dose-response curve and employed as the 30% effective dose ($ED_{30}$). Moreover, the time required for the recovery to the level corresponding to 50% of the maximum decrease in the blood pressure (half duration) was measured at each dose. Then the half duration at the above-mentioned $ED_{30}$ was calculated and employed as the indication of the lasting time of the blood pressure-lowering effect (half duration at $ED_{30}$).

As the test compounds, use was made of the compounds of the present invention obtained in the above Examples. Table C summarizes the results.

Table C

| No. | Blood pressure-lowering effect | | No. | Blood pressure-lowering effect | |
|---|---|---|---|---|---|
| | $ED_{30}$ (mg/kg) | Half duration (min) | | $ED_{30}$ (mg/kg) | Half duration (min) |
| Ex. 1 | 5.5 | 151 | Ex. 2 | 0.9 | 47 |
| Ex. 3 | 0.6 | 37 | Ex. 8 | 0.5 | 7 |
| Ex. 9 | 1.6 | 16 | Ex. 14 | 0.8 | 15 |
| Ex. 17 | 1.4 | 21 | Ex. 18 | 2.0 | 62 |
| Ex. 20 | 1.5 | 28 | Ex. 27 | 0.7 | 34 |
| Ex. 39 | 1.7 | 39 | Ex. 41 | 0.6 | 25 |
| Ex. 42 | 4.0 | 60 | | | |
| Ex. 70 | 0.7 | 41 | Ex. 72 | 1.3 | 47 |
| Ex. 78 | 1.0 | 40 | Ex. 80 | 0.6 | 49 |
| Ex. 85 | 0.3 | 44 | Ex. 119 | 1.2 | 49 |
| Ex. 122 | 0.6 | 48 | Ex. 129 | 1.3 | 42 |

Table C (continued)

| No. | Blood pressure-lowering effect | | No. | Blood pressure-lowering effect | |
|---|---|---|---|---|---|
| | $ED_{30}$ (mg/kg) | Half duration (min) | | $ED_{30}$ (mg/kg) | Half duration (min) |
| Ex. 132 | 0.7 | 44 | Ex. 137 | 0.6 | 46 |
| Ex. 141 | 0.5 | 48 | Ex. 146 | 0.6 | 45 |
| Ex. 155 | 0.2 | 46 | Ex. 163 | 0.2 | 44 |
| Ex. 171 | 0.3 | 45 | Ex. 174 | 0.5 | 43 |
| Ex. 179 | 0.9 | 43 | Ex. 181 | 0.8 | 60 |
| Ex. 183 | 1.0 | 47 | Ex. 185 | 1.5 | 53 |
| Ex. 191 | 1.0 | 58 | Ex. 192 | 1.0 | 49 |
| SIN-1 | 1.0 | 75 | molsidomine | 0.3 | 39 |

[Test Example 3]

Myocardial necrosis inhibitory effect in rat ischemia-reperfusion model

Male Sprague Dawley rats weighing 270 to 410 g were anesthetized with sodium pentobarbital (50 mg/kg, i.p.) and fixed in the supine position. A canula was inserted into the trachea of each animal. Then the blood pressure of the animal was measured (AP-621G, Nippon Kohden Corp.) through a polyethylene tube (PE50, Becton Dickinson) inserted into the right femoral artery via a pressure transducer (Life Kit DTS, DX-300, Nippon Kohden Corp.) and the heart rate was determined from the pulse wave (AT-610G, Nippon Kohden Corp.). Two canulae (PE10 and PE50, Becton Dickinson) for injecting the maintenance anesthetic drug (sodium pentobarbital 20 mg/kg/h) and a coloring matter respectively were inserted into the right jugular vein. The left jugular vein was exposed for blood collection. The chest was opened by vertically sectioning the chest region from the third to fifth left ribs about 10 mm apart from the median line under artificial respiration with room air (Harvard respirator, model 683, rate : 60 strokes/min. tidal volume : 10 ml/kg body weight). Then the operative field was enlarged with a wound retractor and the heart sac was peeled off to thereby expose the heart. By using a curved round needle with thread for vascular tract (8mm, 6-0 black blaid silk, Matsuda Medical Industry), a thread of 4 to 5 mm in length was put in the left ventricle in the depth of 2 mm for ligating the coronary artery. After the completion of the operation, the animal was allowed to stand as such for 20 minutes or longer. After confirming that the blood pressure and the heart rate had been stabilized, the experiment was initiated.

In this experiment, use was made of eight groups of rats including: 1) a physiological saline (i.e., the solvent for SIN-1) group; 2) an SIN-1 (3 μg/kg/min) group; 3) another SIN-1 (10 μg/kg/min) group; 4) 1% DMSO-containing physiological saline (adjusted to pH 5 with hydrochloric acid, i.e., the solvent for the compounds of Examples 1 and 2) group; 5) a group of the compound of Example 1 (10 μg/kg/min); 6) another group of the compound of Example 1 (30 μg/kg/min); 7) a group of the compound of Example 2 (3 μg/kg/min); and 8) another group of the compound of Example 2 (10 μg/kg/min). Each drug was intravenously administered continuously for 75 minutes (from 15 minutes before the ligation of the coronary artery to the completion of the reflow) at a rate of 0.95 ml/h. Fifteen minutes after the initiation of the drug administration, the coronary artery was ligated and maintained for 30 minutes. The coronary artery was ligated by the snare method with the use of a polyethylene tube (PE160, Becton Dickinson) and a vascular clamp (C-17 grooved, Natsume). After 30-minutes reflow, the risk area (RA) and the necrosis area (NA) were measured.

The risk area and the necrosis area formed by ligating the coronary artery were measured by the double staining method with Evans blue and triphenyltetrazolium chloride (TTC) in the following manner. To clarify the risk area, the coronary artery was ligated again after the completion of the reflow. Subsequently, 1 ml of 5% Evans blue (Sigma) and a 1 M solution of KCl were successively injected into the jugular vein until the heart arrest. Thus, the non-risk area was stained dark blue. After the heart arrest, the heart was quickly taken out and washed with physiological saline. Then the left ventricle was separated and cut into circular slices of 3 mm in width with a trimming blade (Feather). To decide the necrosis area, these slices were incubated in a 0.1 M phosphate buffer (81 mM $Na_2HPO_4$: 19 mM $NaH_2PO_4$, pH 7.4) containing 1% TTC at 37°C for 5 minutes. Thus, living cells were exclusively stained red with TTC while the necrosis area was not stained. After staining with TTC, each wet slice was weighed, fixed with 20% formalin and then photographed while allowing to stand in physiological saline. The tissue cross section photograph was retrieved with an image scanner (Seiko Epson GY-6000) and the left ventricle (LV) area, RA and NA were measured by using an image analyzing software, NIH Image 1.41. Based on the obtained data, the area ratios (RA/LV and NA/RA) of each cross section were calculated. Further, the averages of the area ratios of both cross sections were determined and converted into

the volume ratios. Then the weight of each slice (LV weight) was multiplied thereby to give the weight of each area of each slice. Thus, the weight ratios of the areas of the total organ were determined. The formulae employed in the above calculation were as follows.

$$\text{RA/LV (\%)} = \frac{\text{total risk area weight of each slice}}{\text{left ventricle weight}} \times 100$$

*Risk area (RA) weight = {RA(area-A)/LV (area-A) + RA(area-B)/LV (area-B)}/2 x LV weight

RA(area-A): RA in the cardiac apex side section of each slice.

LV(area-A): LV in the cardiac apex side section of each slice.

RA(area-B): RA in the cardiac bottom side section of each slice.

LV(area-B): LV in the cardiac bottom side section of each slice.

$$\text{NA/LV (\%)} = \frac{\text{total necrosis area weight of each slice}}{\text{left ventricle weight}} \times 100$$

*Necrosis area (NA) weight = {NA(area-A)/LV (area-A) + NA(area-B)/LV (area-B))/2 x LV weight

NA(area-A): NA in the cardiac apex side section of each slice.

LV(area-A): LV in the cardiac apex side section of each slice.

NA(area-B): NA in the cardiac bottom side section of each slice.

LV(area-B): LV in the cardiac bottom side section of each slice.

$$\text{NA/RA (\%)} = \frac{\text{total necrosis area weight of each slice}}{\text{total risk area weight of each slice}} \times 100$$

*Necrosis area (NA) weight = {NA(area-A)/LV (area-A) + NA(area-B)/LV (area-B)}/2 x LV weight

NA(area-A): NA in the cardiac apex side section of each slice.

LV(area-A): LV in the cardiac apex side section of each slice.

NA(area-B): NA in the cardiac bottom side section of each slice.

LV(area-B): LV in the cardiac bottom side section of each slice.

*Risk area (RA) weight = {RA(area-A)/LV (area-A) + RA(area-B)/LV (area-B)}/2 x LV weight

RA(area-A): RA in the cardiac apex side section of each slice.

LV(area-A): LV in the cardiac apex side section of each slice.

RA(area-B): RA in the cardiac bottom side section of each slice.

LV(area-B): LV in the cardiac bottom side section of each slice.

After the completion of the reflow, 1 ml of the venous blood was collected from the right jugular vein, fed into a serum separation tube (Separapit Tube, Sekisui Medical) and then centrifuged at 4°C at 1,400 x g for 10 minutes to separate the serum which was stored at -80°C until using. The enzymatic activities of creatine kinase (CK) and lactate dehydrogenase (LDH) were measured in accordance with the method given in the manual of an autoanalyzer (COBAS FARA II).

Each value measured was expressed in mean ± standard error. The significance was examined by the analysis of variance (ANOVA). When a significant difference was observed between two groups, the difference was examined by Fischer's PLSD method and p of less than 0.05 was regarded as significantly different.

Table D summarizes the results.

Table D

| No. | RA/LV | NA/RA | LDH | CK |
|---|---|---|---|---|
| 1) physiological saline | 46.2±4.6 | 33.9±5.7 | 1592±371 | 4794±606 |
| 2) SIN-1 3 μg/kg/min | 49.0±3.0 | 18.5±4.3 | 1082±60 | 3138±207 |
| 3) SIN-1 10 μg/kg/min | 48.9±4.9 | 19.3±5.2 | 1196±166 | 2853±507 |
| 4) 1% DMSO-physiological saline | 39.0±2.3 | 42.3±2.4 | 1482±223 | 2921±427 |
| 5) compd. of Ex. 1 10 μg/kg/min | 34.0±2.6 | 33.4±6.1 | 1538±254 | 3237±803 |
| 6) compd. of Ex. 1 30 μg/kg/min | 36.5±3.7 | 22.3±6.1 p<0.01 vs. 4 | 1339±343 | 2772±496 |
| 7) compd. of Ex. 2 3 μg/kg/min | 28.3±1.9 | 18.0±4.5 p<0.05 vs. 4 | 864±59 | 2409±298 |
| 8) compd. of Ex. 2 10 μg/kg/min | 28.1±3.5 | 16.5±4.7 p<0.01 vs. 4 | 957±192 | 2427±357 |

[Test Example 4]

Inhibitory effect on platelet aggregation in rabbit

Male JW/CSK rabbits weighing 2.6 to 3.1 kg were anesthetized with thiopental (20 mg/kg). Then the blood of each animal was collected from the left carotid artery into a container containing a 3.8% solution of citric acid (9 : 1 v/v). The collected blood was immediately centrifuged at room temperature at 1,300 rpm (230 G) for 10 minutes to give a platelet rich plasma (PRP). Further, a required amount of the blood was centrifuged at 3,000 rpm (1,300 G) for 15 minutes to give a platelet poor plasma (PPP). The PRP to be used in the platelet aggregation test was diluted with PPP to adjust the platelet count to 3 x $10^8$. The aggregability was measured by using a platelet aggregation measuring device (TE-500, ERMA) with the use of arachidonic acid (500 mM) as an aggregation agent. Each test compound was mixed with PRP and pre-incubated for 3 minutes. Next, the aggregation agent was added thereto and the aggregability was measured for 5 minutes. Thus, the activity of the test compound was evaluated by determining the 50% inhibitory concentration ($IC_{50}$; μM) thereof to the maximum aggregation induced by the arachidonic acid. As the test compounds, the compounds of the present invention obtained in the above Examples were employed. As a control drug, use was made of aspirin which has been widely known as a platelet aggregation inhinitor. Table E summarizes the results.

Table E

| Ex. No. | Rabbit antiplatelet aggregation effect $IC_{50}$ (μM) |
|---|---|
| Ex. No. 2 | 15.8 |
| Ex. No. 3 | 2.7 |
| Ex. No. 68 | 10.5 |

Table E (continued)

| Ex. No. | Rabbit antiplatelet aggregation effect $IC_{50}$ (μM) |
|---|---|
| Ex. No. 86 | 1.3 |
| Ex. No. 88 | 1.1 |
| Ex. No. 135 | 12.9 |
| Ex. No. 139 | 15.8 |
| Ex. No. 192 | 3.9 |
| aspirin | 27.5 |
| Invention compound : n = 1 - 3<br>Aspirin : n = 7 | |

[Test Example 5]

Effect on blood coagulability in rat A-V shunt model

Rat A-V shunts models were prepared by reference to the method of Umezu et al. (Thrombos Haemostas. 1978:39:74 - 83). Male SD rats were anesthetized with sodium pentobarbital (50 mg/kg, i.p.) and fixed in the supine position. A thick polyethylene tube (6 cm; Hibiki No. 7, Kunii Co.) containing a silk suture thread was connected at both ends to another thin polyethylene tube (10 cm, Hibiki No. 4, Kunii Co.) and then inserted into the left common carotid artery and the right internal jugular vein of the rat so as to form an external blood circulation system (A-V Shunt). The blood was circulated within the shunt system for 15 minutes and then the blood coagulates adhering to the silk suture thread in the thick tube was weighed. After repeating this operation thrice, the total weight was determined and the activity of each test compound was evaluated based thereon.

(1) Anti-aggregation effect achieved by peroral administration

In this experiment, the anti-aggregation effects achieved from 75 minutes to 2 hours after the peroral administration were examined in a vehicle group (0.3% carboxymethyl cellulose, hereinafter referred to simply as CMC; n = 6), a group of the compound of Example 2 (10 mg/kg; n = 6) and a group of FK-409 (10 mg/kg; n = 6) which had been reported as being capable of inhibiting the formation of blood coagulates (European Journal of Pharmacology, 1995:272:39 - 43). Table F summarizes the results.

Table F

| Test compd. | Blood coagulability in rat A-V shunt model p.o. administration; wt. (mg) of coagulates |
|---|---|
| vehicle | 121.17±7.56 |
| FK409 | 89.17±8.43 # |
| compd. of Ex. 2 | 84.92±9.69 # |
| Each value is expressed as mean ± S.E. (n = 6).<br># means being significantly different from the vehicle ($p < 0.05$; Dunnet's multiple comparison). | |

(2) Drug effect by intraduodenal administration

This experiment was performed on a vehicle group (0.3% CMC; n = 10) and groups of the compound of Example 2 (0.3, 1.0 and 3.0 mg/kg; each n = 6). The drug was intraduodenally administered by preparing the A-V shunt model, sectioning the abdominal region of the animal and inserting into the duodenum a catheter provided with a needle at the tip. Forty-five minutes after the administration of the test compounds, the blood coagulates formed during this period were weighed. Table G summarizes the results.

Table G

| Test compd. | Blood coagulability in rat A-V shunt model i.d. administration; wt. (mg) of coagulates |
|---|---|
| vehicle | 99.7±4.95 |
| compd. of Ex. 2 (0.3 mg/kg) | 87.5±6.19 |
| compd. of Ex. 2 (1.0 mg/kg) | 76.6±6.93 # |
| compd. of Ex. 2 (3.0 mg/kg) | 65.7±7.07 ## |
| Each value is expressed as mean ± S.E. (n = 6-10).<br># means being significantly different from the vehicle (p < 0.05); ## means being significantly different from the vehicle (p < 0.01); (Dunnet's multiple comparison). | |

INDUSTRIAL APPLICABILITY

Because of having a vasodilative effect, a myocardial protective effect, an antiplatelet aggregation effect, etc., the compounds of the present invention or pharmaceutically acceptable salts thereof are useful as drugs such as remedies for angina pectoris.

## Claims

**1.** A compound represented by the following general formula (1) or a pharmaceutically acceptable salt thereof:

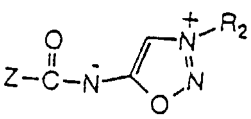

(1)

[wherein Z represents:

$$-A-X-R_1$$

{wherein A represents a linear or branched alkylene group having 1 to 6 carbon atoms optionally substituted by an optionally substituted aryl group having 6 to 12 carbon atoms or an alkylcarbonylamino group carrying, as the alkyl moiety, a linear or branched alkyl group having 1 to 6 carbon atoms;

X represents $S(O)_n$ (n being 0, 1 or 2); or a direct bond; and

$R_1$ represents an aryl group having 6 to 12 carbon atoms optionally mono-, di- or tri-substituted by 1 to 3 hydroxyl groups and/or 1 to 3 linear or branched alkyl groups having 1 to 6 carbon atoms and/or 1 to 3 linear or branched alkoxy groups having 1 to 6 carbon atoms and/or 1 to 3 halogen atoms and/or 1 or 2 nitro groups; an arylcarbonyl group carrying, as the aryl moiety, an aryl group having 6 to 12 carbon atoms optionally mono-, di- or tri-substituted by 1 to 3 linear or branched alkyl groups having 1 to 6 carbon atoms optionally halogenated and/or 1 to 3 linear or branched alkoxy groups having 1 to 6 carbon atoms and/or 1 to 3 halogen atoms; an alkylcarbonyl group having, as the alkyl moiety, a linear or branched alkyl group having 1 to 6 carbon atoms; a linear or branched alkyl group having 1 to 6 carbon atoms; an aralkyloxycarbonyl group carrying, as the aryl moiety, an optionally substituted aryl group having 6 to 12 carbon atoms and, as the alkylene moiety, an alkylene group having 1 or 2 carbon atoms; a cycloalkylcarbonyl group carrying, as the cycloalkyl moiety, an optionally substituted cycloalkyl group having 3 to 6 carbon atoms; an arylcarbamoyl group carrying, as the aryl moiety, an optionally substituted aryl group having 6 to 12 carbon atoms; an alkylcarbamoyl group carrying, as the alkyl moiety, a linear or branched alkyl group having 1 to 6 carbon atoms; or -CO-$R_{14}$ wherein $R_{14}$ represents an optionally substituted monocyclic heterocycle; or a bicyclic heterocycle optionally substituted by a hydroxyl group and/or linear or branched alkyl groups having 1 to 6 carbon atoms}; an optionally substituted monocyclic heterocycle; a bicyclic heterocycle optionally substituted by a hydroxyl group and/or linear or branched alkyl groups having 1 to 6 carbon atoms; or an optionally substituted cycloalkyl group having 3 to 6 carbon atoms; and

$R_2$ represents:

$$-N(R_3)R_4$$

(wherein $R_3$ represents a hydrogen atom; or a linear or branched alkyl group having 1 to 6 carbon atoms; and $R_4$ represents a hydrogen atom; an optionally substituted linear or branched alkyl group having 1 to 6 carbon atoms; an optionally substituted linear or branched alkenyl group having 2 to 6 carbon atoms; an optionally substituted linear or branched alkynyl group having 2 to 6 carbon atoms; an optionally substituted aryl group having 6 to 12 carbon atoms; an optionally substituted cycloalkyl group having 3 to 6 carbon atoms; or -CO-$R_7$ wherein $R_7$ represents an optionally substituted linear or branched alkyl group having 1 to 6 carbon atoms; an optionally substituted linear or branched alkoxy group having 1 to 6 carbon atoms; an optionally substituted monocyclic heterocycle; or an optionally substituted bicyclic heterocycle); or a group represented by the following general formula (2):

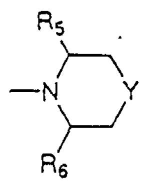

(2)

{wherein $R_5$ and $R_6$ are the same or different and each represents a hydrogen atom; or a linear or branched alkyl group having 1 to 6 carbon atoms; Y represents an oxygen atom; $S(O)_n$ (n being 0, 1 or 2); or a group represented by the following general formula (3):

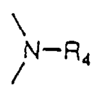

(3)

(Wherein $R_4$ represents a hydrogen atom; an optionally substituted linear or branched alkyl group having 1 to 6 carbon atoms; an optionally substituted linear or branched alkenyl group having 2 to 6 carbon atoms; an optionally substituted linear or branched alkynyl group having 2 to 6 carbon atoms; an optionally substituted aryl group having 6 to 12 carbon atoms; an optionally substituted cycloalkyl group having 3 to 6 carbon atoms; or -CO-$R_7$ wherein $R_7$ represents an optionally substituted linear or branched alkyl group having 1 to 6 carbon atoms; an optionally substituted linear or branched alkoxy group having 1 to 6 carbon atoms; an optionally substituted monocyclic heterocycle; or an optionally substituted bicyclic heterocycle); or a group represented by the following general formula (4):

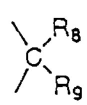

(4)

(wherein $R_8$ represents a hydrogen atom; or a linear or branched alkyl group having 1 to 6 carbon atoms; and $R_9$ represents a hydrogen atom; a hydroxyl group; a thiol group; an alkylcarbonylthio group carrying, as the

alkyl moiety, a linear or branched alkyl group having 1 to 6 carbon atoms; an alkylcarbonyloxy group carrying, as the alkyl moiety, a linear or branched alkyl group having 1 to 6 carbon atoms; an arylcarbonylthio group carrying, as the aryl moiety, an aryl group having 6 to 12 carbon atoms; an arylcarbonyloxy group carrying, as the aryl moiety, an aryl group having 6 to 12 carbon atoms; or a linear or branched alkyl group having 1 to 6 carbon atoms)}].

**2.** The compound as claimed in Claim 1 or a pharmaceutically acceptable salt thereof, wherein, in the general formula (1), Z represents:

$$-A-X-R_1$$

{wherein A represents a linear or branched alkylene group having 1 to 6 carbon atoms optionally substituted by an optionally substituted aryl group having 6 to 12 carbon atoms or an alkylcarbonylamino group carrying, as the alkyl moiety, a linear or branched alkyl group having 1 to 6 carbon atoms;
X represents $S(O)_n$ (n being 0, 1 or 2); or a direct bond; and
$R_1$ represents an aryl group having 6 to 12 carbon atoms optionally mono-, di- or tri-substituted by 1 to 3 hydroxyl groups and/or 1 to 3 linear or branched alkyl groups having 1 to 6 carbon atoms and/or 1 to 3 linear or branched alkoxy groups having 1 to 6 carbon atoms and/or 1 to 3 halogen atoms and/or 1 or 2 nitro groups; an arylcarbonyl group carrying, as the aryl moiety, an aryl group having 6 to 12 carbon atoms optionally mono-, di- or tri-substituted by 1 to 3 linear or branched alkyl groups having 1 to 6 carbon atoms and optionally halogenated and/or 1 to 3 linear or branched alkoxy groups having 1 to 6 carbon atoms and/or 1 to 3 halogen atoms; an alkylcarbonyl group having, as the alkyl moiety, a linear or branched alkyl group having 1 to 6 carbon atoms; a linear or branched alkyl group having 1 to 6 carbon atoms; an aralkyloxycarbonyl group carrying, as the aryl moiety, an optionally substituted aryl group having 6 to 12 carbon atoms and, as the alkylene moiety, an alkylene group having 1 or 2 carbon atoms; a cycloalkylcarbonyl group carrying, as the cycloalkyl moiety, an optionally substituted cycloalkyl group having 3 to 6 carbon atoms; an arylcarbamoyl group carrying, as the aryl moiety, an optionally substituted aryl group having 6 to 12 carbon atoms; an alkylcarbamoyl group carrying, as the alkyl moiety, a linear or branched alkyl group having 1 to 6 carbon atoms; or $-CO-R_{14}$ wherein $R_{14}$ represents an optionally substituted monocyclic heterocycle; or a bicyclic heterocycle optionally substituted by a hydroxyl group and/or linear or branched alkyl groups having 1 to 6 carbon atoms}.

**3.** The compound as claimed in Claim 1 or a pharmaceutically acceptable salt thereof, wherein, in the general formula (1), Z represents:

$$-A-X-R_1$$

{wherein A represents a linear or branched alkylene group having 1 to 4 carbon atoms optionally substituted by an aryl group having 6 to 12 carbon atoms or an alkylcarbonylamino group carrying, as the alkyl moiety, a linear or branched alkyl group having 1 to 3 carbon atoms;
X represents $S(O)_n$ (n being 0, 1 or 2); or a direct bond; and
$R_1$ represents a phenyl group optionally mono-, di- or tri-substituted by 1 to 3 hydroxyl groups and/or 1 to 3 linear or branched alkyl groups having 1 to 4 carbon atoms and/or 1 to 3 linear or branched alkoxy groups having 1 to 3 carbon atoms; a benzoyl group optionally mono- di- or tri-substituted by 1 to 3 optionally halogenated linear or branched alkyl groups having 1 to 4 carbon atoms and/or 1 to 3 linear or branched alkoxy groups having 1 to 3 carbon atoms and/or 1 to 3 halogen atoms; an alkylcarbonyl group having, as the alkyl moiety, a linear or branched alkyl group having 1 to 4 carbon atoms; a linear or branched alkyl group having 1 to 3 carbon atoms; an aralkyloxycarbonyl group carrying, as the aryl moiety, an aryl group having 6 to 12 carbon atoms and, as the alkylene moiety, an alkylene group having 1 or 2 carbon atoms; a cycloalkylcarbonyl group carrying, as the cycloalkyl moiety, a cycloalkyl group having 3 to 6 carbon atoms; an arylcarbamoyl group carrying, as the aryl moiety, an optionally substituted aryl group having 6 to 12 carbon atoms; an alkylcarbamoyl group carrying, as the alkyl moiety, a linear or branched alkyl group having 1 to 4 carbon atoms; or $-CO-R_{14}$ wherein $R_{14}$ represents a bicyclic heterocycle substituted by a hydroxyl group and/or linear or branched alkyl groups having 1 to 4 carbon atoms}; and
$R_2$ represents:

$$-N(R_3)R_4$$

(wherein $R_3$ represents a linear alkyl group having 1 to 6 carbon atoms; and $R_4$ represents a hydrogen atom;

an optionally substituted linear or branched alkyl group having 1 to 4 carbon atoms; an optionally substituted linear or branched alkenyl group having 2 to 4 carbon atoms; an optionally substituted phenyl group; an optionally substituted cycloalkyl group having 3 to 6 carbon atoms; or -CO-$R_7$ wherein $R_7$ represents an optionally substituted linear or branched alkyl group having 1 to 4 carbon atoms; an optionally substituted linear or branched alkoxy group having 1 to 4 carbon atoms; an optionally substituted monocyclic heterocycle; or an optionally substituted bicyclic heterocycle); or a group represented by the following general formula (2):

{wherein $R_5$ and $R_6$ are the same or different and each represents a hydrogen atom; or a linear alkyl group having 1 to 6 carbon atoms; Y represents an oxygen atom; a sulfur atom; or a group represented by the following general formula (3):

(Wherein $R_4$ represents a hydrogen atom; an optionally substituted linear or branched alkyl group having 1 to 4 carbon atoms; an optionally substituted linear or branched alkenyl group having 2 to 4 carbon atoms; an optionally substituted phenyl group; an optionally substituted cycloalkyl group having 3 to 6 carbon atoms; or -CO-$R_7$ wherein $R_7$ represents an optionally substituted linear or branched alkyl group having 1 to 4 carbon atoms; an optionally substituted linear or branched alkoxy group having 1 to 4 carbon atoms; an optionally substituted monocyclic heterocycle; or an optionally substituted bicyclic heterocycle); or a group represented by the following general formula (4):

(wherein $R_8$ represents a hydrogen atom; or a linear alkyl group having 1 to 3 carbon atoms; and $R_9$ represents a hydrogen atom; a hydroxyl group; a thiol group; an alkylcarbonylthio group carrying, as the alkyl moiety, a linear alkyl group having 1 to 6 carbon atoms; an arylcarbonylthio group carrying, as the aryl moiety, an aryl group having 6 to 12 carbon atoms; a linear alkyl group having 1 to 3 carbon atoms; an acetoxy group; or an benzoyloxy group)}.

**4.** The compound as claimed in Claim 1 or a pharmaceutically acceptable salt thereof, wherein, in the general formula (1), Z represents:

$$-A-X-R_1$$

{wherein A represents a linear or branched alkylene group having 1 to 4 carbon atoms optionally substituted by a phenyl group or an alkylcarbonylamino group carrying, as the alkyl moiety, a linear alkyl group having 1 to 3 carbon atoms;

X represents a sulfur atom; or a direct bond; and

$R_1$ represents a phenyl group mono-, di- or tri-substituted by a hydroxyl group and/or 1 or 2 linear or branched alkyl groups having 1 to 4 carbon atoms and/or 1 to 3 alkoxy groups having 1 or 2 carbon atoms; a benzoyl group optionally mono- di- or tri-substituted by 1 or 2 optionally halogenated linear or branched alkyl groups having 1 to 4 carbon atoms and/or 1 to 3 linear or branched alkoxy groups having 1 or 2 carbon atoms and/or 1 to 3 halogen atoms; an alkylcarbonyl group having, as the alkyl moiety, a linear or branched alkyl group having 1 to 4 carbon atoms; an alkyl group having 1 or 2 carbon atoms; an aralkyloxycarbonyl group carrying, as the aryl moiety, an aryl group having 6 to 12 carbon atoms and, as the alkylene moiety, an alkylene group having 1 or 2 carbon atoms; a cycloalkylcarbonyl group carrying, as the cycloalkyl moiety, a cycloalkyl group having 3 to 6 carbon atoms; an arylcarbamoyl group carrying, as the aryl moiety, an aryl group having 6 to 12 carbon atoms; an alkylcarbamoyl group carrying, as the alkyl moiety, a linear or branched alkyl group having 1 to 4 carbon atoms; or -CO-$R_{14}$ wherein $R_{14}$ represents a bicyclic heterocycle substituted by a hydroxyl group and/or linear or branched alkyl groups having 1 to 4 carbon atoms and containing an oxygen atom as the heteroatom}; and

$R_2$ represents:

$$-N(R_3)R_4$$

(wherein $R_3$ represents a linear alkyl group having 1 to 3 carbon atoms; and $R_4$ represents a hydrogen atom; an optionally substituted linear or branched alkyl group having 1 to 4 carbon atoms; an allyl group; a phenyl group, a 2-methoxyphenyl group; a cycloalkyl group having 3 to 6 carbon atoms; or -CO-$R_7$ wherein $R_7$ represents an optionally substituted linear or branched alkyl group having 1 to 4 carbon atoms; a benzyloxy group; or an optionally substituted monocyclic heterocycle; or an optionally substituted bicyclic heterocycle); or a group represented by the following general formula (2):

{(wherein $R_5$ and $R_6$ are the same or different and each represents a hydrogen atom; or a linear alkyl group having 1 to 3 carbon atoms; Y represents an oxygen atom; or a group represented by the following general formula (3):

(Wherein $R_4$ represents a hydrogen atom; an optionally substituted linear or branched alkyl group having 1 to

4 carbon atoms; an allyl group; a phenyl group, a 2-methoxyphenyl group; a cycloalkyl group having 3 to 6 carbon atoms; or -CO-$R_7$ wherein $R_7$ represents an optionally substituted linear or branched alkyl group having 1 to 4 carbon atoms; a benzyloxy group; an optionally substituted monocyclic heterocycle; or an optionally substituted bicyclic heterocycle); or a group represented by the following general formula (4):

(wherein $R_8$ represents a hydrogen atom; or a methyl group; and $R_9$ represents a hydrogen atom; a hydroxyl group; a thiol group; an alkylcarbonylthio group carrying, as the alkyl moiety, a linear alkyl group having 1 to 3 carbon atoms; an arylcarbonylthio group carrying, as the aryl moiety, an aryl group having 6 to 12 carbon atoms; or a methyl group;)}.

5. The compound as claimed in Claim 1 or a pharmaceutically acceptable salt thereof, wherein, in the general formula (1), Z represents:

$$-A-X-R_1$$

{wherein A represents a methylene group; an ethylene group; a trimethylene group; a propylene group; a benzylethylene group; an acetylaminoethylene group or - $C(CH_3)_2$-$CH_2$-;
X represents a sulfur atom; or a direct bond; and
$R_1$ represents a phenyl group mono-, di- or tri-substituted by a hydroxyl group and/or 1 or 2 t-butyl groups and/or 1 to 3 methoxy groups; a benzoyl group optionally mono- di- or tri-substituted by 1 or 2 methyl groups and/or a trifluoromethyl group and/or 1 to 3 methoxy groups and/or halogen atoms; an acetyl group; a pivaloyl group; a methyl group; a benzyloxycarbonyl group; a cyclohexanecarbonyl group; a phenylcarbamoyl group; an ethylcarbamoyl group; a t-butylcarbamoyl group; or -CO-$R_{14}$ wherein $R_{14}$ represents a 3,4-dihydrobenzopyran ring substituted by a hydroxyl group and/or 4 methyl groups); and
$R_2$ represents:

$$-N(R_3)R_4$$

(wherein $R_3$ represents a methyl group; and $R_4$ represents an optionally substituted linear or branched alkyl group having 1 to 4 carbon atoms; or -CO-$R_7$ wherein $R_7$ represents an optionally substituted linear or branched alkyl group having 1 to 4 carbon atoms); or a group represented by the following general formula (2):
{wherein $R_5$ and $R_6$ are the same or different and each represents a hydrogen atom; or a methyl group; and Y represents an oxygen atom; or a group represented by the following general formula (3):
(Wherein $R_4$ represents an optionally substituted linear or branched alkyl group having 1 to 4 carbon atoms; a 2-methoxyphenyl group; a cycloalkyl group having 4 to 6 carbon atoms; or -CO-$R_7$ wherein $R_7$ represents an optionally substituted linear or branched alkyl group having 1 to 4 carbon atoms; or a group represented by the following general formula (4):
(wherein $R_8$ represents a hydrogen atom; and $R_9$ represents a hydrogen atom; a hydroxyl group; a thiol group; an acetylthio group; or a benzoylthio group)}.

6. The compound as claimed in Claim 1 selected from the group consisting of the following compounds or a pharmaceutically acceptable salt thereof: N-((3,5-di-t-butyl-4-hydroxyphenylthio)acetyl)-3-(4-methylpiperazin-1-yl)sydnonimine, N-((S)-3-(benzoylthio)isobutyryl)-3-(4-methylpiperazin-1-yl)sydnonimine, N-((S)-3-(acetylthio)isobutyryl)-3-morpholinosydnonimine, N-((S)-3-(benzoylthio)isobutyryl)-3-(4-benzylpiperazin-1-yl)sydnonimine, N-((benzoylthio)acetyl)-3-(4-methylpiperazin-1-yl)sydnonimine, N-((3,4,5-trimethoxyphenyl)acetyl)-3-(4-methylpiperazin-1-yl)sydnonimine, N-((S)-3-(acetylthio)isobutyryl)-3-(4-isopropylpiperazin-1-yl)sydnonimine, N-(acetylthioacetyl)-3-(4-ethylpiperazin-1-yl)sydnonimine, N-(3-(acetylthio)propionyl)-3-(4-ethylpiperazin-1-yl)sydnonimine, N-((2,6-dimethylbenzoylthio)acetyl)-3-(4-methylpiperazin-1-yl)sydnonimine, N-(3-(2,6-dimethylbenzoylthio)propionyl)-3-(4-methylpiperazin-1-yl)sydnonimine, N-(3-(pivaloylthio)propionyl)-3-(4-methylpiperazin-1-yl)sydnonimine, N-(3-(pivaloylthio)propionyl)-3-(4-ethylpiperazin-1-yl)sydnonimine, N-(3-(ethylcarbamoylthio)propionyl)-3-(4-methylpiperazin-1-yl)sydnonimine, N-(3-(ethylcarbamoylthio)propionyl)-3-(4-ethylpiperazin-1-yl)sydnonimine, N-(3-(ethylcarbamoylthio)propionyl)-3-(4-isopropylpiperazin-1-yl)sydnonimine, N-(3-(phenylcarbamoylthio)propionyl)-3-(4-methylpiperazin-1-yl)sydnonimine, N-(4-(benzoylthio)butyryl)-3-(4-methylpiperazin-1-yl)sydnonimine, N-(3-(3,5-di-t-butyl-4-hydroxyphenylthio)propionyl)-3-(4-methyl-piperazin-1-yl) sydnonimine, and N-((R)-2-acetylamino-3-(acetylthio)propionyl)-3-(4-methylpiperazin-1-yl)sydnonimine.

7. The compound as claimed in Claim 1 or a pharmaceutically acceptable salt thereof, wherein, in the general formula (1), Z represents an optionally substituted monocyclic heterocycle; a bicyclic heterocycle optionally substituted by a hydroxyl group and/or a linear or branched alkyl group having 1 to 6 carbon atoms; or an optionally substituted

cycloalkyl group having 3 to 6 carbon atoms.

8. The compound as claimed in Claim 1 or a pharmaceutically acceptable salt thereof, wherein, in the general formula (1), Z represents a 5- or 6-membered monocyclic heterocycle containing a nitrogen atom and/or a sulfur atom and/or an oxygen atom as the heteroatom optionally having an oxo group; a bicyclic heterocycle containing an oxygen atom as the heteroatom substituted by a hydroxyl group and/or a linear or branched alkyl group having 1 to 4 carbon atoms; or an optionally substituted cycloalkyl group having 4 to 6 carbon atoms; and

$R_2$ represents:

$$-N(R_3)R_4$$

(wherein $R_3$ represents a linear alkyl group having 1 to 6 carbon atoms; and $R_4$ represents a hydrogen atom; an optionally substituted linear or branched alkyl group having 1 to 4 carbon atoms; an optionally substituted linear or branched alkenyl group having 2 to 4 carbon atoms; an optionally substituted phenyl group; an optionally substituted cycloalkyl group having 3 to 6 carbon atoms; or $-CO-R_7$ wherein $R_7$ represents an optionally substituted linear or branched alkyl group having 1 to 4 carbon atoms; an optionally substituted linear or branched alkoxy group having 1 to 4 carbon atoms; an optionally substituted monocyclic heterocycle; or an optionally substituted bicyclic heterocycle); or a group represented by the following general formula (2):
{wherein $R_5$ and $R_6$ are the same or different and each represents a hydrogen atom; or a linear alkyl group having 1 to 6 carbon atoms; and Y represents an oxygen atom; a sulfur atom; or a group represented by the following general formula (3):
(Wherein $R_4$ represents a hydrogen atom; an optionally substituted linear or branched alkyl group having 1 to 4 carbon atoms; an optionally substituted linear or branched alkenyl group having 2 to 4 carbon atoms; an optionally substituted phenyl group; an optionally substituted cycloalkyl group having 3 to 6 carbon atoms; or $-CO-R_7$ wherein $R_7$ represents an optionally substituted linear or branched alkyl group having 1 to 4 carbon atoms; an optionally substituted linear or branched alkoxy group having 1 to 4 carbon atoms; an optionally substituted monocyclic heterocycle; or an optionally substituted bicyclic heterocycle); or a group represented by the following general formula (4):
(wherein $R_8$ represents a hydrogen atom; or a linear alkyl group having 1 to 3 carbon atoms; and $R_9$ represents a hydrogen atom; a hydroxyl group; a thiol group; an alkylcarbonylthio group carrying, as the alkyl moiety, a linear alkyl group having 1 to 6 carbon atoms; an arylcarbonylthio group carrying, as the aryl moiety, an aryl group having 6 to 12 carbon atoms; a linear alkyl group having 1 to 3 carbon atoms; an acetoxy group; or an benzyloxy group)}.

9. The compound as claimed in Claim 1 or a pharmaceutically acceptable salt thereof, wherein, in the general formula (1), Z represents a 5-membered monocyclic heterocycle containing a nitrogen atom and/or a sulfur atom as the heteroatom optionally having an oxo group; a bicyclic heterocycle containing an oxygen atom as the heteroatom substituted by a hydroxyl group and/or a linear or branched alkyl group having 1 to 4 carbon atoms; or a cycloalkyl group having 4 to 6 carbon atoms; and

$R_2$ represents:

$$--N(R_3)R_4$$

(wherein $R_3$ represents a linear alkyl group having 1 to 3 carbon atoms; and $R_4$ represents a hydrogen atom; an optionally substituted linear or branched alkyl group having 1 to 4 carbon atoms; an allyl group; a phenyl group; a 2-methoxyphenyl group; a cycloalkyl having 3 to 6 carbon atoms; or $-CO-R_7$ wherein $R_7$ represents an optionally substituted linear or branched alkyl group having 1 to 4 carbon atoms; a benzyloxy group; an optionally substituted monocyclic heterocycle; or an optionally substituted bicyclic heterocycle); or a group represented by the following general formula (2):
{wherein $R_5$ and $R_6$ are the same or different and each represents a hydrogen atom; or a linear alkyl group having 1 to 3 carbon atoms; and Y represents an oxygen atom; or a group represented by the following general formula (3):
(Wherein $R_4$ represents a hydrogen atom; an optionally substituted linear or branched alkyl group having 1 to 4 carbon atoms; an allyl group; a phenyl group; a 2-methoxyphenyl group; a cycloalkyl group having 3 to 6 carbon atoms; or $-CO-R_7$ wherein $R_7$ represents an optionally substituted linear or branched alkyl group having 1 to 4 carbon atoms; a benzyloxy group; an optionally substituted monocyclic heterocycle; or an optionally substituted bicyclic heterocycle); or a group represented by the following general formula (4):

(wherein $R_8$ represents a hydrogen atom; or a methyl group; and $R_9$ represents a hydrogen atom; a hydroxyl group; a thiol group; an alkylcarbonylthio group carrying, as the alkyl moiety, a linear alkyl group having 1 to 3 carbon atoms; an arylcarbonylthio group carrying, as the aryl moiety, an aryl group having 6 to 12 carbon atoms; or a methyl group)}.

**10.** The compound as claimed in Claim 1 or a pharmaceutically acceptable salt thereof, wherein, in the general formula (1), Z represents an oxothiazolidine ring; a 3,4-dihydrobenzopyran ring substituted by a hydroxyl group and/or 4 methyl groups; or a cyclohexyl group; and

$R_2$ represents:

$$-N(R_3)R_4$$

(wherein $R_3$ represents a methyl group; and $R_4$ represents an optionally substituted linear or branched alkyl group having 1 to 4 carbon atoms; or $-CO-R_7$ wherein $R_7$ represents an optionally substituted linear or branched alkyl group having 1 to 4 carbon atoms); or a group represented by the following general formula (2):
(wherein $R_5$ and $R_6$ are the same or different and each represents a hydrogen atom; or a methyl group; and Y represents an oxygen atom; or a group represented by the following general formula (3):
(Wherein $R_4$ represents an optionally substituted linear or branched alkyl group having 1 to 4 carbon atoms; a 2-methoxyphenyl group; a cycloalkyl group having 4 to 6 carbon atoms; or $-CO-R_7$ wherein $R_7$ represents an optionally substituted linear or branched alkyl group having 1 to 4 carbon atoms); or a group represented by the following general formula (4):
(wherein $R_8$ represents a hydrogen atom; and $R_9$ represents a hydrogen atom; a hydroxyl group; a thiol group; an acetylthio group; or a benzoylthio group)).

**11.** The compound as claimed in Claim 1 selected from the group consisting of the following compounds or a pharmaceutically acceptable salt thereof: N-(6-hydroxy-2,5,7,8-tetramethylchroman-2-carbonyl)-3-(4-methylpiperazin-1-yl)sydnonimine, N-(6-hydroxy-2,5,7,8-tetramethylchroman-2-carbonyl)-3-(4-isopropylpiperazin-1-yl)sydnonimine, and N-((R)-2-oxo-4-thiazolidinecarbonyl)-3-(4-methylpiperazin-1-yl)sydnonimine.

**12.** A compound represented by the following general formula (11) or a pharmaceutically acceptable salt thereof:

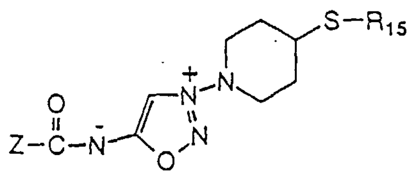

(11)

[wherein Z represents:

$$-A-X-R_1$$

{wherein A represents a linear or branched alkylene group having 1 to 6 carbon atoms optionally substituted by an optionally substituted aryl group having 6 to 12 carbon atoms or an alkylcarbonylamino group carrying, as the alkyl moiety, a linear or branched alkyl group having 1 to 6 carbon atoms;
X represents $S(O)_n$ (n being 0, 1 or 2); or a direct bond; and
$R_1$ represents an aryl group having 6 to 12 carbon atoms optionally mono-, di- or tri-substituted by 1 to 3 hydroxyl groups and/or 1 to 3 linear or branched alkyl groups having 1 to 6 carbon atoms and/or 1 to 3 linear or branched alkoxy groups having 1 to 6 carbon atoms and/or 1 to 3 halogen atoms and/or 1 or 2 nitro groups; an arylcarbonyl group carrying, as the aryl moiety, an aryl group having 6 to 12 carbon atoms optionally mono-, di- or tri-substituted by 1 to 3 linear or branched alkyl groups having 1 to 6 carbon atoms optionally halogenated and/or 1 to 3 linear or branched alkoxy groups having 1 to 6 carbon atoms and/or 1 to 3 halogen atoms; an alkylcarbonyl group having, as the alkyl moiety, a linear or branched alkyl group having 1 to 6 carbon atoms;

a linear or branched alkyl group having 1 to 6 carbon atoms; an aralkyloxycarbonyl group carrying, as the aryl moiety, an optionally substituted aryl group having 6 to 12 carbon atoms and, as the alkylene moiety, an alkylene group having 1 or 2 carbon atoms; a cycloalkylcarbonyl group carrying, as the cycloalkyl moiety, an optionally substituted cycloalkyl group having 3 to 6 carbon atoms; an arylcarbamoyl group carrying, as the aryl moiety, an optionally substituted aryl group having 6 to 12 carbon atoms; an alkylcarbamoyl group carrying, as the alkyl moiety, a linear or branched alkyl group having 1 to 6 carbon atoms; or $-CO-R_{14}$ wherein $R_{14}$ represents an optionally substituted monocyclic heterocycle; or a bicyclic heterocycle optionally substituted by a hydroxyl group and/or linear or branched alkyl groups having 1 to 6 carbon atoms); an optionally substituted monocyclic heterocycle; a bicyclic heterocycle optionally substituted by a hydroxyl group and/or linear or branched alkyl groups having 1 to 6 carbon atoms; or an optionally substituted cycloalkyl group having 3 to 6 carbon atoms; and

$R_{15}$ represents an optionally substituted linear or branched alkyl group having 1 to 6 carbon atoms; a monocyclic heterocycle containing an oxygen atom as the heteroatom; an alkylcarbonyl group carrying, as the alkyl moiety, a linear or branched alkyl group having 1 to 6 carbon atoms; an alkyloxycarbonyl group carrying, as the alkyl moiety, an optionally substituted alkyl group having 1 to 6 carbon atoms; or an arylcarbonyl group carrying, as the aryl moiety, an optionally substituted aryl group having 6 to 12 carbon atoms].

**13.** A compound represented by the general formula (11) or a pharmaceutically acceptable salt thereof wherein Z represents:

$$-A-X-R_1$$

{wherein A represents a linear or branched alkylene group having 1 to 6 carbon atoms optionally substituted by an optionally substituted aryl group having 6 to 12 carbon atoms or an alkylcarbonylamino group carrying, as the alkyl moiety, a linear or branched alkyl group having 1 to 6 carbon atoms;

X represents $S(O)_n$ (n being 0, 1 or 2); or a direct bond; and

$R_1$ represents an aryl group having 6 to 12 carbon atoms optionally mono-, di- or tri-substituted by 1 to 3 hydroxyl groups and/or 1 to 3 linear or branched alkyl groups having 1 to 6 carbon atoms and/or 1 to 3 linear or branched alkoxy groups having 1 to 6 carbon atoms and/or 1 to 3 halogen atoms and/or 1 or 2 nitro groups; an arylcarbonyl group carrying, as the aryl moiety, an aryl group having 6 to 12 carbon atoms optionally mono-, di- or tri-substituted by 1 to 3 linear or branched alkyl groups having 1 to 6 carbon atoms optionally halogenated and/or 1 to 3 linear or branched alkoxy groups having 1 to 6 carbon atoms and/or 1 to 3 halogen atoms; an alkylcarbonyl group having, as the alkyl moiety, a linear or branched alkyl group having 1 to 6 carbon atoms; a linear or branched alkyl group having 1 to 6 carbon atoms; an aralkyloxycarbonyl group carrying, as the aryl moiety, an optionally substituted aryl group having 6 to 12 carbon atoms and, as the alkylene moiety, an alkylene group having 1 or 2 carbon atoms; a cycloalkylcarbonyl group carrying, as the cycloalkyl moiety, an optionally substituted cycloalkyl group having 3 to 6 carbon atoms; an arylcarbamoyl group carrying, as the aryl moiety, an optionally substituted aryl group having 6 to 12 carbon atoms; an alkylcarbamoyl group carrying, as the alkyl moiety, a linear or branched alkyl group having 1 to 6 carbon atoms; or $-CO-R_{14}$ wherein $R_{14}$ represents an optionally substituted monocyclic heterocycle; or a bicyclic heterocycle optionally substituted by a hydroxyl group and/or linear or branched alkyl groups having 1 to 6 carbon atoms); and

$R_{15}$ represents an optionally substituted linear or branched alkyl group having 1 to 6 carbon atoms; a monocyclic heterocycle containing an oxygen atom as the heteroatom; an alkylcarbonyl group carrying, as the alkyl moiety, a linear or branched alkyl group having 1 to 6 carbon atoms; an alkyloxycarbonyl group carrying, as the alkyl moiety, an optionally substituted alkyl group having 1 to 6 carbon atoms; or an arylcarbonyl group carrying, as the aryl moiety, an optionally substituted aryl group having 6 to 12 carbon atoms].

**14.** A compound represented by the general formula (11) or a pharmaceutically acceptable salt thereof wherein Z represents an optionally substituted monocyclic heterocycle; a bicyclic heterocycle optionally substituted by a hydroxyl group and/or a linear or branched alkyl group having 1 to 6 carbon atoms; or an optionally substituted cycloalkyl group having 3 to 6 carbon atoms; and

$R_{15}$ represents an optionally substituted linear or branched alkyl group having 1 to 6 carbon atoms; a monocyclic heterocycle containing an oxygen atom as the heteroatom; an alkylcarbonyl group carrying, as the alkyl moiety, a linear or branched alkyl group having 1 to 6 carbon atoms; an alkyloxycarbonyl group carrying, as the alkyl moiety, an optionally substituted alkyl group having 1 to 6 carbon atoms; or an arylcarbonyl group carrying, as the aryl moiety, an optionally substituted aryl group having 6 to 12 carbon atoms].

**15.** A compound represented by the following general formula (12) or a pharmaceutically acceptable salt thereof:

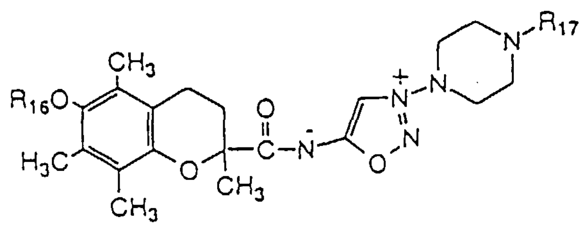

(12)

(wherein $R_{16}$ represents an optionally substituted linear or branched alkyl group having 1 to 6 carbon atoms; a monocyclic heterocycle containing an oxygen atom as the heteroatom; an alkylcarbonyl group carrying, as the alkyl moiety, a linear or branched alkyl group having 1 to 6 carbon atoms; an arylcarbonyl group carrying, as the aryl moiety, an optionally substituted aryl group having 6 to 12 carbon atoms; or a silyl group substituted by a linear or branched alkyl group having 1 to 6 carbon atoms; and $R_{17}$ represents an optionally substituted linear or branched alkyl group having 1 to 6 carbon atoms; or an alkyloxycarbonyl group carrying, as the alkyl moiety, a linear or branched alkyl group having 1 to 6 carbon atoms.

**16.** A drug which comprises, as the active ingredient, a compound as claimed in any of Claims 1 to 11 or a pharmaceutically acceptable salt thereof.

**17.** A therapeutic medicine for angina pectoris which comprises, as the active ingredient, a compound as claimed in any of Claims 1 to 11 or a pharmaceutically acceptable salt thereof.

**18.** A vasodilator agent which comprises, as the active ingredient, a compound as claimed in any of Claims 1 to 11 or a pharmaceutically acceptable salt thereof.

**19.** A myocardial protective agent which comprises, as the active ingredient, a compound as claimed in any of Claims 1 to 11 or a pharmaceutically acceptable salt thereof.

**20.** An antiplatelet aggregation agent which comprises, as the active ingredient, a compound as claimed in any of Claims 1 to 11 or a pharmaceutically acceptable salt thereof.

## INTERNATIONAL SEARCH REPORT

International application No.
PCT/JP96/03226

**A. CLASSIFICATION OF SUBJECT MATTER**
Int. Cl[6] C07D271/04, C07D413/04, C07D413/12, C07D417/12, A61K31/41, A61K31/495, A61K31/535
According to International Patent Classification (IPC) or to both national classification and IPC

**B. FIELDS SEARCHED**

Minimum documentation searched (classification system followed by classification symbols)
Int. Cl[6] C07D271/04, C07D413/04, C07D413/12, C07D417/12, A61K31/41, A61K31/495, A61K31/535

Documentation searched other than minimum documentation to the extent that such documents are included in the fields searched

Electronic data base consulted during the international search (name of data base and, where practicable, search terms used)
CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | JP, 04-312581, A (Kasera AG.), November 4, 1992 (04. 11. 92) & EP, 497122, A & CA, 2060030, A | 1-5, 7-10, 16 |
| X | JP, 02-178275, A (Kasera AG.), July 11, 1990 (11. 07. 90) & EP, 367036, A & US, 5006540, A | 1-5, 7-9, 16, 17, 20 |
| X | JP, 62-73, A (Kasera AG.), January 6, 1987 (06. 01. 87) & EP, 206219, A | 1-5, 7-10, 16 |
| X | JP, 57-158768, A (Kasera AG.), September 30, 1982 (30. 09. 82) & EP, 59356, A & US, 4436743, A | 1-4, 7-9, 16 |
| X | JP, 56-25174, A (Kasera AG.), March 10, 1981 (10. 03. 81) & EP, 23343, A & US, 4305939, A | 1-3, 7, 8, 16 |
| X | Journal of Heterocyclic Chemistry, Vol. 7, | 1, 7-9 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| January 27, 1997 (27. 01. 97) | February 4, 1997 (04. 02. 97) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No. PCT/JP96/03226

C (Continuation). DOCUMENTS CONSIDERED TO BE RELEVANT

| Category* | Citation of document, with indication, where appropriate, of the relevant passages | Relevant to claim No. |
|---|---|---|
| | No. 1 (1970) p. 123-129 (Chemical Abstracts, Vol. 72, No. 90360) | |
| A | JP, 7-196652, A (Kasera AG.), August 1, 1995 (01. 08. 95) & EP, 655450, A | 1, 7-10 |
| A | JP, 6-128156, A (Kasera AG.), May 10, 1994 (10. 05. 94) & EP, 499831, A & CA, 2061488, A | 1-5, 7-10, 16, 20 |
| A | Journal of Cardiovascular Pharmacology Vol. 22 (Suppl. 7) (1993), p. S44-S50 (Chemical Abstracts, Vol. 120, No. 351) | 16, 18, 19 |
| A | Journal of Cardiovascular Pharmacology Vol. 22 (Suppl. 7) (1993), p. S51-S58 (Chemical Abstracts, Vol. 120, No. 352), | 16, 18 |

Form PCT/ISA/210 (continuation of second sheet) (July 1992)